# EUROPEAN PATENT APPLICATION

(11) **EP 4 588 524 A2**
(43) Date of publication of application: **23.07.2025**
(21) Application number: 25172360.7
(22) Date of filing: 01.07.2022
(51) Int. Cl.: A61P 37/04

(54) **ANTI-TIGIT AND ANTI-PVRIG IN MONOTHERAPY AND COMBINATION TREATMENTS**

(30) Priority: 01.07.2021 US 202163217634 P; 28.07.2021 US 202163226640 P; 02.08.2021 US 202163228469 P; 15.10.2021 US 202163256431 P; 24.11.2021 US 202163283097 P
(62) Divisional of application: 22758006.5
(71) Applicant: Compugen Ltd., 5885849 Holon (IL)
(72) Inventor: ALTEBER, Zoya, 7420209 Nes Ziyona (IL); OPHIR, Eran, 4052431 Even Yehuda (IL); FRENKEL, Masha, 7740809 Ashdod (IL); COJOCARU, Gad, S., 4724800 Ramat Hasharon (IL); ADEWOYE Adeboye, Henry, San Francisco, CA 94107 (US)
(74) Representative: Fuchs Patentanwälte Partnerschaft mbB

(57) **Abstract**

The present invention provides combination treatments with anti-PVRIG antibodies and anti-TIGIT antibodies, as well treatments with anti-TIGIT antibodies alone, as described herein.

## Description

### CROSS-REFERENCE TO RELATED APPLICATIONS

This application claims priority to U.S. Provisional Patent Application No. 63/217,634, entitled "Anti-TIGIT and Anti-PVRIG Combination Treatment," filed July 1, 2021, U.S. Provisional Patent Application No. 63/226,640, entitled "Monotherapy with Anti-TIGIT Antibodies," filed July 28, 2021, U.S. Provisional Patent Application No. 63/228,469, entitled "Anti-PVRIG Antibodies for the Treatment of Multiple Myeloma," filed August 2, 2021, U.S. Provisional Patent Application No. 63/256,431, entitled "Monotherapy with Anti-TIGIT Antibodies," filed October 15, 2021, and U.S. Provisional Patent Application No. 63/283,097, entitled "Anti-PVRIG Antibodies for the Treatment of Multiple Myeloma," filed November 24, 2021 which are hereby incorporated by reference in their entireties.

### BACKGROUND OF THE INVENTION

Naive T cells must receive two independent signals from antigen-presenting cells (APC) in order to become productively activated. The first, Signal 1, is antigen-specific and occurs when T cell antigen receptors encounter the appropriate antigen-MHC complex on the APC. The fate of the immune response is determined by a second, antigen-independent signal (Signal 2) which is delivered through a T cell costimulatory molecule that engages its APC-expressed ligand. This second signal could be either stimulatory (positive costimulation) or inhibitory (negative costimulation or coinhibition). In the absence of a costimulatory signal, or in the presence of a coinhibitory signal, T-cell activation is impaired or aborted, which may lead to a state of antigen-specific unresponsiveness (known as T-cell anergy), or may result in T-cell apoptotic death.

Costimulatory molecule pairs usually consist of ligands expressed on APCs and their cognate receptors expressed on T cells. The prototype ligand/receptor pairs of costimulatory molecules are B7/CD28 and CD40/CD40L. The B7 family consists of structurally related, cell-surface protein ligands, which may provide stimulatory or inhibitory input to an immune response. Members of the B7 family are structurally related, with the extracellular domain containing at least one variable or constant immunoglobulin domain.

Both positive and negative costimulatory signals play critical roles in the regulation of cell-mediated immune responses, and molecules that mediate these signals have proven to be effective targets for immunomodulation. Based on this knowledge, several therapeutic approaches that involve targeting of costimulatory molecules have been developed, and were shown to be useful for prevention and treatment of cancer by turning on, or preventing the turning off, of immune responses in cancer patients and for prevention and treatment of autoimmune diseases and inflammatory diseases, as well as rejection of allogenic transplantation, each by turning off uncontrolled immune responses, or by induction of "off signal" by negative costimulation (or coinhibition) in subjects with these pathological conditions.

Manipulation of the signals delivered by B7 ligands has shown potential in the treatment of autoimmunity, inflammatory diseases, and transplant rejection. Therapeutic strategies include blocking of costimulation using monoclonal antibodies to the ligand or to the receptor of a costimulatory pair, or using soluble fusion proteins composed of the costimulatory receptor that may bind and block its appropriate ligand. Another approach is induction of co-inhibition using soluble fusion protein of an inhibitory ligand. These approaches rely, at least partially, on the eventual deletion of auto- or allo-reactive T cells (which are responsible for the pathogenic processes in autoimmune diseases or transplantation, respectively), presumably because in the absence of costimulation (which induces cell survival genes) T cells become highly susceptible to induction of apoptosis. Thus, novel agents that are capable of modulating costimulatory signals, without compromising the immune system's ability to defend against pathogens, are highly advantageous for treatment and prevention of such pathological conditions.

Costimulatory pathways play an important role in tumor development. Interestingly, tumors have been shown to evade immune destruction by impeding T cell activation through inhibition of co-stimulatory factors in the B7-CD28 and TNF families, as well as by attracting regulatory T cells, which inhibit anti-tumor T cell responses (see Wang (2006), "Immune Suppression by Tumor Specific CD4+ Regulatory T cells in Cancer", Semin. Cancer. Biol. 16:73-79; Greenwald, et al. (2005), "The B7 Family Revisited", Ann. Rev. Immunol. 23:515-48; Watts (2005), "TNF/TNFR Family Members in Co-stimulation of T Cell Responses", Ann. Rev. Immunol. 23:23-68; Sadum, et al., (2007) "Immune Signatures of Murine and Human Cancers Reveal Unique Mechanisms of Tumor Escape and New Targets for Cancer Immunotherapy", Clin. Canc. Res. 13(13): 4016-4025). Such tumor expressed co-stimulatory molecules have become attractive cancer biomarkers and may serve as tumor-associated antigens (TAAs). Furthermore, costimulatory pathways have been identified as immunologic checkpoints that attenuate T cell dependent immune responses, both at the level of initiation and effector function within tumor metastases. As engineered cancer vaccines continue to improve, it is becoming clear that such immunologic checkpoints are a major barrier to the vaccines' ability to induce therapeutic anti-tumor responses. In that regard, costimulatory molecules can serve as adjuvants for active (vaccination) and passive (antibody-mediated) cancer immunotherapy, providing strategies to thwart immune tolerance and stimulate the immune system.

Over the past decade, agonists and/or antagonists to various costimulatory proteins have been developed for treating autoimmune diseases, graft rejection, allergy and cancer. For example, CTLA4-Ig (Abatacept, Orencia^{®}) is approved for treatment of RA, mutated CTLA4-Ig (Belatacept, Nulojix^{®}) for prevention of acute kidney transplant rejection and by the anti-CTLA4 antibody (Ipilimumab, Yervoy^{®}), recently approved for the treatment of melanoma. Other costimulation regulators have been approved, such as the anti-PD-1 antibodies of Merck (Keytruda^{®}) and BMS (Opdivo^{®}), have been approved for cancer treatments and are in testing for viral infections as well.

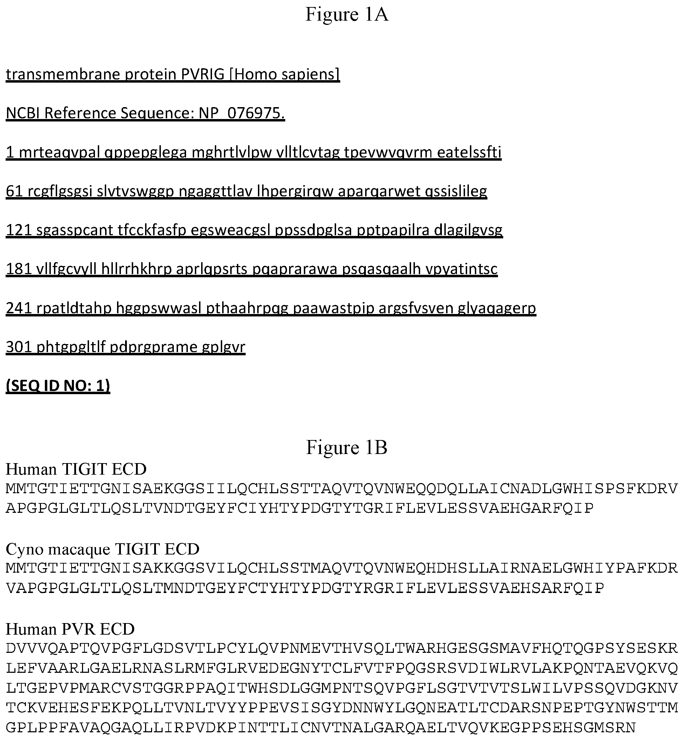

A particular target of interest is PVRIG. PVRIG is a transmembrane domain protein of 326 amino acids in length, with a signal peptide (spanning from amino acid 1 to 40), an extracellular domain (spanning from amino acid 41 to 171), a transmembrane domain (spanning from amino acid 172 to 190) and a cytoplasmic domain (spanning from amino acid 191 to 326). The full length human PVRIG protein is shown in Figure 1. There are two methionines that can be start codons, but the mature proteins are identical.

The PVRIG proteins contain an immunoglobulin (Ig) domain within the extracellular domain, which is a PVR-like Ig fold domain. The PVR-like Ig fold domain may be responsible for functional counterpart binding, by analogy to the other B7 family members. The PVR-like Ig fold domain of the extracellular domain includes one disulfide bond formed between intra domain cysteine residues, as is typical for this fold and may be important for structure-function. These cysteines are located at residues 22 and 93 (or 94). In one embodiment, there is provided a soluble fragment of PVRIG that can be used in testing of PVRIG antibodies. Included within the definition of PVRIG proteins are PVRIG ECD fragments, including know ECD fragments such as those decirbed in U.S. Patent No. 9,714, 289.

PVRIG has also been identified as an inhibitory receptor which recognizes CD112 but not CD155, and it may be involved in negative regulation of the anti-tumor functions mediated by DNAM-1. PVRL2 was identified as the ligand for PVRIG, placing PVRIG in the DNAM/TIGIT immunoreceptor axis (see, Liang et al., Journal of Clinical Oncology 2017 35:15_suppl, 3074-3074).

Anti-PVRIG antibodies (including antigen-binding fragments) that both bind to PVRIG and prevent activation by PVRL2 (e.g. most commonly by blocking the interaction of PVRIG and PVLR2), are used to enhance T cell and/or NK cell activation and be used in treating diseases such as cancer and pathogen infection. As such, formulations for administering such antibodies are needed.

Another target of interest is TIGIT. TIGIT is a coinhibitory receptor that is highly expressed on effector & regulatory (Treg) CD4+ T cells, effector CD8+ T cells, and NK cells. TIGIT has been shown to attenuate immune response by (1) direct signaling, (2) inducing ligand signaling, and (3) competition with and disruption of signaling by the costimulatory receptor CD226 (also known as DNAM-1). TIGIT signaling has been the most well-studied in NK cells, where it has been demonstrated that engagement with its cognate ligand, poliovirus receptor (PVR, also known as CD155) directly suppresses NK cell cytotoxicity through its cytoplasmic ITIM domain. Knockout of the TIGIT gene or antibody blockade of the TIGIT/PVR interaction has shown to enhance NK cell killing in vitro, as well as to exacerbate autoimmune diseases in vivo. In addition to its direct effects on T- and NK cells, TIGIT can induce PVR-mediated signaling in dendritic or tumor cells, leading to the increase in production of anti-inflammatory cytokines such as IL10. In T-cells TIGIT can also inhibit lymphocyte responses by disrupting homodimerization of the costimulatory receptor CD226, and by competing with it for binding to PVR.

TIGIT is highly expressed on lymphocytes, including Tumor Infiltrating Lymphocytes (TILs) and Tregs, that infiltrate different types of tumors. PVR is also broadly expressed in tumors, suggesting that the TIGIT-PVR signaling axis may be a dominant immune escape mechanism for cancer. Notably, TIGIT expression is tightly correlated with the expression of another important coinhibitory receptor, PD1. TIGIT and PD1 are co-expressed on the TILs of numerous human and murine tumors. Unlike TIGIT and CTLA4, PD1 inhibition of T cell responses does not involve competition for ligand binding with a costimulatory receptor.

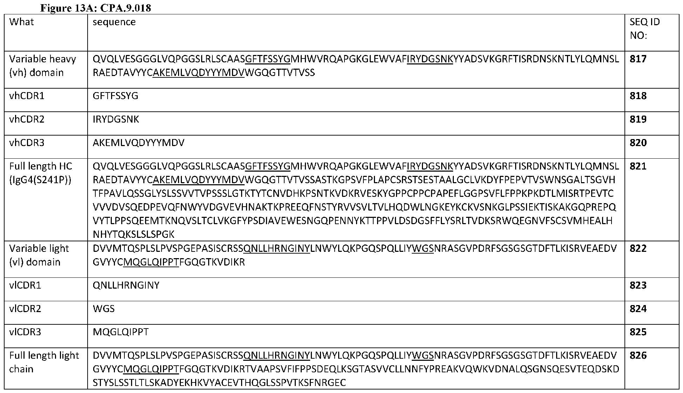

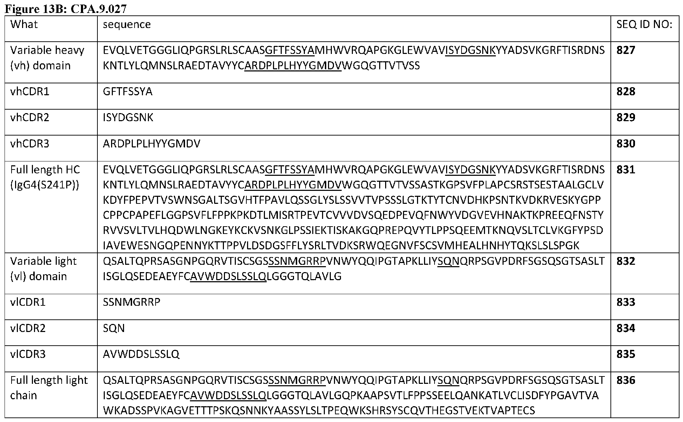

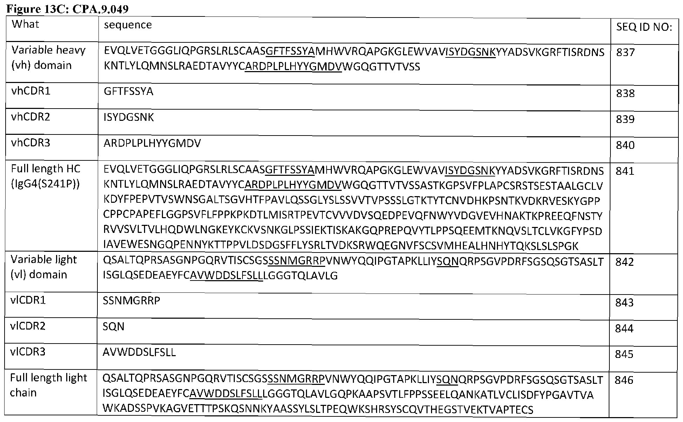

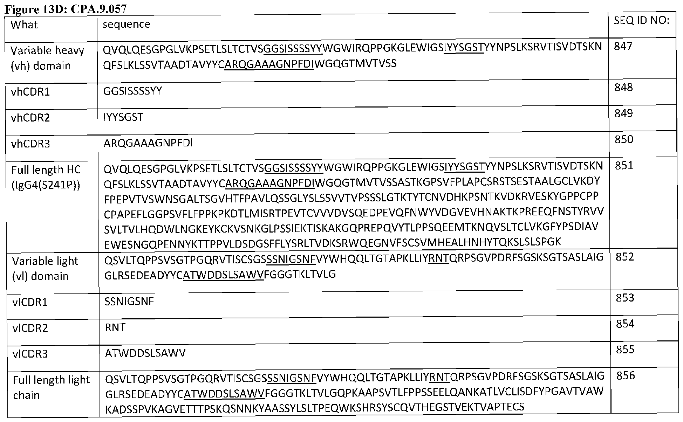

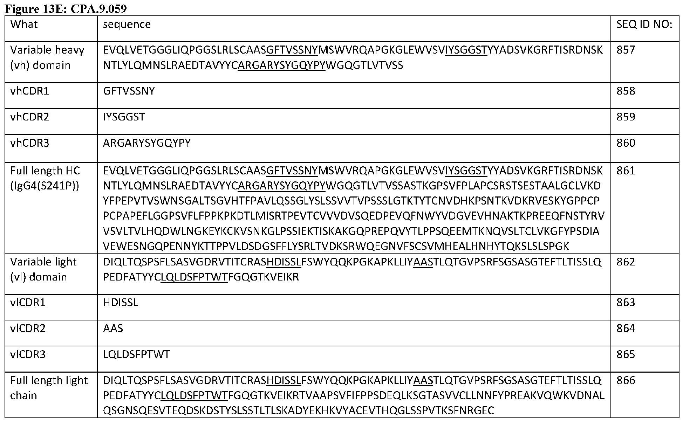

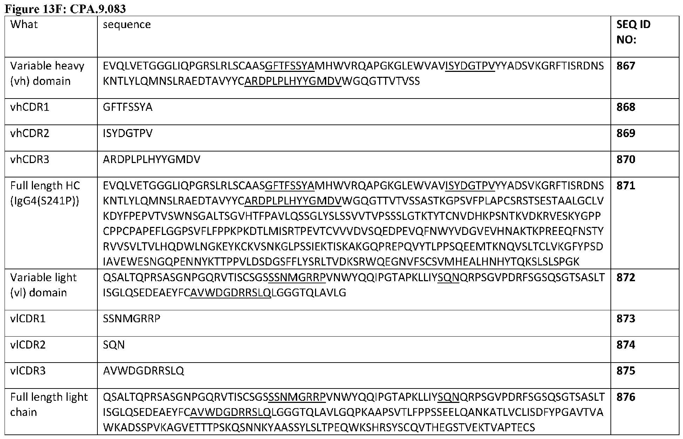

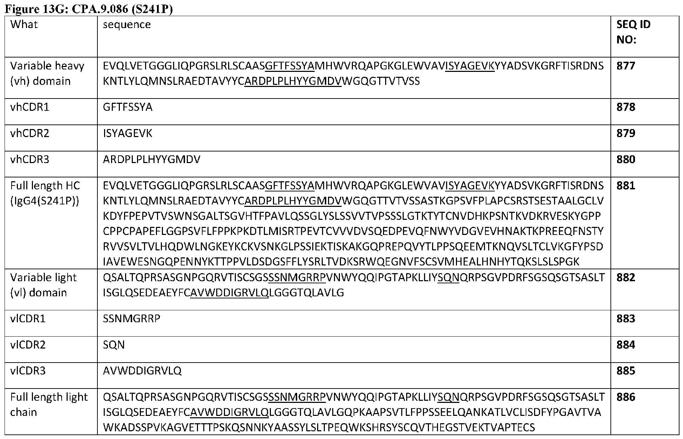

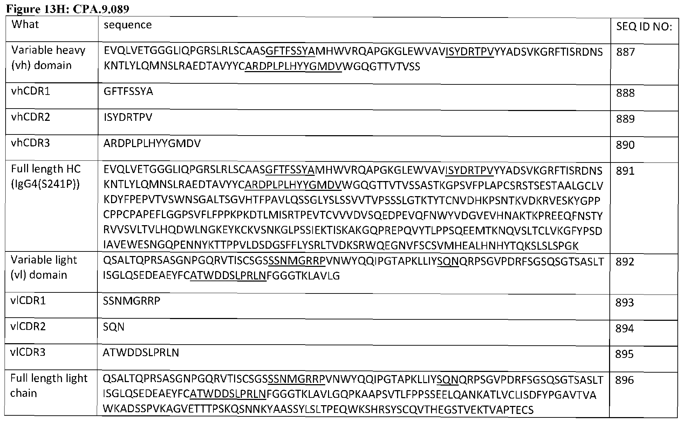

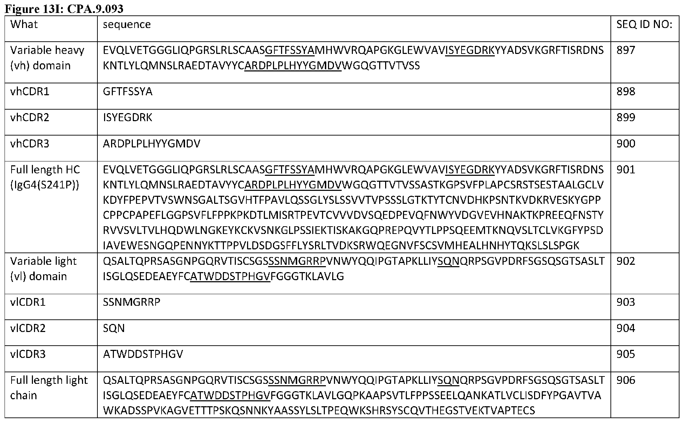

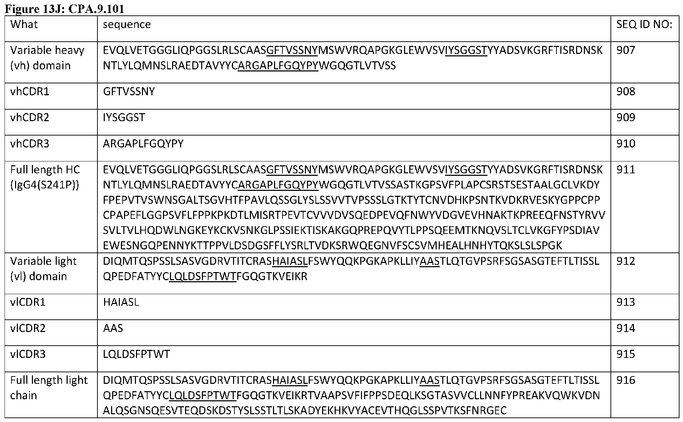

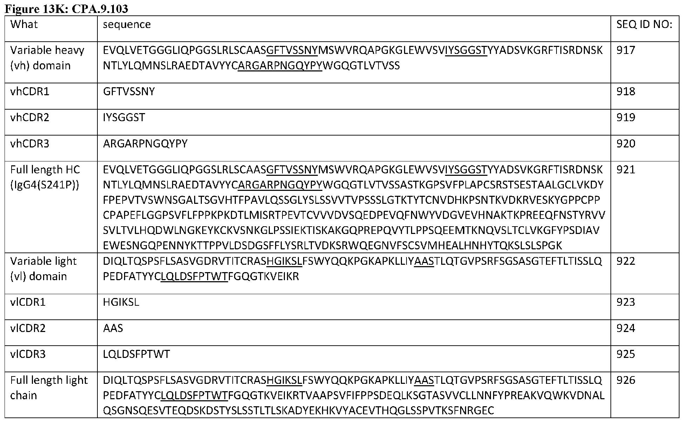

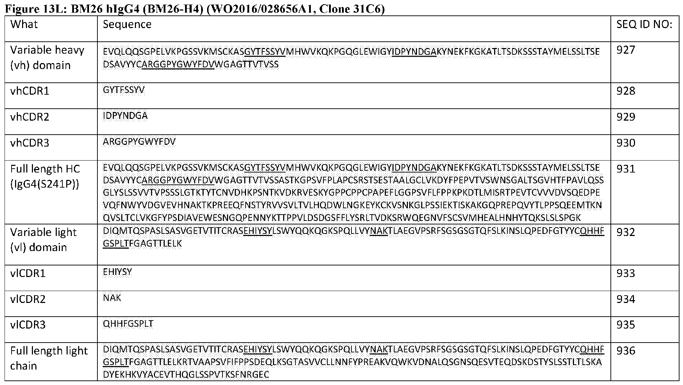

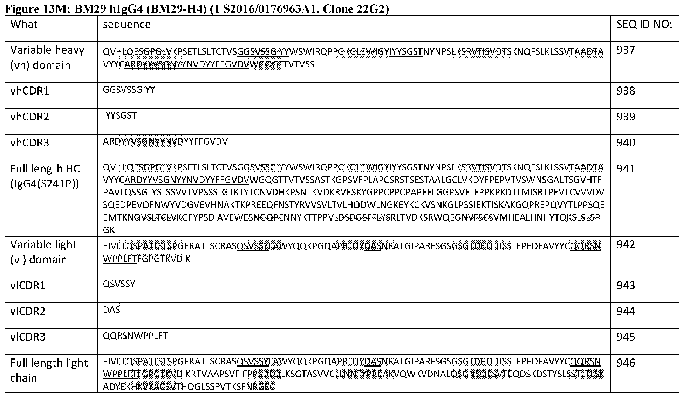

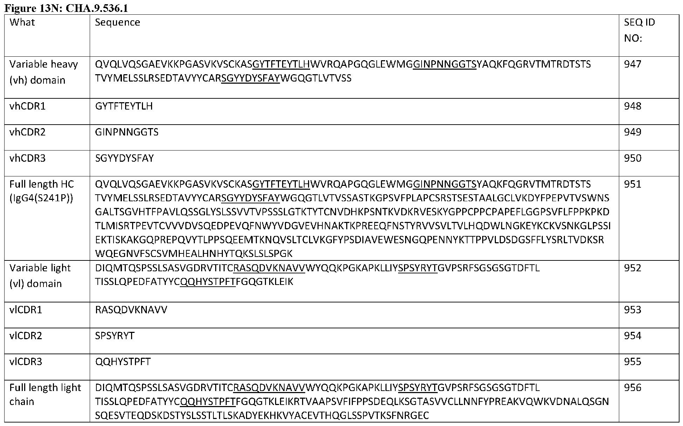

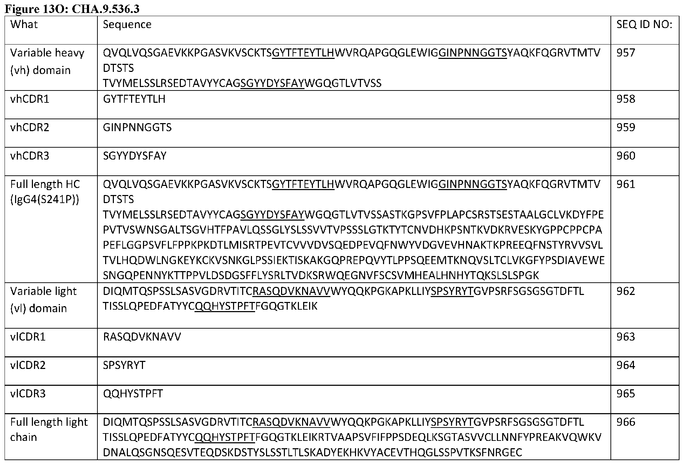

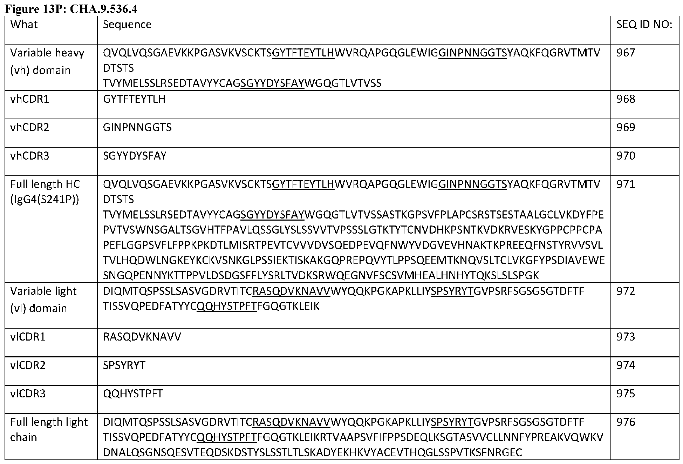

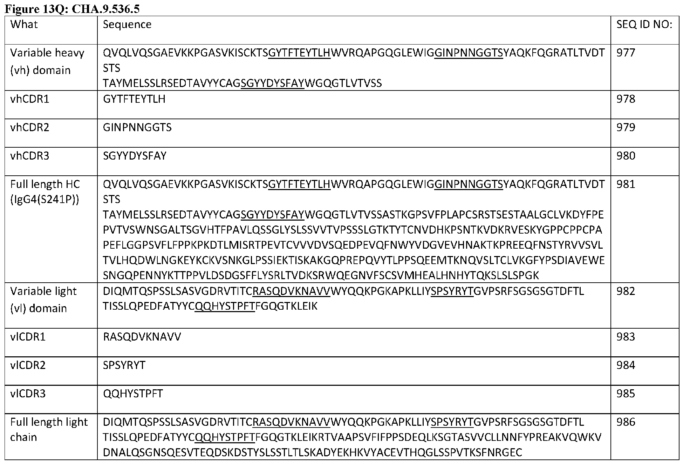

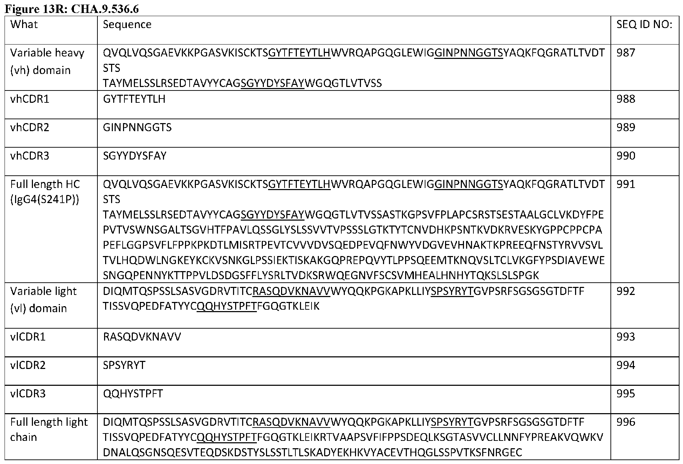

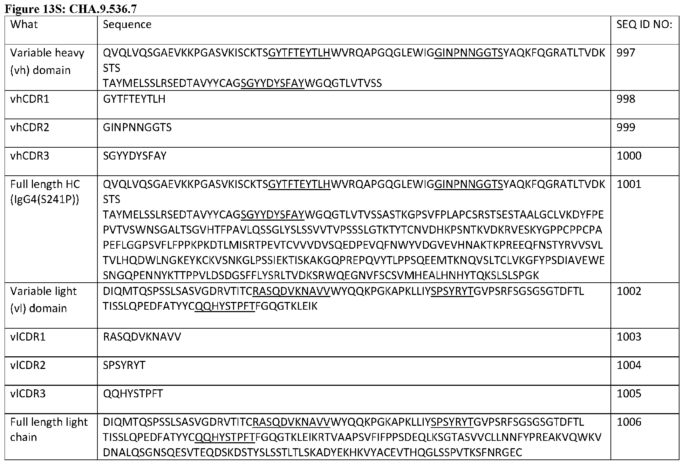

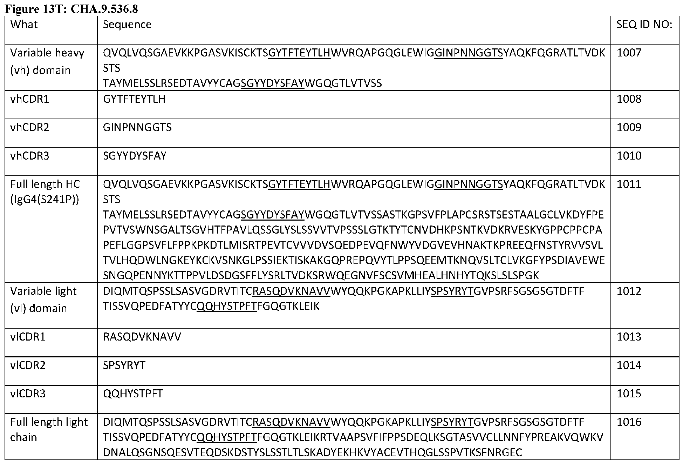

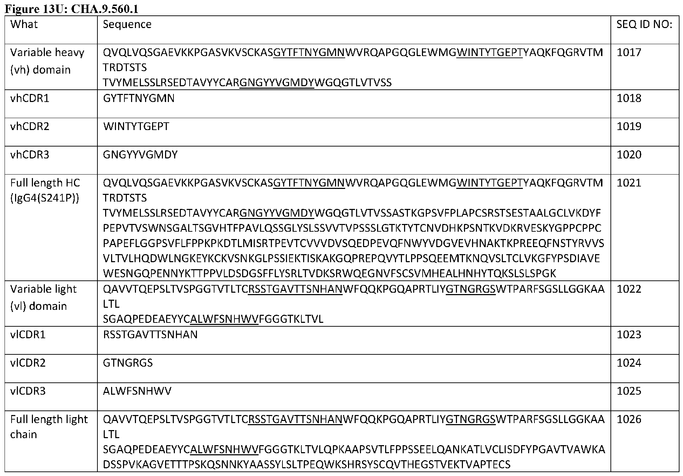

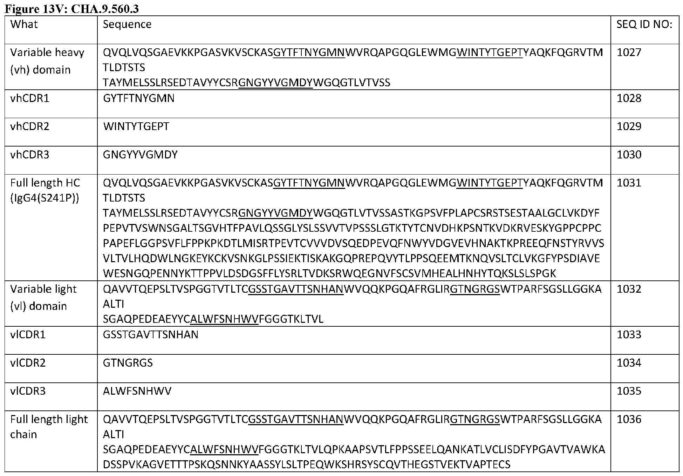

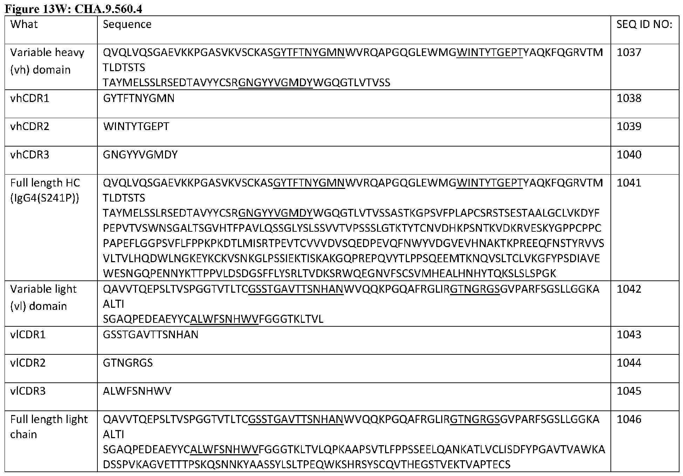

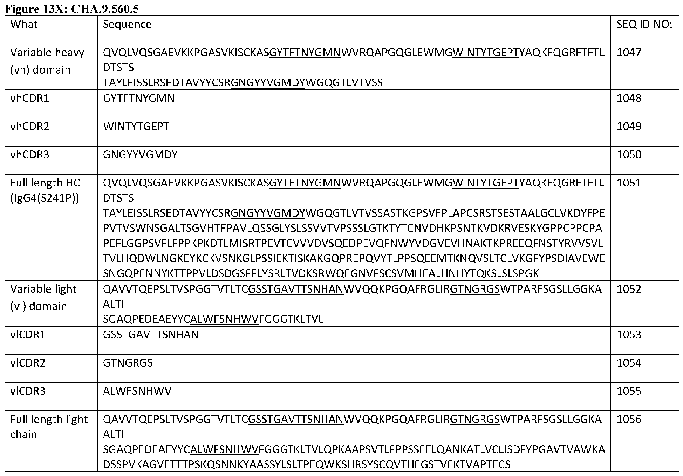

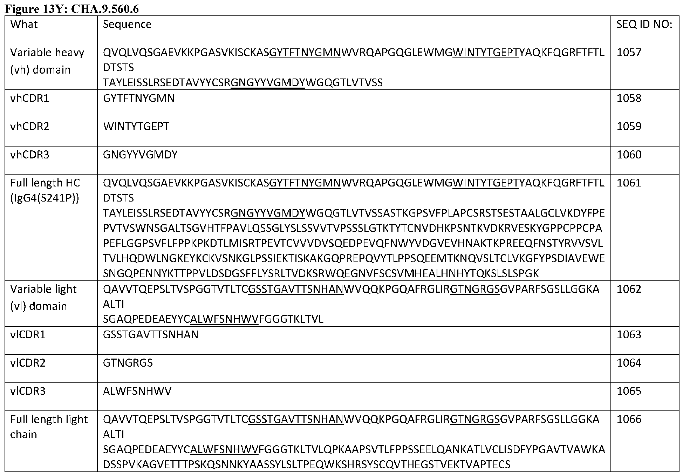

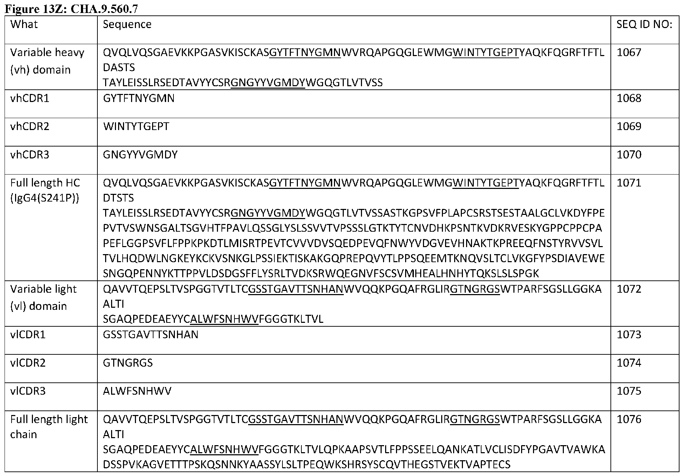

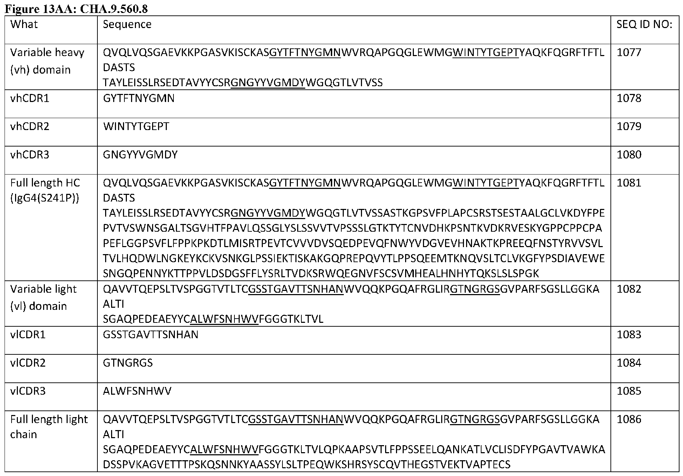

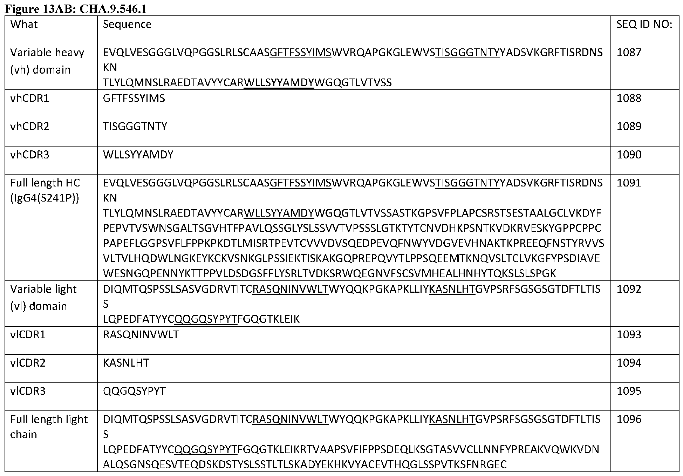

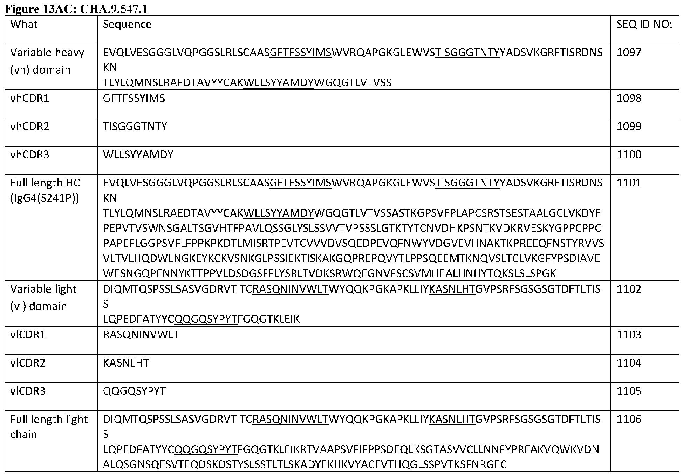

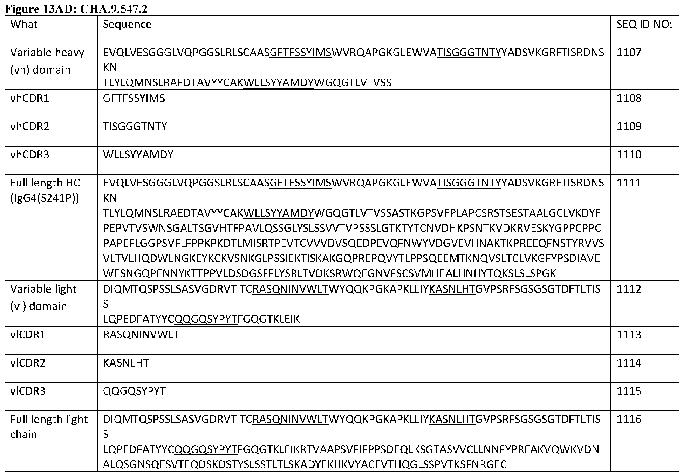

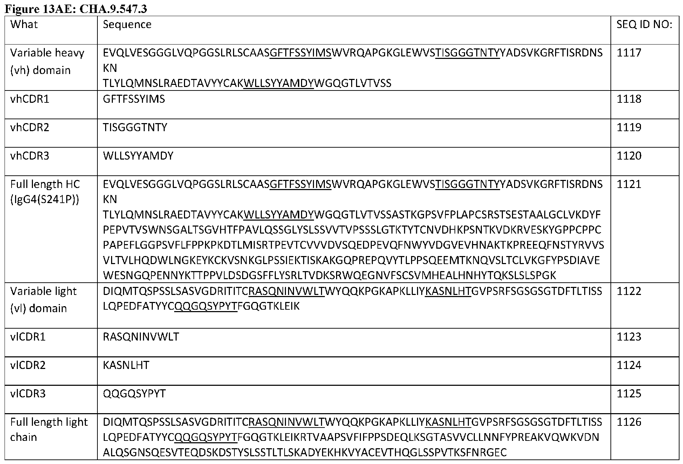

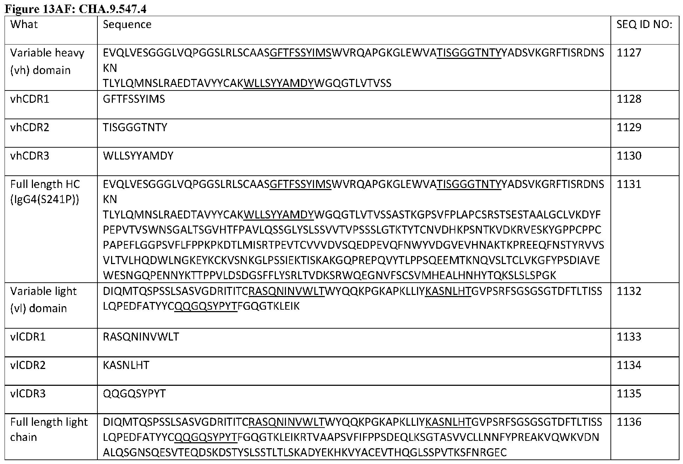

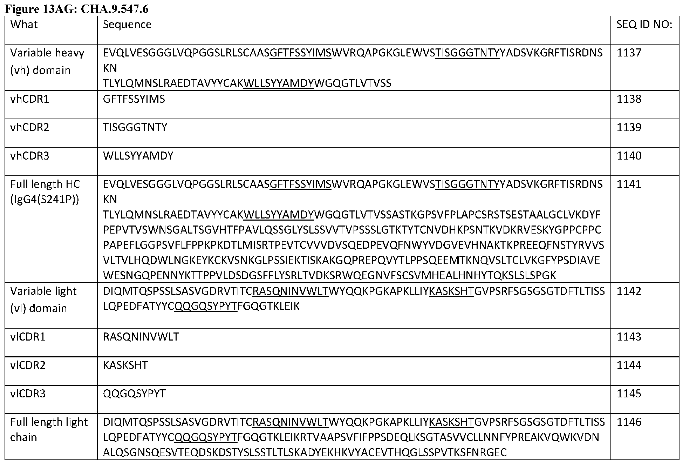

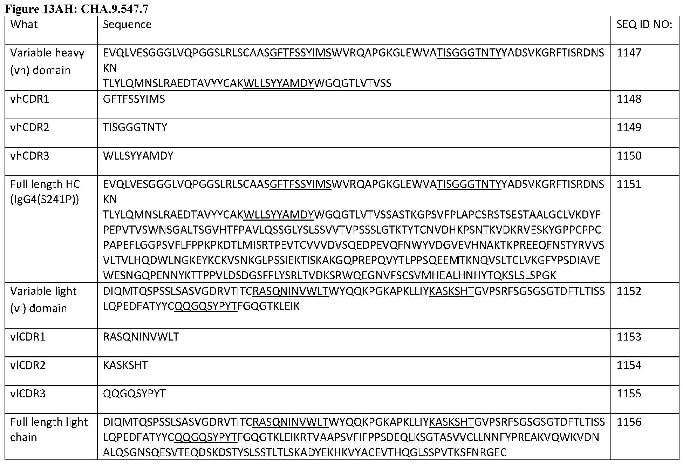

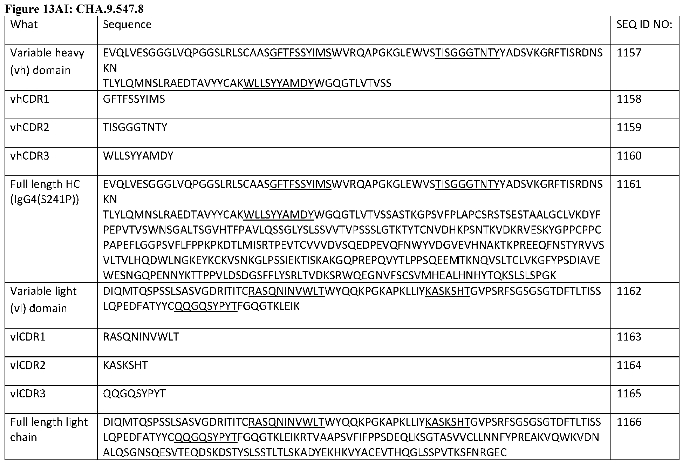

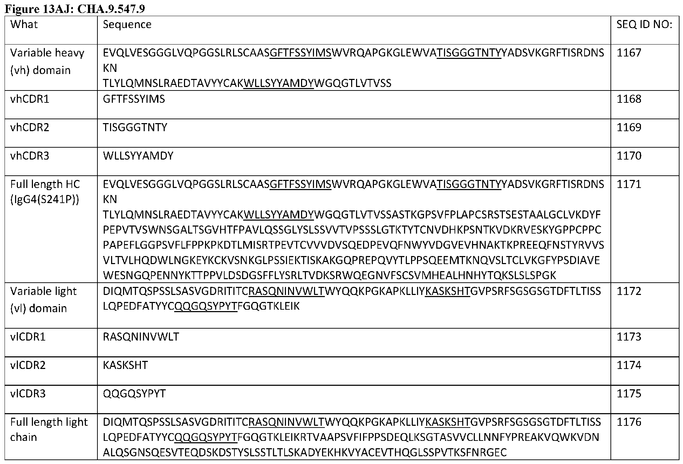

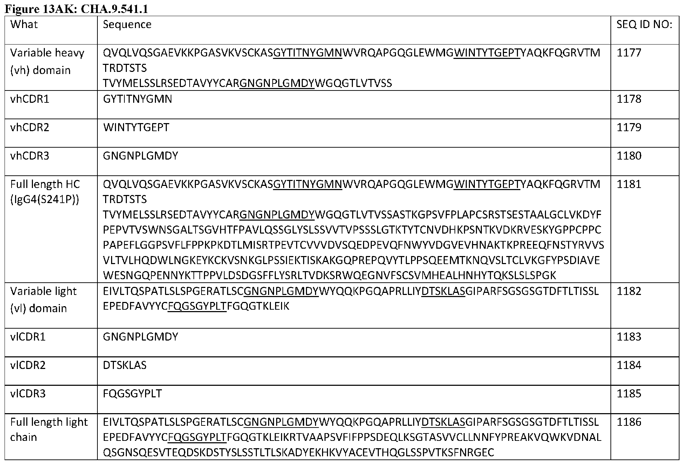

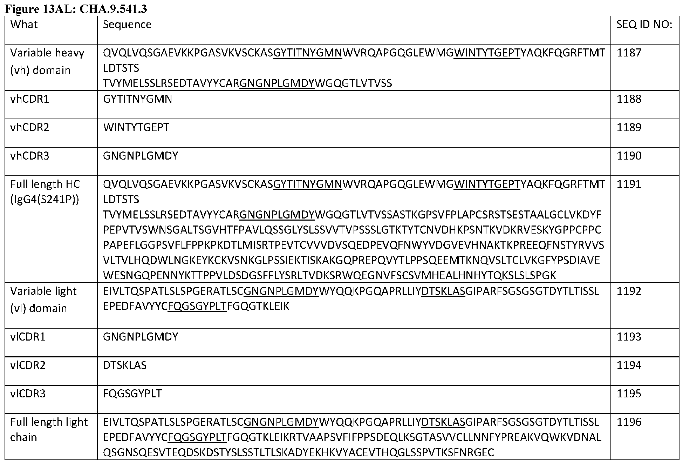

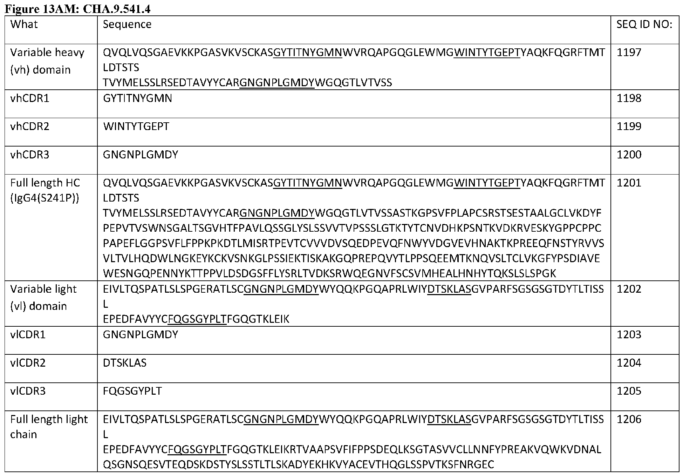

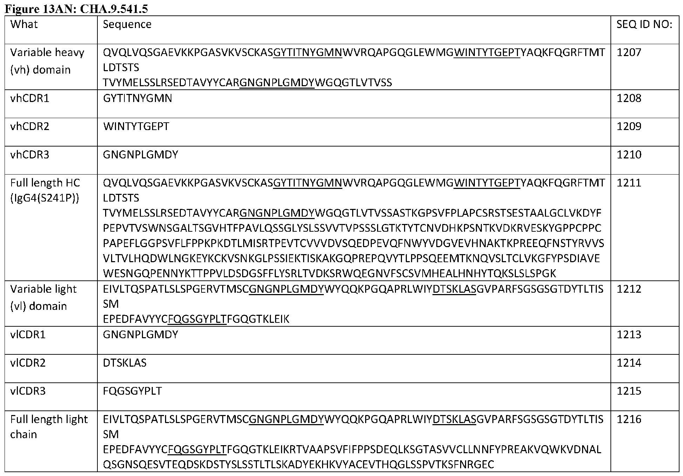

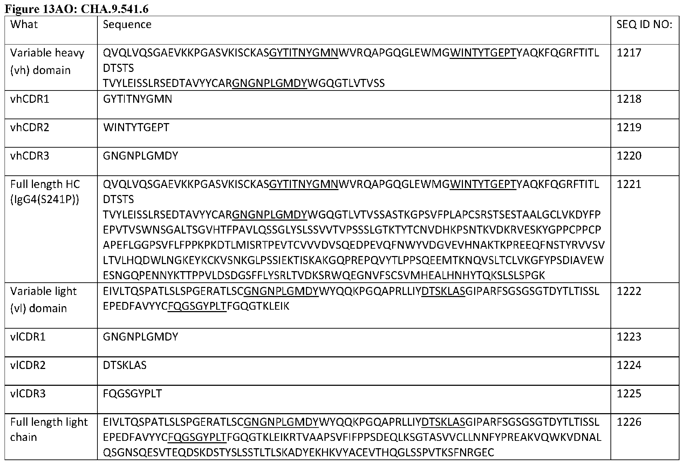

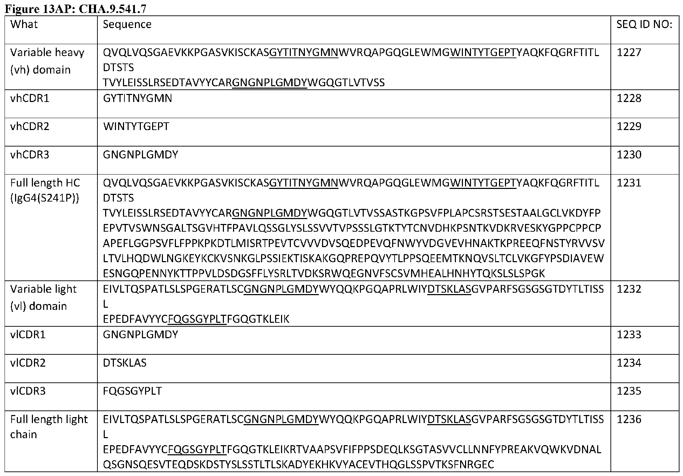

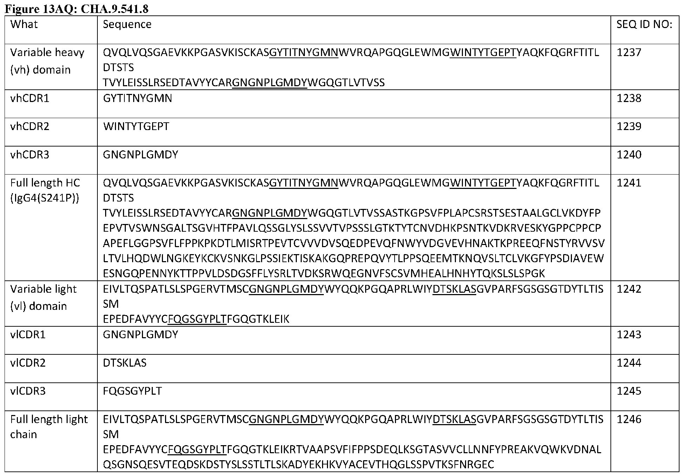

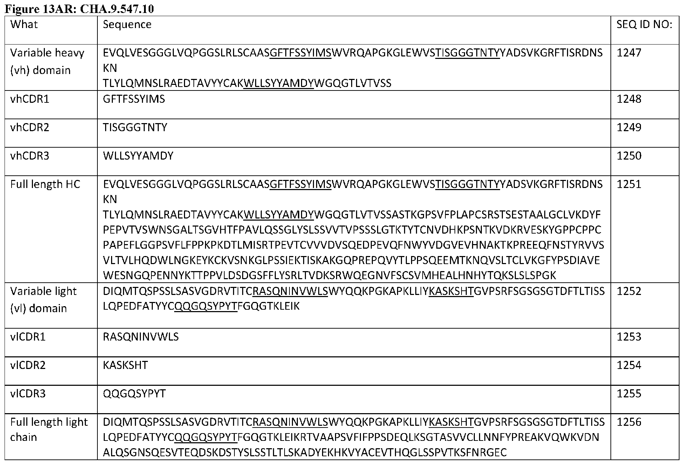

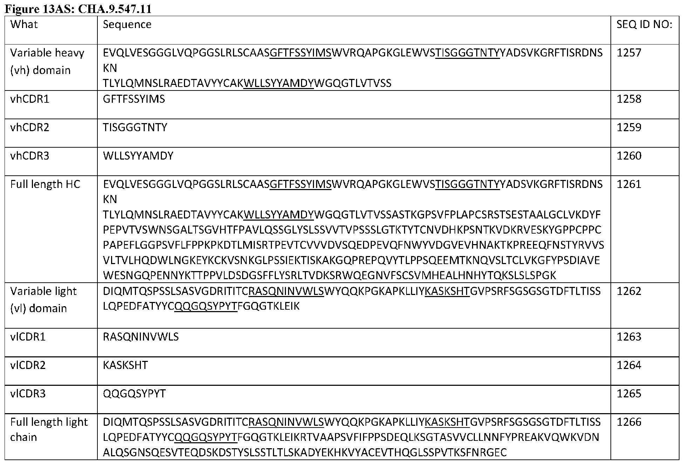

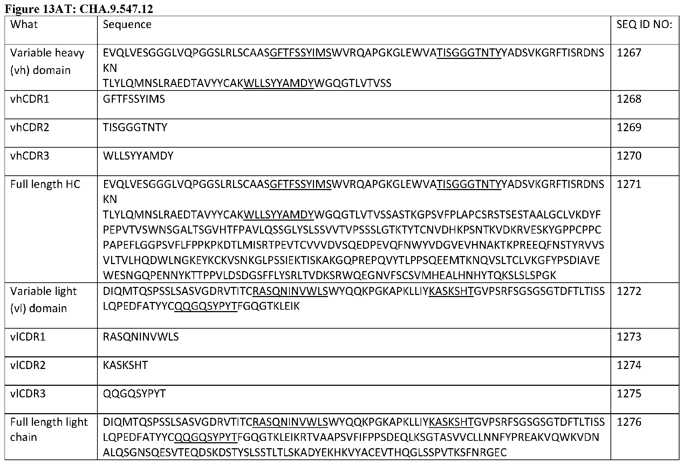

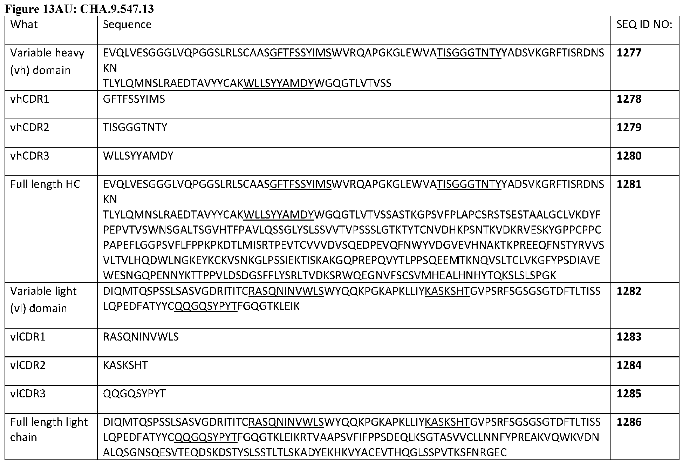

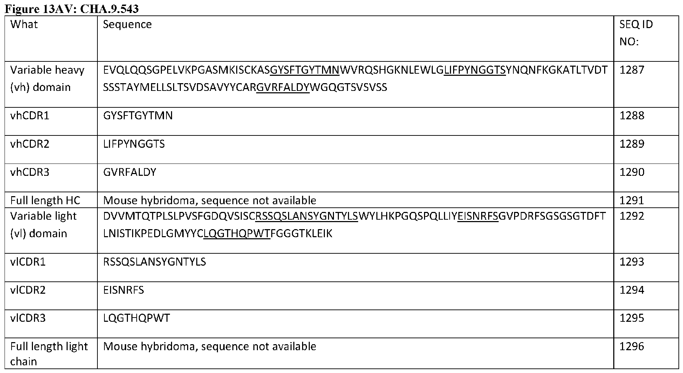

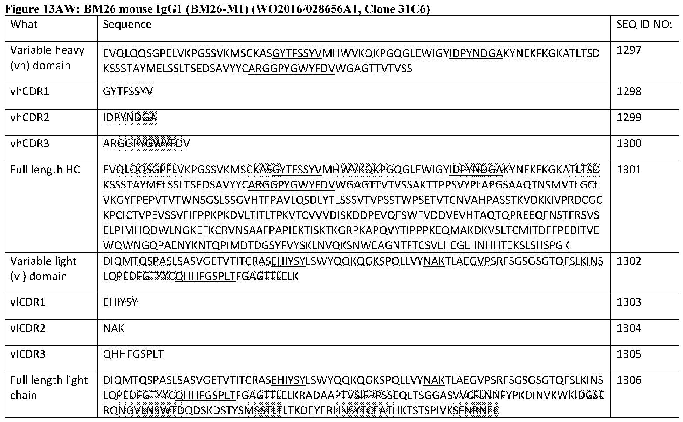

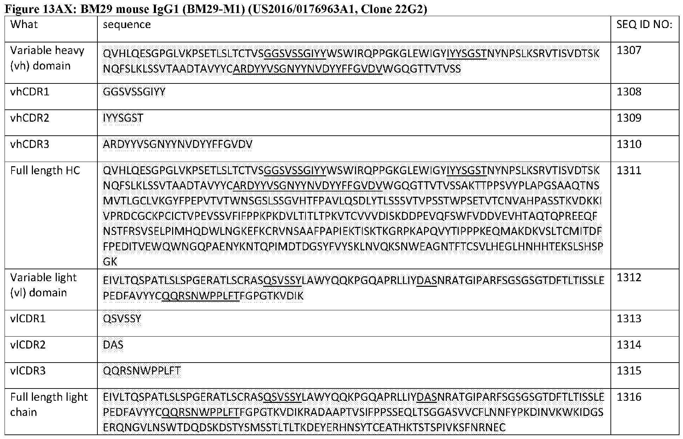

Accordingly, TIGIT is an attractive target for monoclonal antibody therapy and disease treatment, and anti-TIGIT antibodies of the present invention find use in such methods, including, for example, anti-TIGIT antibodies including those with CDRs identical to those shown in Figure 13.

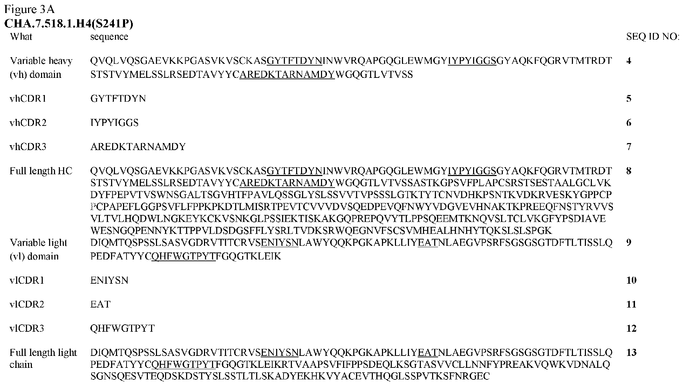

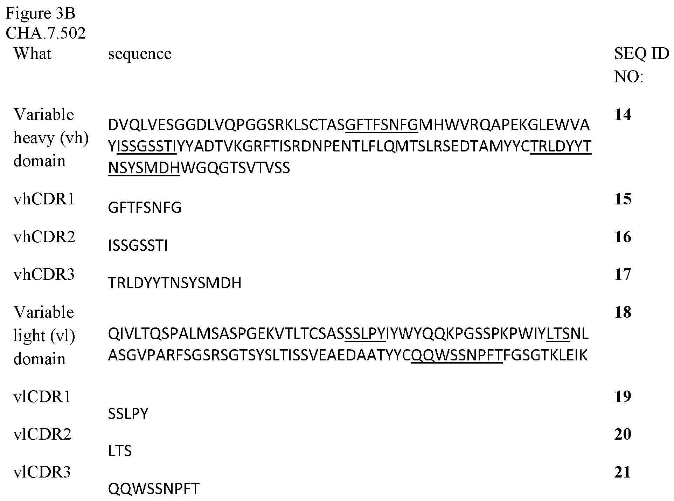

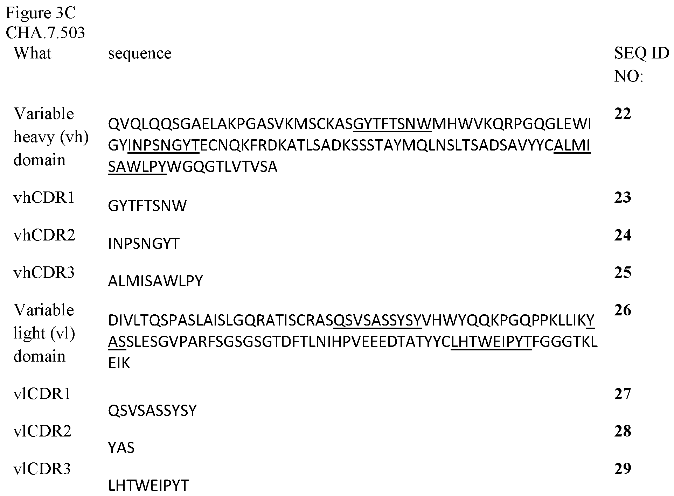

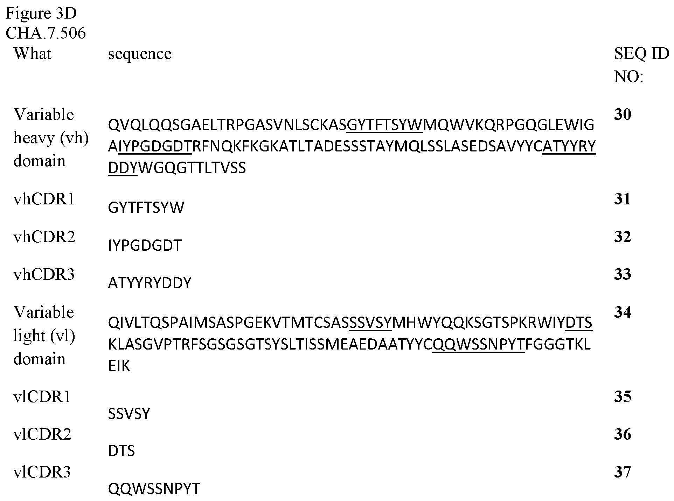

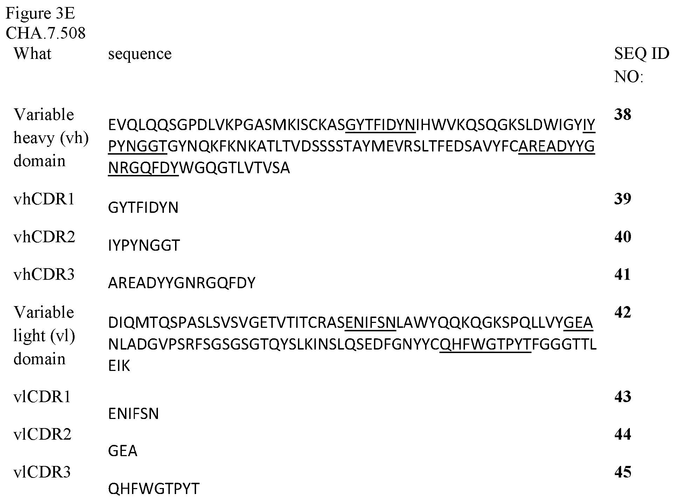

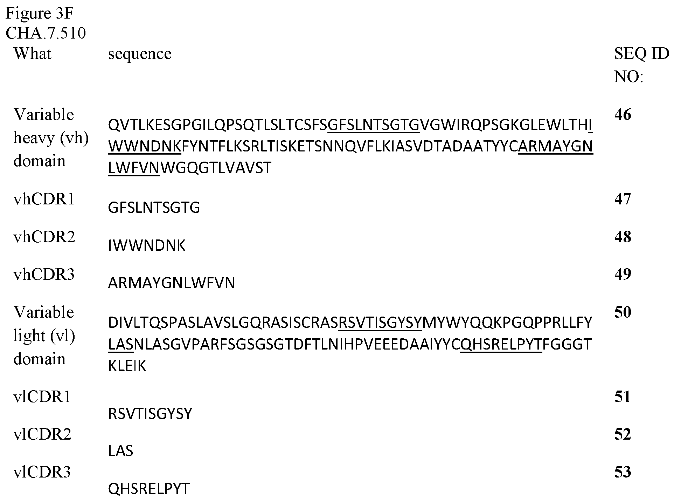

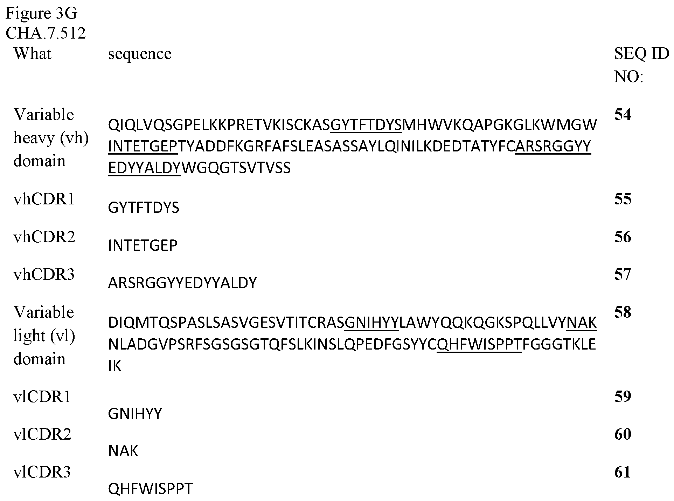

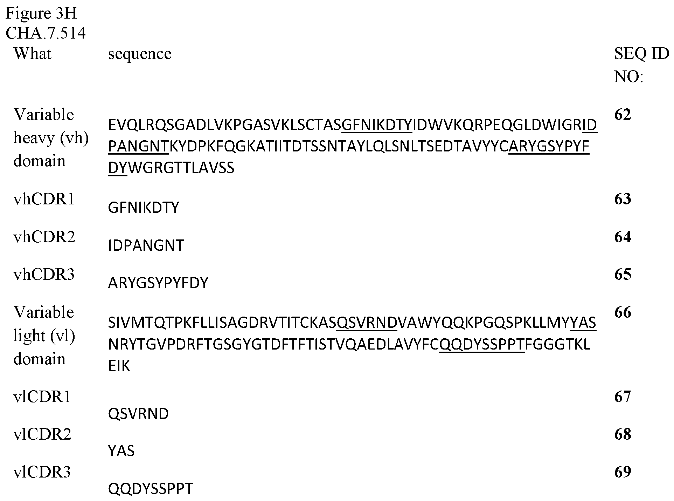

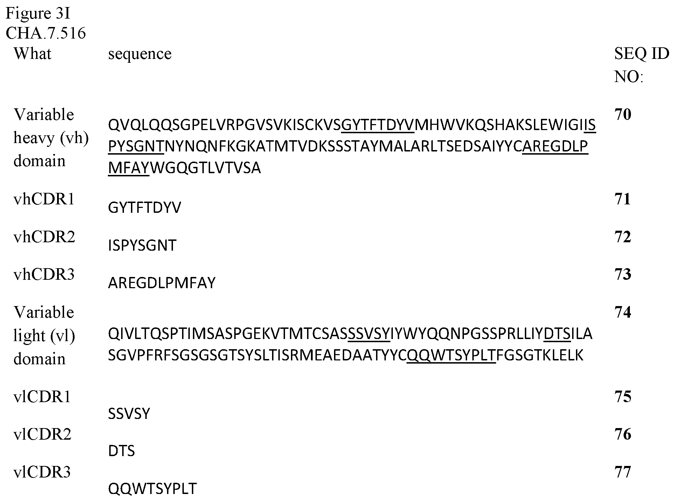

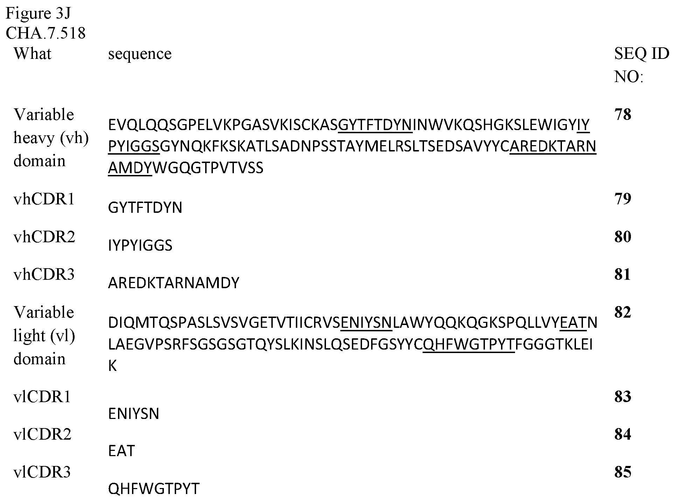

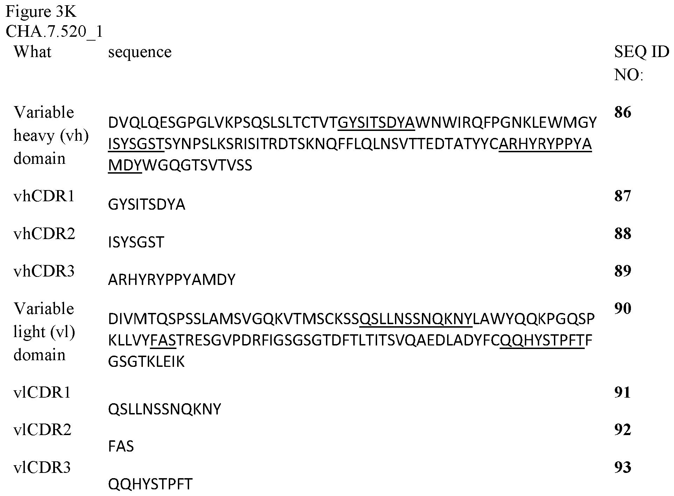

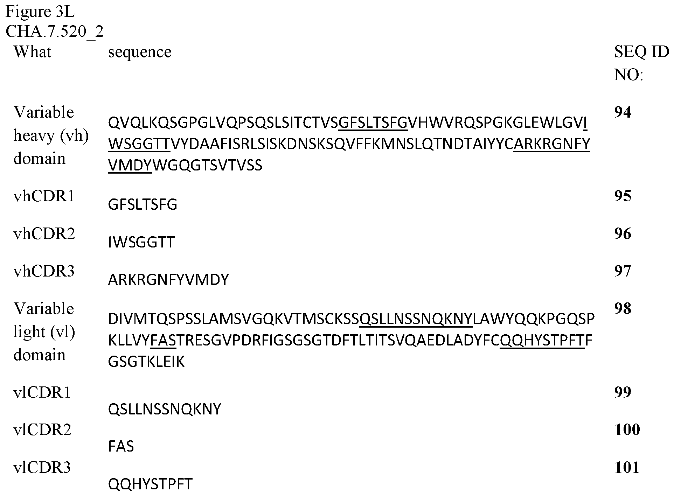

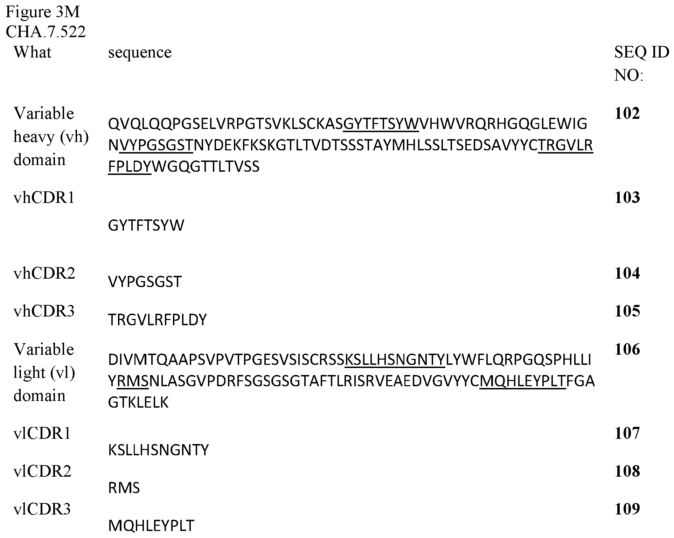

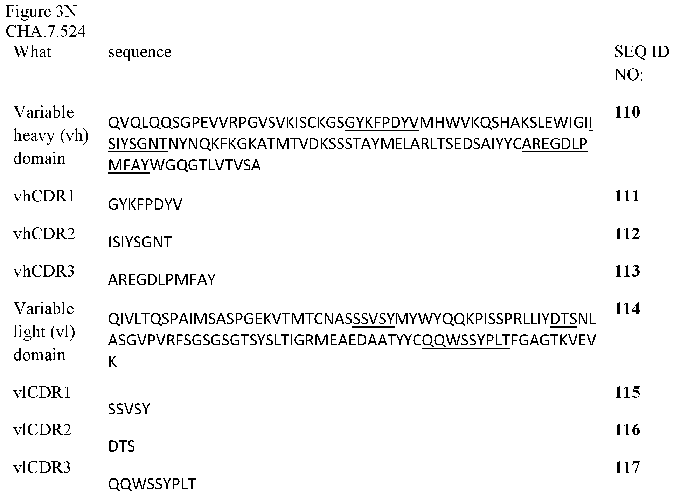

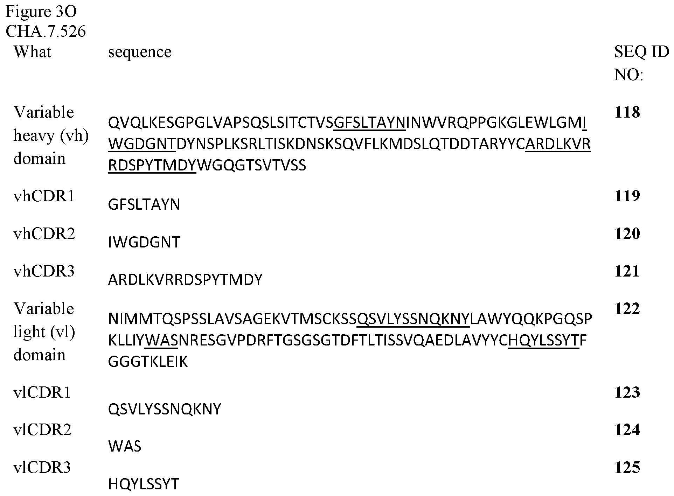

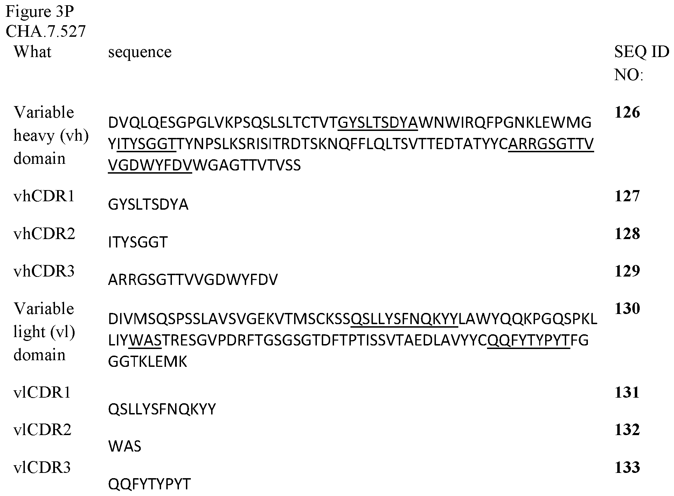

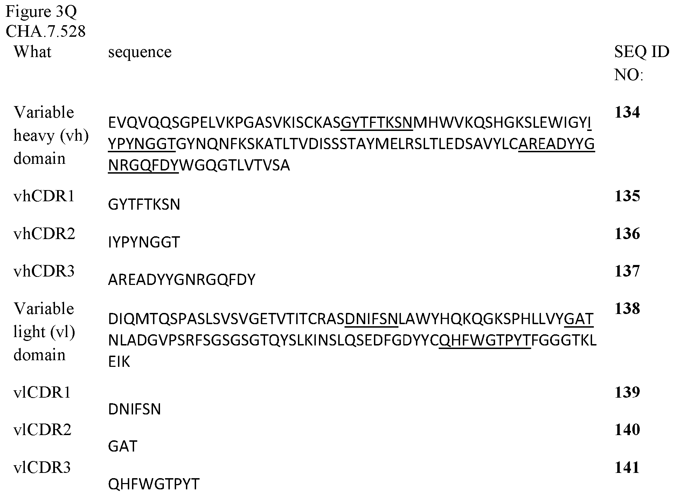

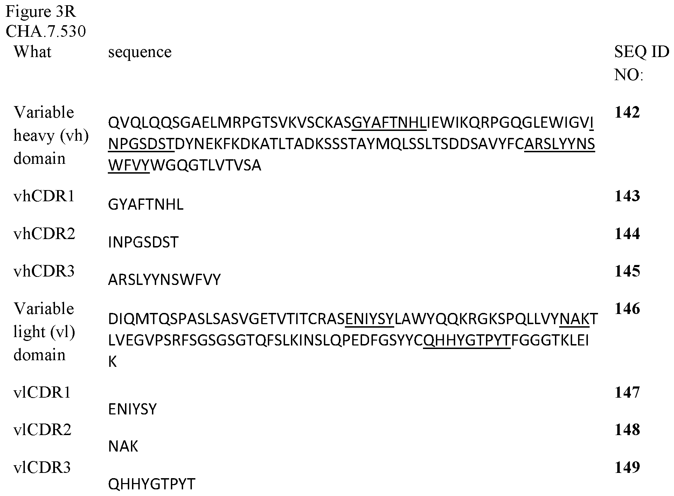

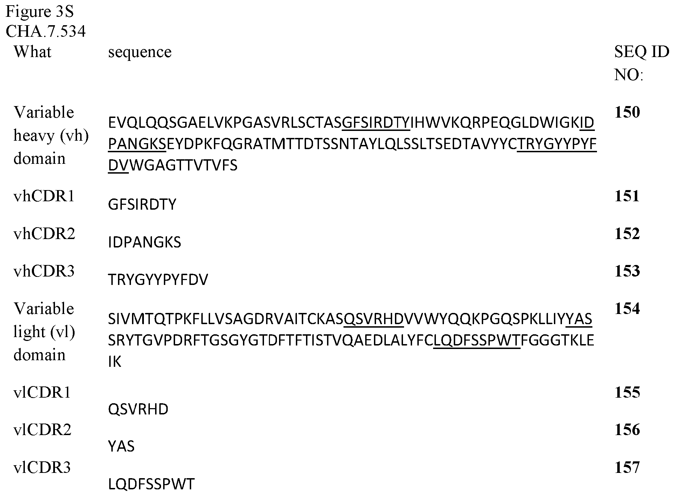

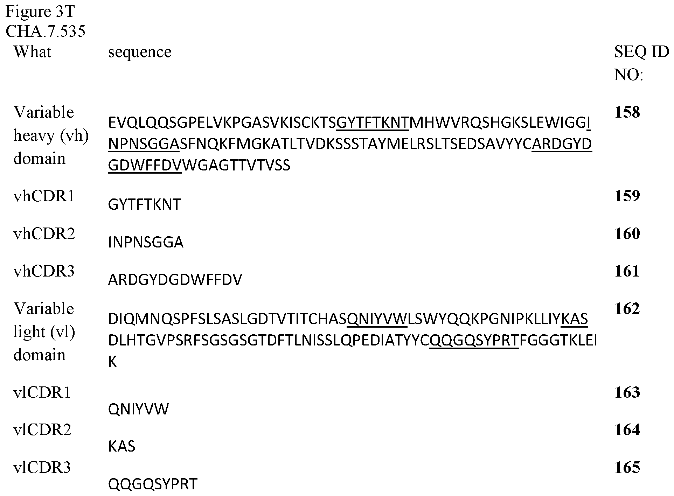

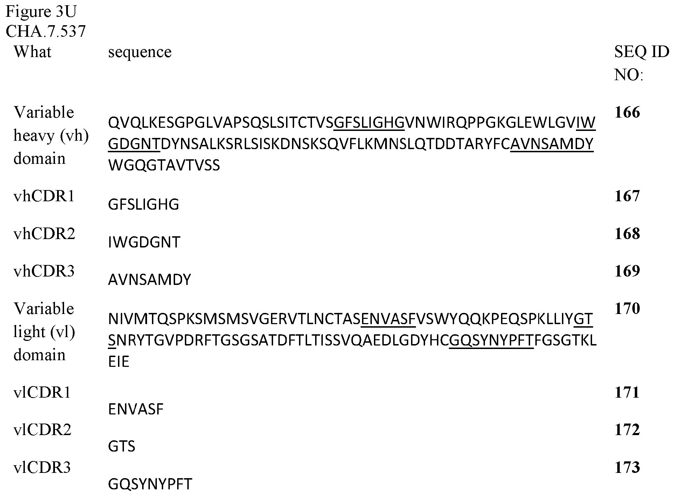

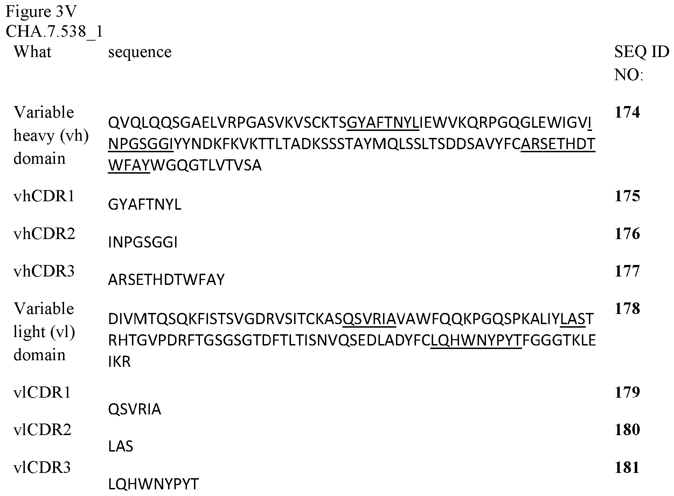

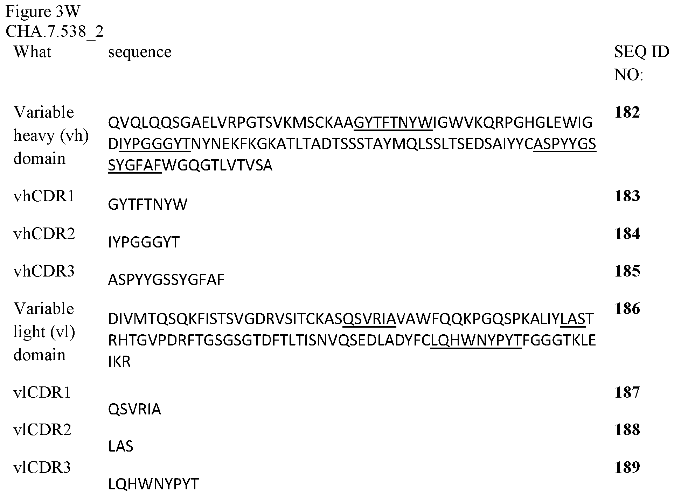

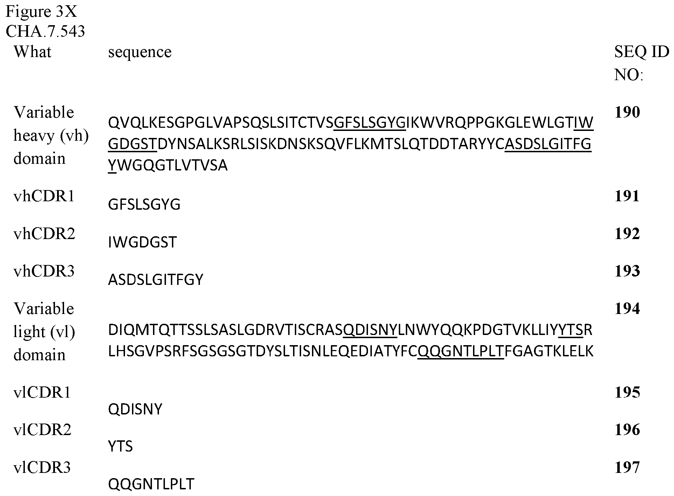

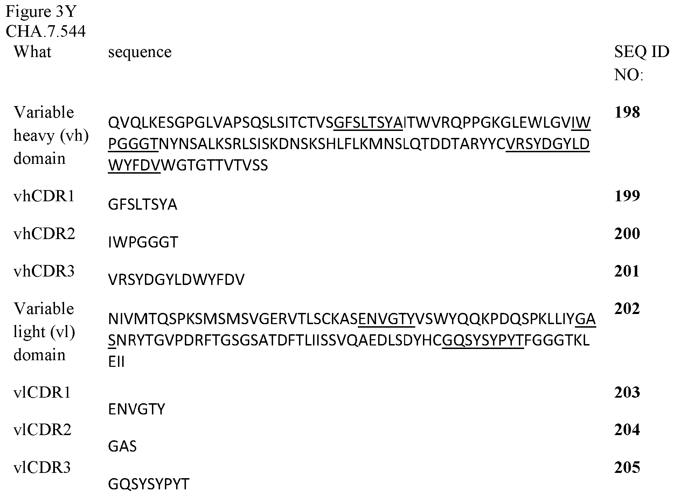

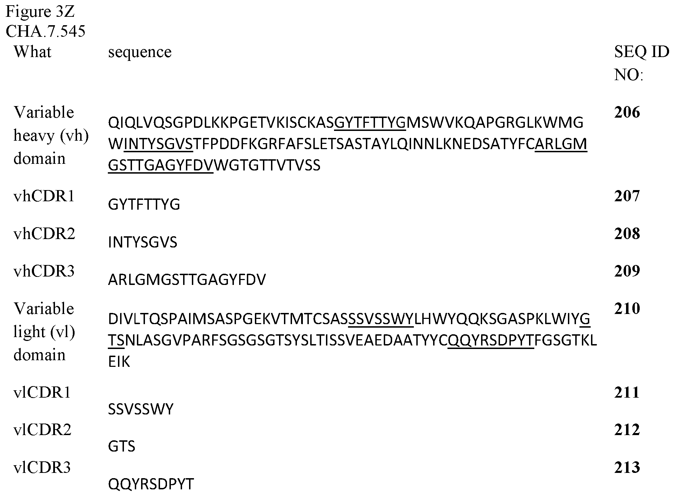

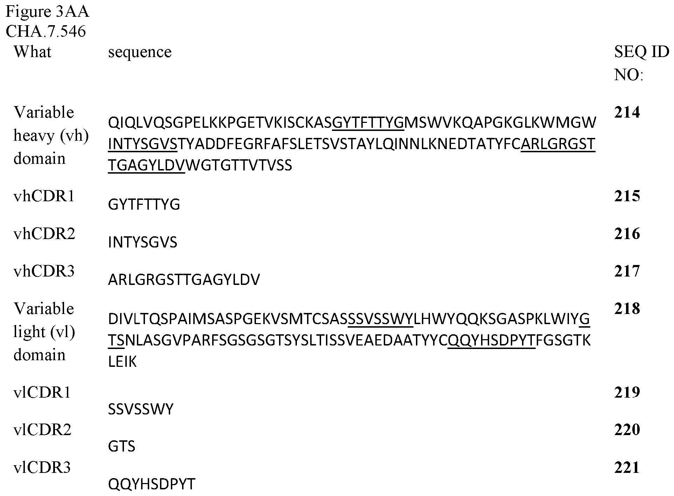

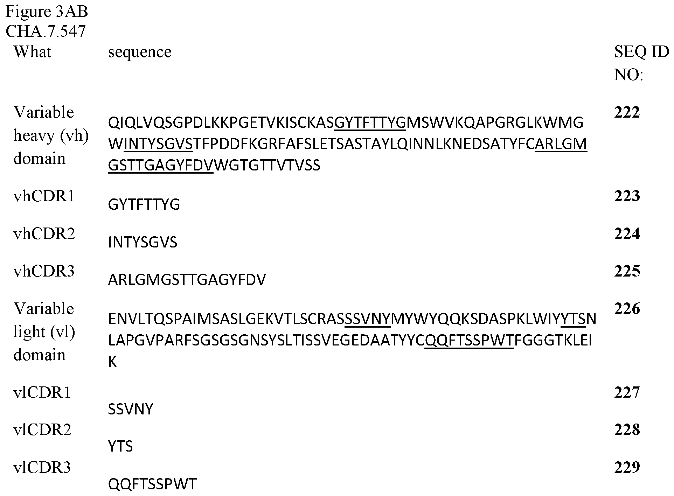

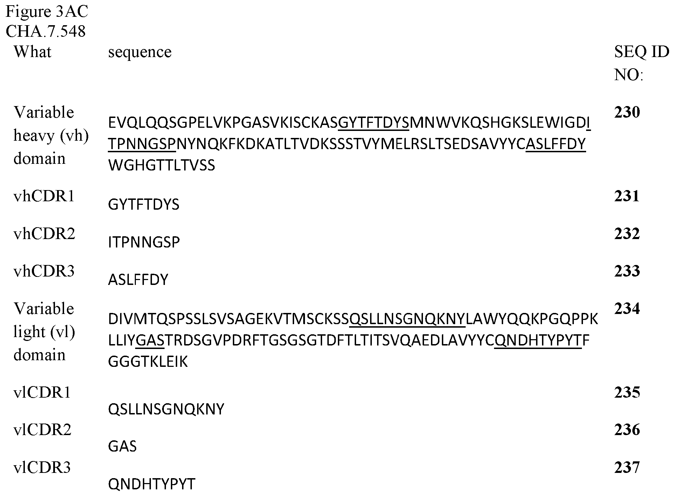

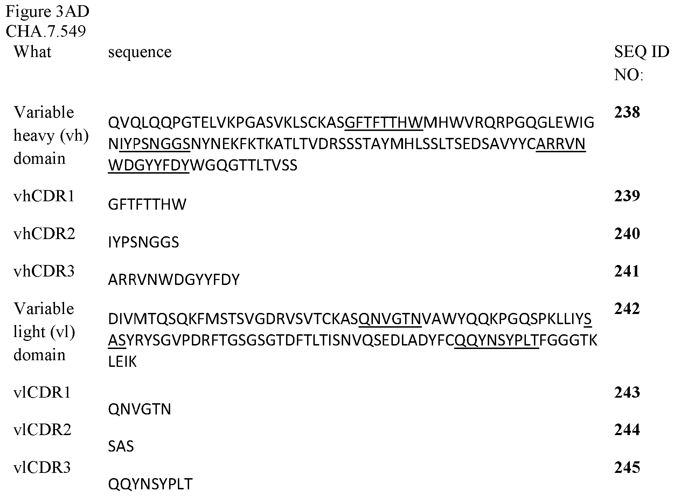

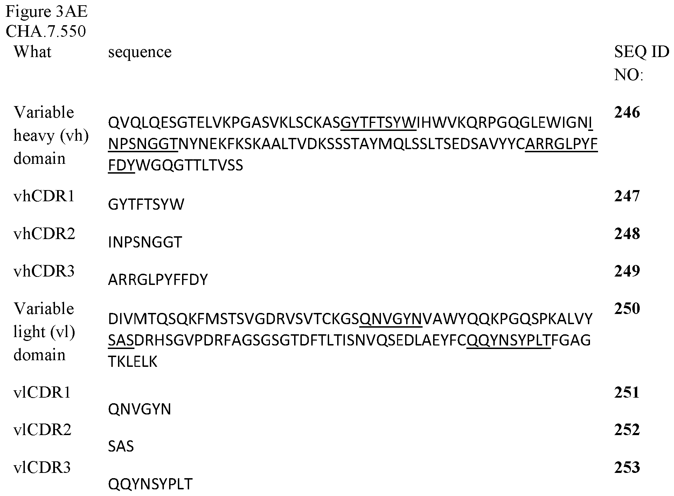

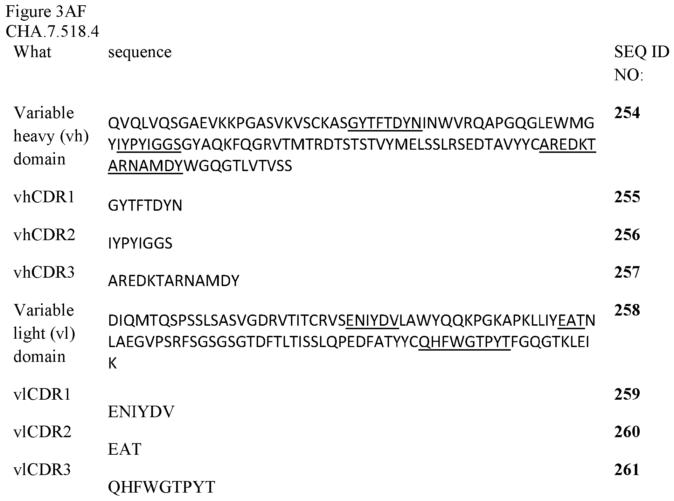

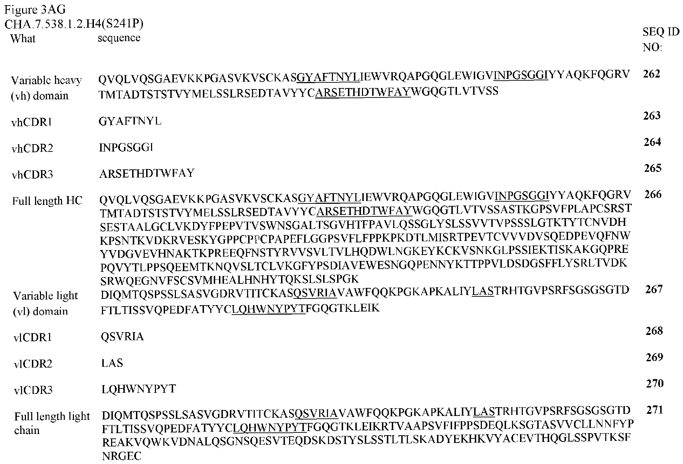

Accordingly, it is also an object of the invention to provide combination therapies as well as stable liquid pharmaceutical formulations comprising anti-PVRIG antibodies for use in disease treatment (e.g., anti-PVRIG antibodies including those with CDRs identical to those shown in Figure 3, and anti-TIGIT antibodies including those with CDRs identical to those shown in Figure 13).

Anti-PVRIG antibodies (including antigen-binding fragments) that both bind to PVRIG and prevent activation by PVRL2 have been identified, however, there remains a need in the art to develop further therapeutic treatments for multiple myeloma. Accordingly, it is a further object of the invention to provide antibodies and combinations thereof for use in the treatment of multiple myeloma, including treatment with anti-PVRIG antibodies, anti-TIGIT antibodies, and combination thereof.

### BRIEF SUMMARY OF THE INVENTION

Accordingly, it is an object of the invention to provide methods of treating cancer comprising administering i) an anti-PVRIG antibody and an anti-TIGIT antibody, or ii) an anti-TIGIT antibody alone, or iii) an anti-PVRIG antibody alone.

In some embodiments, the anti-PVRIG antibody is CHA.7.518.1.H4(S241P) and the anti-TIGIT antibody is CPA.9.086.H4(S241P).

T In some embodiments, the anti-PVRIG antibody comprises:
i) a heavy chain variable domain comprising the vhCDR1, vhCDR2, and vhCDR3 from the heavy chain of CHA.7.518.1.H4(S241P) (SEQ ID NO:4), and
ii) a light chain variable domain comprising the vlCDR1, vlCDR2, and vlCDR3 from the light chain of CHA.7.518.1.H4(S241P) (SEQ ID NO:9).

In some embodiments, the anti-TIGIT antibody comprises:
i) a heavy chain variable domain comprising the vhCDR1, vhCDR2, and vhCDR3 from the heavy chain of CPA.9.086.H4(S241P) (SEQ ID NO:877), and
ii) a light chain variable domain comprising the vlCDR1, vlCDR2, and vlCDR3 from the light chain of CPA.9.086.H4(S241P) (SEQ ID NO:882).

In some embodiments, the anti-TIGIT antibody is administered every 3 weeks or every 4 weeks.

In some embodiments, the anti-TIGIT antibody and the anti-PVRIG antibody are each individually administered every 3 weeks or every 4 weeks.

In some embodiments, the anti-TIGIT and/or the anti-TIGIT antibody is administered intravenously.

In some embodiments, the antiPVRIG anti-PVRIG antibody is administered as a stable liquid pharmaceutical formulation of an anti-PVRIG antibody comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the anti-PVRIG antibody and/or anti-TIGIT antibody comprises a CH1-hinge-CH2-CH3 sequence of IgG4 (SEQ ID NO:17 or SEQ ID NO:50), wherein the hinge region optionally comprises mutations.

In some embodiments, the anti-PVRIG antibody and/or anti-TIGIT antibody comprises the CH1-hinge-CH2-CH3 region from IgG1, IgG2, IgG3, or IgG4, wherein the hinge region optionally comprises mutations.

In some embodiments of the anti-PVRIG antibody the heavy chain variable domain is from the heavy chain of CHA.7.518.1.H4(S241P) (SEQ ID NO:4) and the light chain variable domain is from the light chain of CHA.7.518.1.H4(S241P) (SEQ ID NO:9) and for the anti-TIGIT antibody the heavy chain variable domain is from the heavy chain of CPA.9.086 (S241P) (SEQ ID NO:877) and the light chain variable domain is from the light chain of CPA.9.086 (S241P) (SEQ ID NO:882).

In some embodiments, the anti-PVRIG antibody comprises a CL region of human kappa 2 light chain.

In some embodiments, the pharmaceutical formulation comprises from 10 mM to 80 mM histidine, from 15 mM to 70 mM histidine, from 20 mM to 60 mM histidine, from 20 mM to 50 mM histidine, or from 20 mM to 30 mM histidine.

In some embodiments, the pharmaceutical formulation comprises about 25 mM histidine.

In some embodiments, the pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.

In some embodiments, the pharmaceutical formulation comprises about 60 mM NaCl.

The method according to any one of claims 1-16, wherein the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.

In some embodiments, the pharmaceutical formulation comprises about 100 mM L-arginine.

In some embodiments, the pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.

In some embodiments, the pharmaceutical formulation comprises about 0.01% polysorbate 80.

In some embodiments, the pH is from 6 to 7.0.

In some embodiments, the pH is from 6.3 to 6.8.

In some embodiments, the pH is 6.5 +/- 0.2.

In some embodiments, the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.

In some embodiments, the formulation is stable at 2°C to 8°C for at least 1 week, 2 weeks, 3 weeks, 4 weeks, 5 weeks, 6 weeks, 7 weeks, 8 weeks, 9 weeks, or 10 weeks, 1 month, 3 months, 6 months, 9 months, 12 months, 18 months, 24 months, 30 months, 36 months, or 42 months.

In some embodiments, the formulation is stable at about 20°C to 25 °C for at least 1 week, 2 weeks, 3 weeks, 4 weeks, 5 weeks, or 6 weeks, 1 months, 3 months, 6 months, or 9 months, 12 months, 18 months, or 36 months.

In some embodiments, the formulation is stable at 35°C to 40°C for at least 1 week, 2 weeks, 3 weeks, 4 weeks, or 5 weeks.

In some embodiments, the anti-PVRIG antibody is at a concentration of about 20 mg/mL.

In some embodiments, the anti-PVRIG antibody formulation comprises:
a) a heavy chain comprising:
   i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
b) a light chain comprising:
   i) a VL-CL, wherein the VL from CHA.7.518.1.H4(S241P) (SEQ ID NO:9) and wherein the CL region is from human kappa 2 light chain.

In some embodiments, the hinge region optionally comprises mutations.

In some embodiments, the hinge region optionally comprises mutations.

In some embodiments, the anti-PVRIG antibody formulation comprises:
i) a heavy chain comprising the heavy chain from CHA.7.518.1.H4(S241P) (SEQ ID NO:8); and
ii) a light chain comprising the light chain from CHA.7.518.1.H4(S241P) (SEQ ID NO:13).

In some embodiments, the anti-PVRIG antibody formulation comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the anti-PVRIG antibody formulation comprising:
(a) an anti-PVRIG antibody, wherein the anti-PVRIG antibody comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the anti-PVRIG antibody is administered at a dosage of about 0.01 mg/kg to about 20 mg/kg of the anti-PVRIG antibody or about 0.01 mg/kg to about 10 mg/kg of the anti-PVRIG antibody.

In some embodiments, the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.

In some embodiments, the anti-PVRIG antibody is administered 20 mg/kg every 4 weeks.

In some embodiments, the stable liquid pharmaceutical formulation is administered for the treatment of cancer.

In some embodiments, the present invention provides for use in a method of treating cancer.

In some embodiments, the cancer selected from the group consisting of gastro-esophageal junction cancer, prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, multiple myeloma, and/or Myelodysplastic syndromes (MDS).

In some embodiments, the cancer selected from the group consisting advanced cancer, ovarian cancer, lung cancer, colon cancer, plasma cell neoplasm, multiple myeloma, and HNSCC.

In some embodiments, the cancer selected from the group consisting of HNSCC, CRC (MSS), NSCLC, and multiple myeloma.

In some embodiments, the presnet disclosures provide a method for treating multiple myeloma comprising administering an anti-PVRIG antibody, optionally wherein the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.

In some embodiments, the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.

In some embodiments, the anti-PVRIG antibody comprises a heavy chain variable domain from the heavy chain of CHA.7.518.1.H4(S241P) (SEQ ID NO:8) and a light chain variable domain from the light chain of CHA.7.518.1.H4(S241P) (SEQ ID NO:13).

In some embodiments, the anti-PVRIG antibody comprises a heavy chain variable domain from the heavy chain of CHA.7.538.1.2.H4(S241P) (SEQ ID NO:266) and a light chain variable domain from the light chain of CHA.7.538.1.2.H4(S241P) (SEQ ID NO:271).

In some embodiments, the anti-PVRIG antibody is administered as a stable liquid pharmaceutical formulation of the anti-PVRIG antibody comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:8), and
   ii) a light chain variable domain comprising the vlCDR1, vlCDR2, and vlCDR3 from the light chain of CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the anti-PVRIG antibody comprises a CH1-hinge-CH2-CH3 sequence of IgG4 (SEQ ID NO:657 or SEQ ID NO:658), wherein the hinge region optionally comprises mutations.

In some embodiments, the anti-PVRIG antibody comprises a CH1-hinge-CH2-CH3 region from IgG1, IgG2, IgG3, or IgG4, wherein the hinge region optionally comprises mutations.

In some embodiments, the heavy chain variable domain of the anti-PVRIG antibody is from the heavy chain of CHA.7.518.1.H4(S241P) (SEQ ID NO:8) and the light chain variable domain of the anti-PVRIG antibody is from the light chain of CHA.7.518.1.H4(S241P) (SEQ ID NO:13).

In some embodiments, the anti-PVRIG antibody comprises a CL region of human kappa 2 light chain.

In some embodiments, the stable liquid formulation comprises from 10 mM to 80 mM histidine, from 15 mM to 70 mM histidine, from 20 mM to 60 mM histidine, from 20 mM to 50 mM histidine, or from 20 mM to 30 mM histidine.

In some embodiments, the pharmaceutical formulation comprises about 25 mM histidine.

In some embodiments, the pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.

In some embodiments, the pharmaceutical formulation comprises about 60 mM NaCl.

In some embodiments, the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.

In some embodiments, the pharmaceutical formulation comprises about 100 mM L-arginine.

In some embodiments, the pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.

In some embodiments, the pharmaceutical formulation comprises about 0.01% polysorbate 80.

In some embodiments, the pH is from 6 to 7.0.

In some embodiments, the pH is from 6.3 to 6.8.

In some embodiments, the pH is 6.5 +/- 0.2.

In some embodiments, wherein the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.

In some embodiments, the anti-PVRIG antibody is at a concentration of about 20 mg/mL.

In some embodiments, the anti-PVRIG antibody formulation comprises:
a) a heavy chain comprising:
   i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
b) a light chain comprising:
   i) a VL-CL, wherein the VL from CHA.7.518.1.H4(S241P) (SEQ ID NO:9) and wherein the CL region is from human kappa 2 light chain.

In some embodiments, the hinge region optionally comprises mutations.

In some embodiments, the hinge region optionally comprises mutations.

In some embodiments, the anti-PVRIG antibody formulation comprises:
i) a heavy chain comprising the heavy chain from CHA.7.518.1.H4(S241P) (SEQ ID NO:8); and
ii) a light chain comprising the light chain from CHA.7.518.1.H4(S241P) (SEQ ID NO:13).

In some embodiments, anti-PVRIG antibody formulation comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:8), and
   ii) a light chain variable domain comprising the vlCDR1, vlCDR2, and vlCDR3 from the light chain of CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

The method according to any one of claims 47 to 67, the anti-PVRIG antibody formulation comprising:
(a) an anti-PVRIG antibody, wherein the anti-PVRIG antibody comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the anti-PVRIG antibody is administered at a dosage of about 0.01 mg/kg to about 20 mg/kg of the anti-PVRIG antibody or about 0.01 mg/kg to about 10 mg/kg of the anti-PVRIG antibody.

In some embodiments, the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.

In some embodiments, the anti-PVRIG antibody is administered about 20 mg/kg every 4 weeks.

In some embodiments, the anti-PVRIG antibody is administered in combination with an anti-DNAM antibody.

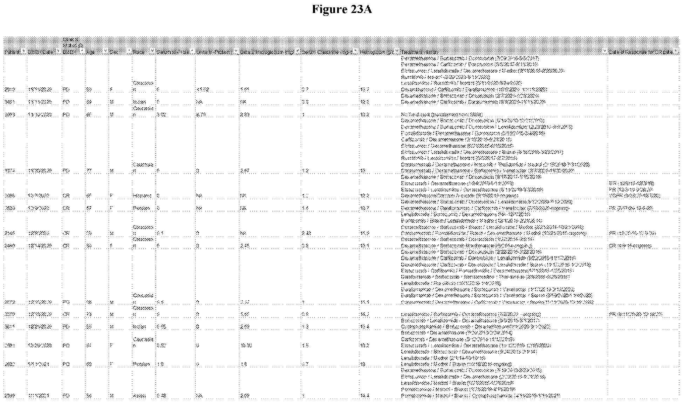

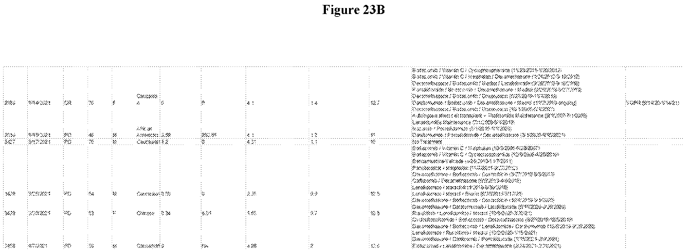

In some embodiments, the anti-DNAM antibody is selected from the group consisting of 10E5, DX11, 11A8.7.4, and the anti-CD226 antibody as shown in Figure 23.

In some embodiments, the anti-PVRIG antibody is administered in combination with an anti-PD-1 antibody.

In some embodiments, the anti-PVRIG antibody is administered in combination with an anti-TIGIT antibody.

In some embodiments, the anti-PVRIG antibody is administered in combination with an anti-PD-1 antibody and an anti-TIGIT antibody.

In some embodiments, the anti-PD-1 antibody is selected from the group consisting of nivolumab and pembrolizumab.

In some embodiments, the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody is selected from the group consisting of CPA.9.083.H4(S241P) and CPA.9.086.H4(S241P).

In some embodiments, the anti-TIGIT antibody comprises:
a) a heavy chain comprising VH-CH1-hinge-CH2-CH3; and
b) a light chain comprising VL-VC, wherein VC is either kappa or lambda.

In some embodiments, the sequence of the CH1-hinge-CH2-CH3 is selected from human IgG1, IgG2 and IgG4, and variants thereof.

In some embodiments, the anti-PVRIG antibody is CHA.7.518.1.H4(S241P), the anti-TIGIT antibody is CPA.9.086.H4(S241P) and the anti-PD-1 antibody is nivolumab.

In some embodiments, the anti-PVRIG antibody is CHA.7.518.1.H4(S241P), the anti-TIGIT antibody is CPA.9.083.H4(S241P) and the anti-PD-1 antibody is nivolumab.

In some embodiments, the anti-PVRIG antibody is CHA.7.518.1.H4(S241P), the anti-TIGIT antibody is CPA.9.086.H4(S241P) and the anti-PD-1 antibody is pembrolizumab.

In some embodiments, the anti-PVRIG antibody is CHA.7.518.1.H4(S241P), the anti-TIGIT antibody is CPA.9.083.H4(S241P) and the anti-PD-1 antibody is pembrolizumab.

In some embodiments, the anti-PVRIG antibody is CHA.7.538.1.2.H4(S241P), the anti-TIGIT antibody is CPA.9.086.H4(S241P) and the anti-PD-1 antibody is nivolumab.

In some embodiments, the anti-PVRIG antibody is CHA.7.538.1.2.H4(S241P), the anti-TIGIT antibody is CPA.9.083.H4(S241P) and the anti-PD-1 antibody is nivolumab.

In some embodiments, the anti-PVRIG antibody is CHA.7.538.1.2.H4(S241P), the anti-TIGIT antibody is CPA.9.086.H4(S241P) and the anti-PD-1 antibody is pembrolizumab.

In some embodiments, the anti-PVRIG antibody is CHA.7.538.1.2.H4(S241P), the anti-TIGIT antibody is CPA.9.083.H4(S241P) and the anti-PD-1 antibody is pembrolizumab.

In some embodiments, the anti-PVRIG antibody and/or the anti-TIGIT antibody is administered in combination with a further multiple myeloma therapy.

In some embodiments, the anti-PVRIG antibody and/or the anti-TIGIT antibody is administered in combination with one or more chemotherapies or chemotherapeutics.

In some embodiments, the one or more chemotherapeutics or chemotherapeutics is selected from the group consisting of cyclophosphamide (cytoxan), etoposide (VP-16), doxorubicin (adriamycin), liposomal doxorubicin (Doxil), melphalan, melphalan flufenamide, melflufen (Pepaxto), and bendamustine (Treanda).

In some embodiments, the anti-PVRIG antibody and/or the anti-TIGIT antibody is administered in combination with one or more corticosteroids.

In some embodiments, the the one or more corticosteroids are selected from the group consisting of dexamethasone and/ prednisone.

In some embodiments, the anti-PVRIG antibody and/or the anti-TIGIT antibody is administered in combination with one or more immunomodulating agents.

In some embodiments, the one or more immunomodulating agents include are selected from the group consisting of thalidomide (Thalomid), lenalidomide (Revlimid), and pomalidomide (Pomalyst).

In some embodiments, the anti-PVRIG antibody and/or the anti-TIGIT antibody is administered in combination with one or more proteasome inhibitors.

In some embodiments, the one or more proteasome inhibitors are selected from the group consisting of bortezomib (Velcade), carfilzomib (Kyprolis), and ixazomib (Ninlaro).

In some embodiments, the anti-PVRIG antibody and/or the anti-TIGIT antibody is administered in combination with one or more histone deacetylase (HDAC) inhibitors.

In some embodiments, the one or more histone deacetylase (HDAC) inhibitors is panobinostat (Farydak).

In some embodiments, the anti-PVRIG antibody and/or the anti-TIGIT antibody is administered in combination with one or more anti-CD38 monoclonal antibodies.

In some embodiments, the one or more anti-CD38 monoclonal antibodies are selected from the group consisting of daratumumab (Darzalex), daratumumab and hyaluronidase (Darzalex Faspro), isatuximab (Sarclisa), and belantamab mafodotin-blmf (Blenrep).

In some embodiments, the anti-PVRIG antibody and/or the anti-TIGIT antibody is administered in combination with one or more anti-SLAMF7 antibodies.

In some embodiments, the anti-SLAMF7 monoclonal antibody is elotuzumab (Empliciti).

In some embodiments, the anti-PVRIG antibody and/or the anti-TIGIT antibody is administered in combination with lenalidomide (or pomalidomide or thalidomide) and dexamethasone.

In some embodiments, the anti-PVRIG antibody and/or the anti-TIGIT antibody is administered in combination with carfilzomib (or ixazomib or bortezomib), lenalidomide, and dexamethasone.

In some embodiments, the anti-PVRIG antibody and/or the anti-TIGIT antibody is administered in combination with bortezomib (or carfilzomib), cyclophosphamide, and dexamethasone.

In some embodiments, the anti-PVRIG antibody and/or the anti-TIGIT antibody is administered in combination with elotuzumab (or daratumumab), lenalidomide, and dexamethasone.

In some embodiments, the anti-PVRIG antibody and/or the anti-TIGIT antibody is administered in combination with bortezomib, liposomal doxorubicin, and dexamethasone.

In some embodiments, the anti-PVRIG antibody and/or the anti-TIGIT antibody is administered in combination with panobinostat, bortezomib, and dexamethasone.

In some embodiments, the anti-PVRIG antibody and/or the anti-TIGIT antibody is administered in combination with elotuzumab, bortezomib, and dexamethasone.

In some embodiments, the anti-PVRIG antibody and/or the anti-TIGIT antibody is administered in combination with melphalan and prednisone (MP), in the presence or absence of thalidomide and/or bortezomib

In some embodiments, the anti-PVRIG antibody and/or the anti-TIGIT antibody is administered in combination with vincristine, doxorubicin (Adriamycin), and dexamethasone (called VAD).

In some embodiments, the anti-PVRIG antibody and/or the anti-TIGIT antibody is administered in combination with dexamethasone, cyclophosphamide, etoposide, and cisplatin (called DCEP).

In some embodiments, the anti-PVRIG antibody and/or the anti-TIGIT antibody is administered in combination with dexamethasone, thalidomide, cisplatin, doxorubicin, cyclophosphamide, and etoposide (called DT-PACE), with or without bortezomib.

In some embodiments, the anti-PVRIG antibody and/or the anti-TIGIT antibody is administered in combination with elinexor, bortezomib, and dexamethasone.

In some embodiments, the subject to be administered the therapy has multiple myleoma with high DNAM expression.

In some embodiments, the subject to be administered the therapy has multiple myleoma with high DNAM expression in CD8+ cells and/or a high percentage of CD8+ cells are DNAM positive.

In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In one aspect, the present disclosures provide a method for treating multiple myeloma comprising administering an anti-TIGIT antibody, optionally wherein the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.80.

In some embodiments, the anti-TIGIT antibody comprises a heavy chain variable domain from the heavy chain of CPA.9.086.H4(S241P) (SEQ ID NO:877) and a light chain variable domain from the light chain of CPA.9.086.H4(S241P) (SEQ ID NO:882).

In some embodiments, the anti-TIGIT antibody comprises a heavy chain variable domain from the heavy chain of CPA.9.086.H4(S241P) and a light chain variable domain from the light chain of CPA.9.086.H4(S241P).

In some embodiments, the anti-TIGIT antibody is administered in combination with an anti-PVRIG antibody provided herein.

In some embodiments, the anti-TIGIT antibody is administered in combination with an anti-PD-1 antibody.

In some embodiments, the anti-PD-1 antibody is selected from the group consisting of nivolumab and pembrolizumab.

In some embodiments, the anti-TIGIT antibody is administered in combination with an anti-DNAM antibody.

In some embodiments, the anti-DNAM antibody is selected from the group consisting of 10E5, DX11, 11A8.7.4, and the anti-CD226 antibody as shown in Figure 23.

In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In one aspect, the present disclosures provide a method of treatment for cancer comprising administering an anti-TIGIT antibody comprising the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8,
wherein the anti-TIGIT antibody is administered at a dosage of about 0.01 mg/kg to about 10 mg/kg.

In some embodiments, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody comprises:
a) a heavy chain comprising VH-CH1-hinge-CH2-CH3; and
b) a light chain comprising VL-VC, wherein VC is either kappa or lambda.

In some embodiments, the sequence of the CH1-hinge-CH2-CH3 is selected from human IgG1, IgG2 and IgG4, and variants thereof.

In some embodiments, the hinge region optionally comprises mutations.

In some embodiments, the anti-TIGIT antibody is selected from the group consisting of CPA.9.083.H4(S241P) and CPA.9.086.H4(S241P).

In some embodiments, the anti-TIGIT antibody is CPA.9.083.H4(S241P).

In some embodiments, the anti-TIGIT antibody is CPA.9.086.H4(S241P).

In some embodiments, the anti-TIGIT antibody is administered every 1 week, 2 weeks, 3 weeks, or 4 weeks.

In some embodiments, the anti-TIGIT antibody is administered every 3 weeks.

In some embodiments, the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody.

In some embodiments, the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody every 1 week, 2 weeks, 3 weeks, or 4 weeks.

In some embodiments, the anti-TIGIT antibody is administered about 10 mg/kg every 3 weeks or every 4 weeks.

In some embodiments, the anti-TIGIT antibody is administered about 3 mg/kg every 3 weeks or every 4 weeks.

In some embodiments, the cancer is selected from the group consisting of vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma (including hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma), post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, malignant melanoma, pleural mesothelioma, chordoma, endometrial sarcoma, chondrosarcoma, uterine sarcoma, synovial sarcoma, uveal melanoma, amyloidosis, AL-amyloidosis, astrocytoma, and/or Myelodysplastic syndromes (MDS).

In some embodiments, the use of an anti-TIGIT for the treatment of cancer is provided herein.

In some embodiments, the cancer is selected from the group consisting of prostate cancer, liver cancer (HCC), rectal cancer, colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), uveal melanoma, glioma, renal cell cancer (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cell cancer, MSI-high cancer, KRAS mutant tumors, adult T-cell leukemia/lymphoma, pleural mesothelioma, anal SCC, neuroendocrine lung cancer (including neuroendocrine lung carcinoma), small cell lung cancer, NSCLC, NSCLC large cell, NSCLC squamous cell, NSCLC adenocarcinoma, atypical carcinoid lung cancer, NSCLC with PDL1 >=50% TPS, cervical SCC, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, chordoma, sarcoma, endometrial sarcoma, chondrosarcoma, uterine sarcoma, plasma cell disorders, multiple myeloma, amyloidosis, AL-amyloidosis, glioblastoma, astrocytoma, and fallopian tube cancer.

In another aspect, the present disclosures provide a method of treating cancer comprising administering a therapeutic combination selected from the group consisting of:
i) an anti-PVRIG antibody, an anti-TIGIT antibody, and an immune cell engager;
ii) an anti-TIGIT antibody and an immune cell engager; and
iii) an anti-PVRIG antibody and an immune cell engager.

In some embodiments, the immune cell engager is bispecific or is a bispecifc antibody.

In some embodiments, the immune cell engager comprises a tumor-targeting moiety.

In some embodiments, the tumor-targeting moiety comprises an antibody molecule or a binding portion thereof, optionally which specifically binds to the targeted tumor and/or tumor cells.

In some embodiments, the immune cell engager comprises an immune cell-targeting moiety.

In some embodiments, the immune cell-targeting moiety comprises an antibody molecule or a binding portion thereof, optionally which specifically binds to a target immune cell antigen.

In some embodiments, the immune cell antigen is selected from the group consisting of a T cell antigen, a NK cell antigen, a B cell antigen, a dendritic cell antigen, and a macrophage cell antigen.

In some embodiments, the immune cell engager is a T cell engager.

In some embodiments, the T cell engager is selected from the group consising of a Bispecific T cell engager (BiTE), a F(ab')2, F(ab')-ScFv2, a di-scFv, a diabody, a minibody, a scFv-Fc, a DART, a TandAb, a ScDiabody, a ScDiabody-CH3, Diabody-CH3, a triple body, a miniantibody, a minibody, a TriBi minibody, a ScFv-CH3 KIH (knobs in holes), a Fab-ScFv, a SCFv-CH-CL-scFv, a scFv-KIH, a Fab-scFv-Fc, a Tetravalent HCAb, a scDiabody-Fc, a Diabody-Fc, a intrabody, dock and lock antibodies, a ImmTAC, a HSAbody, a ScDiabody-HAS, a humabody and a Tandem ScFv-toxic.

In some embodiments, the immune cell engager comprises a fusion protein comprising two or more polypeptides.

In some embodiments, the immune cell engager comprises two or more single-chain variable fragments (scFvs), wehrein at least two of the scFvs comprise binding domains from different antibodies, and wherein at least one scFv comprises an effector cell surface molecule binding domain and binds to an effector cell surface molecule and at least one scFv binds to a tumor cell, optionally a tumor specific cell surface molecule binding domain and binds to a tumor specific cell surface molecule.

In some embodiments, the effector cell surface molecule is selected from the group consisting of CD3, CD28, CD5, CD16, NKG2D, CD64, CD32, CD89, NKG2C, CD44v6, IL-12, PD-L1, Vγ9Vδ2, NKp46, and BCMA.

In some embodiments, the tumor specific cell surface molecule is selected from the group consisting of BCMA, B7H3, CD10, CD19, CD20, CD22, CD24, CD30, CD33, CD34, CD38, CD44, CD79a, CD79b, CD123, CD138, CD179b, CEA, CLDN18.2, CLEC12A, CS-1, DLL3, EGFR, EGFRvIII, EGFRxFcγRI, EGFRvIIIxCD3, EGFRxCD3, EPCAM, FLT-3, FOLR1, FOLR3, GD2, gpA33, GPC3, HER2, HM1.24, LGR5, MSLN, MCSP, MICA/B, MUC 17 (mucin-17), PSMA, PAMA, P-cadherin, ROR1, PD-1, CLEC12A, WT1, EphA2, ADAM17, and PSCA.

In some embodiments, the immune cell engager effector cell surface molecule: tumor specific cell surface molecule pairs are selected from the group consisting of CD3:CD19, CD16:CD30, CD64:CD30, CD16:BCMA, CD64:BCMA, CD3:BCMA, and CD3:CD33.

In some embodiments, the effector cell surface molecule: tumor specific cell surface molecule pair is CD3:BCMA.

In some embodiments, the immune cell engager further comprises a linker, optionally between the effector cell surface molecule binding domain and the tumor specific cell antigen binding domain.

In some embodiments, the immune cell engager is a bi-specific T cell engager (BiTE)

In some embodiments, the BiTE is specific for the CD3ε subunit of the TCR complex of a T-cell and tumor-targeting moiety.

In some embodiments, the BiTEis selected from the group consisting of Blinatumomab (Blyncyto^{®}), Solitomab, Epcoritamab, Odronextamab, Mosunetuzumab, XmAb13676, Glofitamab, scIgG, IGM-2323, AMG 330, AMG 673, AMG 420, AMG 701, Teclistamab, REGN5458, PF-06863135, TNB-383B, Cevostamab, Talquetamab, Flotetuzumab, Pasotuxizumab, AMG 160, AMG 596, AMG 757, AMG 199, AMG 910, AMV564, Cibisatamab, M802, M701, ERY974, MGD007, ES414, Hu3F8-bsAb, ISB 1302, BTRC4017A, GEN1044, CAdDuo, CAdTrio, Vγ9Vδ2, bscEphA2xCD, A300E, PSCA, EGFRxFcγRI, EGFRvIIIxCD3, and EGFRxCD3.

In some embodiments, the immune cell engager binds to CD3 and BCMA.

In some embodiments, the immune cell engager is a bispecific antibody targeting CD3 and BCMA.

In some embodiments, the treatment further comprises administering one or more additional immmmune checkpoint inhibitor antibodies.

In some embodiments, the immmmune checkpoint inhibitor antibody is selected from the group consisting of anti-PVRIG antibodies, anti- PD-1 antibodies, anti-CTLA-4 antibodies, anti-PD-L1 antibodies, anti-LAG-3 antibodies, anti-TIM-3 antibodies, anti-BTLA antibodies, anti- DNAM1 antibodies, anti-ICOS antibodies, anti-4-1bb antibodies, anti-GITR antibodies, anti-OX40 antibodies, anti-CD96 antibodies, anti-B7-H4 antibodies, anti-B7-H3 antibodies, anti-VISTA antibodies, anti-CD27 antibodies, anti-CD40 antibodies, and anti-CD137 antibodies.

In some embodiments, the the anti-PVRIG antibody and/or the anti-TIGIT antibody, and/or the immune cell engager and/or the immmmune checkpoint inhibitor antibody are each administered sequentially or simultaneously, including in any order.

In some embodiments, the cancer is selected from the group consisting of vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma (including hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma), post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, malignant melanoma, pleural mesothelioma, chordoma, endometrial sarcoma, chondrosarcoma, uterine sarcoma, synovial sarcoma, uveal melanoma, amyloidosis, AL-amyloidosis, astrocytoma, and Myelodysplastic syndromes (MDS).

In some embodiments, the cancer is selected from the group consisting of prostate cancer, liver cancer (HCC), rectal cancer, colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), uveal melanoma, glioma, renal cell cancer (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cell cancer, MSI-high cancer, KRAS mutant tumors, adult T-cell leukemia/lymphoma, pleural mesothelioma, anal SCC, neuroendocrine lung cancer (including neuroendocrine lung carcinoma), small cell lung cancer, NSCLC, NSCLC large cell, NSCLC squamous cell, NSCLC adenocarcinoma, atypical carcinoid lung cancer, NSCLC with PDL1 >=50% TPS, cervical SCC, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, chordoma, sarcoma, endometrial sarcoma, chondrosarcoma, uterine sarcoma, plasma cell disorders, multiple myeloma, amyloidosis, AL-amyloidosis, glioblastoma, astrocytoma, and fallopian tube cancer.

In some embodiments, the anti-PVRIG antibody and/or the anti-TIGIT antibody is an antibody provided herein.

In some embodiments, the method is for use in the treatment of cancer.

In some embodiments, the present disclosures provide ase of the anti-PVRIG antibodies and/or anti-TIGIT antibodies and/or immune cell engagers.

In some embodiments, the anti-PVRIG antibodies and/or anti-TIGIT antibodies and/or immune cell engagers disclosed herein are used for the preparation of a medicament for use in a method for treating cancer.

### BRIEF DESCRIPTION OF THE DRAWINGS

Figure 1A depicts the full-length sequence of human PVRIG. Figure 1B depicts the sequences of human and cynomolgus macaque (referred to as cyno) TIGIT ECD and of the human PVR ECD proteins.

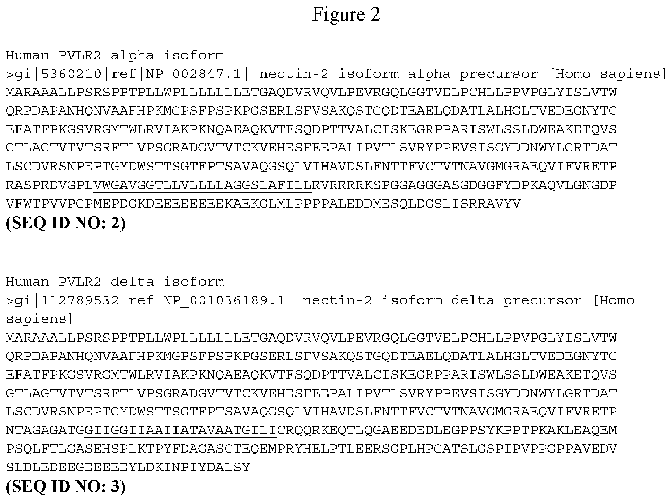

Figure 2 depicts the sequence of the human Poliovirus receptor-related 2 protein (PVLR2, also known as nectin-2, CD112 or herpesvirus entry mediator B, (HVEB)), the binding partner of PVRIG. PVLR2 is a human plasma membrane glycoprotein.
Figure 3A-3AG depicts the variable heavy and light chains as well as the vhCDR1, vhCDR2, vhCDR3, vlCDR1, vlCDR2 and vlCDR3 sequences of the PVRIG antibodies of the invention, including CHA.7.518.1.H4(S241P).
Figures 4A-4AA shows the amino acid sequences of the variable heavy and light domains, the full length heavy and light chains, and the variable heavy and variable light CDRs for the enumerated human CPA anti-PVRIG sequences of the invention that both bind PVRIG and block binding of PVRIG and PVLR2.
Figures 5A-5H depicts the amino acid sequences of the variable heavy and light domains, the full length heavy and light chains, and the variable heavy and variable light CDRs for eight human CPA anti-PVRIG sequences of the invention that bind PVRIG and but do not block binding of PVRIG and PVLR2.

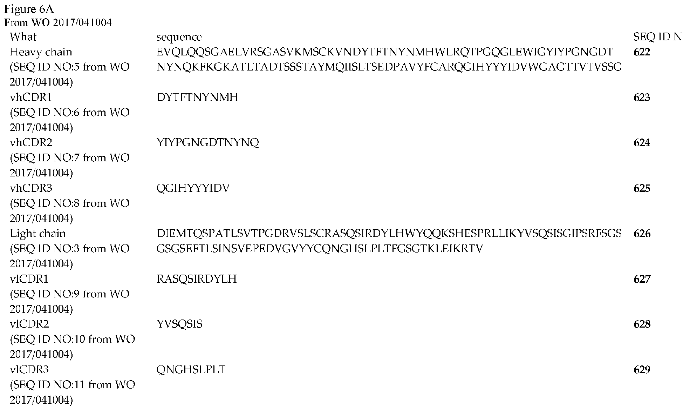

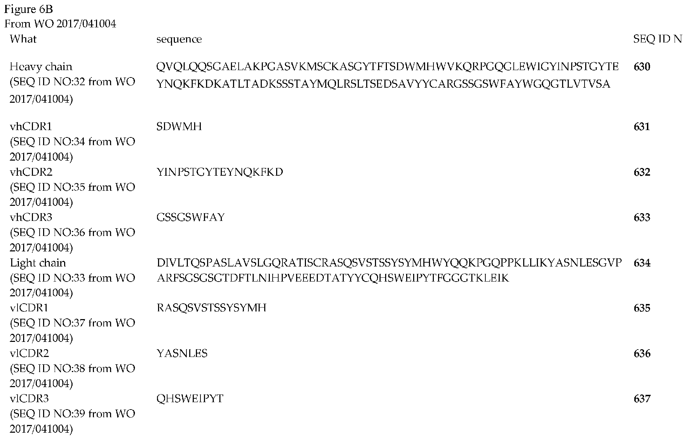

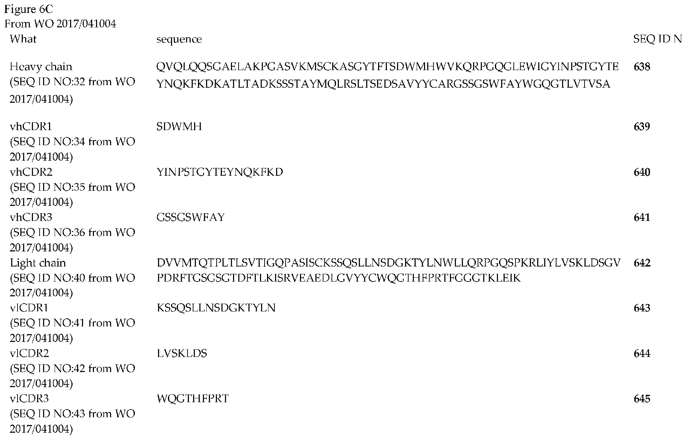

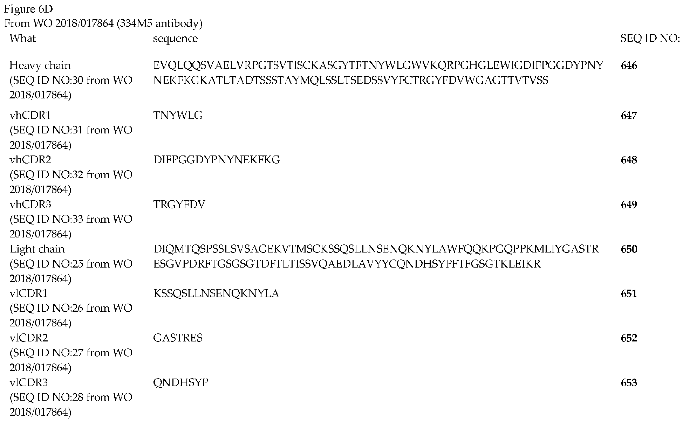

Figures 6A-6D depicts the sequences of other PVRIG antibodies that can be formulated according to stable liquid formulations of an anti-PVRIG antibody of the present invention and can be used as anti-PVRIG treatment antibodies.
Figure 7 depicts the sequences of human IgG1, IgG2, IgG3 and IgG4.

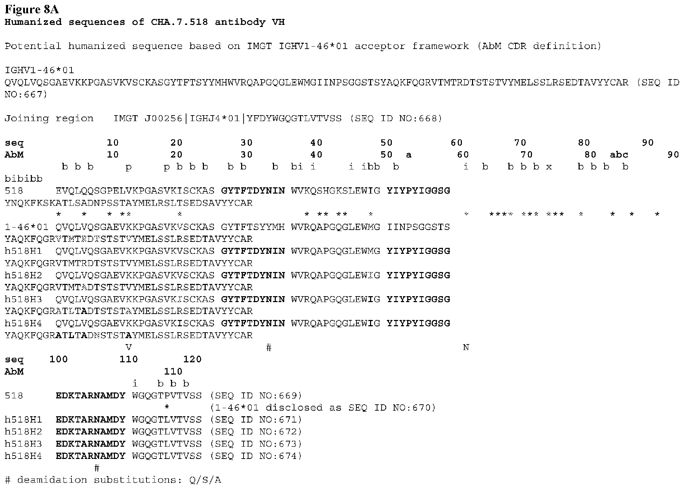

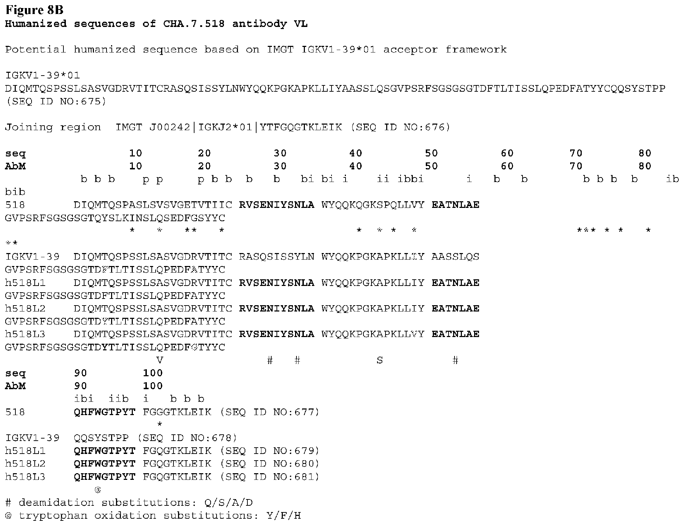

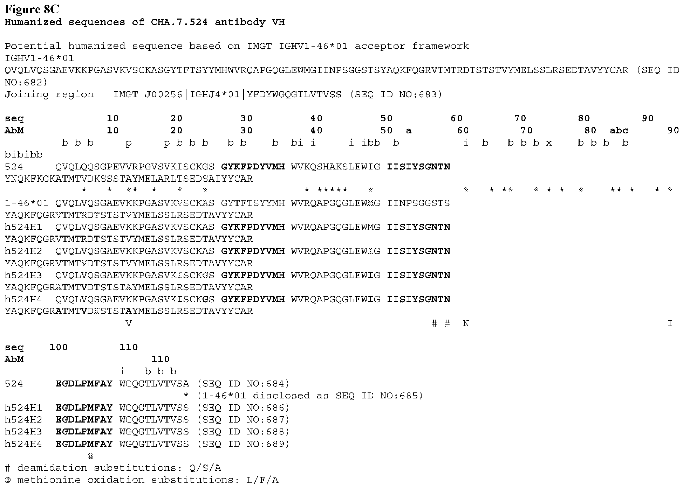

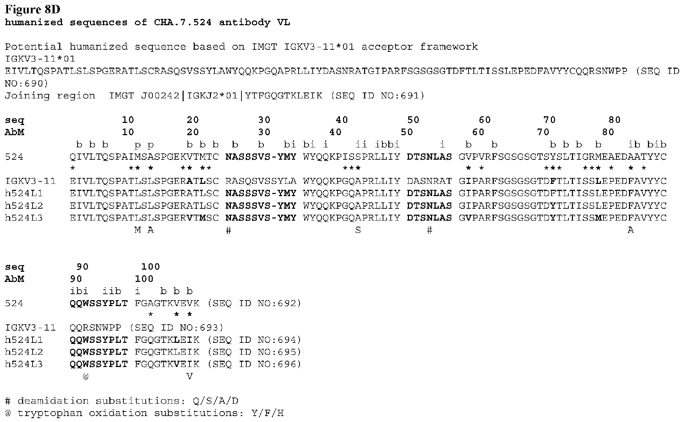

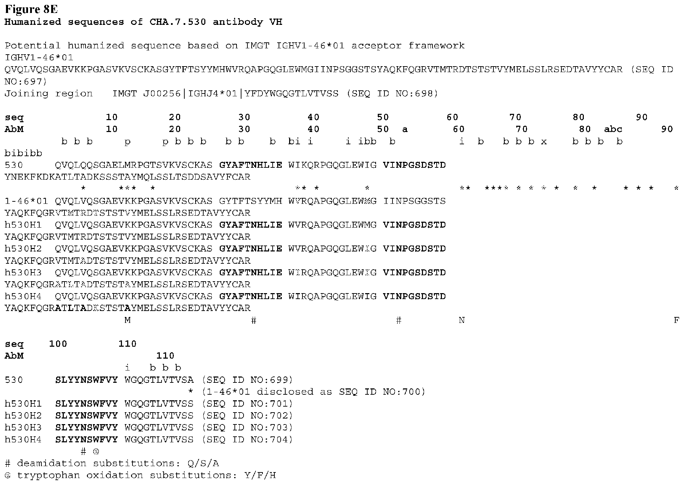

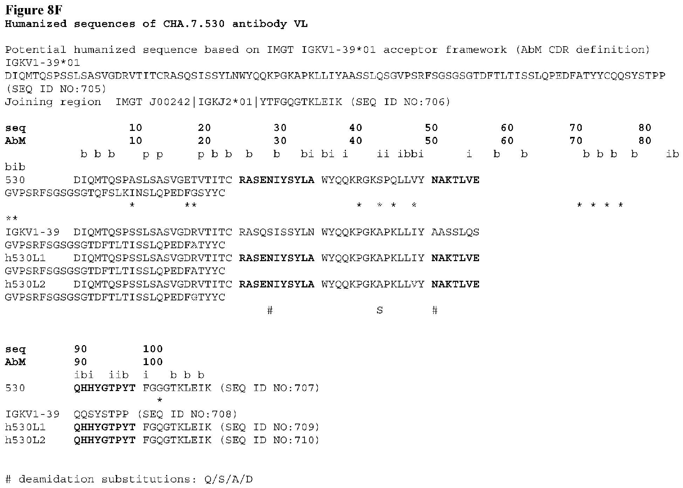

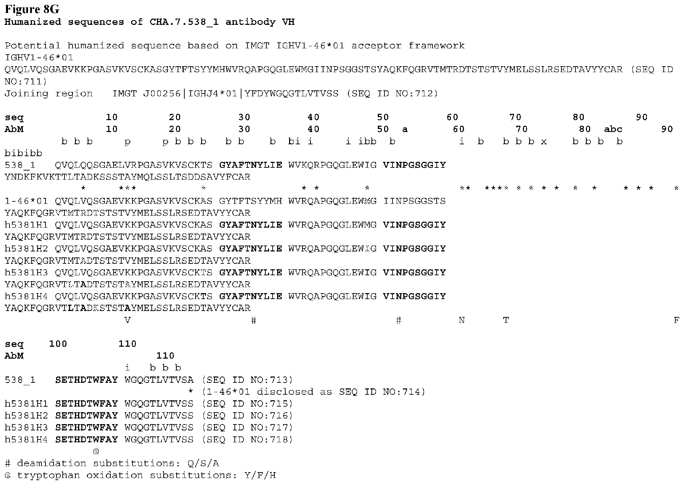

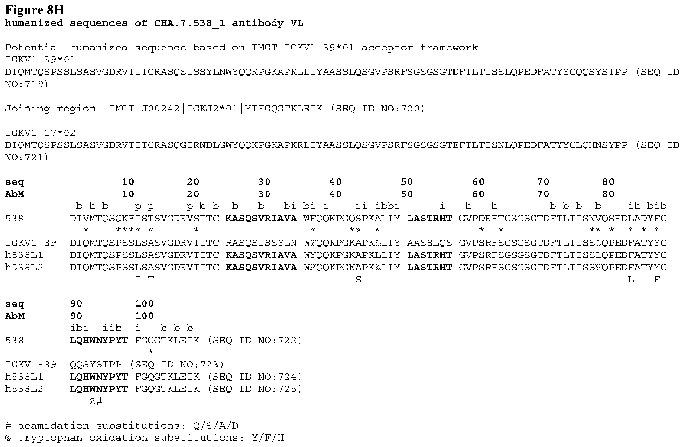

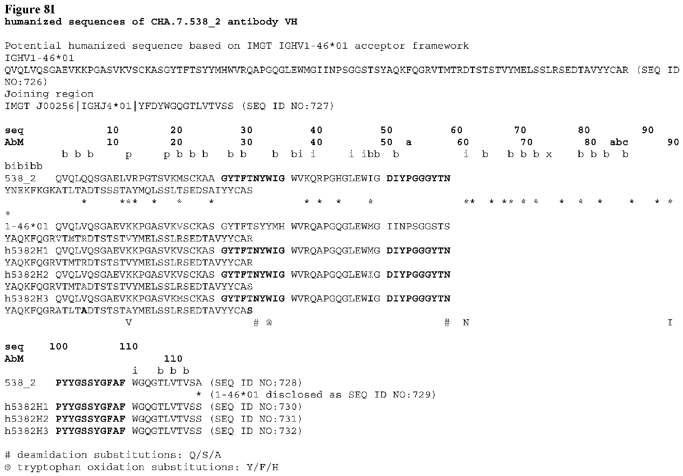

Figures 8A-8I depict a collation of the humanized sequences of five CHA antibodies.

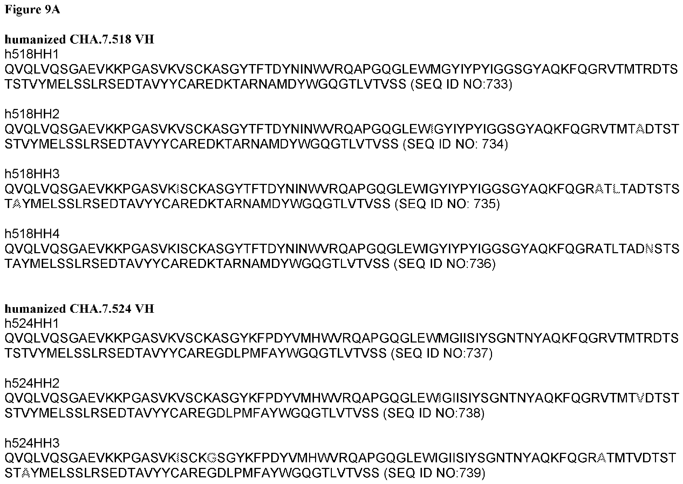

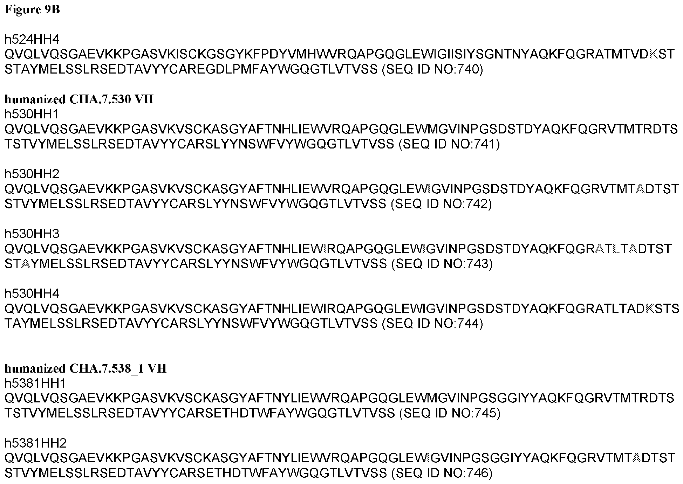

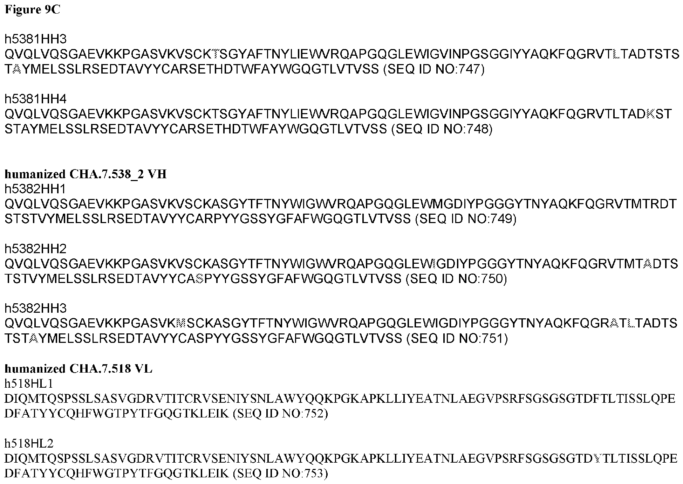

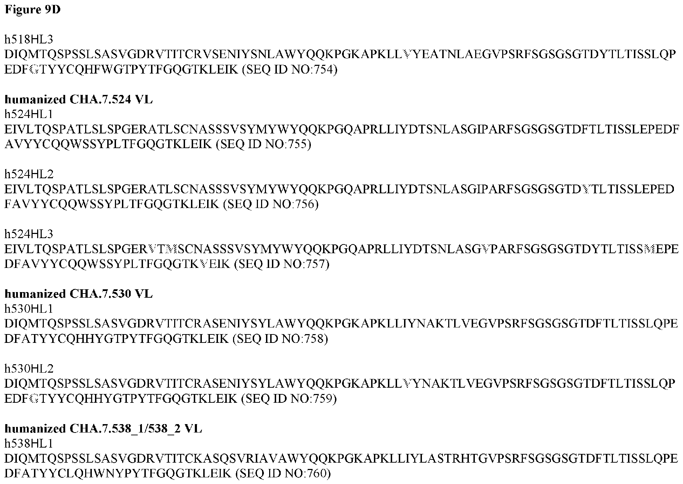

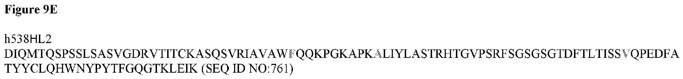

Figures 9A-9E depict a collation of the humanized sequences of five CHA antibodies.

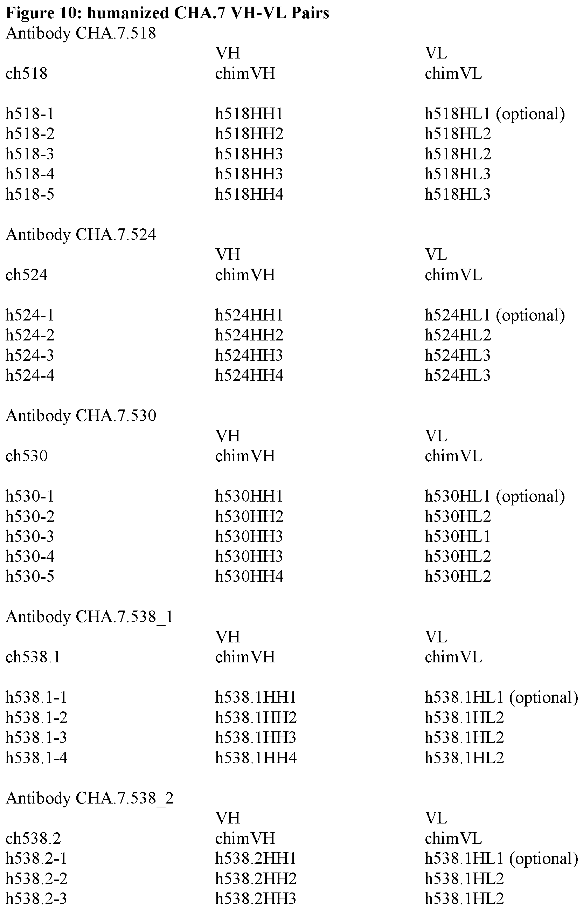

Figure 10 depicts schemes for combining the humanized VH and VL CHA antibodies of Figures 8A-8I and Figures 9A-9E. The "chimVH" and "chimVL" are the mouse variable heavy and light sequences attached to a human IgG constant domain.

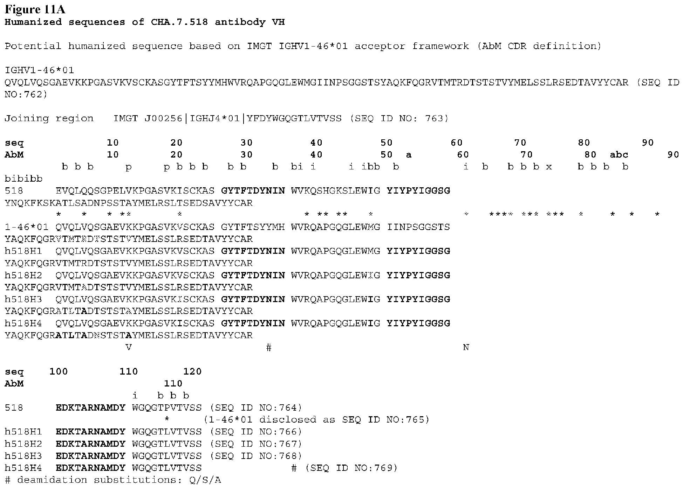

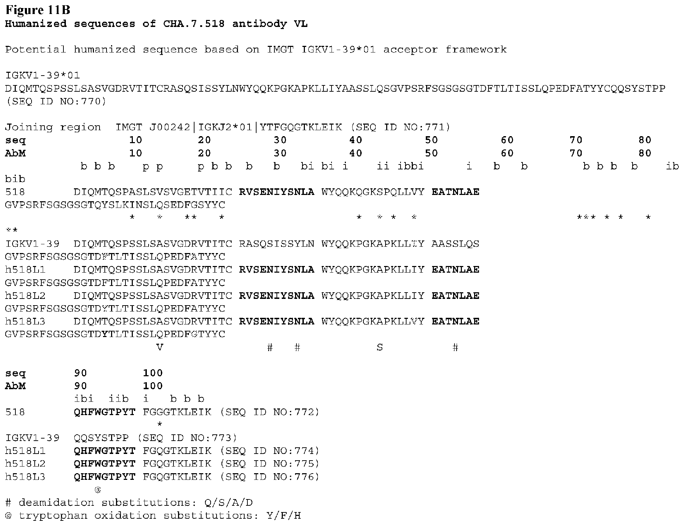

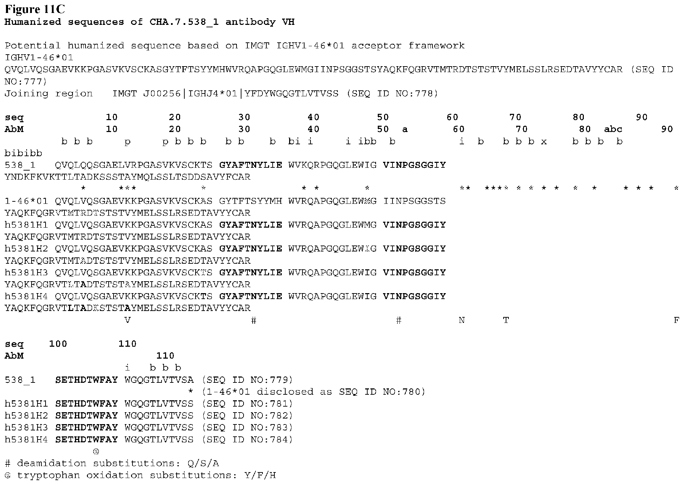

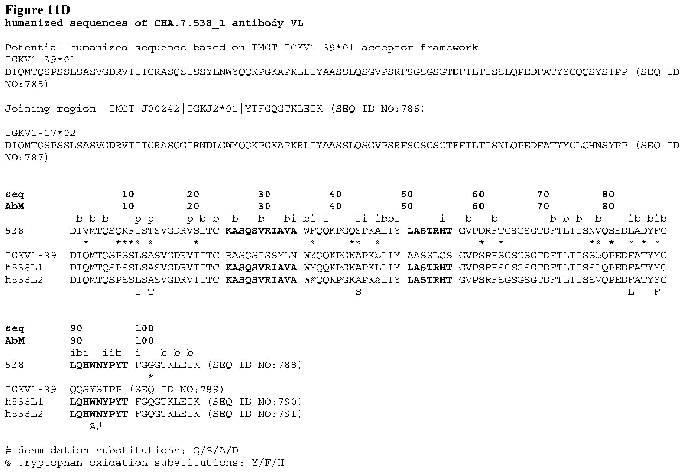

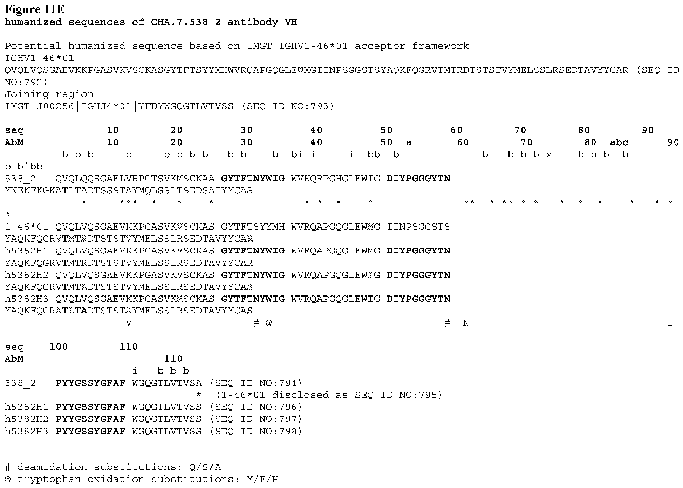

Figures 11A-11E depict four humanized sequences for each of CHA.7.518, CHA.7.524, CHA.7.530, CHA.7.538_1 and CHA.7.538_2. All humanized antibodies comprise the H4(S241P) substitution. Note that the light chain for CHA.7.538_2 is the same as for CHA.7.538_1. The "H1" of each is a "CDR swap" with no changes to the human framework. Subsequent sequences alter framework changes shown in larger bold font. CDR sequences are noted in bold. CDR definitions are AbM from website www.bioinf.org.uk/abs/. Human germline and joining sequences from IMGT^{®} the international ImMunoGeneTics^{®} information system www.imgt.org (founder and director: Marie-Paule Lefranc, Montpellier, France). Residue numbering shown as sequential (seq) or according to Chothia from website www.bioinf.org.uk/abs/ (AbM). "b" notes buried sidechain; "p" notes partially buried; "i" notes sidechain at interface between VH and VL domains. Sequence differences between human and murine germlines noted by asterisk (*). Potential additional mutations in frameworks are noted below sequence. Potential changes in CDR sequences noted below each CDR sequence as noted on the figure (# deamidation substitutions: Q/S/A; these may prevent asparagine (N) deamidation. @ tryptophan oxidation substitutions: Y/F/H; these may prevent tryptophan oxidation; @ methionine oxidation substitutions: L/F/A).

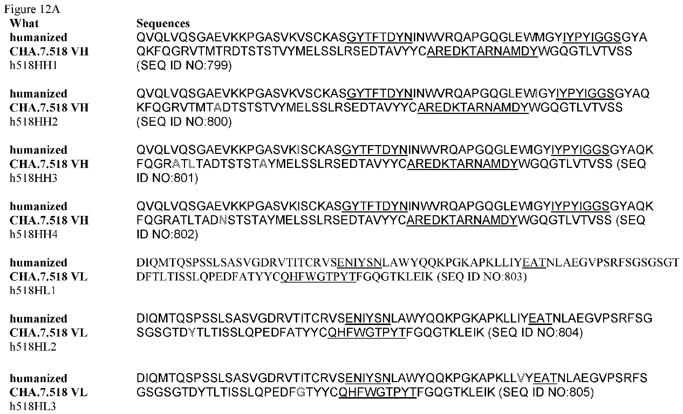

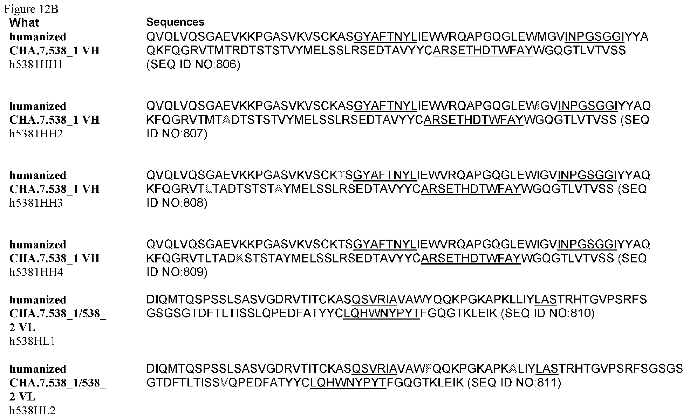

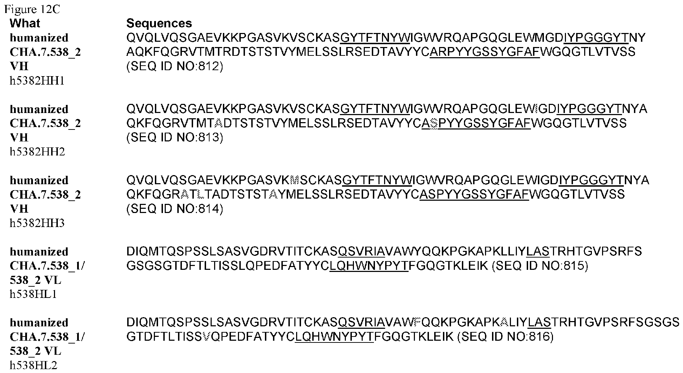

Figure 12A to 12C depicts a collation of the humanized sequences of three CHA antibodies: CHA.7.518, CHA.7.538.1, and CHA.7.538.2.
Figures 13A-13AX depict the sequences of anti-TIGIT antibodies. Unless otherwise noted, the CDRs utilize the IMGT numbering (including the antibodies of the sequence listing.
Figures 14-14B: Dominant DNAM-1 axis receptor expression in MM BM aspirates.
Figures 15A-15B: PVRL2 is expressed on BM PC cells in MM patients.
Figures 16A-16D: DNAM-1 axis receptor co-expression on CD8+ T cells.
Figure 17: The expression of PVRIG, TIGIT, PD-1 and DNAM-1 on CD4+, CD8+ T cells and NK, NKT cells. The expression of the immune check points was analyzed on BM CD3+CD8+, CD3+CD4+ T cells, NK and NKT cells from MM patients by flow cytometry. **A.** The expression is presented as Log10 of the fold of stain mean florescence intensity (MFI) over isotype control MFI. **B.** The expression is presented as percentages of the population. **C.** Representative histogram showing the expression of PVRIG on MM patient BM indicated lymphocyte subsets.
Figure 18: PVRIG and TIGIT are co-expressed on CD4+, CD8+ T cells and NK, NKT cells. The percent of PVRIG/TIGIT double positive cells is shown for each of the populations- CD3+CD8+, CD3+CD4+ T cells, NK and NKT cells from MM patients, as analyzed by flow cytometry.
Figure 19: PVRIG and DNAM-1 are co-expressed on CD4+, CD8+ T cells and NK, NKT cells. The percent of PVRIG/DNAM-1 double positive cells is shown for each of the populations- CD3+CD8+, CD3+CD4+ T cells, NK and NKT cells from MM patients, as analyzed by flow cytometry.
Figure 20: TIGIT and DNAM-1 are co-expressed on CD4+, CD8+ T cells and NK, NKT cells. The percent of TIGIT/DNAM-1 double positive cells is shown for each of the populations- CD3+CD8+, CD3+CD4+ T cells, NK and NKT cells from MM patients, as analyzed by flow cytometry.
Figure 21: DNAM-1 expression levels on CD8+ T cells positively correlate with complete response in MM patients. The expression of DNAM-1 was analyzed on BM CD3+CD8+ T cells from CR and PR MM patients, by flow cytometry. Box plot (bold line represents median value, rectangle 25 and 75 percentile, and whiskers max and min value) comparing the precent of DNAM positive CD8⁺ T cells between complete responders to progressing multiple myeloma in our dataset.
Figure 22: PVRIG and PVRL2 expression by IHC in BM biopsy of MM patient. PVRL2 stained endothelial cells (blue arrows) and some malignant plasma cells (orange arrows), in malignant plasma cells PVRL2 could be detected on membrane and/or cytoplasmatic speckles. PVRIG stains infiltrating lymphocytes (red arrows). Out of the 4 biopsies on the TMA, two had lymphocytic infiltration and two were absent of infiltration, hance no PVRIG could be evaluated. The two positive samples are presented.
Figure 23: Clinical data and treatment history of patients participating in the study.

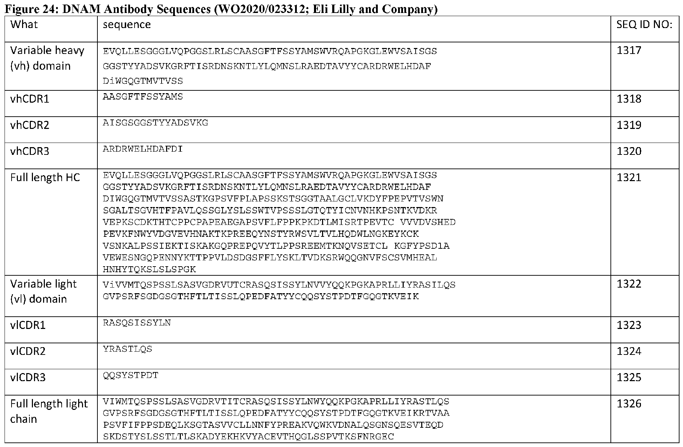

Figures 24: Depicts the sequences of anti-DNAM (*i.e.,* anti-CD226) antibodies. Unless otherwise noted, the CDRs utilize the IMGT numbering (including the antibodies of the sequence listing.
Figure 25 depicts a study design schematic.
Figure 26 depicts the demographics of the study.
Figure 27 depicts the patient disposition summary.
Figure 28 depicts the summary of adverse events.
Figure 29 depicts the incidence of treatment emergent adverse events (TEAEs) in ≥2 patients.

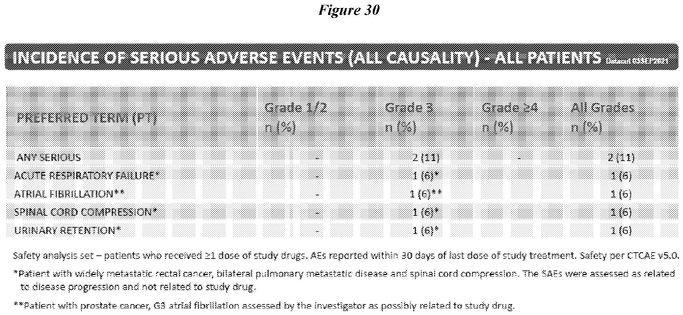

Figure 30 depicts the incidence of serious adverse events (all causality) - all patients.

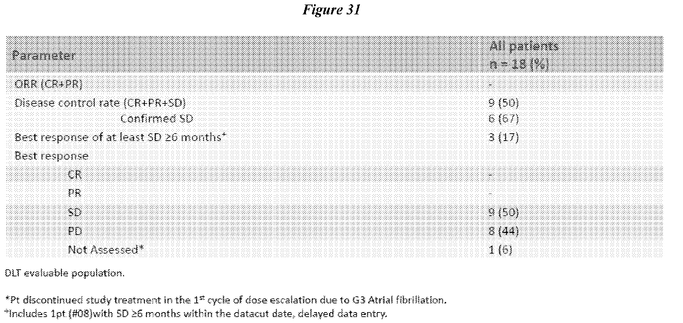

Figure 31 depicts the summary of investigator assessed response (RECIST v1.1) assessment.

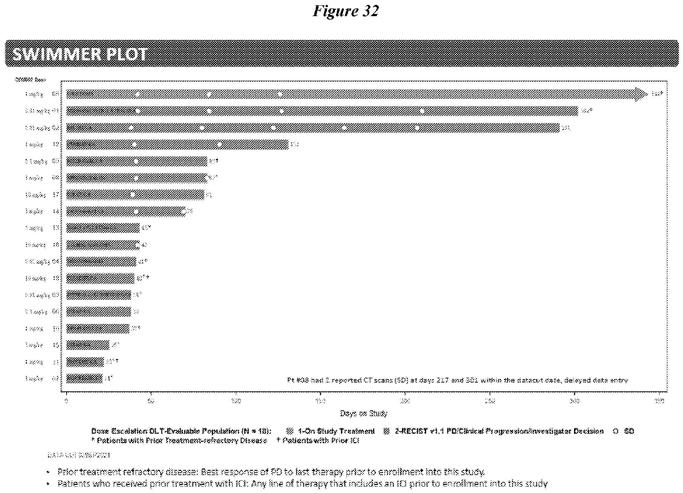

Figure 32 depicts a swimmer plot with the data.

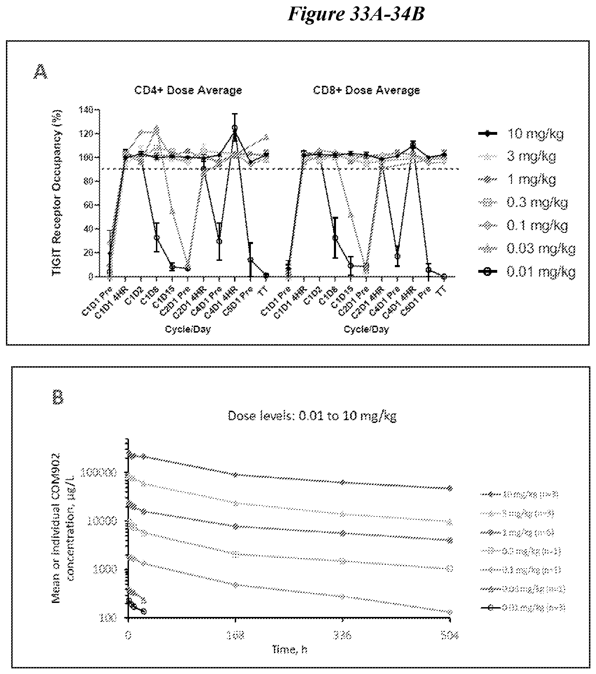

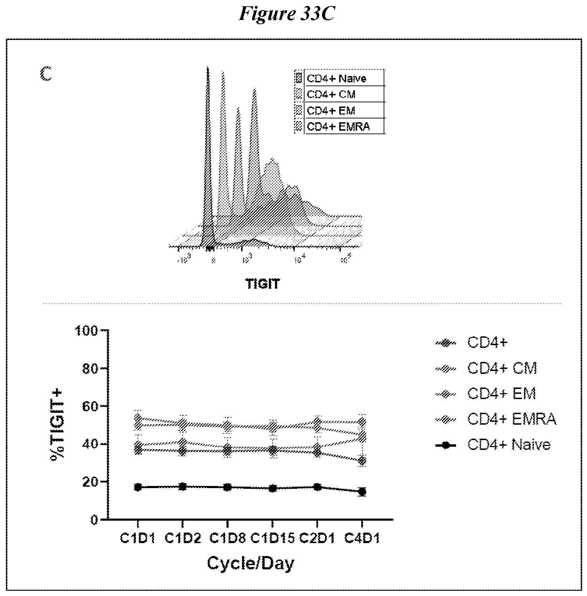

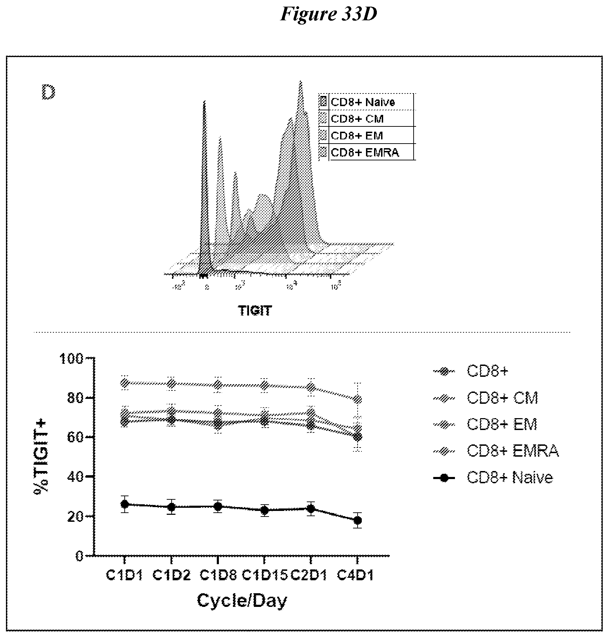

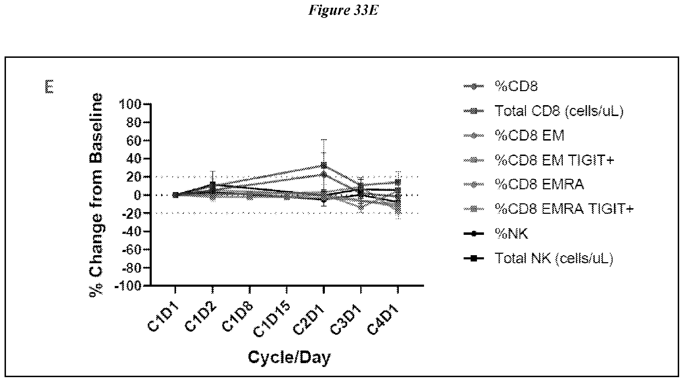

Figure 33A-33E depicts the CPA.9.086.H4(S241P) Peripheral Receptor Occupancy and CPA.9.086.H4(S241P) PK Profile. No significant changes in TIGIT+ CD4 and TIGIT+ CD8 T cell subsets were observed. No significant changes in CD8 T cell subsets and NK cells were observed. A) CPA.9.086.H4(S241P) peripheral RO of TIGIT >90% from 0.1 mg/kg dose level at trough. B) Preliminary CPA.9.086.H4(S241P) PK profiles with a dose-proportional increase in circulating concentrations at doses > 0.1 mg/kg. Flow cytometry analysis of %TIGIT positive cells out of CD4 (C) and CD8 (D) T cell subsets. Shown in each panel are representative histograms of TIGIT+ T cell subsets and the average of all patients for each subset. TIGIT detection was carried out by labeled secondary antibody (anti-hIgG4) following blood samples saturation by CPA.9.086.H4(S241P), T cell subsets were defined by CD45RA and CCR7 staining (CM: CD45RA-CCR7+ ; EM: CD45RA-CCR7- ; EMRA: CD45RA+ CCR7- ; Naïve: CD45RA+CCR7+). Flow cytometry analysis of percent of change compared to baseline for CD8, CD8 EM, CD8 EMRA T cells and NK cells and of TIGIT+ CD8 EM and CD8 EMRA T cells (the most highly TIGIT expressor subsets) (E). Average of all patients for each subset is shown. Parental CD8 and NK (CD3-CD56+/CD16+) cells were gated out of lymphocytes, TIGIT detection was carried out by labeled secondary antibody (anti-hIgG4) following blood samples saturation by CPA.9.086.H4(S241P), T cell subsets were defined by CD45RA and CCR7 staining (EM: CD45RA-CCR7- ; EMRA: CD45RA+ CCR7-).
Figure 34A-34B: Combination of T cell engager (*i.e.,* anti-BCMA/CD3 bispecific antibody) with anti-PVRIG antibody and/or anti-TIGIT antibody induce immune activation in MM patients.
Figure 35: Violi: plots of selected set of immune checkpoints in CD8 in synovial sarcoma scRNAseq. Boxplot: box represents the IQR (interquartile range), whiskers represent maximal expression based on Q3 + 1.5*IQR, bold dots represents outlier values.
Figure 36: Boxplot showing expression of PVRIG divided by CD8 across TCGA, demonstrating the highest expression of PVRIG in synovial sarcoma.

### DETAILED DESCRIPTION OF THE INVENTION

### I. INTRODUCTION

Cancer can be considered as an inability of the patient to recognize and eliminate cancerous cells. In many instances, these transformed (*e*.*g*., cancerous) cells counteract immune surveillance. There are natural control mechanisms that limit T-cell activation in the body to prevent unrestrained T-cell activity, which can be exploited by cancerous cells to evade or suppress the immune response. Restoring the capacity of immune effector cells-especially T cells-to recognize and eliminate cancer is the goal of immunotherapy. The field of immuno-oncology, sometimes referred to as "immunotherapy" is rapidly evolving, with several recent approvals of T cell checkpoint inhibitory antibodies such as Yervoy, Keytruda and Opdivo. These antibodies are generally referred to as "checkpoint inhibitors" because they block normally negative regulators of T cell immunity. It is generally understood that a variety of immunomodulatory signals, both costimulatory and coinhibitory, can be used to orchestrate an optimal antigen-specific immune response. Generally, these antibodies bind to checkpoint inhibitor proteins such as CTLA-4 and PD-1, which under normal circumstances prevent or suppress activation of cytotoxic T cells (CTLs). By inhibiting the checkpoint protein, for example through the use of antibodies that bind these proteins, an increased T cell response against tumors can be achieved. That is, these cancer checkpoint proteins suppress the immune response; when the proteins are blocked, for example using antibodies to the checkpoint protein, the immune system is activated, leading to immune stimulation, resulting in treatment of conditions such as cancer and infectious disease. PVRIG is expressed on the cell surface of NK and T-cells and shares several similarities to other known immune checkpoints. As indicated above, PVRL2 has been identified as the ligand for PVRIG.

The present invention is directed to biomarkers for use in determining populations for treatment with antibodies to human Poliovirus Receptor Related Immunoglobulin Domain Containing Protein, or "PVRIG", sometimes also referred to herein as "PV protein" (e.g., anti-PVRIG antibodies including those with CDRs identical to those shown in Figure 3). Such biomarkers include, for example PVRIG and/or PVRL2 expression, as described herein, as well as DNAM expression. In some embodiments, the biomarker is PVRIG expression. In some embodiments, the biomarker is PVRL2 expression. In some embodiments, the biomarker is DNAM expression.

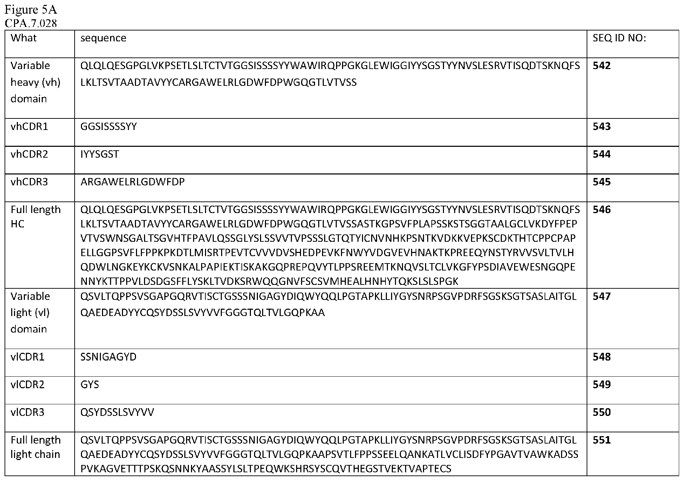

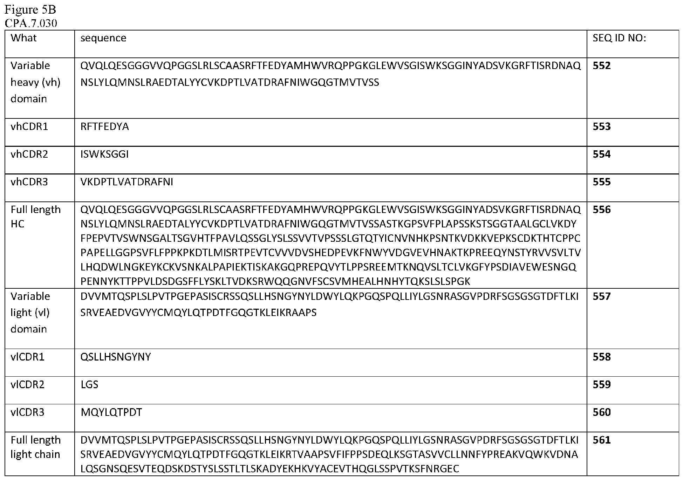

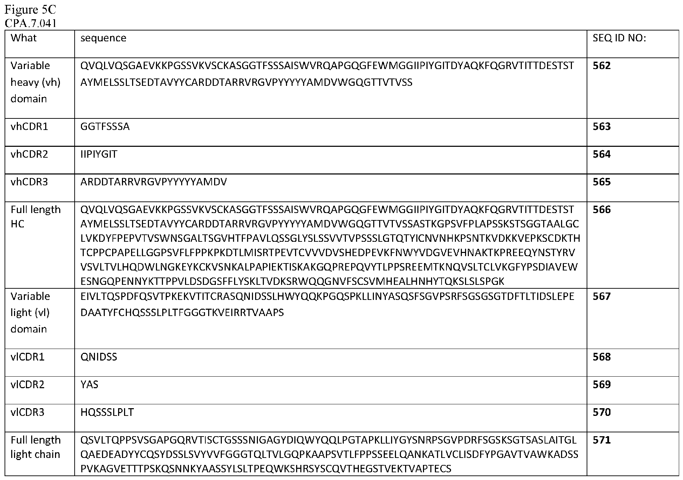

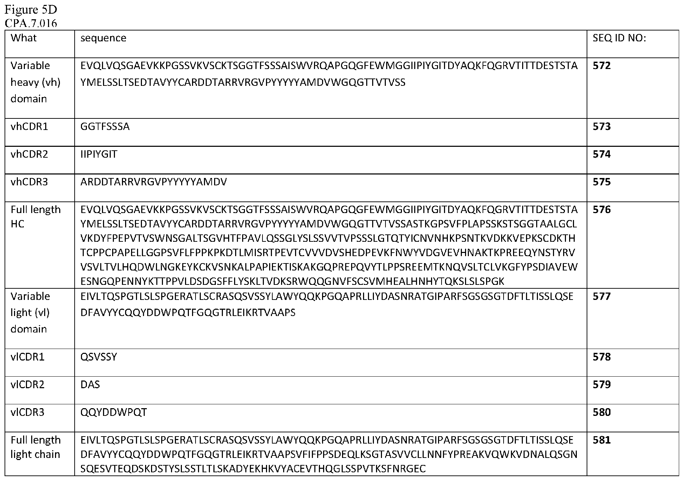

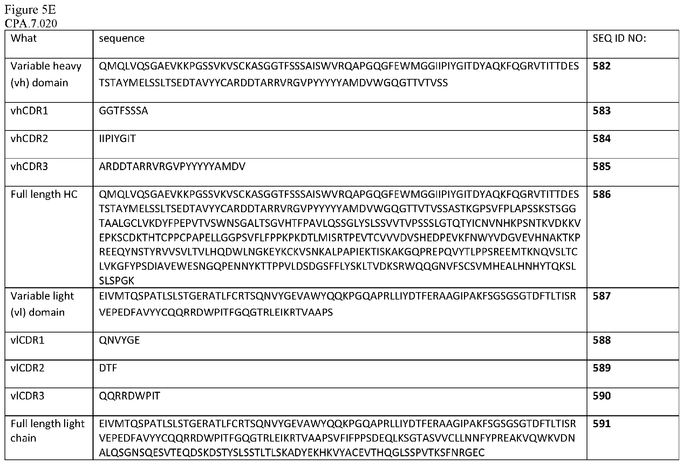

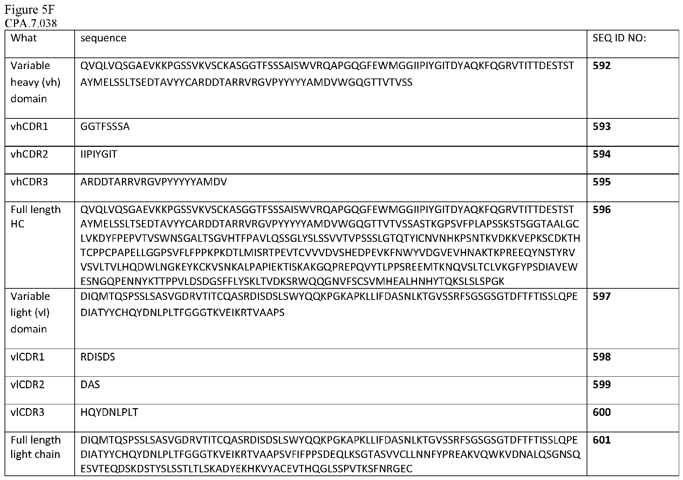

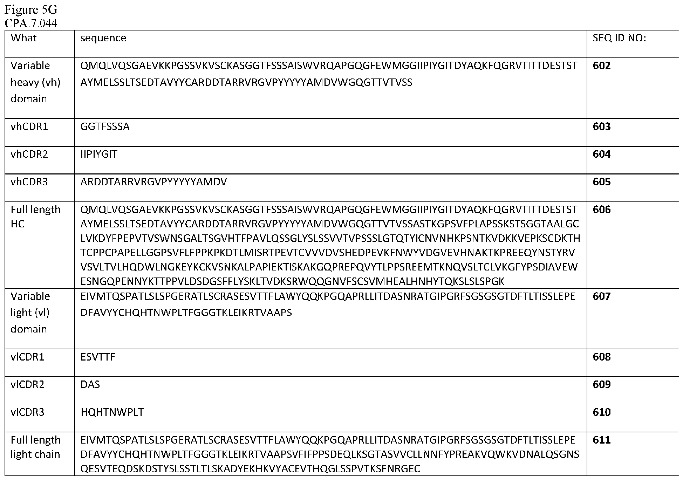

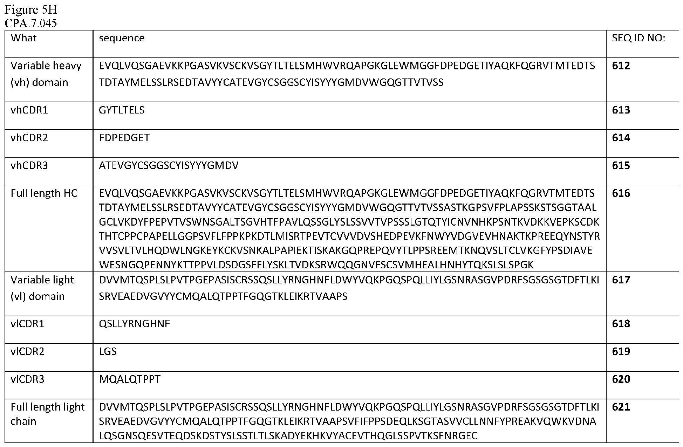

The present invention also provides biomarkers for use with formulations comprising anti-PVRIG antibodies, including antigen binding domains, that bind to the human PVRIG and peptides thereof and methods of activating T cells and/or NK cells to treat diseases such as cancer and infectious diseases, and other conditions where increased immune activity results in treatment. In particular, these formulations comprise antibodies comprising heavy and light chains as well as the vhCDR1, vhCDR2, vhCDR3, vlCDR1, vlCDR2 and vlCDR3 sequences from CHA.7.518.1.H4(S241P). In some embodiments, anti-PVRIG antibodies include those with CDRs identical to those shown in Figure 3. In some embodiments, anti-PVRIG antibodies include those with CDRs identical to those shown in Figures 5A-5D, as well as anti-PVRIG antibodies comprising the heavy and light chains as provided in Figures 5A-5D.

### II. PVRIG PROTEINS

The present invention provides formulations comprising antibodies that specifically bind to PVRIG proteins. "Protein" in this context is used interchangeably with "polypeptide", and includes peptides as well. The present invention provides antibodies that specifically bind to PVRIG proteins. PVRIG is a transmembrane domain protein of 326 amino acids in length, with a signal peptide (spanning from amino acid 1 to 40), an extracellular domain (spanning from amino acid 41 to 171), a transmembrane domain (spanning from amino acid 172 to 190) and a cytoplasmic domain (spanning from amino acid 191 to 326). The full length human PVRIG protein is shown in Figure 1. There are two methionines that can be start codons, but the mature proteins are identical.

Accordingly, as used herein, the term "PVRIG" or "PVRIG protein" or "PVRIG polypeptide" may optionally include any such protein, or variants, conjugates, or fragments thereof, including but not limited to known or wild type PVRIG, as described herein, as well as any naturally occurring splice variants, amino acid variants or isoforms, and in particular the ECD fragment of PVRIG. The term "soluble" form of PVRIG is also used interchangeably with the terms "soluble ectodomain (ECD)" or "ectodomain" or "extracellular domain (ECD) as well as "fragments of PVRIG polypeptides", which may refer broadly to one or more of the following optional polypeptides:

The PVRIG proteins contain an immunoglobulin (Ig) domain within the extracellular domain, which is a PVR-like Ig fold domain. The PVR-like Ig fold domain may be responsible for functional counterpart binding, by analogy to the other B7 family members. The PVR-like Ig fold domain of the extracellular domain includes one disulfide bond formed between intra domain cysteine residues, as is typical for this fold and may be important for structure-function. These cysteines are located at residues 22 and 93 (or 94). In one embodiment, there is provided a soluble fragment of PVRIG that can be used in testing of PVRIG antibodies. Included within the definition of PVRIG proteins are PVRIG ECD fragments, including know ECD fragments such as those described in U.S. Patent No. 9,714, 289, incorporate by reference herein in its entirety for all purposes.

As noted herein and more fully described below, the anti-PVRIG antibodies (including antigen-binding fragments) that both bind to PVRIG and prevent activation by PVRL2 (*e*.*g*., most commonly by blocking the interaction of PVRIG and PVLR2), are used to enhance T cell and/or NK cell activation and be used in treating diseases such as cancer and pathogen infection.

### III. TIGIT PROTEINS

The present invention provides antibodies that specifically bind to TIGIT proteins and prevent activation by its ligand protein, PVR, poliovirus receptor (*aka,* CD155) a human plasma membrane glycoprotein. TIGIT, or T cell immunoreceptor with Ig and ITIM domains, is a coinhibitory receptor protein also known as WUCAM, Vstm3 or Vsig9. TIGIT has an immunoglobulin variable domain, a transmembrane domain, and an immunoreceptor tyrosine-based inhibitory motif (ITIM) and contains signature sequence elements of the PVR protein family. The extracellular domain (ECD) sequences of TIGIT and of PVR are shown in Figure 1B. The antibodies of the invention are specific for the TIGIT ECD such that the binding of TIGIT and PVR is blocked

Accordingly, as used herein, the term "TIGIT" or "TIGIT protein" or "TIGIT polypeptide" may optionally include any such protein, or variants, conjugates, or fragments thereof, including but not limited to known or wild type TIGIT, as described herein, as well as any naturally occurring splice variants, amino acid variants or isoforms, and in particular the ECD fragment of TIGIT.

As noted herein and more fully described below, anti-TIGIT antibodies (including antigen-binding fragments) that both bind to TIGIT and prevent activation by PVR (e.g., most commonly by blocking the interaction of TIGIT and PVR), are used to enhance T cell and/or NK cell activation and be used in treating diseases such as cancer and pathogen infection.

### IV. ANTIBODIES

Accordingly, the invention provides anti-PVRIG antibodies that can be formulated according to the formulations and invention described herein and which are provided in Figure 3 (*e*.*g*., including anti-PVRIG antibodies including those with CDRs identical to those shown in Figure 3). PVRIG, also called Poliovirus Receptor Related Immunoglobulin Domain Containing Protein, Q6DKI7 or C7orf15, relates to amino acid and nucleic acid sequences shown in RefSeq accession identifier NP_076975, shown in Figure 1. The antibodies of the invention are specific for the PVRIG extracellular domain.

As is discussed below, the term "antibody" is used generally. Antibodies that find use in the present invention can take on a number of formats as described herein, including traditional antibodies as well as antibody derivatives, fragments and mimetics, described below. In general, the term "antibody" includes any polypeptide that includes at least one antigen binding domain, as more fully described below. Antibodies may be polyclonal, monoclonal, xenogeneic, allogeneic, syngeneic, or modified forms thereof, as described herein, with monoclonal antibodies finding particular use in many embodiments. In some embodiments, antibodies of the invention bind specifically or substantially specifically to PVRIG molecules. The terms "monoclonal antibodies" and "monoclonal antibody composition", as used herein, refer to a population of antibody molecules that contain only one species of an antigen-binding site capable of immunoreacting with a particular epitope of an antigen, whereas the term "polyclonal antibodies" and "polyclonal antibody composition" refer to a population of antibody molecules that contain multiple species of antigen-binding sites capable of interacting with a particular antigen. A monoclonal antibody composition, typically displays a single binding affinity for a particular antigen with which it immunoreacts.

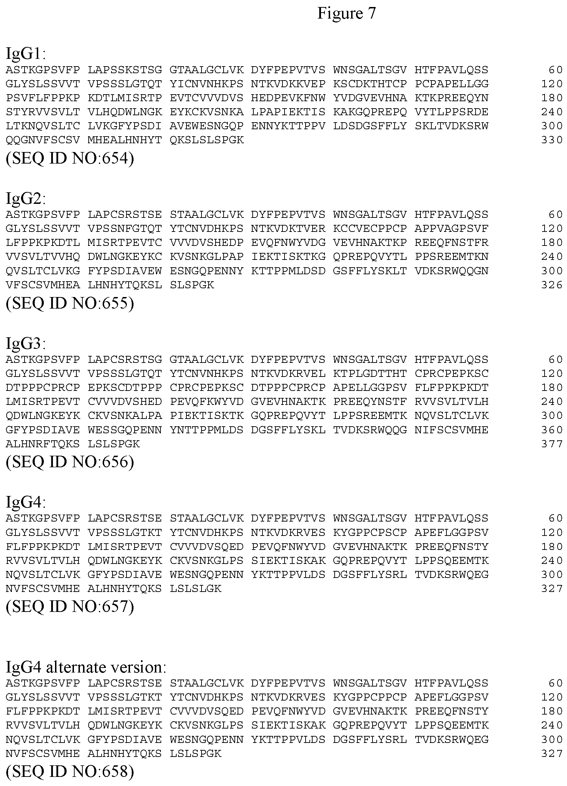

Traditional full length antibody structural units typically comprise a tetramer. Each tetramer is typically composed of two identical pairs of polypeptide chains, each pair having one "light" (typically having a molecular weight of about 25 kDa) and one "heavy" chain (typically having a molecular weight of about 50-70 kDa). Human light chains are classified as kappa and lambda light chains. The present invention is directed to the IgG class, which has several subclasses, including, but not limited to IgG1, IgG2, IgG3, and IgG4. Thus, "isotype" as used herein is meant any of the subclasses of immunoglobulins defined by the chemical and antigenic characteristics of their constant regions. While the exemplary antibodies herein designated "CPA" are based on IgG1 heavy constant regions, as shown in Figure 7, the anti-PVRIG antibodies of the invention include those using IgG2, IgG3 and IgG4 sequences, or combinations thereof. For example, as is known in the art, different IgG isotypes have different effector functions which may or may not be desirable. Accordingly, the CPA antibodies of the invention can also swap out the IgG1 constant domains for IgG2, IgG3 or IgG4 constant domains (depicted in Figure 7), with IgG2 and IgG4 finding particular use in a number of situations, for example for ease of manufacture or when reduced effector function is desired, the latter being desired in some situations.

The amino-terminal portion of each chain includes a variable region of about 100 to 110 or more amino acids primarily responsible for antigen recognition, generally referred to in the art and herein as the "Fv domain" or "Fv region". In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions".

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region, although sometimes the numbering is shifted slightly as will be appreciated by those in the art; Kabat et al., SEQUENCES OF PROTEINS OF IMMUNOLOGICAL INTEREST, 5 th Ed. Public Health Service, National Institutes of Health, Bethesda, Md. (1991) and/or those residues forming a hypervariable loop (*e*.*g*., residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the invention are described below and shown in Figure 3.

The carboxy-terminal portion of each chain defines a constant region primarily responsible for effector function. Kabat et al. collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5th edition, NIH publication, No. 91-3242, E. A. Kabat et al., entirely incorporated by reference).

In the IgG subclass of immunoglobulins, there are several immunoglobulin domains in the heavy chain. By "immunoglobulin (Ig) domain" herein is meant a region of an immunoglobulin having a distinct tertiary structure. Of interest in the present invention are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat.

Accordingly, the invention provides variable heavy domains, variable light domains, heavy constant domains, light constant domains and Fc domains to be used as outlined herein. By "variable region" as used herein is meant the region of an immunoglobulin that comprises one or more Ig domains substantially encoded by any of the Vκ or Vλ, and/or VH genes that make up the kappa, lambda, and heavy chain immunoglobulin genetic loci respectively. Accordingly, the variable heavy domain comprises vhFR1-vhCDR1-vhFR2-vhCDR2-vhFR3-vhCDR3-vhFR4, and the variable light domain comprises vlFR1-vlCDR1-vlFR2-vlCDR2-vlFR3-vlCDR3-vlFR4. By "heavy constant region" herein is meant the CH1-hinge-CH2-CH3 portion of an antibody. By "Fc" or "Fc region" or "Fc domain" as used herein is meant the polypeptide comprising the constant region of an antibody excluding the first constant region immunoglobulin domain and in some cases, part of the hinge. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, the Fc domain comprises immunoglobulin domains Cγ2 and Cγ3 (Cγ2 and Cγ3) and the lower hinge region between Cγ1 (Cγ1) and Cγ2 (Cγ2). Although the boundaries of the Fc region may vary, the human IgG heavy chain Fc region is usually defined to include residues C226 or P230 to its carboxyl-terminus, wherein the numbering is according to the EU index as in Kabat. In some embodiments, as is more fully described below, amino acid modifications are made to the Fc region, for example to alter binding to one or more FcγR receptors or to the FcRn receptor.

Thus, "Fc variant" or "variant Fc" as used herein is meant a protein comprising an amino acid modification in an Fc domain. The Fc variants of the present invention are defined according to the amino acid modifications that compose them. Thus, for example, N434S or 434S is an Fc variant with the substitution serine at position 434 relative to the parent Fc polypeptide, wherein the numbering is according to the EU index. Likewise, M428L/N434S defines an Fc variant with the substitutions M428L and N434S relative to the parent Fc polypeptide. The identity of the WT amino acid may be unspecified, in which case the aforementioned variant is referred to as 428L/434S. It is noted that the order in which substitutions are provided is arbitrary, that is to say that, for example, 428L/434S is the same Fc variant as M428L/N434S, and so on. For all positions discussed in the present invention that relate to antibodies, unless otherwise noted, amino acid position numbering is according to the EU index.

By "Fab" or "Fab region" as used herein is meant the polypeptide that comprises the VH, CH1, VL, and CL immunoglobulin domains. Fab may refer to this region in isolation, or this region in the context of a full length antibody, antibody fragment or Fab fusion protein. By "Fv" or "Fv fragment" or "Fv region" as used herein is meant a polypeptide that comprises the VL and VH domains of a single antibody. As will be appreciated by those in the art, these generally are made up of two chains.

Throughout the present specification, either the IMTG numbering system or the Kabat numbering system is generally used when referring to a residue in the variable domain (approximately, residues 1-107 of the light chain variable region and residues 1-113 of the heavy chain variable region) (*e*.*g*., Kabat et al., supra (1991)). EU numbering as in Kabat is generally used for constant domains and/or the Fc domains.

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and nonconformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning". Specific bins are described below.

Included within the definition of "antibody" is an "antigen-binding portion" of an antibody (also used interchangeably with "antigen-binding fragment", "antibody fragment" and "antibody derivative"). That is, for the purposes of the invention, an antibody of the invention has a minimum functional requirement that it bind to a PVRIG antigen. As will be appreciated by those in the art, there are a large number of antigen fragments and derivatives that retain the ability to bind an antigen and yet have alternative structures, including, but not limited to, (i) the Fab fragment consisting of VL, VH, CL and CH1 domains, (ii) the Fd fragment consisting of the VH and CH1 domains, (iii) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., 1988, Science 242:423-426, Huston et al., 1988, Proc. Natl. Acad. Sci. U.S.A. 85:5879-5883, entirely incorporated by reference), (iv) "diabodies" or "triabodies", multivalent or multispecific fragments constructed by gene fusion (Tomlinson et. al., 2000, Methods Enzymol. 326:461-479; WO94/13804; Holliger et al., 1993, Proc. Natl. Acad. Sci. U.S.A. 90:6444-6448, all entirely incorporated by reference), (v) "domain antibodies" or "dAb" (sometimes referred to as an "immunoglobulin single variable domain", including single antibody variable domains from other species such as rodent (for example, as disclosed in WO 00/29004), nurse shark and Camelid V-HH dAbs, (vi) SMIPs (small molecule immunopharmaceuticals), camelbodies, nanobodies and IgNAR. The present invention is directed to monoclonal antibodies that generally are based on the IgG class, which has several subclasses, including, but not limited to IgG1, IgG2, IgG3, and IgG4. In general, IgG1, IgG2 and IgG4 are used more frequently than IgG3. It should be noted that IgG1 has different allotypes with polymorphisms at 356 (D or E) and 358 (L or M). The sequences depicted herein use the 356D/358M allotype, however the other allotype is included herein. That is, any sequence inclusive of an IgG1 Fc domain included herein can have 356E/358L replacing the 356D/358M allotype.

The amino-terminal portion of each chain includes a variable region of about 100 to 110 or more amino acids primarily responsible for antigen recognition, generally referred to in the art and herein as the "Fv domain" or "Fv region". In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions" that are each 9-15 amino acids long or longer.

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region; Kabat et al., SEQUENCES OF PROTEINS OF IMMUNOLOGICAL INTEREST, 5th Ed. Public Health Service, National Institutes of Health, Bethesda, Md. (1991) and/or those residues forming a hypervariable loop (e.g. residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the invention are described below.

As will be appreciated by those in the art, the exact numbering and placement of the CDRs can be different among different numbering systems. However, it should be understood that the disclosure of a variable heavy and/or variable light sequence includes the disclosure of the associated (inherent) CDRs. Accordingly, the disclosure of each variable heavy region is a disclosure of the vhCDRs (e.g. vhCDR1, vhCDR2 and vhCDR3) and the disclosure of each variable light region is a disclosure of the vlCDRs (e.g. vlCDR1, vlCDR2 and vlCDR3). A useful comparison of CDR numbering is as below, see Lafranc et al., Dev. Comp. Immunol. 27(1):55-77 (2003):

| | Kabat+Clothia | IMGT | Kabat | AbM | Chothia | Contact |
|---|---|---|---|---|---|---|
| vhCDR1 | 26-35 | 27-38 | 31-35 | 26-35 | 26-32 | 30-35 |
| vhCDR2 | 50-65 | 56-65 | 50-65 | 50-58 | 53-55 | 47-58 |
| vhCDR3 | 95-102 | 105-117 | 95-102 | 95-102 | 96-101 | 93-101 |
| vlCDR1 | 24-34 | 27-38 | 24-34 | 24-34 | 26-32 | 30-36 |
| vlCDR2 | 50-56 | 56-65 | 50-56 | 50-56 | 50-52 | 46-55 |
| vlCDR3 | 89-97 | 105-117 | 89-97 | 89-97 | 91-96 | 89-96 |

Throughout the present specification, the Kabat numbering system is generally used when referring to a residue in the variable domain (approximately, residues 1-107 of the light chain variable region and residues 1-113 of the heavy chain variable region) and the hinge and the EU numbering system for Fc regions (e.g, Kabat et al., supra (1991)).

The present invention provides a large number of different CDR sets. In this case, a "full CDR set" comprises the three variable light and three variable heavy CDRs, e.g. a vlCDR1, vlCDR2, vlCDR3, vhCDR1, vhCDR2 and vhCDR3. These can be part of a larger variable light or variable heavy domain, respectfully. In addition, as more fully outlined herein, the variable heavy and variable light domains can be on separate polypeptide chains, when a heavy and light chain is used, or on a single polypeptide chain in the case of scFv sequences.

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and non-conformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning." As outlined below, the invention not only includes the enumerated antigen binding domains and antibodies herein, but those that compete for binding with the epitopes bound by the enumerated antigen binding domains.

The carboxy-terminal portion of each chain defines a constant region primarily responsible for effector function. Kabat et al. collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5th edition, NIH publication, No. 91-3242, E.A. Kabat et al., entirely incorporated by reference).

In the IgG subclass of immunoglobulins, there are several immunoglobulin domains in the heavy chain. By "immunoglobulin (Ig) domain" herein is meant a region of an immunoglobulin having a distinct tertiary structure. Of interest in the present invention are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat.

Another type of Ig domain of the heavy chain is the hinge region. By "hinge" or "hinge region" or "antibody hinge region" or "immunoglobulin hinge region" herein is meant the flexible polypeptide comprising the amino acids between the first and second constant domains of an antibody. Structurally, the IgG CH1 domain ends at EU position 220, and the IgG CH2 domain begins at residue EU position 237. Thus for IgG the antibody hinge is herein defined to include positions 221 (D221 in IgG1) to 236 (G236 in IgG1), wherein the numbering is according to the EU index as in Kabat.

The light chain generally comprises two domains, the variable light domain (containing the light chain CDRs and together with the variable heavy domains forming the Fv region), and a constant light chain region (often referred to as CL or Cκ). In general, either the constant lambda or constant kappa domain can be used, with lambda generally finding use in the invention.

Another region of interest for additional substitutions, outlined below, is the Fc region.

Still further, an antibody or antigen-binding portion thereof (antigen-binding fragment, antibody fragment, antibody portion) may be part of a larger immunoadhesion molecules (sometimes also referred to as "fusion proteins"), formed by covalent or noncovalent association of the antibody or antibody portion with one or more other proteins or peptides. Examples of immunoadhesion molecules include use of the streptavidin core region to make a tetrameric scFv molecule and use of a cysteine residue, a marker peptide and a C-terminal polyhistidine tag to make bivalent and biotinylated scFv molecules. Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

In general, the anti-PVRIG antibodies of the invention are recombinant. "Recombinant" as used herein, refers broadly with reference to a product, *e.g.,* to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "recombinant antibody", as used herein, includes all antibodies that are prepared, expressed, created or isolated by recombinant means, such as (a) antibodies isolated from an animal (*e*.*g*., a mouse) that is transgenic or transchromosomal for human immunoglobulin genes or a hybridoma prepared therefrom (described further below), (b) antibodies isolated from a host cell transformed to express the human antibody, *e*.*g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, in vivo somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

### A. Chimeric and Humanized Antibodies

In some embodiments, the anti-PVRIG antibodies and/or anti-TIGIT antibodies herein can be derived from a mixture from different species, e.g. a chimeric antibody and/or a humanized antibody. In general, both "chimeric antibodies" and "humanized antibodies" refer to antibodies that combine regions from more than one species. For example, "chimeric antibodies" traditionally comprise variable region(s) from a mouse (or rat, in some cases) and the constant region(s) from a human. "Humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536, all entirely incorporated by reference. "Backmutation" of selected acceptor framework residues to the corresponding donor residues is often required to regain affinity that is lost in the initial grafted construct (US 5530101; US 5585089; US 5693761; US 5693762; US 6180370; US 5859205; US 5821337; US 6054297; US 6407213, all entirely incorporated by reference). The humanized antibody optimally also will comprise at least a portion, and usually all, of an immunoglobulin constant region, typically that of a human immunoglobulin, and thus will typically comprise a human Fc region. Humanized antibodies can also be generated using mice with a genetically engineered immune system. Roque et al., 2004, Biotechnol. Prog. 20:639-654, entirely incorporated by reference. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein, all entirely incorporated by reference). Humanization methods include but are not limited to methods described in Jones et al., 1986, Nature 321:522-525; Riechmann et al., 1988; Nature 332:323-329; Verhoeyen et al., 1988, Science, 239:1534-1536; Queen et al., 1989, Proc Natl Acad Sci, USA 86:10029-33; He et al., 1998, J. Immunol. 160: 1029-1035; Carter et al., 1992, Proc Natl Acad Sci USA 89:4285-9, Presta et al., 1997, Cancer Res. 57(20):4593-9; Gorman et al., 1991, Proc. Natl. Acad. Sci. USA 88:4181-4185; O'Connor et al., 1998, Protein Eng 11:321-8, all entirely incorporated by reference. Humanization or other methods of reducing the immunogenicity of nonhuman antibody variable regions may include resurfacing methods, as described for example in Roguska et al., 1994, Proc. Natl. Acad. Sci. USA 91:969-973, entirely incorporated by reference.

Thus, the vhCDRs and vlCDRs from any of the enumerated antibodies herein may be humanized (or "rehumanized", for those that were already humanized).

In certain embodiments, the antibodies of the invention comprise a heavy chain variable region from a particular germline heavy chain immunoglobulin gene and/or a light chain variable region from a particular germline light chain immunoglobulin gene. For example, such antibodies may comprise or consist of a human antibody comprising heavy or light chain variable regions that are "the product of" or "derived from" a particular germline sequence. A human antibody that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (i.e., greatest % identity) to the sequence of the human antibody. A human antibody that is "the product of" or "derived from" a particular human germline immunoglobulin sequence may contain amino acid differences as compared to the germline sequence, due to, for example, naturally-occurring somatic mutations or intentional introduction of site-directed mutation. However, a humanized antibody typically is at least 90% identical in amino acids sequence to an amino acid sequence encoded by a human germline immunoglobulin gene and contains amino acid residues that identify the antibody as being derived from human sequences when compared to the germline immunoglobulin amino acid sequences of other species (*e*.*g*., murine germline sequences). In certain cases, a humanized antibody may be at least 95, 96, 97, 98 or 99%, or even at least 96%, 97%, 98%, or 99% identical in amino acid sequence to the amino acid sequence encoded by the germline immunoglobulin gene excluding the CDRs. That is, the CDRs may be murine, but the framework regions of the variable region (either heavy or light) can be at least 96%, 97%, 98%, or 99% identical in amino acid sequence to the framework amino acids encoded by one human germline immunoglobulin gene.

Typically, a humanized antibody derived from a particular human germline sequence will display no more than 10-20 amino acid differences from the amino acid sequence encoded by the human germline immunoglobulin gene. In certain cases, the humanized antibody may display no more than 5, or even no more than 4, 3, 2, or 1 amino acid difference from the amino acid sequence encoded by the germline immunoglobulin gene (again, prior to the introduction of any variants herein; that is, the number of variants is generally low).

In one embodiment, the parent antibody has been affinity matured, as is known in the art. Structure-based methods may be employed for humanization and affinity maturation, for example as described in USSN 11/004,590. Selection based methods may be employed to humanize and/or affinity mature antibody variable regions, including but not limited to methods described in Wu et al., 1999, J. Mol. Biol. 294:151-162; Baca et al., 1997, J. Biol. Chem. 272(16):10678-10684; Rosok et al., 1996, J. Biol. Chem. 271(37): 22611-22618; Rader et al., 1998, Proc. Natl. Acad. Sci. USA 95: 8910-8915; Krauss et al., 2003, Protein Engineering 16(10):753-759, all entirely incorporated by reference. Other humanization methods may involve the grafting of only parts of the CDRs, including but not limited to methods described in USSN 09/810,510; Tan et al., 2002, J. Immunol. 169:1119-1125; De Pascalis et al., 2002, J. Immunol. 169:3076-3084, all entirely incorporated by reference.

### B. PVRIG Antibodies

The present invention provides anti-PVRIG antibodies. (For convenience, "anti-PVRIG antibodies" and "PVRIG antibodies" are used interchangeably). The anti-PVRIG antibodies of the invention specifically bind to human PVRIG, and preferably the ECD of human PVRIG, as depicted in Figure 1.

Specific binding for PVRIG or a PVRIG epitope can be exhibited, for example, by an antibody having a KD of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the PVRIG antigen or epitope.

However, as shown in the Examples, for optimal binding to PVRIG expressed on the surface of NK and T-cells, the antibodies preferably have a KD less 50 nM and most preferably less than 1 nM, with less than 0.1 nM and less than 1 pM and 0.1 pM finding use in the methods of the invention.

Also, specific binding for a particular antigen or an epitope can be exhibited, for example, by an antibody having a KA or Ka for a PVRIG, antigen or epitope of at least 20-, 50-, 100-, 500-, 1000-, 5,000-, 10,000- or more times greater for the epitope relative to a control, where KA or Ka refers to an association rate of a particular antibody-antigen interaction.

In some embodiments, the anti-PVRIG antibodies of the invention bind to human PVRIG with a K_{D} of 100 nM or less, 50 nM or less, 10 nM or less, or 1 nM or less (that is, higher binding affinity), or 1pM or less, wherein K_{D} is determined by known methods, e.g. surface plasmon resonance (SPR, e.g. Biacore assays), ELISA, KINEXA, and most typically SPR at 25°C or 37°C.

### C. Specific anti-PVRIG antibodies

The invention provides antigen binding domains, including full length antibodies, which contain a number of specific, enumerated sets of 6 CDRs.

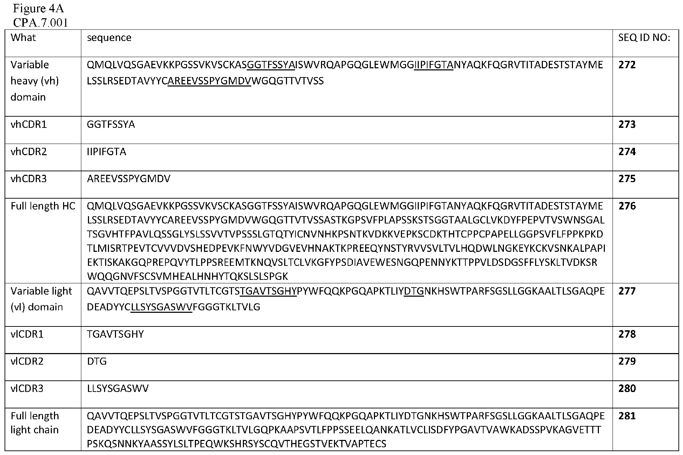

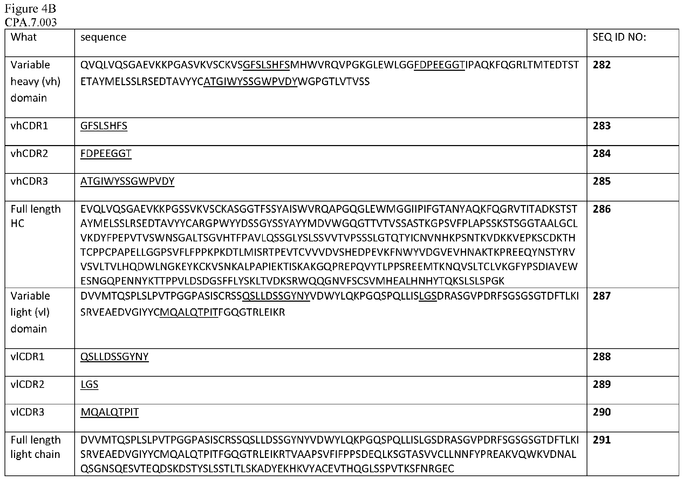

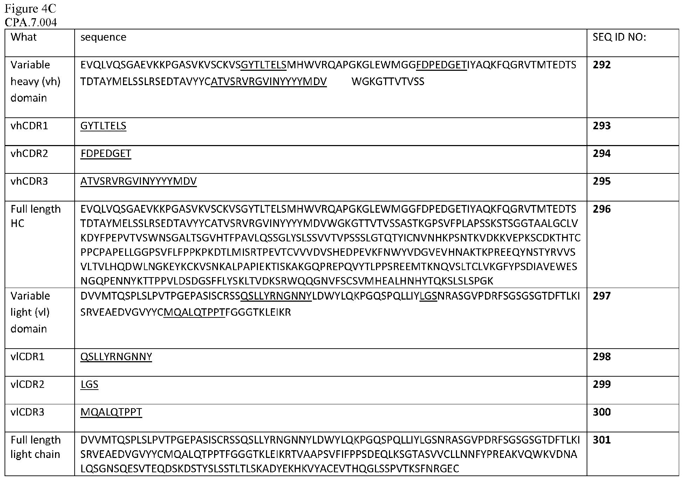

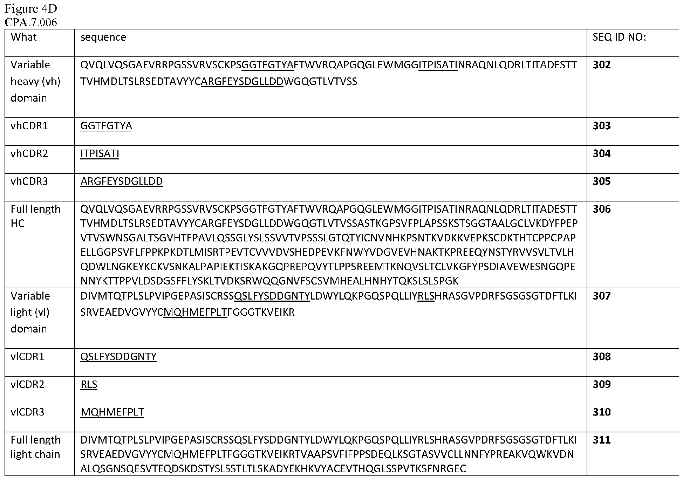

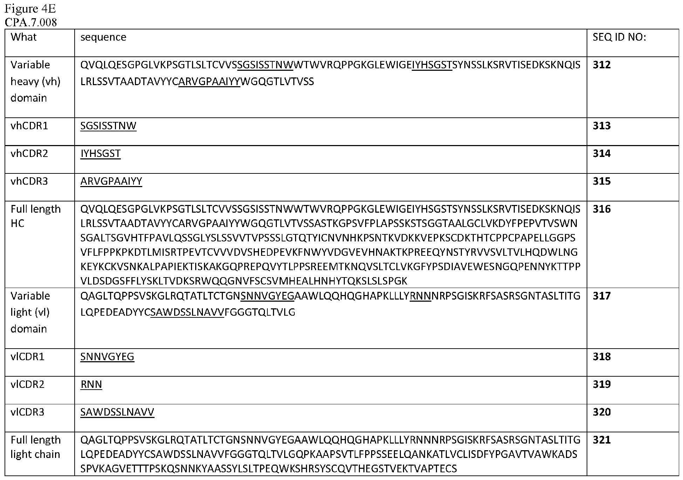

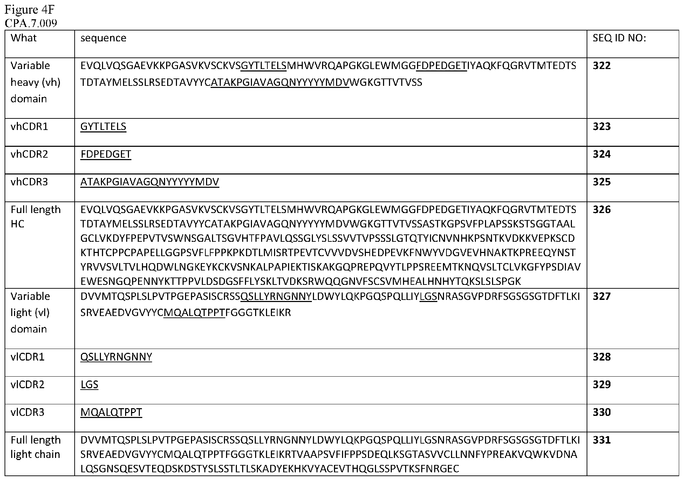

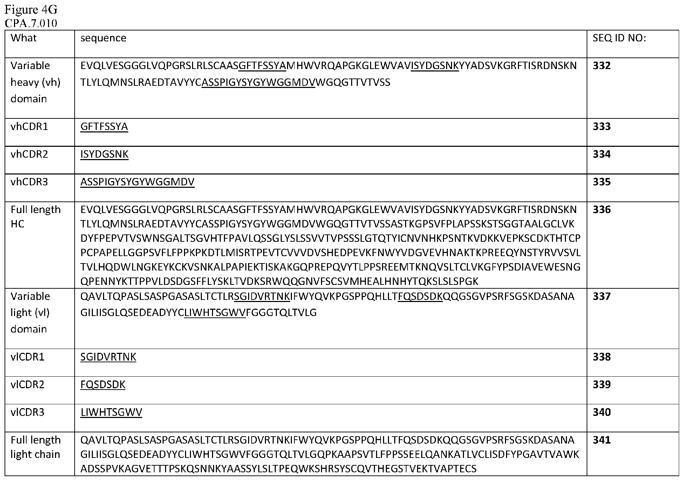

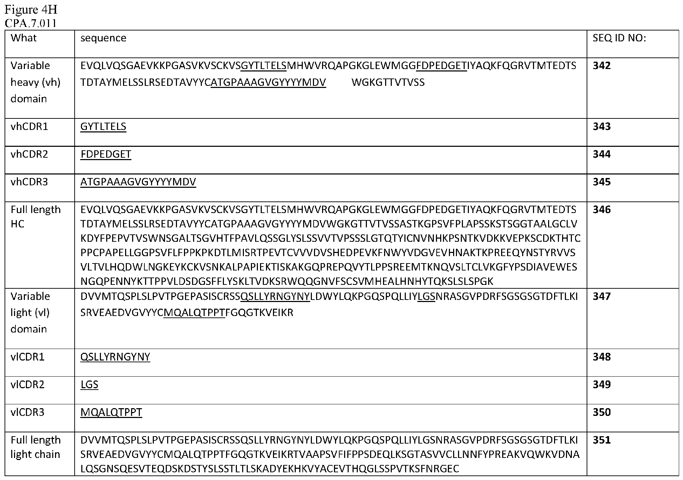

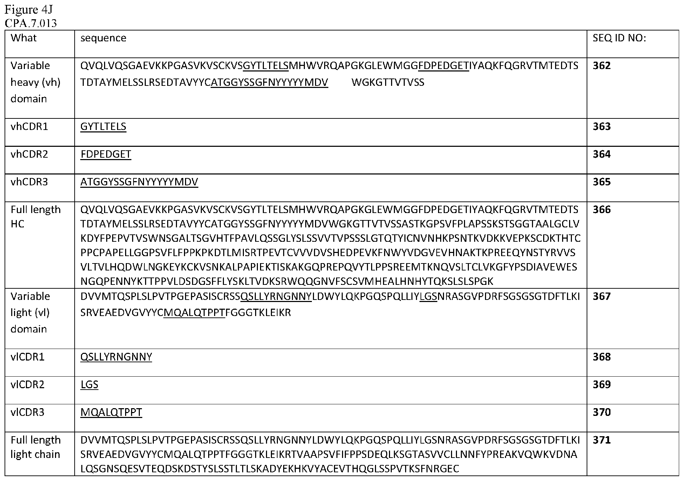

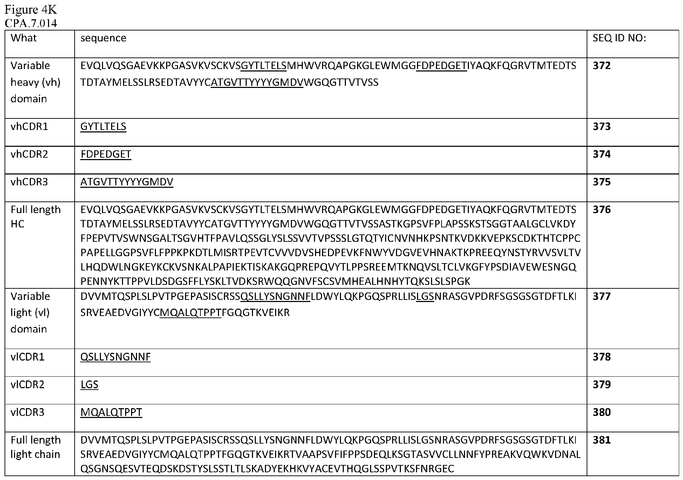

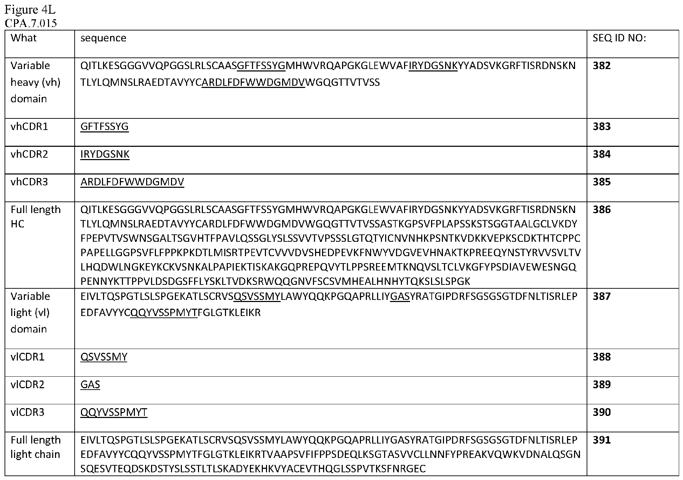

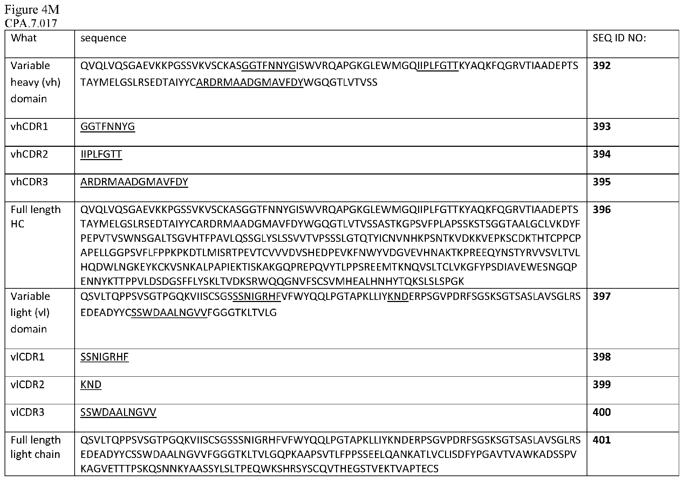

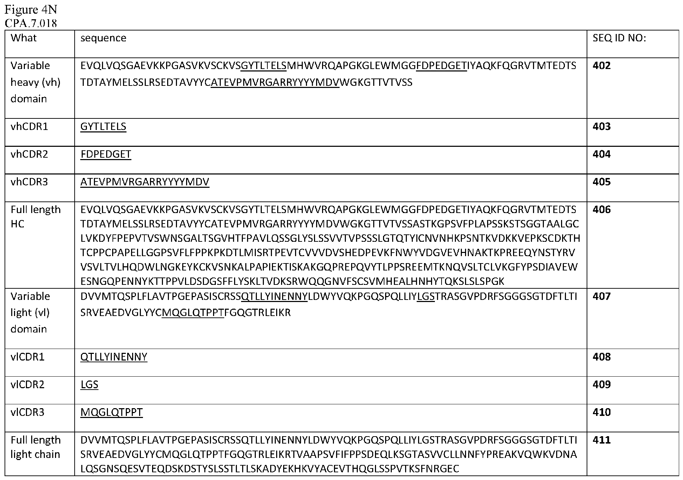

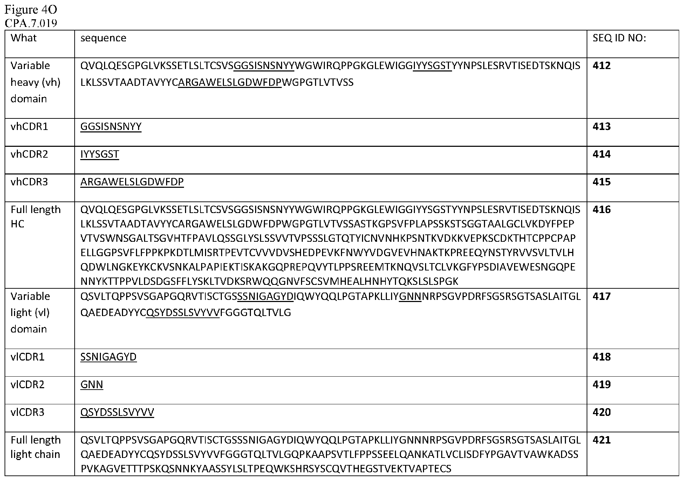

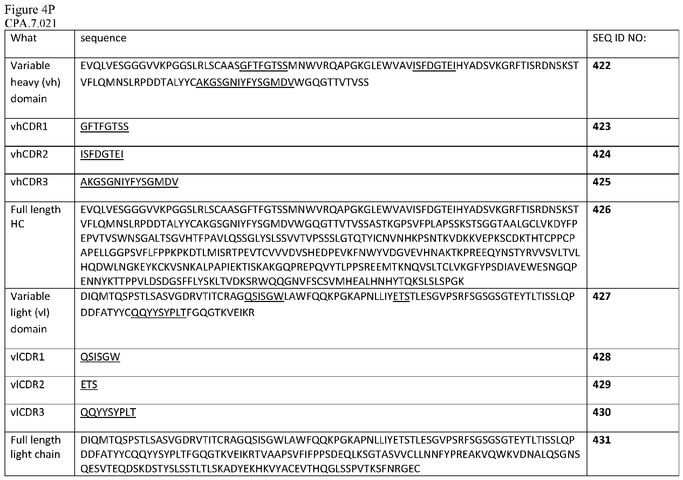

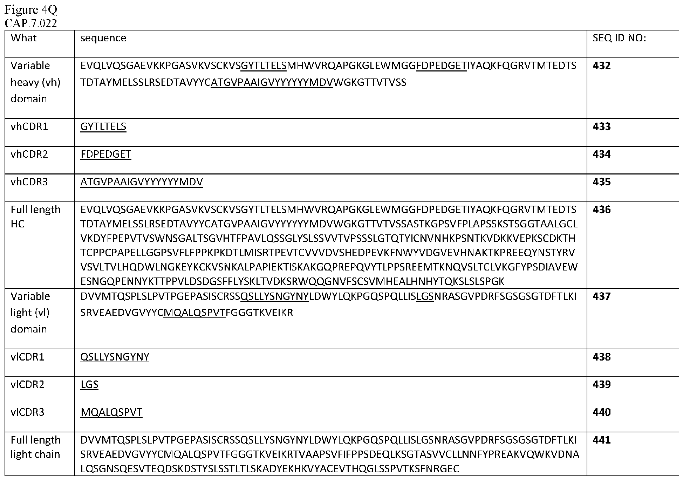

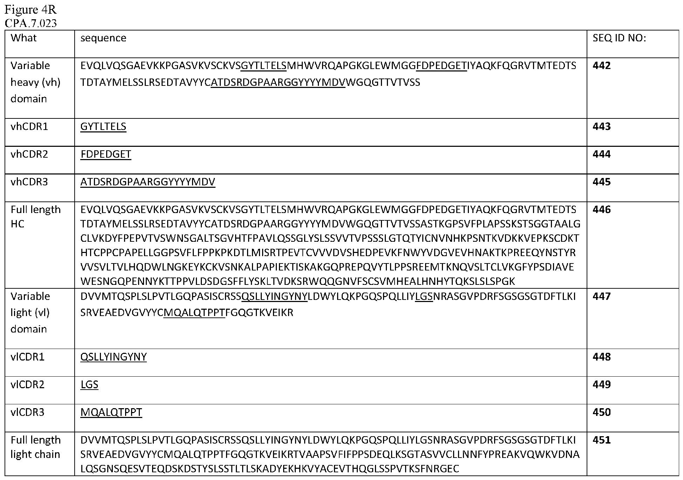

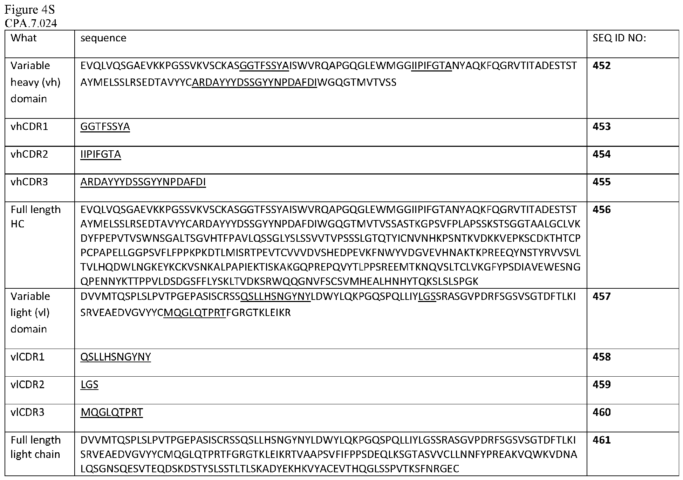

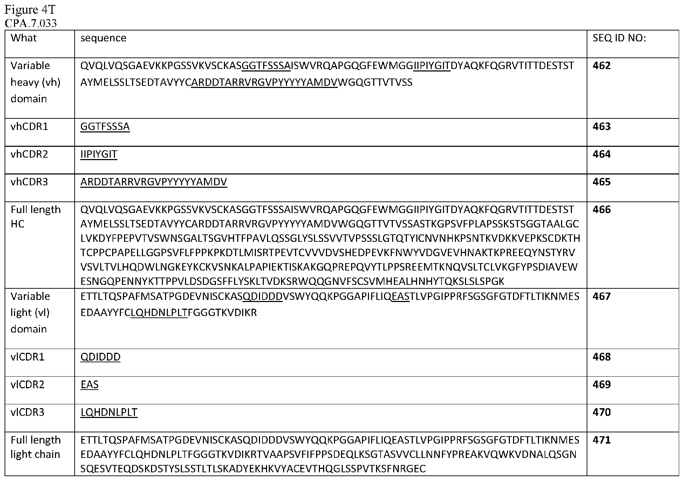

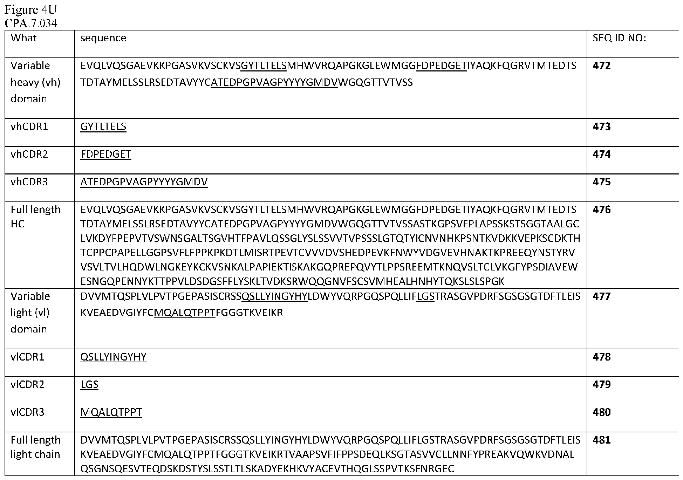

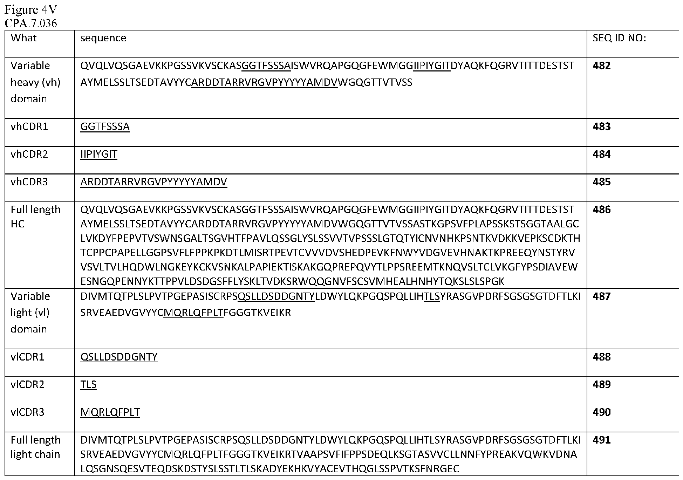

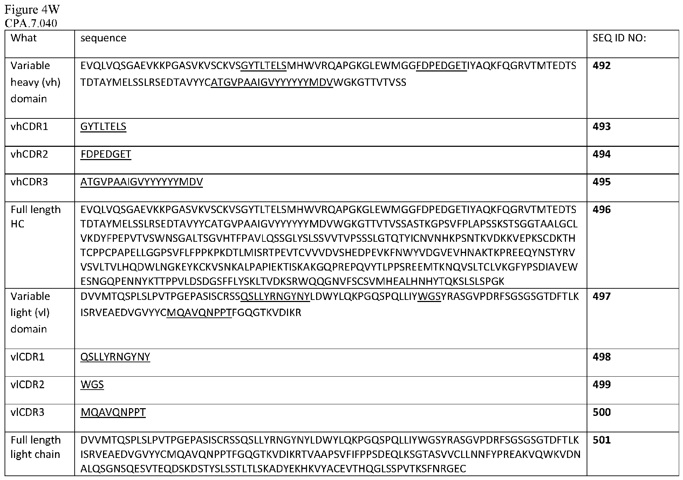

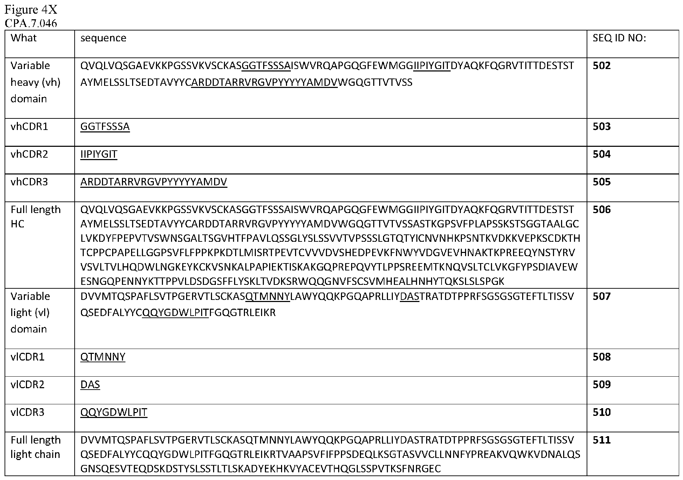

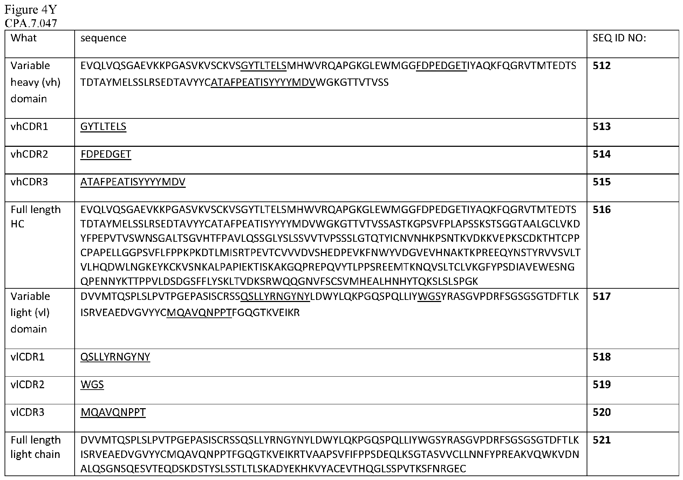

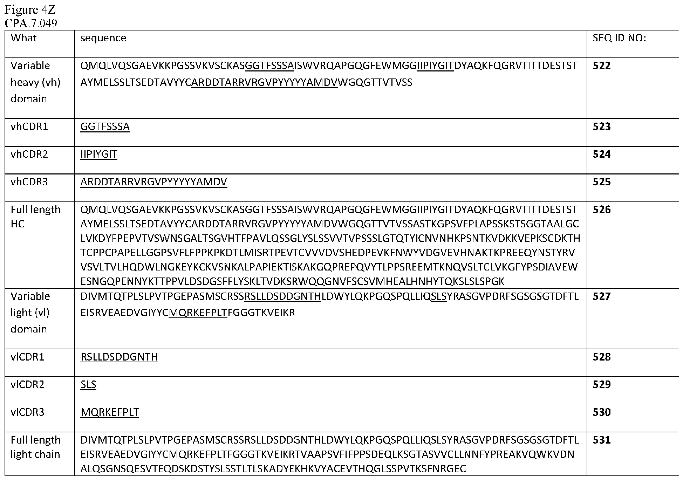

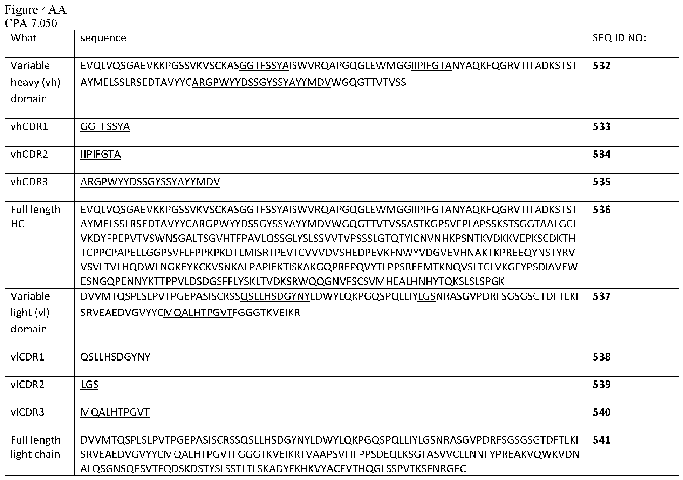

The antibodies described herein as labeled as follows. The antibodies have reference numbers, for example "CPA.7.013". This represents the combination of the variable heavy and variable light chains, as depicted in Figure 4A-4AA and Figure 5A-5H, for example. "CPA.7.013.VH" refers to the variable heavy portion of CPA.7.013, while "CPA.7.013.VL" is the variable light chain. "CPA.7.013.vhCDR1", "CPA.7.013.vhCDR2", "CPA.7.013.vhCDR3", "CPA.7.013.vlCDR1", "CPA.7.013.vlCDR2", and "CPA.7.013.vICDR3", refers to the CDRs are indicated. "CPA.7.013.HC" refers to the entire heavy chain (e.g. variable and constant domain) of this molecule, and "CPA.7.013.LC" refers to the entire light chain (e.g. variable and constant domain) of the same molecule. "CPA.7.013.H1" refers to a full length antibody comprising the variable heavy and light domains, including the constant domain of **H**uman IgG**1** (hence, the H1; IgG1, IgG2, IgG3 and IgG4 sequences are shown in Figure 7). Accordingly, "CPA.7.013.H2" would be the CPA.7.013 variable domains linked to a **H**uman IgG**2**. "CPA.7.013.H3" would be the CPA.7.013 variable domains linked to a **H**uman IgG**3**, and "CPA.7.013.H4" would be the CPA.7.013 variable domains linked to a **H**uman IgG**4**.

The invention further provides variable heavy and light domains as well as full length heavy and light chains.

In many embodiments, the antibodies of the invention are human (derived from phage) and block binding of PVRIG and PVLR2. As shown in Figure 4A-4AA, the CPA antibodies that both bind and block the receptor-ligand interaction are as below, with their components outlined as well:
CPA.7.001, CPA.7.001.VH, CPA.7.001.VL, CPA.7.001.HC, CPA.7.001.LC and CPA.7.001.H1, CPA.7.001.H2, CPA.7.001.H3, CPA.7.001.H4; CPA.7.001.vhCDR1, CPA.7.001.vhCDR2, CPA.7.001.vhCDR3, CPA.7.001.vlCDR1, CPA.7.001.vlCDR2, and CPA.7.001.vlCDR3;
CPA.7.003, CPA.7.003.VH, CPA.7.003.VL, CPA.7.003.HC, CPA.7.003.LC, CPA.7.003.H1, CPA.7.003.H2, CPA.7.003.H3, CPA.7.003.H4; CPA.7.003.vhCDR1, CPA.7.003.vhCDR2, CPA.7.003.vhCDR3, CPA.7.003.vlCDR1, CPA.7.003.vlCDR2, and CPA.7.003.vlCDR3;
CPA.7.004, CPA.7.004.VH, CPA.7.004.VL, CPA.7.004.HC, CPA.7.004.LC, CPA.7.004.H1, CPA.7.004.H2, CPA.7.004.H3 CPA.7.004.H4; CPA.7.004.vhCDR1, CPA.7.004.vhCDR2, CPA.7.004.vhCDR3, CPA.7.004.vlCDR1, CPA.7.004.vlCDR2, and CPA.7.004.vlCDR3;
CPA.7.006, CPA.7.006.VH, CPA.7.006.VL, CPA.7.006.HC, CPA.7.006.LC, CPA.7.006.H1, CPA.7.006.H2, CPA.7.006.H3 CPA.7.006.H4; CPA.7.006.vhCDR1, CPA.7.006.vhCDR2, CPA.7.006.vhCDR3, CPA.7.006.vlCDR1, CPA.7.006.vlCDR2, and CPA.7.006.vlCDR3;
CPA.7.008, CPA.7.008.VH, CPA.7.008.VL, CPA.7.008.HC, CPA.7.008.LC, CPA.7.008.H1, CPA.7.008.H2, CPA.7.008.H3 CPA.7.008.H4; CPA.7.008.vhCDR1, CPA.7.008.vhCDR2, CPA.7.008.vhCDR3, CPA.7.008.vlCDR1, CPA.7.008.vlCDR2, and CPA.7.008.vlCDR3;
CPA.7.009, CPA.7.009.VH, CPA.7.009.VL, CPA.7.009.HC, CPA.7.009.LC, CPA.7.009.H1, CPA.7.009.H2, CPA.7.009.H3 CPA.7.009.H4; CPA.7.009.vhCDR1, CPA.7.009.vhCDR2, CPA.7.009.vhCDR3, CPA.7.009.vlCDR1, CPA.7.009.vlCDR2, and CPA.7.009.vlCDR3;
CPA.7.010, CPA.7.010.VH, CPA.7.010.VL, CPA.7.010.HC, CPA.7.010.LC, CPA.7.010.H1, CPA.7.010.H2, CPA.7.010.H3 CPA.7.010.H4; CPA.7.010.vhCDR1, CPA.7.010.vhCDR2, CPA.7.010.vhCDR3, CPA.7.010.vlCDR1, CPA.7.010.vlCDR2, and CPA.7.010.vlCDR3;
CPA.7.011, CPA.7.011.VH, CPA.7.011.VL, CPA.7.011.HC, CPA.7.011.LC, CPA.7.011.H1, CPA.7.011.H2, CPA.7.011.H3 CPA.7.011.H4; CPA.7.011.vhCDR1, CPA.7.011.vhCDR2, CPA.7.011.vhCDR3, CPA.7.011.vlCDR1, CPA.7.011.vlCDR2, and CPA.7.011.vlCDR3;
CPA.7.012, CPA.7.012.VH, CPA.7.012.VL, CPA.7.012.HC, CPA.7.012.LC, CPA.7.012.H1, CPA.7.012.H2, CPA.7.012.H3 CPA.7.012.H4; CPA.7.012.vhCDR1, CPA.7.012.vhCDR2, CPA.7.012.vhCDR3, CPA.7.012.vlCDR1, CPA.7.012.vlCDR2, and CPA.7.012.vlCDR3;
CPA.7.013, CPA.7.013.VH, CPA.7.013.VL, CPA.7.013.HC, CPA.7.013.LC, CPA.7.013.H1, CPA.7.013.H2, CPA.7.013.H3 CPA.7.013.H4; CPA.7.013.vhCDR1, CPA.7.013.vhCDR2, CPA.7.013.vhCDR3, CPA.7.013.vlCDR1, CPA.7.013.vlCDR2, and CPA.7.013.vlCDR3;
CPA.7.014, CPA.7.014.VH, CPA.7.014.VL, CPA.7.014.HC, CPA.7.014.LC, CPA.7.014.H1, CPA.7.014.H2, CPA.7.014.H3 CPA.7.014.H4; CPA.7.014.vhCDR1, CPA.7.014.vhCDR2, CPA.7.014.vhCDR3, CPA.7.014.vlCDR1, CPA.7.014.vlCDR2, and CPA.7.014.vlCDR3;
CPA.7.015, CPA.7.015.VH, CPA.7.015.VL, CPA.7.015.HC, CPA.7.015.LC, CPA.7.015.H1, CPA.7.015.H2, CPA.7.015.H3 CPA.7.015.H4; CPA.7.015.vhCDR1, CPA.7.015.vhCDR2, CPA.7.015.vhCDR3, CPA.7.015.vlCDR1, CPA.7.015.vlCDR2, and CPA.7.015.vlCDR3;
CPA.7.017, CPA.7.017.VH, CPA.7.017.VL, CPA.7.017.HC, CPA.7.017.LC, CPA.7.017H1, CPA.7.017.H2, CPA.7.017.H3 CPA.7.017.H4; CPA.7.017.vhCDR1, CPA.7.000171.vhCDR2, CPA.7.017.vhCDR3, CPA.7.017.vlCDR1, CPA.7.017.vlCDR2, and CPA.7.017.vlCDR3;
CPA.7.018, CPA.7.018.VH, CPA.7.018.VL, CPA.7.018.HC, CPA.7.018.LC, CPA.7.018.H1, CPA.7.018.H2, CPA.7.018.H3 CPA.7.018.H4; CPA.7.017.vhCDR1, CPA.7.017.vhCDR2, CPA.7.017.vhCDR3, CPA.7.017.vlCDR1, CPA.7.017.vlCDR2, and CPA.7.017.vlCDR3;
CPA.7.019, CPA.7.019.VH, CPA.7.019.VL, CPA.7.019.HC, CPA.7.019.LC, CPA.7.019.H1, CPA.7.019.H2, CPA.7.019.H3 CPA.7.019.H4; CPA.7.019.vhCDR1, CPA.7.019.vhCDR2, CPA.7.019.vhCDR3, CPA.7.019.vlCDR1, CPA.7.019.vlCDR2, and CPA.7.019.vlCDR3;
CPA.7.021, CPA.7.021.VH, CPA.7.021.VL, CPA.7.021.HC, CPA.7.021.LC, CPA.7.021.H1, CPA.7.021.H2, CPA.7.021.H3 CPA.7.021.H4; CPA.7.021.vhCDR1, CPA.7.021.vhCDR2, CPA.7.021.vhCDR3, CPA.7.021.vlCDR1, CPA.7.021.vlCDR2, and CPA.7.021.vlCDR3;
CPA.7.022, CPA.7.022.VH, CPA.7.022.VL, CPA.7.022.HC, CPA.7.022.LC, CPA.7.022.H1, CPA.7.022.H2, CPA.7.022.H3 CPA.7.022.H4; CPA.7.022.vhCDR1, CPA.7.022.vhCDR2, CPA.7.002201.vhCDR3, CPA.7.022.vlCDR1, CPA.7.022.vlCDR2, and CPA.7.022.vlCDR3;
CPA.7.023, CPA.7.023.VH, CPA.7.023.VL, CPA.7.023.HC, CPA.7.023.LC, CPA.7.023.H1, CPA.7.023.H2, CPA.7.023.H3 CPA.7.023.H4; CPA.7.023.vhCDR1, CPA.7.023.vhCDR2, CPA.7.023.vhCDR3, CPA.7.023.vlCDR1, CPA.7.023.vlCDR2, and CPA.7.023.vlCDR3;
CPA.7.024, CPA.7.024.VH, CPA.7.024.VL, CPA.7.024.HC, CPA.7.024.LC, CPA.7.024.H1, CPA.7.024.H2, CPA.7.024.H3 CPA.7.024.H4; CPA.7.024.vhCDR1, CPA.7.024.vhCDR2, CPA.7.024.vhCDR3, CPA.7.024.vlCDR1, CPA.7.024.vlCDR2, and CPA.7.024.vlCDR3;
CPA.7.033, CPA.7.033.VH, CPA.7.033.VL, CPA.7.033.HC, CPA.7.033.LC, CPA.7.033.H1, CPA.7.033.H2, CPA.7.033.H3 CPA.7.033.H4; CPA.7.033.vhCDR1, CPA.7.033.vhCDR2, CPA.7.033.vhCDR3, CPA.7.033.vlCDR1, CPA.7.033.vlCDR2, and CPA.7.033.vlCDR3;
CPA.7.034, CPA.7.034.VH, CPA.7.034.VL, CPA.7.034.HC, CPA.7.034.LC, CPA.7.034.H1, CPA.7.034.H2, CPA.7.034.H3 CPA.7.034.H4; CPA.7.034.vhCDR1, CPA.7.034.vhCDR2, CPA.7.034.vhCDR3, CPA.7.034.vlCDR1, CPA.7.034.vlCDR2, and CPA.7.034.vlCDR3;
CPA.7.036, CPA.7.036.VH, CPA.7.036.VL, CPA.7.036.HC, CPA.7.036.LC, CPA.7.036.H1, CPA.7.036.H2, CPA.7.036.H3 CPA.7.036.H4; CPA.7.036.vhCDR1, CPA.7.036.vhCDR2, CPA.7.036.vhCDR3, CPA.7.036.vlCDR1, CPA.7.036.vlCDR2, and CPA.7.036.vlCDR3;
CPA.7.040, CPA.7.040.VH, CPA.7.040.VL, CPA.7.040.HC, CPA.7.040.LC, CPA.7.040.H1, CPA.7.040.H2, CPA.7.040.H3 and CPA.7.040.H4; CPA.7.040.vhCDR1, CPA.7.040.vhCDR2, CPA.7.040.vhCDR3, CPA.7.040.vlCDR1, CPA.7.040.vlCDR2, and CPA.7.040.vlCDR3;
CPA.7.046, CPA.7.046.VH, CPA.7.046.VL, CPA.7.046.HC, CPA.7.046.LC, CPA.7.046.H1, CPA.7.046.H2, CPA.7.046.H3 CPA.7.046.H4; CPA.7.046.vhCDR1, CPA.7.046.vhCDR2, CPA.7.046.vhCDR3, CPA.7.046.vlCDR1, CPA.7.046.vlCDR2, and CPA.7.046.vlCDR3;
CPA.7.047, CPA.7.047.VH, CPA.7.047.VL, CPA.7.047.HC, CPA.7.047.LC, CPA.7.047.H1, CPA.7.047.H2, CPA.7.047.H3 CPA.7.047.H4; CPA.7.047.vhCDR1, CPA.7.047.vhCDR2, CPA.7.047.vhCDR3, CPA.7.047.vlCDR1, CPA.7.004701.vlCDR2, and CPA.7.047.vlCDR3;
CPA.7.049, CPA.7.049.VH, CPA.7.049.VL, CPA.7.049.HC, CPA.7.049.LC, CPA.7.049.H1, CPA.7.049.H2, CPA.7.049.H3 CPA.7.049.H4; CPA.7.049.vhCDR1, CPA.7.049.vhCDR2, CPA.7.049.vhCDR3, CPA.7.049.vlCDR1, CPA.7.049.vlCDR2, and CPA.7.049.vlCDR3; and
CPA.7.050, CPA.7.050.VH, CPA.7.050.VL, CPA.7.050.HC, CPA.7.050.LC, CPA.7.050.H1, CPA.7.050.H2, CPA.7.050.H3 CPA.7.050.H4, CPA.7.050.vhCDR1, CPA.7.050.vhCDR2, CPA.7.050.vhCDR3, CPA.7.050.vlCDR1, CPA.7.050.vICDR2, and CPA.7.050.vICDR3.

In addition, there are a number of CPA antibodies generated herein that bound to PVRIG but did not block the interaction of PVRIG and PVLR2 as shown in Figure 5A-5H, the components of which are :
CPA.7.028, CPA.7.028.VH, CPA.7.028.VL, CPA.7.028.HC, CPA.7.028.LC, CPA.7.028.H1, CPA.7.028.H2, CPA.7.028.H3 and CPA.7.028.H4; CPA.7.028.vhCDR1, CPA.7.028.vhCDR2, CPA.7.028.vhCDR3, CPA.7.028.vlCDR1, CPA.7.028.vlCDR2, and CPA.7.028.vlCDR3.
CPA.7.030, CPA.7.030.VH, CPA.7.030.VL, CPA.7.030.HC, CPA.7.030.LC, CPA.7.030.H1, CPA.7.030.H2, CPA.7.030.H3 and CPA.7.030.H4; CPA.7.030.vhCDR1, CPA.7.030.vhCDR2, CPA.7.030.vhCDR3, CPA.7.030.vlCDR1, CPA.7.030.vlCDR2, and CPA.7.030.vlCDR3.
CPA.7.041, CPA.7.041.VH, CPA.7.041.VL, CPA.7.041.HC, CPA.7.041.LC, CPA.7.041.H1, CPA.7.041.H2, CPA.7.041.H3 and CPA.7.041.H4; CPA.7.041.vhCDR1, CPA.7.041.vhCDR2, CPA.7.041.vhCDR3, CPA.7.041.vlCDR1, CPA.7.041.vlCDR2, and CPA.7.041.vlCDR3.
CPA.7.016, CPA.7.016.VH, CPA.7.016.VL, CPA.7.016.HC, CPA.7.016.LC, CPA.7.016.H1, CPA.7.016.H2, CPA.7.016.H3 and CPA.7.016.H4; CPA.7.016.vhCDR1, CPA.7.016.vhCDR2, CPA.7.016.vhCDR3, CPA.7.016.vlCDR1, CPA.7.016.vlCDR2, and CPA.7.016.vlCDR3.
CPA.7.020, CPA.7.020.VH, CPA.7.020.VL, CPA.7.020.HC, CPA.7.020.LC, CPA.7.020.H1, CPA.7.020.H2, CPA.7.020.H3 and CPA.7.020.H4; CPA.7.020.vhCDR1, CPA.7.020.vhCDR2, CPA.7.020.vhCDR3, CPA.7.020.vlCDR1, CPA.7.020.vlCDR2, and CPA.7.020.vlCDR3.
CPA.7.038, CPA.7.038.VH, CPA.7.038.VL, CPA.7.038.HC, CPA.7.038.LC, CPA.7.038.H1, CPA.7.038.H2, CPA.7.038.H3 and CPA.7.038.H4; CPA.7.038.vhCDR1, CPA.7.038.vhCDR2, CPA.7.038.vhCDR3, CPA.7.038.vlCDR1, CPA.7.038.vlCDR2, and CPA.7.038.vlCDR3.
CPA.7.044, CPA.7.044.VH, CPA.7.044.VL, CPA.7.044.HC, CPA.7.044.LC, CPA.7.044.H1, CPA.7.044.H2, CPA.7.044.H3 and CPA.7.044.H4; CPA.7.044.vhCDR1, CPA.7.044.vhCDR2, CPA.7.044.vhCDR3, CPA.7.044.vlCDR1, CPA.7.044.vlCDR2, and CPA.7.044.vlCDR3.
CPA.7.045, CPA.7.045.VH, CPA.7.045.VL, CPA.7.045.HC, CPA.7.045.LC, CPA.7.045.H1, CPA.7.045.H2, CPA.7.045.H3 and CPA.7.045.H4; CPA.7.045.vhCDR1, CPA.7.045.vhCDR2, CPA.7.045.vhCDR3, CPA.7.045.vlCDR1, CPA.7.045.vlCDR2, and CPA.7.045.vlCDR3.

As discussed herein, the invention further provides variants of the above components, including variants in the CDRs, as outlined above. In addition, variable heavy chains can be 80%, 90%, 95%, 98% or 99% identical to the "VH" sequences herein, and/or contain from 1, 2, 3, 4, 5, 6, 7, 8, 9, 10 amino acid changes, or more, when Fc variants are used. Variable light chains are provided that can be 80%, 90%, 95%, 98% or 99% identical to the "VL" sequences herein, and/or contain from 1, 2, 3, 4, 5, 6, 7, 8, 9, 10 amino acid changes, or more, when Fc variants are used. Similarly, heavy and light chains are provided that are 80%, 90%, 95%, 98% or 99% identical to the "HC" and "LC" sequences herein, and/or contain from 1, 2, 3, 4, 5, 6, 7, 8, 9, 10 amino acid changes, or more, when Fc variants are used.

Furthermore, the present invention provides a number of CHA antibodies, which are murine antibodies generated from hybridomas. As is well known the art, the six CDRs are useful when put into either human framework variable heavy and variable light regions or when the variable heavy and light domains are humanized.

Accordingly, the present invention provides antibodies, usually full length or scFv domains, that comprise the following CHA sets of CDRs, the sequences of which are shown in Figure 3A-3AE:
CHA.7.502.vhCDR1, CHA.7.502.vhCDR2, CHA.7.502.vhCDR3, CHA.7.502.vlCDR1, CHA.7.502.vlCDR2, and CHA.7.502.vlCDR3.
CHA.7.503.vhCDR1, CHA.7.503.vhCDR2, CHA.7.503.vhCDR3, CHA.7.503.vlCDR1, CHA.7.503.vlCDR2, and CHA.7.503.vlCDR3.
CHA.7.506.vhCDR1, CHA.7.506.vhCDR2, CHA.7.506.vhCDR3, CHA.7.506.vlCDR1, CHA.7.506.vlCDR2, and CHA.7.506.vlCDR3.
CHA.7.508.vhCDR1, CHA.7.508.vhCDR2, CHA.7.508.vhCDR3, CHA.7.508.vlCDR1, CHA.7.508.vlCDR2, and CHA.7.508.vlCDR3.
CHA.7.510.vhCDR1, CHA.7.510.vhCDR2, CHA.7.510.vhCDR3, CHA.7.510.vlCDR1, CHA.7.510.vlCDR2, and CHA.7.510.vlCDR3.
CHA.7.512.vhCDR1, CHA.7.512.vhCDR2, CHA.7.512.vhCDR3, CHA.7.512.vlCDR1, CHA.7.512.vlCDR2, and CHA.7.512.vlCDR3.
CHA.7.514.vhCDR1, CHA.7.514.vhCDR2, CHA.7.514.vhCDR3, CHA.7.514.vlCDR1, CHA.7.514.vlCDR2, and CHA.7.514.vlCDR3.
CHA.7.516.vhCDR1, CHA.7.516.vhCDR2, CHA.7.516.vhCDR3, CHA.7.516.vlCDR1, CHA.7.516.vlCDR2, and CHA.7.516.vlCDR3.
CHA.7.518.vhCDR1, CHA.7.518.vhCDR2, CHA.7.518.vhCDR3, CHA.7.518.vlCDR1, CHA.7.518.vlCDR2, and CHA.7.518.vlCDR3.
CHA.7.520_1.vhCDR1, CHA.7.520_1.vhCDR2, CHA.7.520_1.vhCDR3, CHA.7.520_1.vlCDR1, CHA.7.520_1.vlCDR2, and CHA.7.520_1.vlCDR3.
CHA.7.520_2.vhCDR1, CHA.7.520_2.vhCDR2, CHA.7.520_2.vhCDR3, CHA.7.520_2.vlCDR1, CHA.7.520_2.vlCDR2, and CHA.7.520_2.vlCDR3.
CHA.7.522.vhCDR1, CHA.7.522.vhCDR2, CHA.7.522.vhCDR3, CHA.7.522.vlCDR1, CHA.7.522.vlCDR2, and CHA.7.522.vlCDR3.
CHA.7.524.vhCDR1, CHA.7.524.vhCDR2, CHA.7.524.vhCDR3, CHA.7.524.vlCDR1, CHA.7.524.vlCDR2, and CHA.7.524.vlCDR3.
CHA.7.526.vhCDR1, CHA.7.526.vhCDR2, CHA.7.526.vhCDR3, CHA.7.526.vlCDR1, CHA.7.526.vlCDR2, and CHA.7.526.vlCDR3.
CHA.7.527.vhCDR1, CHA.7.527.vhCDR2, CHA.7.527.vhCDR3, CHA.7.527.vlCDR1, CHA.7.527.vlCDR2, and CHA.7.527.vlCDR3.
CHA.7.528.vhCDR1, CHA.7.528.vhCDR2, CHA.7.528.vhCDR3, CHA.7.528.vlCDR1, CHA.7.528.vlCDR2, and CHA.7.528.vlCDR3.
CHA.7.530.vhCDR1, CHA.7.530.vhCDR2, CHA.7.530.vhCDR3, CHA.7.530.vlCDR1, CHA.7.530.vlCDR2, and CHA.7.530.vlCDR3.
CHA.7.534.vhCDR1, CHA.7.534.vhCDR2, CHA.7.534.vhCDR3, CHA.7.534.vlCDR1, CHA.7.534.vlCDR2, and CHA.7.534.vlCDR3.
CHA.7.535.vhCDR1, CHA.7.535.vhCDR2, CHA.7.535.vhCDR3, CHA.7.535.vlCDR1, CHA.7.535.vlCDR2, and CHA.7.535.vlCDR3.
CHA.7.537.vhCDR1, CHA.7.537.vhCDR2, CHA.7.537.vhCDR3, CHA.7.537.vlCDR1, CHA.7.537.vlCDR2, and CHA.7.537.vlCDR3.
CHA.7.538_1.vhCDR1, CHA.7.538_1.vhCDR2, CHA.7.538_1.vhCDR3, CHA.7.538_1.vlCDR1, CHA.7.538_1.vlCDR2, and CHA.7.538_1.vlCDR3.
CHA.7.538_2.vhCDR1, CHA.7.538_2.vhCDR2, CHA.7.538_2.vhCDR3, CHA.7.538_2.vlCDR1, CHA.7.538_2.vlCDR2, and CHA.7.538_2.vlCDR3.
CHA.7.543.vhCDR1, CHA.7.543.vhCDR2, CHA.7.543.vhCDR3, CHA.7.543.vlCDR1, CHA.7.543.vlCDR2, and CHA.7.543.vlCDR3.
CHA.7.544.vhCDR1, CHA.7.544.vhCDR2, CHA.7.544.vhCDR3, CHA.7.544.vlCDR1, CHA.7.544.vlCDR2, and CHA.7.544.vlCDR3.
CHA.7.545.vhCDR1, CHA.7.545.vhCDR2, CHA.7.545.vhCDR3, CHA.7.545.vlCDR1, CHA.7.545.vlCDR2, and CHA.7.545.vlCDR3.
CHA.7.546.vhCDR1, CHA.7.546.vhCDR2, CHA.7.546.vhCDR3, CHA.7.546.vlCDR1, CHA.7.546.vlCDR2, and CHA.7.546.vlCDR3.
CHA.7.547.vhCDR1, CHA.7.547.vhCDR2, CHA.7.547.vhCDR3, CHA.7.547.vlCDR1, CHA.7.547.vlCDR2, and CHA.7.547.vlCDR3.
CHA.7.548.vhCDR1, CHA.7.548.vhCDR2, CHA.7.548.vhCDR3, CHA.7.548.vlCDR1, CHA.7.548.vlCDR2, and CHA.7.548.vlCDR3.
CHA.7.549.vhCDR1, CHA.7.549.vhCDR2, CHA.7.549.vhCDR3, CHA.7.549.vlCDR1, CHA.7.549.vlCDR2, and CHA.7.549.vlCDR3.
CHA.7.550.vhCDR1, CHA.7.550.vhCDR2, CHA.7.550.vhCDR3, CHA.7.550.vlCDR1, CHA.7.550.vlCDR2, and CHA.7.550.vICDR3.

As above, these sets of CDRs may also be amino acid variants as described above.

In addition, the framework regions of the variable heavy and variable light chains can be humanized as is known in the art (with occasional variants generated in the CDRs as needed), and thus humanized variants of the VH and VL chains of Figure 3A-3AE can be generated. Furthermore, the humanized variable heavy and light domains can then be fused with human constant regions, such as the constant regions from IgG1, IgG2, IgG3 and IgG4.

In particular, as is known in the art, murine VH and VL chains can be humanized as is known in the art, for example, using the IgBLAST program of the NCBI website, as outlined in Ye et al. Nucleic Acids Res. 41:W34-W40 (2013), herein incorporated by reference in its entirety for the humanization methods. IgBLAST takes a murine VH and/or VL sequence and compares it to a library of known human germline sequences. As shown herein, for the humanized sequences generated herein, the databases used were IMGT human VH genes (F+ORF, 273 germline sequences) and IMGT human VL kappa genes (F+ORF, 74 germline sequences). An exemplary five CHA sequences were chosen: CHA.7.518, CHA.7.530, CHA.7.538_1, CHA.7.538_2 and CHA.7.524 (see Figure 3A-3AE for the VH and VL sequences). For this embodiment of the humanization, human germline IGHV1-46(allele1) was chosen for all 5 as the acceptor sequence and the human heavy chain IGHJ4(allele1) joining region (J gene). For three of four (CHA.7.518, CHA.7.530, CHA.7.538_1 and CHA.7.538_2), human germline IGKV1-39(allele 1) was chosen as the acceptor sequence and human light chain IGKJ2(allele1) (J gene) was chosen. The J gene was chosen from human joining region sequences compiled at IMGT^{®} the international ImMunoGeneTics information system as www.imgt.org. CDRs were defined according to the AbM definition (see www.bioinfo.org.uk/abs/).

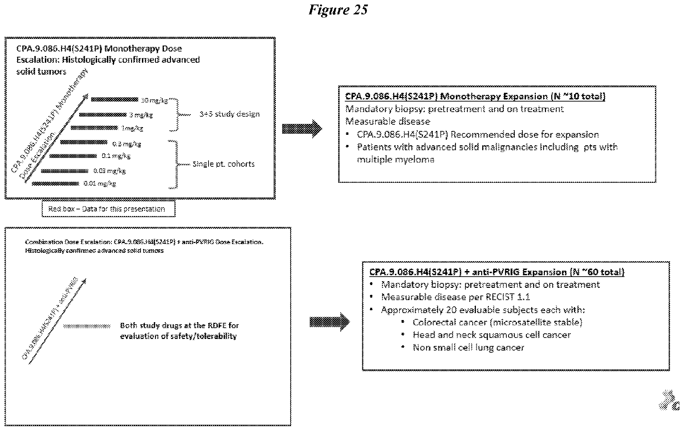

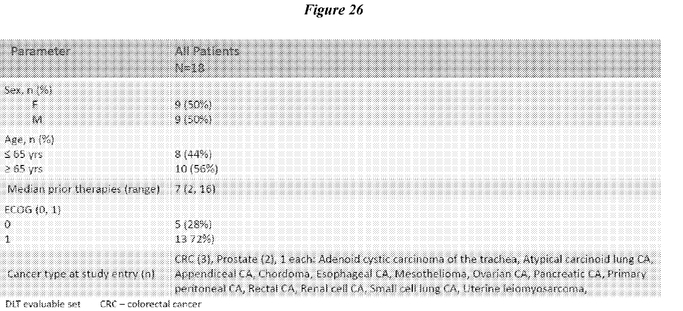

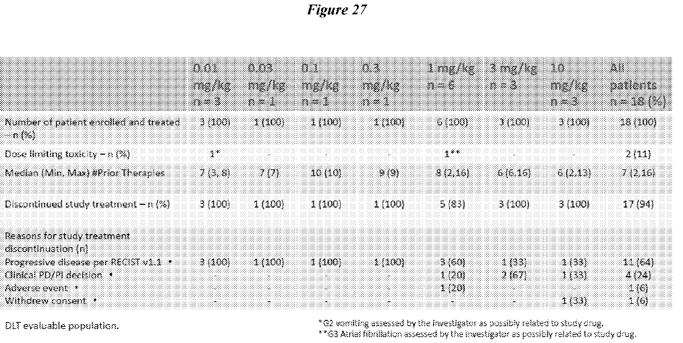

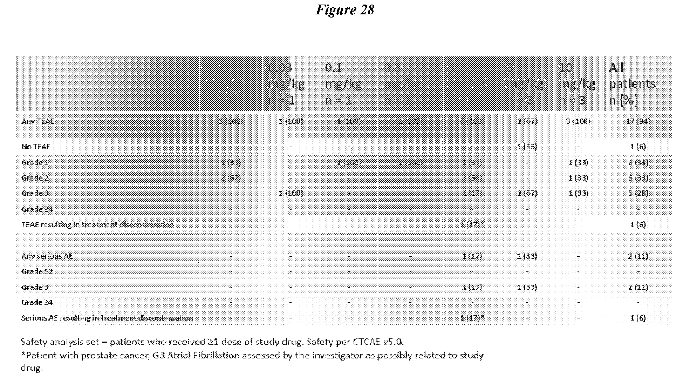

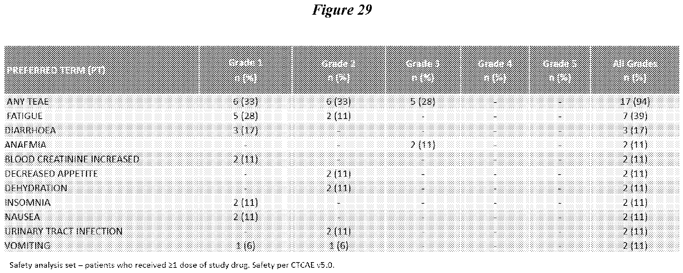

Specific humanized antibodies of CHA antibodies include those shown in Figures 25, 26, 27, 28, and 29. As will be appreciated by those in the art, each humanized variable heavy (**H**umanized **H**eavy; HH) and variable light (**H**umanized **L**ight, HL) sequence can be combined with the constant regions of human IgG1, IgG2, IgG3 and IgG4. That is, CHA.7.518.HH1 is the first humanized variable heavy chain, and CHA.7.518.HH1.**1** is the full length heavy chain, comprising the "HH1" humanized sequence with a IgG**1** constant region (CHA.7.518.HH1.**2** is CHA.7.518.HH1 with IgG2, etc,).

In some embodiments, the anti-PVRIG antibodies of the present invention include anti-PVRIG antibodies wherein the V_{H} and V_{L} sequences of different anti-PVRIG antibodies can be "mixed and matched" to create other anti-PVRIG antibodies. PVRIG binding of such "mixed and matched" antibodies can be tested using the binding assays described above. e.g., ELISAs). In some embodiments, when V_{H} and V_{L} chains are mixed and matched, a V_{H} sequence from a particular V_{H}/V_{L} pairing is replaced with a structurally similar V_{H} sequence. Likewise, in some embodiments, a V_{L} sequence from a particular V_{H}/V_{L} pairing is replaced with a structurally similar V_{L} sequence. For example, the V_{H} and V_{L} sequences of homologous antibodies are particularly amenable for mixing and matching.

Accordingly, the antibodies of the invention comprise CDR amino acid sequences selected from the group consisting of (a) sequences as listed herein; (b) sequences that differ from those CDR amino acid sequences specified in (a) by 1, 2, 3, 4, 5, 6, 7, 8, 9, 10 or more amino acid substitutions; (c) amino acid sequences having 90% or greater, 95% or greater, 98% or greater, or 99% or greater sequence identity to the sequences specified in (a) or (b); (d) a polypeptide having an amino acid sequence encoded by a polynucleotide having a nucleic acid sequence encoding the amino acids as listed herein.

Additionally included in the definition of PVRIG antibodies are antibodies that share identity to the PVRIG antibodies enumerated herein. That is, in certain embodiments, an anti-PVRIG antibody according to the invention comprises heavy and light chain variable regions comprising amino acid sequences that are homologous to isolated anti-PVRIG amino acid sequences of preferred anti-PVRIG immune molecules, respectively, wherein the antibodies retain the desired functional properties of the parent anti-PVRIG antibodies. The percent identity between the two sequences is a function of the number of identical positions shared by the sequences (i.e., % homology=# of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

In general, the percentage identity for comparison between PVRIG antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-PVRIG antibodies of the invention are those that share identity along the entire variable region (for example, where the identity is 95 or 98% identical along the variable regions), or along the entire constant region, or along just the Fc domain.

In addition, also included are sequences that may have the identical CDRs but changes in the variable domain (or entire heavy or light chain). For example, PVRIG antibodies include those with CDRs identical to those shown in described herein but whose identity along the variable region can be lower, for example 95 or 98% percent identical.

### 1. PVRIG Antibodies that Compete for binding with Enumerated Antibodies

The present invention provides not only the enumerated antibodies but additional antibodies that compete with the enumerated antibodies (the CPA and CHA numbers enumerated herein that specifically bind to PVRIG) to specifically bind to the PVRIG molecule. The PVRIG antibodies of the invention "bin" into different epitope bins. There are four separate bins outlined herein; 1) the epitope bin into which CPA.7.002, CPA.7.003, CPA.7.005, CPA.7.007, CPA.7.010, CPA.7.012, CPA.7.015, CPA.7.016, CPA.7.017, CPA.7.019, CPA.7.020, CPA.7.021, CPA.7.024, CPA.7.028, CPA.7.032, CPA.7.033, CPA.7.036, CPA.7.037, CPA.7.038, CPA.7.043, CPA.7.046 and CPA.7.041 all fall into; 2) the epitope bin into which CPA.7.004, CPA.7.009, CPA.7.011, CPA.7.014, CPA.7.018, CPA.7.022, CPA.7.023, CPA.7.034, CPA.7.040, CPA.7.045 and CPA.7.047 all fall; 3) CPA.7.039, which defines the distinction between bin 1 and bin 2, in that bin 1 blocks CPA.7.039 binding and bin 2 sandwiches the ligand with CPA.7.039, and bin 4) with CPA.7.050.

Thus, the invention provides anti-PVRIG antibodies that compete for binding with antibodies that are in bin 1, with antibodies that are in bin 2, with antibodies that are in bin 3 and/or with antibodies that are in bin 4.

Additional antibodies that compete with the enumerated antibodies are generated, as is known in the art and generally outlined below. Competitive binding studies can be done as is known in the art, generally using SPR/Biacore^{®} binding assays, as well as ELISA and cell-based assays.

### D. TIGIT Antibodies

The present invention provides anti-TIGIT antibodies. (For convenience, "anti-TIGIT antibodies" and "TIGIT antibodies" are used interchangeably). The anti- TIGIT antibodies of the invention specifically bind to human TIGIT, and preferably the ECD of human TIGIT. The invention further provides antigen binding domains, including full length antibodies, which contain a number of specific, enumerated sets of 6 CDRs that bind to TIGIT.

Specific binding for TIGIT or a TIGIT epitope can be exhibited, for example, by an antibody having a K_{D} of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10° ¹⁰ M, at least about 10⁻¹¹ M, at least about 10⁻¹² M, at least about 10⁻¹³ M, at least about 10⁻¹⁴ M, at least about 10⁻¹⁵ M, or greater, where K_{D} refers to the equilibrium dissociation constant of a particular antibody-antigen interaction. Typically, an antibody that specifically binds an antigen will have a K_{D} that is 20-, 50-, 100-, 500-, 1000-, 5,000-, 10,000- or more times greater for a control molecule relative to the TIGIT antigen or epitope.

However, for optimal binding to TIGIT expressed on the surface of NK and T-cells, the antibodies preferably have a KD less 50 nM and most preferably less than 1 nM, with less than 0.1 nM and less than 1 pM finding use in the methods of the invention

Also, specific binding for a particular antigen or an epitope can be exhibited, for example, by an antibody having a ka (referring to the association rate constant) for a TIGIT antigen or epitope of at least 20-, 50-, 100-, 500-, 1000-, 5,000-, 10,000- or more times greater for the epitope relative to a control, where ka refers to the association rate constant of a particular antibody-antigen interaction.

In some embodiments, the anti-TIGIT antibodies of the invention bind to human TIGIT with a K_{D} of 100 nM or less, 50 nM or less, 10 nM or less, or 1 nM or less (that is, higher binding affinity), or 1pM or less, wherein K_{D} is determined by known methods, e.g. surface plasmon resonance (SPR, e.g. Biacore assays), ELISA, KINEXA, and most typically SPR at 25° or 37° C.

The TIGIT antibodies described herein are labeled as follows. The antibodies have reference numbers, for example "CPA.9.086". This represents the combination of the variable heavy and variable light chains, as depicted in Figure 13, for example, with the understanding that these antibodies include two heavy chains and two light chains. "CPA.9.086.VH" refers to the variable heavy portion of CPA. 9. 086, while "CPA. 9. 086.VL" is the variable light chain. "CPA. 9. 086.vhCDR1", "CPA. 9. 086.vhCDR2", "CPA. 9. 086.vhCDR3", "CPA. 9. 086.vlCDR1", "CPA. 9. 086.vlCDR2", and "CPA. 9. 086.vlCDR3", refers to the CDRs are indicated. "CPA. 9. 086.HC" refers to the entire heavy chain (e.g. variable and constant domain) of this molecule, and "CPA. 9. 086.LC" refers to the entire light chain (e.g. variable and constant domain) of the same molecule. In general, the human kappa light chain is used for the constant domain of each phage (or humanized hybridoma) antibody herein, although in some embodiments the lambda light constant domain is used. "CPA. 9. 086.H1" refers to a full-length antibody comprising the variable heavy and light domains, including the constant domain of **H**uman IgG**1** (hence, the H1; IgG1, IgG2, IgG3 and IgG4 sequences are shown in Figure 7). Accordingly, "CPA. 9. 086.H2" would be the CPA. 9. 086 variable domains linked to a **H**uman IgG**2**. "CPA. 9. 086.H3" would be the CPA. 9. 086 variable domains linked to a **H**uman IgG**3**, and "CPA. 9. 086.H4" would be the CPA. 9. 086 variable domains linked to a **H**uman IgG**4**. Note that in some cases, the human IgGs may have additional mutations, such are described below, and this can be annotated. For example, in many embodiments, there may be a S241P mutation in the human IgG4, and this can be annotated as "CPA.9.086.H4(S241P)" for example. The human IgG4 sequence with this S241P hinge variant is shown in Figure 7. Other potential variants are IgG1(N297A), (or other variants that ablate glycosylation at this site and thus many of the effector functions associated with FcγRIIIa binding), and IgG1(D265A), which reduces binding to FcγR receptors.

The invention further provides variable heavy and light domains as well as full length heavy and light chains.

In some embodiments, the invention provides scFvs that bind to TIGIT comprising a variable heavy domain and a variable light domain linked by an scFv linker as outlined above. The VL and VH domains can be in either orientation, e.g., from N- to C-terminus "VH-linker-VL" or "VL-linker" or "VH". These are named by their component parts; for example, "scFv-CPA. 9.086 VH-linker-VL" or "scFv-CPA.9.086.VL-linker-VH." Thus, "scFv-CPA.9.086" can be in either orientation.

In many embodiments, the antibodies of the invention are human (derived from phage) and block binding of TIGIT and PVR. As shown in Figure 13, the CPA antibodies that both bind and block the receptor-ligand interaction are as below, with their components outlined as well (as discussed in the "Sequence" section, the sequences of all but the scFv constructs are in the sequence listing):
CPA.9.018, CPA.9.018.VH, CPA.9.018.VL, CPA.9.018.HC, CPA.9.018.LC, CPA.9.018.H1, CPA.9.018.H2, CPA.9.018.H3, CPA.9.018.H4; CPA.9.018.H4(S241P); CPA.9.018.vhCDR1, CPA.9.018.vhCDR2, CPA.9.018.vhCDR3, CPA.9.018.vlCDR1, CPA.9.018.vlCDR2, CPA.9.018.vlCDR3 and scFv-CPA.9.018;
CPA.9.027, CPA.9.027.VH, CPA.9.027.VL, CPA.9.027.HC, CPA.9.027.LC, CPA.9.027.H1, CPA.9.027.H2, CPA.9.027.H3, CPA.9.027.H4; CPA.9.018.H4(S241P); CPA.9.027.vhCDR1, CPA.9.027.vhCDR2, CPA.9.027.vhCDR3, CPA.9.027.vlCDR1, CPA.9.027.vlCDR2, CPA.9.027.vlCDR3 and scFv-CPA.9.027;
CPA.9.049, CPA.9.049.VH, CPA.9.049.VL, CPA.9.049.HC, CPA.9.049.LC, CPA.9.049.H1, CPA.9.049.H2, CPA.9.049.H3; CPA.9.049.H4; CPA.9.049.H4(S241P); CPA.9.049.vhCDR1, CPA.9.049.vhCDR2, CPA.9.049.vhCDR3, CPA.9.049.vlCDR1, CPA.9.049.vlCDR2, CPA.9.049.vlCDR3 and scFv-CPA.9.049;
CPA.9.057, CPA.9.057.VH, CPA.9.057.VL, CPA.9.057.HC, CPA.9.057.LC, CPA.9.057.H1, CPA.9.057.H2, CPA.9.057.H3; CPA.9.057.H4; CPA.9.057.H4(S241P); CPA.9.057.vhCDR1, CPA.9.057.vhCDR2, CPA.9.057.vhCDR3, CPA.9.057.vlCDR1, CPA.9.057.vlCDR2, CPA.9.057.vlCDR3 and scFv-CPA.9.057;
CPA.9.059, CPA.9.059.VH, CPA.9.059.VL, CPA.9.059.HC, CPA.9.059.LC, CPA.9.059.H1, CPA.9.059.H2, CPA.9.059.H3; CPA.9.059.H4; CPA.9.059.H4(S241P); CPA.9.059.vhCDR1, CPA.9.059.vhCDR2, CPA.9.059.vhCDR3, CPA.9.059.vlCDR1, CPA.9.059.vlCDR2, CPA.9.059.vlCDR3 and scFv-CPA.9.059;
CPA.9.083, CPA.9.083.VH, CPA.9.083.VL, CPA.9.083.HC, CPA.9.083.LC, CPA.9.083.H1, CPA.9.083.H2, CPA.9.083.H3; CPA.9.083.H4; CPA.9.083.H4(S241P); CPA.9.083.vhCDR1, CPA.9.083.vhCDR2, CPA.9.083.vhCDR3, CPA.9.083.vlCDR1, CPA.9.083.vlCDR2, CPA.9.083.vlCDR3 and scFv-CPA.9.083;
CPA.9.086, CPA.9.086.VH, CPA.9.086.VL, CPA.9.086.HC, CPA.9.086.LC, CPA.9.086.H1, CPA.9.086.H2, CPA.9.086.H3; CPA.9.086.H4; CPA.9.086.H4(S241P); CPA.9.086.vhCDR1, CPA.9.086.vhCDR2, CPA.9.086.vhCDR3, CPA.9.086.vlCDR1, CPA.9.086.vlCDR2, CPA.9.086.vlCDR3 and scFv-CPA.9.086;
CPA.9.089, CPA.9.089.VH, CPA.9.089.VL, CPA.9.089.HC, CPA.9.089.LC, CPA.9.089.H1, CPA.9.089.H2, CPA.9.089.H3; CPA.9.089.H4; CPA.9.089.H4(S241P); CPA.9.089.vhCDR1, CPA.9.089.vhCDR2, CPA.9.089.vhCDR3, CPA.9.089.vlCDR1, CPA.9.089.vlCDR2, CPA.9.089.vlCDR3 and scFv-CPA.9.089;
CPA.9.093, CPA.9.093.VH, CPA.9.093.VL, CPA.9.093.HC, CPA.9.093.LC, CPA.9.093.H1, CPA.9.093.H2, CPA.9.093.H3; CPA.9.093.H4; CPA.9.093.H4(S241P); CPA.9.093.vhCDR1, CPA.9.093.vhCDR2, CPA.9.093.vhCDR3, CPA.9.093.vlCDR1, CPA.9.093.vlCDR2, CPA.9.093.vlCDR3 and scFv-CPA.9.093;
CPA.9.101, CPA.9.101.VH, CPA.9.101.VL, CPA.9.101.HC, CPA.9.101.LC, CPA.9.101.H1, CPA.9.101.H2, CPA.9.101.H3; CPA.9.101.H4; CPA.9.101.H4(S241P); CPA.9.101.vhCDR1, CPA.9.101.vhCDR2, CPA.9.101.vhCDR3, CPA.9.101.vlCDR1, CPA.9.101.vlCDR2, CPA.9.101.vlCDR3 and scFv-CPA.9.101; and
CPA.9.103, CPA.9.103.VH, CPA.9.103.VL, CPA.9.103.HC, CPA.9.103.LC, CPA.9.103.H1, CPA.9.103.H2, CPA.9.103.H3; CPA.9.103.H4; CPA.9.103.H4(S241P); CPA.9.103.vhCDR1, CPA.9.103.vhCDR2, CPA.9.103.vhCDR3, CPA.9.103.vlCDR1, CPA.9.103.vlCDR2, CPA.9.103.vlCDR3 and scFv-CPA.9.103.

Furthermore, the present invention provides a number of CHA antibodies, which are murine antibodies generated from hybridomas. As is well known the art, the six CDRs are useful when put into either human framework variable heavy and variable light regions or when the variable heavy and light domains are humanized.

Accordingly, the present invention provides antibodies, usually full length or scFv domains, that comprise the following sets of CDRs, the sequences of which are shown in Figure 13 and/or the sequence listing:
CHA.9.536.1, CHA.9.536.1.VH, CHA.9.536.1.VL, CHA.9.536.1.HC, CHA.9.536.1.LC, CHA.9.536.1.H1, CHA.9.536.1.H2, CHA.9.536.1.H3; CHA.9.536.1.H4, CHA.9.536.1.H4(S241P), CHA.9.536.1.vhCDR1, CHA.9.536.1.vhCDR2, CHA.9.536.1.vhCDR3, CHA.9.536.1.vlCDR1, CHA.9.536.1.vlCDR2 and CHA.9.536.1.vhCDR3;
CHA.9.536.3, CHA.9.536.3.VH, CHA.9.536.3.VL, CHA.9.536.3.HC, CHA.9.536.3.LC, CHA.9.536.3.H1, CHA.9.536.3.H2, CHA.9.536.3.H3; CHA.9.536.3.H4, CHA.9.536.3.H4(S241P); CHA.9.536.3.vhCDR1, CHA.9.536.3.vhCDR2, CHA.9.536.3.vhCDR3, CHA.9.536.3.vlCDR1, CHA.9.536.3.vlCDR2 and CHA.9.536.3.vhCDR3;
CHA.9.536.4, CHA.9.536.4.VH, CHA.9.536.4.VL, CHA.9.536.4.HC, CHA.9.536.4.LC, CHA.9.536.4.H1, CHA.9.536.4.H2, CHA.9.536.4.H3; CHA.9.536.4.H4, CHA.9.536.4.H4(S241P), CHA.9.536.4.vhCDR1, CHA.9.536.4.vhCDR2, CHA.9.536.4.vhCDR3, CHA.9.536.4.vlCDR1, CHA.9.536.4.vlCDR2 and CHA.9.536.4.vhCDR3;
CHA.9.536.5, CHA.9.536.5.VH, CHA.9.536.5.VL, CHA.9.536.5.HC, CHA.9.536.5.LC, CHA.9.536.5.H1, CHA.9.536.5.H2, CHA.9.536.5.H3; CHA.9.536.5.H4, CHA.9.536.5.H4(S241P), CHA.9.536.5.vhCDR1, CHA.9.536.5.vhCDR2, CHA.9.536.5.vhCDR3, CHA.9.536.5.vlCDR1, CHA.9.536.5.vlCDR2 and CHA.9.536.5.vhCDR3;
CHA.9.536.6, CHA.9.536.6.VH, CHA.9.536.6.VL, CHA.9.536.6.HC, CHA.9.536.6.LC, CHA.9.536.6.H1, CHA.9.536.6.H2, CHA.9.536.6.H3; CHA.9.536.6.H4, CHA.9.536.6.vhCDR1, CHA.9.536.6.vhCDR2, CHA.9.536.6.vhCDR3, CHA.9.536.6.vlCDR1, CHA.9.536.6.vlCDR2 and CHA.9.536.6.vhCDR3;
CHA.9.536.7, CHA.9.536.7.VH, CHA.9.536.7.VL, CHA.9.536.7.HC, CHA.9.536.7.LC, CHA.9.536.7.H1, CHA.9.536.7.H2, CHA.9.536.7.H3; CHA.9.536.7.H4, CHA.9.536.5.H4(S241P); CHA.9.536.7.vhCDR1, CHA.9.536.7.vhCDR2, CHA.9.536.7.vhCDR3, CHA.9.536.7.vlCDR1, CHA.9.536.7.vlCDR2 and CHA.9.536.7.vhCDR3;
CHA.9.536.8, CHA.9.536.8.VH, CHA.9.536.8.VL, CHA.9.536.8.HC, CHA.9.536.8.LC, CHA.9.536.8.H1, CHA.9.536.8.H2, CHA.9.536.8.H3; CHA.9.536.8.H4, CHA.9.536.8.H4(S241P), CHA.9.536.8.vhCDR1, CHA.9.536.8.vhCDR2, CHA.9.536.8.vhCDR3, CHA.9.536.8.vlCDR1, CHA.9.536.8.vlCDR2 and CHA.9.536.8.vhCDR3;
CHA.9.560.1, CHA. 9.560.1VH, CHA. 9.560.1.VL, CHA. 9.560.1.HC, CHA. 9.560.1.LC, CHA. 9.560.1.H1, CHA. 9.560.1.H2, CHA. 9.560.1.H3; CHA. 9.560.1.H4, CHA. 9.560.1.H4(S241P), CHA. 9.560.1.vhCDR1, CHA. 9.560.1.vhCDR2, CHA. 9.560.1.vhCDR3, CHA. 9.560.1.vlCDR1, CHA. 9.560.1.vlCDR2 and CHA. 9.560.1.vhCDR3;
CHA.9.560.3, CHA. 9.560. 3VH, CHA. 9.560. 3.VL, CHA. 9.560. 3.HC, CHA. 9.560. 3.LC, CHA. 9.560. 3.H1, CHA. 9.560. 3.H2, CHA. 9.560. 3.H3; CHA.9.560.3.H4, CHA.9.560.3.H4(S241P); CHA. 9.560. 3.vhCDR1, CHA. 9.560. 3.vhCDR2, CHA. 9.560. 3.vhCDR3, CHA. 9.560. 3.vlCDR1, CHA. 9.560. 3.vlCDR2 and CHA. 9.560. 3.vhCDR3;
CHA.9.560.4, CHA. 9.560. 4VH, CHA. 9.560. 4.VL, CHA. 9.560. 4.HC, CHA. 9.560. 4.LC, CHA. 9.560. 4.H1, CHA. 9.560. 4.H2, CHA. 9.560. 4.H3; CHA.9.560.4.H4, CHA.9.560.4.H4(S241P), CHA. 9.560. 4.vhCDR1, CHA. 9.560. 4.vhCDR2, CHA. 9.560. 4.vhCDR3, CHA. 9.560. 4.vlCDR1, CHA. 9.560. 4.vlCDR2 and CHA. 9.560. 4.vhCDR3;
CHA.9.560.5, CHA. 9.560. 5VH, CHA. 9.560. 5.VL, CHA. 9.560. 5.HC, CHA. 9.560. 5.LC, CHA. 9.560. 5.H1, CHA. 9.560. 5.H2, CHA. 9.560. 5.H3; CHA. 9.560. 5.H4, CHA. 9.560. 5.vhCDR1, CHA. 9.560. 5.vhCDR2, CHA. 9.560. 5.vhCDR3, CHA. 9.560. 5.vlCDR1, CHA. 9.560. 5.vlCDR2 and CHA. 9.560. 5.vhCDR3;
CHA.9.560.6, CHA. 9.560. 6VH, CHA. 9.560. 6.VL, CHA. 9.560. 6.HC, CHA. 9.560. 6.LC, CHA. 9.560. 6.H1, CHA. 9.560. 6.H2, CHA. 9.560. 6.H3; CHA.9.560.6.H4, CHA.9.560.6.H4(S241P), CHA. 9.560. 6.vhCDR1, CHA. 9.560. 6.vhCDR2, CHA. 9.560. 6.vhCDR3, CHA. 9.560. 6.vlCDR1, CHA. 9.560. 6.vlCDR2 and CHA. 9.560. 6.vhCDR3;
CHA.9.560.7, CHA. 9.560. 7VH, CHA. 9.560. 7.VL, CHA. 9.560. 7.HC, CHA. 9.560. 7.LC, CHA. 9.560. 7.H1, CHA. 9.560. 7.H2, CHA. 9.560. 7.H3; CHA.9.560.7.H4; CHA.9.560.7.H4(S241P); CHA. 9.560. 7.vhCDR1, CHA. 9.560. 7.vhCDR2, CHA. 9.560. 7.vhCDR3, CHA. 9.560. 7.vlCDR1, CHA. 9.560. 7.vlCDR2 and CHA. 9.560. 7.vhCDR3;
CHA.9.560.8, CHA. 9.560. 8VH, CHA. 9.560. 8.VL, CHA. 9.560. 8.HC, CHA. 9.560. 8.LC, CHA. 9.560. 8.H1, CHA. 9.560. 8.H2, CHA. 9.560. 8.H3; CHA.9.560.8.H4, CHA.9.560.8.H4(S241P); CHA. 9.560. 8.vhCDR1, CHA. 9.560. 8.vhCDR2, CHA. 9.560. 8.vhCDR3, CHA. 9.560. 8.vlCDR1, CHA. 9.560. 8.vlCDR2 and CHA. 9.560. 8.vhCDR3;
CHA.9.546.1, CHA. 9. 546.1VH, CHA. 9. 546.1.VL, CHA. 9. 546.1.HC, CHA. 9. 546.1.LC, CHA. 9. 546.1.H1, CHA. 9. 546.1.H2, CHA. 9. 546.1.H3; CHA.9.546.1.H4, CHA.9.546.1.H4(S241P), CHA. 9. 546.1.vhCDR1, CHA. 9. 546.1.vhCDR2, CHA. 9. 546.1.vhCDR3, CHA. 9. 546.1.vlCDR1, CHA. 9. 546.1.vlCDR2 and CHA. 9. 546.1.vhCDR3;
CHA.9.547.1, CHA. 9. 547.1VH, CHA. 9. 547.1.VL, CHA. 9. 547.1.HC, CHA. 9. 547.1.LC, CHA. 9. 547.1.H1, CHA. 9. 547.1.H2, CHA. 9. 547.1.H3; CHA.9.547.1.H4, CHA.9.547.1.H4(S241P), CHA. 9. 547.1.vhCDR1, CHA. 9. 547.1.vhCDR2, CHA. 9. 547.1.vhCDR3, CHA. 9. 547.1.vlCDR1, CHA. 9. 547.1.vlCDR2 and CHA. 9. 547.1.vhCDR3;
CHA.9.547.2, CHA. 9. 547. 2VH, CHA. 9. 547. 2.VL, CHA. 9. 547. 2.HC, CHA. 9. 547. 2.LC, CHA. 9. 547. 2.H1, CHA. 9. 547. 2.H2, CHA. 9. 547. 2.H3; CHA.9.547.2.H4, CHA.9.547.2.H4(S241P), CHA. 9. 547. 2.vhCDR1, CHA. 9. 547. 2.vhCDR2, CHA. 9. 547. 2.vhCDR3, CHA. 9. 547. 2.vlCDR1, CHA. 9. 547. 2.vlCDR2 and CHA. 9. 547. 2.vhCDR3;
CHA.9.547.3, CHA. 9. 547. 3VH, CHA. 9. 547. 3.VL, CHA. 9. 547. 3.HC, CHA. 9. 547. 3.LC, CHA. 9. 547. 3.H1, CHA. 9. 547. 3.H2, CHA. 9. 547. 3.H3; CHA.9.547.3.H4, CHA.9.547.3.H4(S241P), CHA. 9. 547. 3.vhCDR1, CHA. 9.547. 3.vhCDR2, CHA. 9. 547. 3.vhCDR3, CHA. 9. 547. 3.vlCDR1, CHA. 9. 547. 3.vlCDR2 and CHA. 9. 547. 3.vhCDR3;
CHA.9.547.4, CHA. 9. 547. 4VH, CHA. 9. 547. 4.VL, CHA. 9. 547. 4.HC, CHA. 9.547. 4.LC, CHA. 9. 547. 4.H1, CHA. 9. 547. 4.H2, CHA. 9. 547. 4.H3; CHA.9.547.4.H4, CHA.9.547.4.H4(S241P), CHA. 9. 547. 4.vhCDR1, CHA. 9. 547. 4.vhCDR2, CHA. 9. 547. 4.vhCDR3, CHA. 9. 547. 4.vlCDR1, CHA. 9. 547. 4.vlCDR2 and CHA. 9. 547. 4.vhCDR3;
CHA.9.547.6, CHA. 9. 547. 6 VH, CHA. 9. 547. 6.VL, CHA. 9. 547. 6.HC, CHA. 9. 547. 6.LC, CHA. 9. 547. 6.H1, CHA. 9. 547. 6.H2, CHA. 9. 547. 6.H3; CHA.9.547.6.H4, CHA.9.547.6.H4(S241P), CHA. 9. 547. 6.vhCDR1, CHA. 9. 547. 6.vhCDR2, CHA. 9. 547. 6.vhCDR3, CHA. 9. 547. 6.vlCDR1, CHA. 9. 547. 6.vlCDR2 and CHA. 9. 547. 6.vhCDR3;
CHA.9.547.7, CHA. 9. 547. 7VH, CHA. 9. 547. 7.VL, CHA. 9. 547. 7.HC, CHA. 9. 547. 7.LC, CHA. 9. 547. 7.H1, CHA. 9. 547. 7.H2, CHA. 9. 547. 7.H3; CHA.9.547.7.H4, CHA.9.547.7.H4(S241P), CHA. 9. 547. 7.vhCDR1, CHA. 9. 547. 7.vhCDR2, CHA. 9. 547. 7.vhCDR3, CHA. 9. 547. 7.vlCDR1, CHA. 9. 547. 7.vlCDR2 and CHA. 9. 547. 7.vhCDR3;
CHA.9.547.8, CHA. 9. 547. 8VH, CHA. 9. 547. 8.VL, CHA. 9. 547. 8.HC, CHA.9.547.8.LC, CHA. 9. 547. 8.H1, CHA. 9. 547. 8.H2, CHA. 9. 547. 8.H3; CHA.9.547.8.H4, CHA.9.547.8.H4(S241P), CHA. 9. 547. 8.vhCDR1, CHA. 9. 547. 8.vhCDR2, CHA. 9. 547. 8.vhCDR3, CHA. 9. 547. 8.vlCDR1, CHA. 9. 547. 8.vlCDR2 and CHA. 9. 547. 8.vhCDR3;
CHA.9.547.9, CHA.9.547.9, CHA.9.547.9VH, CHA.9.547.9.VL, CHA.9. 547.9.HC, CHA.9.547.9.LC, CHA.9.547.9.H1, CHA.9.547.9.H2, CHA.9.547.9.H3; CHA.9.547.9.H4, CHA.9.547.9.H4, CHA.9.547.9.H4(S241P), CHA.9.547.9.H4(S241P), CHA.9.547.9.vhCDR1, CHA.9.547.9.vhCDR2, CHA.9.547.9.vhCDR3, CHA.9.547.9.vlCDR1, CHA.9.547.9.vlCDR2 and CHA.9.547.9.vhCDR3;
CHA.9.547.13, CHA.9.547.13, CHA.9.547. 13VH, CHA.9. 547.13.VL, CHA.9. 547.13.HC, CHA. 9.547.13.LC, CHA. 9.547.13.H1, CHA.9.547.13.H2, CHA.9. 547.13.H3; CHA.9.547.13.H4, CHA.9.547.13.H4, CHA.9.547.13.H4(S241P), CHA.9.547.13.H4(S241P), CHA. 9. 547.13.vhCDR1, CHA.9.547.13.vhCDR2, CHA.9.547. 13.vhCDR3, CHA. 9. 547.13.vlCDR1, CHA. 9. 547.13.vlCDR2 and CHA. 9. 547. 13.vhCDR3;
CHA.9.541.1, CHA. 9. 541.1.VH, CHA. 9. 541.1.VL, CHA. 9. 541.1.HC, CHA. 9. 541.1.LC, CHA. 9. 541.1.H1, CHA. 9. 541.1.H2, CHA. 9. 541.1.H3; CHA.9.541.1.H4, CHA.9.541.1.H4(S241P), CHA. 9. 541.1.vhCDR1, CHA. 9. 541.1.vhCDR2, CHA. 9. 541.1.vhCDR3, CHA. 9. 541.1.vlCDR1, CHA. 9. 541.1.vlCDR2 and CHA. 9.541.1.vhCDR3;
CHA.9.541.3, CHA. 9. 541. 3.VH, CHA. 9. 541. 3.VL, CHA. 9. 541. 3.HC, CHA. 9. 541. 3.LC, CHA. 9. 541. 3.H1, CHA. 9. 541. 3.H2, CHA. 9. 541. 3.H3; CHA.9.541.3.H4, CHA.9.541.3.H4(S241P), CHA. 9. 541. 3.vhCDR1, CHA. 9. 541. 3.vhCDR2, CHA. 9. 541. 3.vhCDR3, CHA. 9. 541. 3.vlCDR1, CHA. 9. 541. 3.vlCDR2 and CHA. 9.541. 3.vhCDR3;
CHA.9.541.4, CHA. 9. 541.4.VH, CHA. 9. 541. 4.VL, CHA. 9. 541. 4.HC, CHA. 9. 541. 4.LC, CHA. 9. 541. 4.H1, CHA. 9. 541. 4.H2, CHA. 9. 541. 4.H3; CHA.9.541.4.H4, CHA.9.541.4.H4(S241P), CHA. 9. 541. 4.vhCDR1, CHA. 9. 541. 4.vhCDR2, CHA. 9. 541. 4.vhCDR3, CHA. 9. 541. 4.vlCDR1, CHA. 9. 541. 4.vlCDR2 and CHA. 9.541. 4.vhCDR3;
CHA.9.541.5, CHA. 9. 541. 5.VH, CHA. 9. 541. 5.VL, CHA. 9. 541. 5.HC, CHA. 9. 541. 5.LC, CHA. 9. 541. 5.H1, CHA. 9. 541. 5.H2, CHA. 9. 541. 5.H3; CHA.9.541.5.H4, CHA.9.541.5.H4(S241P), CHA. 9. 541. 5.vhCDR1, CHA. 9. 541. 5.vhCDR2, CHA. 9. 541. 5.vhCDR3, CHA. 9. 541. 5.vlCDR1, CHA. 9. 541. 5.vlCDR2 and CHA. 9.541. 5.vhCDR3;
CHA.9.541.6, CHA. 9. 541. 6.VH, CHA. 9. 541. 6.VL, CHA. 9. 541. 6.HC, CHA. 9. 541. 6.LC, CHA. 9. 541. 6.H1, CHA. 9. 541. 6.H2, CHA. 9. 541.6.H3; CHA.9.541.6.H4, CHA.9.541.6.H4(S241P), CHA. 9. 541. 6.vhCDR1, CHA. 9. 541. 6.vhCDR2, CHA. 9. 541. 6.vhCDR3, CHA. 9. 541. 6.vlCDR1, CHA. 9. 541. 6.vlCDR2 and CHA. 9.541. 6.vhCDR3;
CHA.9.541.7, CHA. 9. 541. 7.VH, CHA. 9. 541. 7.VL, CHA. 9. 541. 7.HC, CHA. 9. 541. 7.LC, CHA. 9. 541. 7.H1, CHA. 9. 541. 7.H2, CHA. 9. 541. 7.H3; CHA.9.541.7.H4, CHA.9.541.7.H4(S241P), CHA. 9. 541. 7.vhCDR1, CHA. 9. 541. 7.vhCDR2, CHA. 9. 541. 7.vhCDR3, CHA. 9. 541. 7.vlCDR1, CHA. 9. 541. 7.vlCDR2 and CHA. 9.541. 7.vhCDR3; and
CHA.9.541.8, CHA. 9. 541. 8.VH, CHA. 9. 541. 8.VL, CHA. 9. 541. 8.HC, CHA. 9. 541. 8.LC, CHA. 9. 541. 8.H1, CHA. 9. 541. 8.H2, CHA. 9. 541. 8.H3; CHA.9.541.8.H4, CHA.9.541.8.H4(S241P); CHA. 9. 541. 8vhCDR1, CHA. 9. 541. 8.vhCDR2, CHA. 9. 541. 8.vhCDR3, CHA. 9. 541. 8.vlCDR1, CHA. 9. 541. 8.vlCDR2 and CHA. 9.541. 8.vhCDR3.

In the case of scFvs comprising the CDRs of the antibodies above, these are labeled as scFvs that include a scFv comprising a variable heavy domain with the vhCDRs, a linker and a variable light domain with the vlCDRs, again as above in either orientation. Thus the invention includes scFv-CHA.9.536.3.1, scFv-CHA.9.536.3, scFv-CHA.9.536.4, scFv-CHA.9.536.5, scFv-CHA.9.536.7, scFv-CHA.9.536.8, scFv-CHA.9.560.1, scFv-CHA.9.560.3, scFv-CHA.9.560.4, scFv-CHA.9.560.5, scFv-CHA.9.560.6, scFv-CHA.9.560.7, scFv-CHA.9.560.8, scFv-CHA.9.546.1, scFv-CHA.9.547.1, scFv-CHA.9.547.2, scFv-CHA.9.547.3, scFv-CHA.9.547.4, scFv-CHA.9.547.6, scFv-CHA.9.547.7, scFv-CHA.9.547.8, scFv-CHA.9.547.9, scFv-CHA.9.547.13, scFv-CHA.9.541.1, scFv-CHA.9.541.3, scFv-CHA.9.541.4, scFv-CHA.9.541.5, scFv-CHA.9.541.6, scFv-CHA.9.541.7 and scFv-CHA.9.541.8.

In addition, CHA.9.543 binds to TIGIT but does not block the TIGIT-PVR interaction.

As discussed herein, the invention further provides variants of the above components (CPA and CHA), including variants in the CDRs, as outlined above. Thus, the invention provides antibodies comprising a set of 6 CDRs as outlined herein that can contain one, two or three amino acid differences in the set of CDRs, as long as the antibody still binds to TIGIT. Suitable assays for testing whether an anti-TIGIT antibody that contains mutations as compared to the CDR sequences outlined herein are known in the art, such as Biacore assays.

In addition, the invention further provides variants of the above variable heavy and light chains. In this case, the variable heavy chains can be 80%, 90%, 95%, 98% or 99% identical to the "VH" sequences herein, and/or contain from 1, 2, 3, 4, 5, 6, 7, 8, 9, 10 amino acid changes, or more, when Fc variants are used. Variable light chains are provided that can be 80%, 90%, 95%, 98% or 99% identical to the "VL" sequences herein (and in particular CPA.9.086), and/or contain from 1, 2, 3, 4, 5, 6, 7, 8, 9, 10 amino acid changes, or more, when Fc variants are used. In these embodiments, the invention includes these variants as long as the antibody still binds to TIGIT. Suitable assays for testing whether an anti-TIGIT antibody that contains mutations as compared to the CDR sequences outlined herein are known in the art, such as Biacore assays.

Similarly, heavy and light chains are provided that are 80%, 90%, 95%, 98% or 99% identical to the full length "HC" and "LC" sequences herein (and in particular CPA.9.086), and/or contain from 1, 2, 3, 4, 5, 6, 7, 8, 9, 10 amino acid changes, or more, when Fc variants are used. In these embodiments, the invention includes these variants as long as the antibody still binds to TIGIT. Suitable assays for testing whether an anti-TIGIT antibody that contains mutations as compared to the CDR sequences outlined herein are known in the art, such as Biacore assays.

In addition, the framework regions of the variable heavy and variable light chains of either the CPA or CHA antibodies herein can be humanized (or, in the case of the CHA antibodies, "rehumanized", to the extent that alternative humanization methods can be done) as is known in the art (with occasional variants generated in the CDRs as needed), and thus humanized variants of the VH and VL chains of Figure 13 can be generated (and in particular CPA.9.086). Furthermore, the humanized variable heavy and light domains can then be fused with human constant regions, such as the constant regions from IgG1, IgG2, IgG3 and IgG4 (including IgG4(S241P)).

In particular, as is known in the art, murine VH and VL chains can be humanized as is known in the art, for example, using the IgBLAST program of the NCBI website, as outlined in Ye et al. Nucleic Acids Res. 41:W34-W40 (2013), herein incorporated by reference in its entirety for the humanization methods. IgBLAST takes a murine VH and/or VL sequence and compares it to a library of known human germline sequences. As shown herein, for the humanized sequences generated herein, the databases used were IMGT human VH genes (F+ORF, 273 germline sequences) and IMGT human VL kappa genes (F+ORF, 74 germline sequences). An exemplary five CHA sequences were chosen: CHA.9.536, CHA9.560, CHA.9.546, CHA.9.547 and CHA.9.541 (see Figure 13). For this embodiment of the humanization, human germline IGHV1-46(allele1) was chosen for all 5 as the acceptor sequence and the human heavy chain IGHJ4(allele1) joining region (J gene). For three of four (CHA.7.518, CHA.7.530, CHA.7.538_1 and CHA.7.538_2), human germline IGKV 1-39(allele 1) was chosen as the acceptor sequence and human light chain IGKJ2(allele1) (J gene) was chosen. The J gene was chosen from human joining region sequences compiled at IMGT^{®} the international ImMunoGeneTics information system as www.imgt.org. CDRs were defined according to the AbM definition (see www.bioinfo.org.uk/abs/).

In some embodiments, the anti-TIGIT antibodies of the present invention include anti-TIGIT antibodies wherein the V_{H} and V_{L} sequences of different anti-TIGIT antibodies can be "mixed and matched" to create other anti-TIGIT antibodies. TIGIT binding of such "mixed and matched" antibodies can be tested using the binding assays described above. e.g., ELISAs or Biacore assays). In some embodiments, when V_{H} and V_{L} chains are mixed and matched, a V_{H} sequence from a particular V_{H}/V_{L} pairing is replaced with a structurally similar V_{H} sequence. Likewise, in some embodiments, a V_{L} sequence from a particular V_{H}/V_{L} pairing is replaced with a structurally similar V_{L} sequence. For example, the V_{H} and V_{L} sequences of homologous antibodies are particularly amenable for mixing and matching.

Accordingly, the TIGIT antibodies of the invention comprise CDR amino acid sequences selected from the group consisting of (a) sequences as listed herein; (b) sequences that differ from those CDR amino acid sequences specified in (a) by 1, 2, 3, 4, 5, 6, 7, 8, 9, 10 or more amino acid substitutions; (c) amino acid sequences having 90% or greater, 95% or greater, 98% or greater, or 99% or greater sequence identity to the sequences specified in (a) or (b); (d) a polypeptide having an amino acid sequence encoded by a polynucleotide having a nucleic acid sequence encoding the amino acids as listed herein. In particular, the CPA.9.086 antibody can have sequences selected from (a), (b), (c) or (d).

Additionally included in the definition of TIGIT antibodies are antibodies that share identity to the TIGIT antibodies enumerated herein. That is, in certain embodiments, an anti-TIGIT antibody according to the invention comprises heavy and light chain variable regions comprising amino acid sequences that are identical to all or part of the anti-TIGIT amino acid sequences of preferred anti-TIGIT antibodies, respectively, wherein the antibodies retain the desired functional properties of the parent anti-TIGIT antibodies. The percent identity between the two sequences is a function of the number of identical positions shared by the sequences (i.e., % homology=# of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

In general, the percentage identity for comparison between TIGIT antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-TIGIT antibodies of the invention are those that share identity along the entire variable region (for example, where the identity is 95 or 98% identical along the variable regions), or along the entire constant region, or along just the Fc domain. In particular, the invention provides TIGIT antibodies that have at least 75%, at least 80%, at least 90%, with at least about 95, 96, 97, 98 or 99% percent identity being preferred, with the CPA.9.086 antibody.

In addition, also included are sequences that may have the identical CDRs but changes in the framework portions of the variable domain (or entire heavy or light chain). For example, TIGIT antibodies include those with CDRs identical to those shown in Figure 13 but whose identity along the variable region can be lower, for example 95 or 98% percent identical. In particular, the invention provides TIGIT antibodies that have identical CDRs to CPA.9.086 but with framework regions that are 95 or 98% identical to CPA.9.086.

### 1. TIGIT Antibodies That Compete For Binding

The present invention provides not only the enumerated antibodies but additional antibodies that compete with the enumerated antibodies (the CPA numbers enumerated herein that specifically bind to TIGIT) to specifically bind to the TIGIT molecule. The TIGIT antibodies of the invention "bin" into different epitope bins. Among the 44 TIGIT antibodies in the epitope binning study, there are four communities, each having related pairwise blocking patterns, which separate into 12 total discrete bins outlined herein. There are twelve discrete bins outlined herein; 1) BM9-H4, CHA.9.525, CPA.9.081-H4, CHA.9.538, CHA.9.553, CPA.9.069-H4, CHA.9.543, CHA.9.556, CPA.9.077-H4 and CHA.9.561; 2) CHA.9.560 andCHA.9.528; 3) CHA.9.552, CHA.9.521, CHA.9.541, CHA.9.529, CHA.9.519, CHA.9.527 and CHA.9.549;4) CPA.9.057-H4 and CHA.9.554; 5) CHA.9.546, CPA.9.012-H4, CHA.9.547, CPA.9.013-H4, CPA.9.018-H4, MBSA43-M1, Sino PVR-Fc(ligand), CHA.9.555, PVR-Fc M2A(ligand), BM29-H4, CPA.9.027-H4, CPA.9.049-H4 and CPA.9.053-H4; 6) CPA.9.064-H4; 7) BM26-H4; 8) CPA.9.059-H4; 9) CHA.9.535 and CPA.9.009-H4; 10) CHA.9.536, CHA.9.522 and CPA.9.015-H4; 11) CPA.9.011-H4 and BM8-H4 and 12) CPA.9.071-H4.

Thus, the invention provides anti-TIGIT antibodies that compete for binding with antibodies that are in discrete epitope bins 1 to 12. In a particular embodiment, the invention provides anti-TIGIT antibodies that compete for binding with CPA.9.086 and are at least 95, 96, 97, 98, or 99% identical to CPA.9.086.

Additional antibodies that compete with the enumerated antibodies are generated, as is known in the art and generally outlined below. Competitive binding studies can be done as is known in the art, generally using SPR/Biacore^{®} binding assays, as well as ELISA and cell-based assays.

### E. Optional Antibody Engineering

The anti-PVRIG antibodies (e.g., anti-PVRIG antibodies including those with CDRs identical to those shown in Figure 3) or the anti-TIGIT antibodies (e.g., anti-TIGIT antibodies including those with CDRs identical to those shown in Figure 13) of the invention can be modified, or engineered, to alter the amino acid sequences by amino acid substitutions.

By "amino acid substitution" or "substitution" herein is meant the replacement of an amino acid at a particular position in a parent polypeptide sequence with a different amino acid. In particular, in some embodiments, the substitution is to an amino acid that is not naturally occurring at the particular position, either not naturally occurring within the organism or in any organism. For example, the substitution E272Y refers to a variant polypeptide, in this case an Fc variant, in which the glutamic acid at position 272 is replaced with tyrosine. For clarity, a protein which has been engineered to change the nucleic acid coding sequence but not change the starting amino acid (for example exchanging CGG (encoding arginine) to CGA (still encoding arginine) to increase host organism expression levels) is not an "amino acid substitution"; that is, despite the creation of a new gene encoding the same protein, if the protein has the same amino acid at the particular position that it started with, it is not an amino acid substitution.

As discussed herein, amino acid substitutions can be made to alter the affinity of the CDRs for the PVRIG protein or the TIGIT protein (including both increasing and decreasing binding, as is more fully outlined below), as well as to alter additional functional properties of the antibodies. For example, the antibodies may be engineered to include modifications within the Fc region, typically to alter one or more functional properties of the antibody, such as serum half-life, complement fixation, Fc receptor binding, and/or antigen-dependent cellular cytotoxicity. Furthermore, an antibody according to at least some embodiments of the invention may be chemically modified (e.g., one or more chemical moieties can be attached to the antibody) or be modified to alter its glycosylation, again to alter one or more functional properties of the antibody. Such embodiments are described further below. The numbering of residues in the Fc region is that of the EU index of Kabat.

In one embodiment, the hinge region of C_{H1} is modified such that the number of cysteine residues in the hinge region is altered, e.g., increased or decreased. This approach is described further in U.S. Pat. No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired *Staphylococcyl* protein A (SpA) binding relative to native Fc-hinge domain SpA binding. This approach is described in further detail in U.S. Pat. No. 6,165,745 by Ward et al.

In some embodiments, amino acid substitutions can be made in the Fc region, in general for altering binding to FcγR receptors. By "Fc gamma receptor", "FcγR" or "FcgammaR" as used herein is meant any member of the family of proteins that bind the IgG antibody Fc region and is encoded by an FcγR gene. In humans this family includes but is not limited to FcγRI (CD64), including isoforms FcγRIa, FcγRIb, and FcγRIc; FcγRII (CD32), including isoforms FcγRIIa (including allotypes H131 and R131), FcγRIIb (including FcγRIIb-1 and FcγRIIb-2), and FcγRIIc; and FcγRIII (CD16), including isoforms FcγRIIIa (including allotypes V158 and F158) and FcγRIIIb (including allotypes FcγRIIIb-NA1 and FcyRIIIb-NA2) (Jefferis et al., 2002, Immunol Lett 82:57-65, entirely incorporated by reference), as well as any undiscovered human FcγRs or FcγR isoforms or allotypes. An FcγR may be from any organism, including but not limited to humans, mice, rats, rabbits, and monkeys. Mouse FcγRs include but are not limited to FcγRI (CD64), FcγRII (CD32), FcγRIII-1 (CD16), and FcγRIII-2 (CD16-2), as well as any undiscovered mouse FcγRs or FcγR isoforms or allotypes.

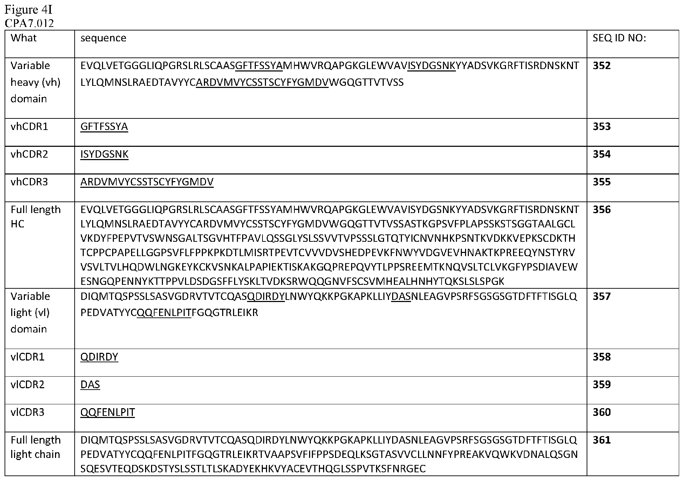

There are a number of useful Fc substitutions that can be made to alter binding to one or more of the FcγR receptors. Substitutions that result in increased binding as well as decreased binding can be useful. For example, it is known that increased binding to FcγRIIIa generally results in increased ADCC (antibody dependent cell-mediated cytotoxicity; the cell-mediated reaction wherein nonspecific cytotoxic cells that express FcγRs recognize bound antibody on a target cell and subsequently cause lysis of the target cell. Similarly, decreased binding to FcγRIIb (an inhibitory receptor) can be beneficial as well in some circumstances. Amino acid substitutions that find use in the present invention include those listed in U.S. Ser. Nos. 11/124,620 (particularly FIG. 4I) and U.S. Patent No. 6,737,056, both of which are expressly incorporated herein by reference in their entirety and specifically for the variants disclosed therein. Particular variants that find use include, but are not limited to, 236A, 239D, 239E, 332E, 332D, 239D/332E, 267D, 267E, 328F, 267E/328F, 236A/332E, 239D/332E/330Y, 239D, 332E/330L, 299T and 297N.

In addition, the antibodies of the invention are modified to increase its biological half-life. Various approaches are possible. For example, one or more of the following mutations can be introduced: T252L, T254S, T256F, as described in U.S. Pat. No. 6,277,375 to Ward. Alternatively, to increase the biological half-life, the antibody can be altered within the C_{H1} or C_{L} region to contain a salvage receptor binding epitope taken from two loops of a CH2 domain of an Fc region of an IgG, as described in U.S. Pat. Nos. 5,869,046 and 6,121,022 by Presta et al. Additional mutations to increase serum half-life are disclosed in U.S. Patent Nos. 8,883,973, 6,737,056 and 7,371,826, and include 428L, 434A, 434S, and 428L/434S.

In yet other embodiments, the Fc region is altered by replacing at least one amino acid residue with a different amino acid residue to alter the effector functions of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another example, one or more amino acids selected from amino acid residues 329, 331 and 322 can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another example, one or more amino acid residues within amino acid positions 231 and 239 are altered to thereby alter the ability of the antibody to fix complement. This approach is described further in PCT Publication WO 94/29351 by Bodmer et al.

In yet another example, the Fc region is modified to increase the ability of the antibody to mediate antibody dependent cellular cytotoxicity (ADCC) and/or to increase the affinity of the antibody for an Fcy receptor by modifying one or more amino acids at the following positions: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Moreover, the binding sites on human IgG1 for FcyRI, FcγRII, FcγRIII and FcRn have been mapped and variants with improved binding have been described (see Shields, R. L. et al. (2001) J. Biol. Chem. 276:6591-6604). Specific mutations at positions 256, 290, 298, 333, 334 and 339 are shown to improve binding to FcyRIII. Additionally, the following combination mutants are shown to improve FcyRIII binding: T256A/S298A, S298A/E333A, S298A/K224A and S298A/E333A/K334A. Furthermore, mutations such as M252Y/S254T/T256E or M428L/N434S improve binding to FcRn and increase antibody circulation half-life (see Chan CA and Carter PJ (2010) Nature Rev Immunol 10:301-316).

In still another embodiment, the antibody can be modified to abrogate *in vivo* Fab arm exchange. Specifically, this process involves the exchange of IgG4 half-molecules (one heavy chain plus one light chain) between other IgG4 antibodies that effectively results in bispecific antibodies which are functionally monovalent. Mutations to the hinge region and constant domains of the heavy chain can abrogate this exchange (*see,* Aalberse, RC, Schuurman J., 2002, Immunology 105:9-19).

In still another embodiment, the glycosylation of an antibody is modified. For example, an aglycosylated antibody can be made (*i.e.,* the antibody lacks glycosylation). Glycosylation can be altered to, for example, increase the affinity of the antibody for antigen or reduce effector function such as ADCC. Such carbohydrate modifications can be accomplished by, for example, altering one or more sites of glycosylation within the antibody sequence, for example N297. For example, one or more amino acid substitutions can be made that result in elimination of one or more variable region framework glycosylation sites to thereby eliminate glycosylation at that site.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies according to at least some embodiments of the invention to thereby produce an antibody with altered glycosylation. For example, the cell lines Ms704, Ms705, and Ms709 lack the fucosyltransferase gene, FUT8 (α (1,6) fucosyltransferase), such that antibodies expressed in the Ms704, Ms705, and Ms709 cell lines lack fucose on their carbohydrates. The Ms704, Ms705, and Ms709 FUT8 cell lines are created by the targeted disruption of the FUT8 gene in CHO/DG44 cells using two replacement vectors (see U.S. Patent Publication No. 20040110704 by Yamane et al. and Yamane-Ohnuki et al. (2004) Biotechnol Bioeng 87:614-22). As another example, EP 1,176,195 by Hanai et al. describes a cell line with a functionally disrupted FUT8 gene, which encodes a fucosyl transferase, such that antibodies expressed in such a cell line exhibit hypofucosylation by reducing or eliminating the α 1,6 bond-related enzyme. Hanai et al. also describe cell lines which have a low enzyme activity for adding fucose to the N-acetylglucosamine that binds to the Fc region of the antibody or does not have the enzyme activity, for example the rat myeloma cell line YB2/0 (ATCC CRL 1662). PCT Publication WO 03/035835 by Presta describes a variant CHO cell line, Lec13 cells, with reduced ability to attach fucose to Asn(297)-linked carbohydrates, also resulting in hypofucosylation of antibodies expressed in that host cell (see also Shields, R. L. et al. (2002) J. Biol. Chem. 277:26733-26740). PCT Publication WO 99/54342 by Umana et al. describes cell lines engineered to express glycoprotein-modifying glycosyl transferases (*e.g.,* β(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180). Alternatively, the fucose residues of the antibody may be cleaved off using a fucosidase enzyme. For example, the fucosidase α-L-fucosidase removes fucosyl residues from antibodies (Tarentino, A. L. et al. (1975) Biochem. 14:5516-23).

Another modification of the antibodies herein that is contemplated by the invention is pegylation or the addition of other water soluble moieties, typically polymers, *e.g.,* in order to enhance half-life. An antibody can be pegylated to, for example, increase the biological (*e.g.,* serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C₁-C₁₀) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies according to at least some embodiments of the invention. See for example, EP 0 154 316 by Nishimura et al. and EP 0 401 384 by Ishikawa et al.

In addition to substitutions made to alter binding affinity to FcγRs and/or FcRn and/or increase in vivo serum half-life, additional antibody modifications can be made, as described in further detail below.

In some cases, affinity maturation is done. Amino acid modifications in the CDRs are sometimes referred to as "affinity maturation". An "affinity matured" antibody is one having one or more alteration(s) in one or more CDRs which results in an improvement in the affinity of the antibody for antigen, compared to a parent antibody which does not possess those alteration(s). In some cases, although rare, it may be desirable to decrease the affinity of an antibody to its antigen, but this is generally not preferred.

In some embodiments, one or more amino acid modifications are made in one or more of the CDRs of the PVRIG antibodies of the invention. In general, only 1 or 2 or 3-amino acids are substituted in any single CDR, and generally no more than from 1, 2, 3. 4, 5, 6, 7, 8 9 or 10 changes are made within a set of CDRs. However, it should be appreciated that any combination of no substitutions, 1, 2 or 3 substitutions in any CDR can be independently and optionally combined with any other substitution.

Affinity maturation can be done to increase the binding affinity of the antibody for the PVRIG antigen by at least about 10% to 50-100-150% or more, or from 1 to 5 fold as compared to the "parent" antibody. Preferred affinity matured antibodies will have nanomolar or even picomolar affinities for the PVRIG antigen. Affinity matured antibodies are produced by known procedures. See, for example, Marks et al., 1992, Biotechnology 10:779-783 that describes affinity maturation by variable heavy chain (VH) and variable light chain (VL) domain shuffling. Random mutagenesis of CDR and/or framework residues is described in: Barbas, et al. 1994, Proc. Nat. Acad. Sci, USA 91:3809-3813; Shier et al., 1995, Gene 169:147-155; Yelton et al., 1995, J. Immunol. 155:1994-2004; Jackson et al., 1995, J. Immunol. 154(7):3310-9; and Hawkins et al, 1992, J. Mol. Biol. 226:889-896, for example.

In some embodiments, one or more amino acid modifications are made in one or more of the CDRs of the TIGIT antibodies of the invention. In general, only 1 or 2 or 3-amino acids are substituted in any single CDR, and generally no more than from 1, 2, 3. 4, 5, 6, 7, 8 9 or 10 changes are made within a set of CDRs. However, it should be appreciated that any combination of no substitutions, 1, 2 or 3 substitutions in any CDR can be independently and optionally combined with any other substitution.

Affinity maturation can be done to increase the binding affinity of the antibody for the TIGIT antigen by at least about 10% to 50-100-150% or more, or from 1 to 5 fold as compared to the "parent" antibody. Preferred affinity matured antibodies will have nanomolar or even picomolar affinities for the TIGIT antigen. Affinity matured antibodies are produced by known procedures. See, for example, Marks et al., 1992, Biotechnology 10:779-783 that describes affinity maturation by variable heavy chain (VH) and variable light chain (VL) domain shuffling. Random mutagenesis of CDR and/or framework residues is described in: Barbas, et al. 1994, Proc. Nat. Acad. Sci, USA 91:3809-3813; Shier et al., 1995, Gene 169:147-155; Yelton et al., 1995, J. Immunol. 155:1994-2004; Jackson et al., 1995, J. Immunol. 154(7):3310-9; and Hawkins et al, 1992, J. Mol. Biol. 226:889-896, for example.

Alternatively, amino acid modifications can be made in one or more of the CDRs of the antibodies of the invention that are "silent", *e.g.,* that do not significantly alter the affinity of the antibody for the antigen. These can be made for a number of reasons, including optimizing expression (as can be done for the nucleic acids encoding the antibodies of the invention).

Thus, included within the definition of the CDRs and antibodies of the invention are variant CDRs and antibodies; that is, the antibodies of the invention can include amino acid modifications in one or more of the CDRs of the enumerated antibodies of the invention. In addition, as outlined below, amino acid modifications can also independently and optionally be made in any region outside the CDRs, including framework and constant regions.

### V. PVRIG TREATMENT ANTIBODIES

The present invention provides anti-PVRIG antibodies for treatments purposes and uses. (For convenience, "anti-PVRIG antibodies" and "PVRIG antibodies" are used interchangeably). The anti-PVRIG antibodies of the invention specifically bind to human PVRIG, and preferably the ECD of human PVRIG, as depicted in Figure 3, including, e.g., anti-PVRIG antibodies including those with CDRs identical to those shown in Figure 3.

Specific binding for PVRIG or a PVRIG epitope can be exhibited, for example, by an antibody having a KD of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the PVRIG antigen or epitope.

However, as shown in the Examples, for optimal binding to PVRIG expressed on the surface of NK and T-cells, the antibodies preferably have a KD less 50 nM and most preferably less than 1 nM, with less than 0.1 nM and less than 1 pM and 0.1 pM finding use in the methods of the invention.

Also, specific binding for a particular antigen or an epitope can be exhibited, for example, by an antibody having a KA or Ka for a PVRIG antigen or epitope of at least 20-, 50-, 100-, 500-, 1000-, 5,000-, 10,000- or more times greater for the epitope relative to a control, where KA or Ka refers to an association rate of a particular antibody-antigen interaction. s

In some embodiments, the anti-PVRIG antibodies of the invention bind to human PVRIG with a K_{D} of 100 nM or less, 50 nM or less, 10 nM or less, or 1 nM or less (that is, higher binding affinity), or 1pM or less, wherein K_{D} is determined by known methods, *e.g.,* surface plasmon resonance (SPR, *e.g.,* Biacore assays), ELISA, KINEXA, and most typically SPR at 25° or 37° C.

The invention provides antigen binding domains, including full length antibodies, which contain a number of specific, enumerated sets of 6 CDRs, as provided in Figure 3. The invention provides antigen binding domains, including full length antibodies, which contain a number of specific, enumerated sets of 6 CDRs, as provided in Figure 3.

The invention further provides variable heavy and light domains as well as full length heavy and light chains.

As discussed herein, the invention further provides variants of the above components, including variants in the CDRs, as outlined above. In addition, variable heavy chains can be at least 80%, at least 90%, at least 95%, at least 98% or at least 99% identical to the "VH" sequences herein, and/or contain from 1, 2, 3, 4, 5, 6, 7, 8, 9, 10 amino acid changes, or more, when Fc variants are used. Variable light chains are provided that can be at least 80%, at least 90%, at least 95%, at least 98% or at least 99% identical to the "VL" sequences herein, and/or contain from 1, 2, 3, 4, 5, 6, 7, 8, 9, 10 amino acid changes, or more, when Fc variants are used. Similarly, heavy and light chains are provided that are at least 80%, at least 90%, at least 95%, at least 98% or at least 99% identical to the "HC" and "LC" sequences herein, and/or contain from 1, 2, 3, 4, 5, 6, 7, 8, 9, 10 amino acid changes, or more, when Fc variants are used.

Accordingly, the present invention provides antibodies, usually full length or scFv domains, that comprise the following CHA sets of CDRs, the sequences of which are shown in Figure 3:
CHA.7.518.1.H4(S241P)vhCDR1, CHA.7.518.1.H4(S241P)vhCDR2,
CHA.7.518.1.H4(S241P)vhCDR3, CHA.7.518.1.H4(S241P)vlCDR1,
CHA.7.518.1.H4(S241P)vlCDR2, and CHA.7.518.1.H4(S241P)vlCDR3.

In addition, the framework regions of the variable heavy and variable light chains can be humanized as is known in the art (with occasional variants generated in the CDRs as needed), and thus humanized variants of the VH and VL chains of Figure 3 can be generated. Furthermore, the humanized variable heavy and light domains can then be fused with human constant regions, such as the constant regions from IgG1, IgG2, IgG3 and IgG4.

In addition, also included are sequences that may have the identical CDRs but changes in the variable domain (or entire heavy or light chain). For example, PVRIG antibodies include those with CDRs identical to those shown in Figure 3 or Figures 5A-5D but whose identity along the variable region can be lower, for example 95 or 98% percent identical. For example, PVRIG antibodies include those with CDRs identical to those shown in Figure 3 but whose identity along the variable region can be lower, for example 95 or 98% percent identical, and in some embodiments at least 95% or at least 98%.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e.g.,* XBLAST and NBLAST) can be used.

In general, the percentage identity for comparison between PVRIG antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-PVRIG antibodies of the invention are those that share identity along the entire variable region (for example, where the identity is 95 or 98% identical along the variable regions, and in some embodiments at least 95% or at least 98%), or along the entire constant region, or along just the Fc domain.

### VI. TIGIT TREATMENT ANTIBODIES

The present invention provides anti-TIGIT antibodies for treatments purposes and uses. (For convenience, "anti- TIGIT antibodies" and "TIGIT antibodies" are used interchangeably). The anti-TIGIT antibodies of the invention specifically bind to human TIGIT, and preferably the ECD of human TIGIT, as depicted in Figure 13, including, *e.g.,* anti-TIGIT antibodies including those with CDRs identical to those shown in Figure 13.

Specific binding for PVRIG or a PVRIG epitope can be exhibited, for example, by an antibody having a KD of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the TIGIT antigen or epitope.

Also, specific binding for a particular antigen or an epitope can be exhibited, for example, by an antibody having a KA or Ka for a TIGIT antigen or epitope of at least 20-, 50-, 100-, 500-, 1000-, 5,000-, 10,000- or more times greater for the epitope relative to a control, where KA or Ka refers to an association rate of a particular antibody-antigen interaction.

In some embodiments, the anti-TIGIT antibodies of the invention bind to human PVRIG with a K_{D} of 100 nM or less, 50 nM or less, 10 nM or less, or 1 nM or less (that is, higher binding affinity), or 1pM or less, wherein K_{D} is determined by known methods, *e.g.,* surface plasmon resonance (SPR, *e.g.,* Biacore assays), ELISA, KINEXA, and most typically SPR at 25° or 37° C.

The invention provides antigen binding domains, including full length antibodies, which contain a number of specific, enumerated sets of 6 CDRs, as provided in Figure 3. The invention provides antigen binding domains, including full length antibodies, which contain a number of specific, enumerated sets of 6 CDRs, as provided in Figure 13.

The invention further provides variable heavy and light domains as well as full length heavy and light chains.

As discussed herein, the invention further provides variants of the above components, including variants in the CDRs, as outlined above. In addition, variable heavy chains can be at least 80%, at least 90%, at least 95%, at least 98% or at least 99% identical to the "VH" sequences herein, and/or contain from 1, 2, 3, 4, 5, 6, 7, 8, 9, 10 amino acid changes, or more, when Fc variants are used. Variable light chains are provided that can be at least 80%, at least 90%, at least 95%, at least 98% or at least 99% identical to the "VL" sequences herein, and/or contain from 1, 2, 3, 4, 5, 6, 7, 8, 9, 10 amino acid changes, or more, when Fc variants are used. Similarly, heavy and light chains are provided that are at least 80%, at least 90%, at least 95%, at least 98% or at least 99% identical to the "HC" and "LC" sequences herein, and/or contain from 1, 2, 3, 4, 5, 6, 7, 8, 9, 10 amino acid changes, or more, when Fc variants are used.

Accordingly, the present invention provides antibodies, usually full length or scFv domains, that comprise the following CHA sets of CDRs, the sequences of which are shown in Figure 13:
CPA.9.086.H4(S241P)vhCDR1, CPA.9.086.H4(S241P)vhCDR2,
CPA.9.086.H4(S241P)vhCDR3, CPA.9.086.H4(S241P)vlCDR1,
CPA.9.086.H4(S241P)vlCDR2, and CPA.9.086.H4(S241P)vlCDR3.

In addition, the framework regions of the variable heavy and variable light chains can be humanized as is known in the art (with occasional variants generated in the CDRs as needed), and thus humanized variants of the VH and VL chains of Figure 13 can be generated. Furthermore, the humanized variable heavy and light domains can then be fused with human constant regions, such as the constant regions from IgG1, IgG2, IgG3 and IgG4.

In addition, also included are sequences that may have the identical CDRs but changes in the variable domain (or entire heavy or light chain). For example, PVRIG antibodies include those with CDRs identical to those shown in Figure 13 but whose identity along the variable region can be lower, for example 95 or 98% percent identical. For example, TIGIT antibodies include those with CDRs identical to those shown in Figure 13 but whose identity along the variable region can be lower, for example 95 or 98% percent identical, and in some embodiments at least 95% or at least 98%.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al. (1997) Nucleic Acids Res. 25(17):3389-3402. When utilizing BLAST and Gapped BLAST programs, the default parameters of the respective programs (*e.g.,* XBLAST and NBLAST) can be used.

In general, the percentage identity for comparison between PVRIG antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-PVRIG antibodies of the invention are those that share identity along the entire variable region (for example, where the identity is 95 or 98% identical along the variable regions, and in some embodiments at least 95% or at least 98%), or along the entire constant region, or along just the Fc domain.

### VII. IMMUNE CELL ENGAGER

As used herein, the term "immune cell engager" (sometimes referred to as an "engager") refers to a molecule, *e.g.,* a fusion polypeptide, which is capable of forming a link between an immune cell, *e.g.,* a T cell, a NK cell, a NKT cell, a B cell, a macrophage, a neutrophil, and a tumor cell; and activating the immune cell. Examples of engagers include, but are not limited to, bi-specific T cell engagers (BiTEs), bi-specific killer cell engagers (BiKEs), tri-specific killer cell engagers, or multi-specific killer cell engagers, or universal engagers compatible with multiple immune cell types. Further as used herein, an immune cell engager that binds to a T cell is referred to as a "T cell engager".

As used herein, the term "surface triggering receptor" refers to a receptor capable of triggering or initiating an immune response, *e.g.,* a cytotoxic response. Surface triggering receptors may be engineered, and may be expressed on effector cells, *e.g.,* a T cell, a NK cell, a NKT cell, a B cell, a macrophage, a dendritic cell, a neutrophil. In some embodiments, the surface triggering receptor facilitates bi- or multi-specific antibody engagement between the effector cells and specific target cell *e.g.,* a tumor cell, independent of the effector cell's natural receptors and cell types. By "universal", it is meant that the surface triggering receptor can be expressed in, and activate, any effector cells irrespective of the cell type, and all effector cells expressing the universal receptor can be coupled or linked to the engagers having the same epitope recognizable by the surface triggering receptor, regardless of the engager's tumor binding specificities. In some embodiments, engagers having the same tumor targeting specificity are used to couple with the universal surface triggering receptor. In some embodiments, engagers having different tumor targeting specificity are used to couple with the universal surface triggering receptor. As such, one or multiple effector cell types can be engaged to kill one specific type of tumor cells in some case, and to kill two or more types of tumors in some other cases. A surface triggering receptor generally comprises a co-stimulatory domain for effector cell activation and an anti-epitope that is specific to the epitope of an engager. A bi-specific engager is specific to the anti-epitope of a surface triggering receptor on one end and is specific to a tumor antigen on the other end.

In some embodiments, the present disclosures provide for a T cell engager that targets both a tumor cell and a T cell. In some embodiments, the T cell engager of the present disclosures is multispecific. In some embodiments, the T cell engager of the present disclosures is bispecific.

In some embodiments, the immune cell engager, including a T cell engager, comprises a tumor-targeting moiety. The tumor-targeting moiety can be an antibody molecule or a portion thereof such as an antigen binding domain, a receptor molecule (*e.g.,* a full length receptor, receptor fragment, or fusion thereof *(e.g.,* a receptor-Fc fusion)), or a ligand molecule *(e.g.,* a full length ligand, ligand fragment, or fusion thereof *(e.g.,* a ligand-Fc fusion)) that binds to the cancer antigen (e.g., the tumor and/or the stromal antigen). In embodiments, the tumor-targeting moiety specifically binds to the target tumor, *e.g.,* binds preferentially to the target tumor. For example, when the tumor-targeting moiety is an antigen binding domain, it binds to the cancer antigen (*e.g.,* the tumor antigen and/or the stromal antigen) with a dissociation constant of less than about 10 nM, and more typically, 10-100 pM.

In some embodiments, the engager comprises an immune cell-targeting moiety. The immune cell-targeting moiety can be an antibody molecule or a portion thereof such as an antigen binding domain, a receptor molecule (*e.g.,* a full length receptor, receptor fragment, or fusion thereof (*e.g.,* a receptor-Fc fusion)), or a ligand molecule (e.g., a full length ligand, ligand fragment, or fusion thereof *(e.g.,* a ligand-Fc fusion)) that binds to the immune cell antigen (*e.g.,* the T cell antigen, the NK cell antigen, the B cell antigen, the dendritic cell antigen, and/or the macrophage cell antigen). In embodiments, the immune cell-targeting moiety specifically binds to the target immune cell, *e.g.,* binds preferentially to the target immune cell. For example, when the immune cell-targeting moiety is an antigen binding domain, it binds to the immune cell antigen (*e.g.,* the T cell antigen, the NK cell antigen, the B cell antigen, the dendritic cell antigen, and/or the macrophage cell antigen) with a dissociation constant of less than about 10 nM, and more typically, 10-100 pM.

Examples of antibody formats for the engager include but are not limited to Bispecific T cell engager (BiTE), F(ab')2, F(ab')-ScFv2, di-scFv, diabody, minibody, scFv-Fc, DART, TandAb, ScDiabody, ScDiabody-CH3, Diabody-CH3, triple body, miniantibody, minibody, TriBi minibody, ScFv-CH3 KIH (knobs in holes), Fab-ScFv, SCFv-CH-CL-scFv, scFv-KIH, Fab-scFv-Fc, Tetravalent HCAb, scDiabody-Fc, Diabody-Fc, intrabody, dock and lock antibodies, ImmTAC, HSAbody, ScDiabody-HAS, humabody and Tandem ScFv-toxic (see, for example, Spiess et al., Molecular Immunology 67: 95-106 (2015)).

Without being bound by any theory, the multispecific molecules disclosed herein are expected to localize *(e.g.,* bridge) and/or activate an immune cell *(e.g.,* an immune effector cell chosen from an NK cell, a B cell, a dendritic cell or a macrophage), in the presence of the cancer cell. Increasing the proximity and/or activity of the immune cell, in the presence of the cancer cell, using the multispecific molecules described herein is expected to enhance an immune response against the target cancer cell, thereby providing a more effective cancer therapy. Accordingly, provided herein are, inter alia, multispecific molecules (*e.g.,* multispecific antibody molecules) that include the aforesaid moieties, nucleic acids encoding the same, methods of producing the aforesaid molecules, and methods of treating a cancer using the aforesaid molecules.

In some embodiments, bi- or multi-specific engagers of the present disclosures can be fusion proteins consisting of two or more single-chain variable fragments (scFvs) of different antibodies, with at least one scFv binds to an effector cell surface molecule, and at least another to a tumor cell via a tumor specific cell surface molecule. The exemplary effector cell surface molecules, or surface triggering receptor, that can be used for bi- or multispecific engager recognition, or coupling, include, but are not limited to, CD3, CD28, CD5, CD16, NKG2D, CD64, CD32, CD89, NKG2C, CD44v6, IL-12, PD-L1, Vγ9Vδ2, NKp46, BCMA and a chimeric Fc receptor as disclosed herein. In some embodiments, the CD16 expressed on the surface of effector cells for engager recognition is a hnCD16, comprising CD16 (containing F176V and optionally S197P) or CD64 extracellular domain, and native or non-native transmembrane, stimulatory and/or signaling domains as described in section I.2. In some embodiments, the CD16 expressed on the surface of effector cells for engager recognition is a hnCD16 based chimeric Fc receptor (CFcR). In some embodiments, the hnCD16 based CFcR comprises a transmembrane domain of NKG2D, a stimulatory domain of 2B4, and a signaling domain of CD3ζ; wherein the extracellular domain of the hnCD16 is derived from a full length or partial sequence of the extracellular domain of CD64 or CD16; and wherein the extracellular domain of CD16 comprises F176V and optionally S197P. The exemplary tumor specific cell surface molecules for bi- or multi-specific engager recognition include, but are not limited to, BCMA (B-cell maturation antigen), B7H3, CD10, CD19, CD20, CD22, CD24, CD30, CD33, CD34, CD38, CD44, CD79a, CD79b, CD123, CD138, CD179b, CEA, CLDN18.2, CLEC12A, CS-1, DLL3, EGFR, EGFRvIII, EGFRxFcγRI, EGFRvIIIxCD3, EGFRxCD3, EPCAM, FLT-3, FOLR1, FOLR3, GD2, gpA33, GPC3, HER2, HM1.24, LGR5, MSLN, MCSP, MICA/B, MUC 17 (mucin-17), PSMA, PAMA, P-cadherin, ROR1, PD-1, CLEC12A, WT1, EphA2, ADAM17, PSCA. In one embodiment, the bispecific antibody is CD3-CD19. In another embodiment, the bispecific antibody is CD16-CD30 or CD64-CD30. In another embodiment, the bispecific antibody is CD16-BCMA or CD64-BCMA. In still another embodiment, the bispecific antibody is CD3-CD33. In yet another embodiment, the bispecific antibody further comprises a linker between the effector cell and tumor cell antigen binding domains, for example, a modified IL15 as a linker for effector NK cells to facilitate effector cell expansion (called TriKE, or Trispecific Killer Engager, in some publications). In one embodiment, the TriKE is CD16-IL15-EPCAM or CD64-IL15-EPCAM. In another embodiment, the TriKE is CD16-IL15-CD33 or CD64-IL15-CD33. In yet another embodiment, the TriKE is NKG2C-IL15-CD33.

In some embodiments, bi-specific T cell engagers (BiTEs) provided herein are specific for the CD3ε subunit of the TCR complex of a T-cell and also a target an antigen of interest, such as a cancer antigen. Since BiTEs are specific for the TCR complex, this enables BiTEs to activate resident T cells to kill cells expressing a particular target antigen on their cell surface, for example cancer cells. An important property of BiTEs is their ability to make CD4+ and non-activated CD8+ T cells target cancer cells. In other words, T cells activated by BiTES can be made to kill cells independent of MHC expression on the cell surface. This is important because some tumor cells downregulate MHC which makes them resistant to agents such as CAR-T cells and immTACs.

In some embodiments, the BiTEs of the present disclosures include but is not limited to Blinatumomab (Blyncyto^{®}), Solitomab, Epcoritamab, Odronextamab, Mosunetuzumab, XmAb13676, Glofitamab, IGM-2323, AMG 330, AMG 673, AMG 420, AMG 701, Teclistamab, REGN5458, PF-06863135, TNB-383B, Cevostamab, scIgG, Talquetamab, Flotetuzumab, Pasotuxizumab, AMG 160, AMG 596, AMG 757, AMG 199, AMG 910, AMV564, Cibisatamab, M802, M701, ERY974, MGD007, ES414, Hu3F8-bsAb, ISB 1302, BTRC4017A, GEN1044, CAdDuo, CAdTrio, CD44v6, Vγ9Vδ2, NKp46, BI 836909, PD-L1, CLEC12A, WT1, bscEphA2xCD, A300E, PSCA, EGFRxFcγRI, EGFRvIIIxCD3, and EGFRxCD3.

In some embodiments, the mmune cell engager, including a T cell engager, of the present disclosure binds to CD3 and BCMA. In some embodiments, the mmune cell engager, including a T cell engager, is a bispecific antibody targeting CD3 and BCMA.

In some embodiments, the immune cell engager, including a T cell engager, is used alone for cancer treatment. In some embodiments, the immune cell engager, including a T cell engager, is used in combiantion with one or more thereapeutic modalities for cancer treatment. In some embodiments, the immune cell engager, including a T cell engager, is used in combination with one or more antibodies targeting an immune checkpoint receptor and function as immune checkpoint inhibitor antibodies. The immune checkpoint inhibitor antibodies of the present disclosure include but are not limited to anti-PVRIG antibodies, anti- PD-1 antibodies, anti-CTLA-4 antibodies, anti-PD-L1 antibodies, anti-LAG-3 antibodies, anti-TIM-3 antibodies, anti-BTLA antibodies, anti- DNAM1 antibodies, anti-ICOS antibodies, anti-4-1bb antibodies, anti-GITR antibodies, anti-OX40 antibodies, anti-CD96 antibodies, anti-B7-H4 antibodies, anti-B7-H3 antibodies, anti-VISTA antibodies, anti-CD27 antibodies, anti-CD40 antibodies, and/or anti-CD137 antibodies.

In some embodiments, the immune cell engager, including a T cell engager, is used in combination with an anti-PVRIG antibody. In some embodiments, the T immune cell engager, including a T cell engager, is used in combination with an anti-TIGIT antibody. In some embodiments, the immune cell engager, including a T cell engager, is used in combination with an anti-PVRIG antibody and an anti-TIGIT antibody. In some embodiments, a CD3/BCMA bispecific antibody is used in combination with an anti-PVRIG antibody and an anti-TIGIT antibody.

Exemplary disclosures of immune cell engager, including T cell engager, and their use thereof are provided in US Application Nos. 15/465,564, 15/518,922, 16/329,098, 16/765,369, 16/389,356, 17/369,831 and US Patent Nos. 11,267,897 and 10,301,391, each of which is incorporated herein by reference in its entirety.

Exemplary disclosures of immune cell engager, including T cell engager, and their use thereof are provided in Herrmann M et al., 2018, Porter CE et al., 2020, de Bruin RCG et al., 2017, Gauthier L et al., 2019, Pollyea and Jordan 2017, Zhao X *et al.,* 2010, Lapillonne H *et al.,* 2006, Dao T et al., 2015, Hipp S et al., 2017, Hammond SA *et al.,* 2007, Yamamoto K *et al.,* 2012 and Singh K, *et al.,* 2021 , each of which is incorporated herein by reference in its entirety.

### VIII. FORMULATIONS OF ANTI-PVRIG ANTIBODIES

The anti-PVRIG antibodies and/or antigen binding portions thereof compositions (e.g., anti-PVRIG antibodies including those with CDRs identical to those shown in Figure 3) can be formulated into pharmaceutical compositions comprising a carrier suitable for the desired delivery method. Suitable carriers include any material that when combined with the therapeutic composition retains the anti-tumor function of the therapeutic composition and is generally non-reactive with the patient's immune system. Examples include, but are not limited to, any of a number of standard pharmaceutical carriers such as sterile phosphate buffered saline solutions, bacteriostatic water, and the like (see, generally, Remington's Pharmaceutical Sciences 16th Edition, A. Osal., Ed., 1980). Acceptable carriers, excipients, or stabilizers are nontoxic to recipients at the dosages and concentrations employed, and include buffers such as phosphate, citrate, acetate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl orbenzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; sweeteners and other flavoring agents; fillers such as microcrystalline cellulose, lactose, corn and other starches; binding agents; additives; coloring agents; salt-forming counter-ions such as sodium; metal complexes (*e.g.,* Zn-protein complexes); and/or non-ionic surfactants such as TWEEN^{™}, PLURONICS^{™}, or polyethylene glycol (PEG).

In a preferred embodiment, the pharmaceutical composition that comprises anti-PVRIG antibodies including those with CDRs identical to those shown in Figure 3) of the invention may be in a water-soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The formulations to be used for in vivo administration are preferably sterile. This is readily accomplished by filtration through sterile filtration membranes or other methods.

As used herein, the term "activity" refers to a functional activity or activities of anti-PVRIG antibodies and/or antigen binding portions thereof. Functional activities include, but are not limited to, biological activity and/or binding affinity.

As used herein, the term "stability" is used in a structural context, e.g., relating to the structural integrity of an anti-PVRIG antibody and/or antigen binding portion thereof, or in a functional context, e.g., relating to a an anti-PVRIG antibody and/or antigen binding portion thereof 's ability to retain its function and/or activity over time (e.g., including anti-PVRIG antibody and/or antigen binding portion thereof stability or anti-PVRIG antibody and/or antigen binding portion thereof formulation stability, wherein the anti-PVRIG antibody includes those with CDRs identical to those shown in Figure 3). As will be appreciated, the anti-PVRIG antibody and/or antigen binding portion thereof under discussion may be contained within a formulation in accordance with the methods and compositions described herein, and the stability of that protein refers to its stability in that formulation. In some embodiments, the stability of an anti-PVRIG antibody and/or antigen binding portion thereof composition is determined by measuring the binding activity of the composition, including for example, using the assays described in the application and figures provided herewith, as well as other applicable assays known in the art. In some embodiments, the stability of an anti-PVRIG antibody and/or antigen binding portion thereof composition is formulated with sugar, sugar alcohol, and/or non-ionic surfactant, as described herein, is compared to an anti-PVRIG antibody and/or antigen binding portion thereof composition formulated without the at least one amino acid, salt, and/or non-ionic surfactant and/or with a different combination of components. In some embodiments, the formulation does not comprise a sugar and/or sugar alcohol.

As used herein, a "storage stable" aqueous an anti-PVRIG antibody and/or antigen binding portion thereof composition refers to an anti-PVRIG antibody and/or antigen binding portion thereof comprising solution that has been formulated to increase the stability of the protein in solution, for example by at least 10%, over a given storage time. In the context of the present disclosure, an anti-PVRIG antibody and/or antigen binding portion thereof can be made "storage stable" by the addition of at least one amino acid, salt, or non-ionic surfactant as a stabilizing agent. In some embodiments, the stability of the an anti-PVRIG antibody and/or antigen binding portion thereof in any given formulation can be measured, for example, by monitoring the formation of aggregates, loss of bulk binding activity, or formation of degradation products, over a period of time. The absolute stability of a formulation, and the stabilizing effects of the sugar, sugar alcohol, or non-ionic surfactant, will vary dependent upon the particular composition being stabilized. In one embodiment, the stability of an anti-PVRIG antibody and/or antigen binding portion thereof composition is determined by measuring the anti-PVRIG antibody and/or antigen binding portion thereof binding activity of the composition. For example, by using an ELISA or other binding activity assay. In one embodiment, the stability of an anti-PVRIG antibody and/or antigen binding portion thereof composition formulated with sugar, sugar alcohol, and/or non-ionic surfactant, as described herein, is compared to an anti-PVRIG antibody and/or antigen binding portion thereof composition formulated without the at least one amino acid, salt, and/or non-ionic surfactant and/or with a different combination of components. In some embodiments, the formulation does not comprise a sugar and/or sugar alcohol.

As used herein, "shelf-life" refers to the period of time a formulation maintains a predetermined level of stability at a predetermined temperature. In particular embodiments, the predetermined temperature refers to frozen *(e.g.,* -80°C, -25°C, 0°C), refrigerated *(e.g.,* 0° to 10°C), or room temperature *(e.g.,* 18°C to 32° C) storage.

As used herein, the term "time of stability" refers to the length of time a formulation is considered stable. For example, the time of stability for a formulation may refer to the length of time for which the level of protein aggregation and/or degradation in the formulation remains below a certain threshold (*e.g.,* 1 %, 2%, 3%, 4%, 5%, 6%, 7%, 8%, 9%, 10%, 1 1 %, 12%, 13%, 14%, 15%, 16%, 17%, 18%, 19%, 20%, etc.), and/or the length of time a formulation maintains biological activity above a certain threshold (*e.g.,* 100%, 95%, 90%, 85%, 80%, 75%, 70%, 65%, 60%, 55%, 50%, etc.) of the amount of activity (including, for example, binding activity) present in the formulation at the start of the storage period.

In the context of the present disclosure, a storage stable aqueous composition of a an anti-PVRIG antibody and/or antigen binding portion thereof formulated with a sugar, sugar alcohol, and/or non-ionic surfactant will have a longer time of stability than a composition of the same an anti-PVRIG antibody and/or antigen binding portion thereof formulated without the at least one amino acid, salt, and/or non-ionic surfactant. In some embodiments, a storage stable aqueous composition of an anti-PVRIG antibody and/or antigen binding portion thereof, will have a time of stability that is, for example, at least 10% greater than the time of stability for the an anti-PVRIG antibody and/or antigen binding portion thereof composition formulated in the absence of the at least one amino acid, salt, and/or non-ionic surfactant, or at least 15%, 20%, 25%, 30%, 35%, 40%, 45%, 50%, 55%, 60%, 65%, 70%, 75%, 80%, 85%, 90%, 95%, 100%, 1 10%, 120%, 130%, 140%, 150%, 160%, 170%, 180%, 190% greater, or at least 2 times greater, or at least 2.5 times, 3.0 times, 3.5 times, 4.0 times, 4.5 times, 5.0 times, 5.5 times, 6.0 times, 6.5 times, 7.0 times, 7.5 times, 8.0 times, 8.5 times, 9.0 times, 9.5 times, 10 times, or more times greater than the time of stability for the an anti-PVRIG antibody and/or antigen binding portion thereof composition formulated in the absence of the at least amino acid, salt, and/or non-ionic surfactant.

As used herein, "BDS" refers to "Bulk Drug Substance."

### A. Stabilized Liquid Formulations

In some embodiments, the present disclosure provides stabilized aqueous formulations of an anti-PVRIG antibody and/or antigen binding portion thereof (e.g., anti-PVRIG antibodies including those with CDRs identical to those shown in Figure 3). The following embodiments are based in part on the discovery that inclusion of at least one amino acid, salt, and/or non-ionic surfactant stabilizes the liquid anti-PVRIG antibody and/or antigen binding portion thereof compositions, as compared to compositions lacking the at least one amino acid, salt, and/or non-ionic surfactant. In some embodiments, the formulation does not comprise a sugar and/or sugar alcohol.

As will be recognized by one of skill in the art, an anti-PVRIG antibody and/or antigen binding portion thereof formulated according to the embodiments provided herein may contain, in addition to the components explicitly disclosed, counter ions contributed by the inclusion of solution components or pH modifying agents, for example, sodium or potassium contributed from an acetate salt, sodium hydroxide, or potassium hydroxide or chloride contributed by calcium chloride or hydrochloric acid. In the context of the present disclosure, a storage stable an anti-PVRIG antibody and/or antigen binding portion thereof composition consisting of or consisting essentially of a given formulation may further comprise one or more counter ion, as necessitated by the formulation process at a particular pH.

In one embodiment, a storage stable anti-PVRIG antibody and/or antigen binding portion provided herein will be stabilized at refrigerated temperature *(i.e.,* between 2°C and 10°C) for a period of time. For example, in one embodiment, a stable liquid pharmaceutical formulations comprising an anti-PVRIG antibody or antigen binding fragment thereof will be stable when stored at refrigerated temperature for at least 4 days. In other embodiments, the anti-PVRIG antibody and/or antigen binding portion composition will be stable at refrigerated temperature for at least 1, 2, 3, 4, 5, 6, 7, 8, 9, 10, 11, 12, 13, 14, 21, 28, or more days. In some embodiments, the anti-PVRIG antibody and/or antigen binding portion composition will be stable for at least 1 week, 2 weeks, 3 weeks, 4 weeks, 5 weeks, 6 weeks, 7 weeks, 8 weeks, 9 weeks, 10 weeks, 1 month, 3 months, 6 months, 9 months, 12 months, 18 months, 24 months, 30 months, 36 months, or 42 months or more. In some embodiments, the anti-PVRIG antibody and/or antigen binding portion composition will be stable for at least 1 month. In some embodiments, the composition will be stable for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or more months. In some embodiments, the anti-PVRIG antibody and/or antigen binding portion composition will be stable for an extended period of time when stored at a temperature between 2°C and 8°C.

In one embodiment, a stable liquid pharmaceutical formulations comprising an anti-PVRIG antibody or antigen binding fragment thereof provided herein will be stabilized at room temperature *(i.e.,* between 18°C and 32°C) for a period of time. For example, in one embodiment, a stable liquid pharmaceutical formulations comprising an anti-PVRIG antibody or antigen binding fragment thereof will be stable when stored at room temperature for at least 4 days. In some embodiments, the anti-PVRIG antibody and/or antigen binding portion composition will be stable at room temperature for at least 1, 2, 3, 4, 5, 6, 7, 8, 9, 10, 11, 12, 13, 14, 21, 28, or more days. In some embodiments, the anti-PVRIG antibody and/or antigen binding portion composition will be stable for at least 1 week, 2 weeks, 3 weeks, 4 weeks, 5 weeks, 6 weeks, 1 months, 3 months, 6 months, or 9 months, 12 months, 18 months, or 36 months or more. In some embodiments, the anti-PVRIG antibody and/or antigen binding portion composition will be stable for at least 1 month. In yet other embodiments, the composition will be stable for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or more months. In some embodiments, room temperature refers to between 20°C and 30°C, between 21°C and 29°C, between 22°C and 28°C, between 23°C and 27°C, between 24°C and 26°C, or about 25°C. In some embodiments, the anti-PVRIG antibody and/or antigen binding portion composition will be stable for an extended period of time when stored at a temperature between 20°C and 25°C. In some embodiments, the anti-PVRIG antibody and/or antigen binding portion composition will be stable for an extended period of time when stored at a temperature of about 25°C.

In one embodiment, a storage stable anti-PVRIG antibody and/or antigen binding portion provided herein will be stabilized at elevated temperature *(i.e.,* between 32°C and 42°C) for a period of time. For example, in one embodiment, a stable liquid pharmaceutical formulations comprising an anti-PVRIG antibody or antigen binding fragment thereof will be stable when stored at elevated temperature for at least 4 days. In some embodiments, the anti-PVRIG antibody and/or antigen binding portion composition will be stable at elevated temperature for at least 1, 2, 3, 4, 5, 6, 7, 8, 9, 10, 11, 12, 13, 14, 21, 28, or more days. In some embodiments, the anti-PVRIG antibody and/or antigen binding portion composition will be stable for at least 1 week, 2 weeks, 3 weeks, 4 weeks, 5 weeks, or more. In some embodiments, the anti-PVRIG antibody and/or antigen binding portion composition will be stable for at least 1 month. In yet other embodiments, the anti-PVRIG antibody and/or antigen binding portion composition will be stable for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or more months. In some embodiments, the anti-PVRIG antibody and/or antigen binding portion composition will be stable for an extended period of time when stored at a temperature between 35°C and 40°C.

In one embodiment, a stored anti-PVRIG antibody and/or antigen binding portion composition is considered storage stable as long as the composition maintains at least 40% of the antibody binding activity present at the start of the storage period *(e.g.,* at time = 0). In another embodiment, a stored composition is considered stable as long as the composition maintains at least 45%, 50%, 55%, 60%, 65%, 70%, 75%, 80%, 85%, 90%, 95% or more of the antibody binding activity present at the start of the storage period *(e.g.,* at time = 0). In one embodiment, antibody binding activity is measure using any assay known in the art.

In some embodiments, an anti-PVRIG antibody and/or antigen binding portion composition is considered to have been stabilized by the addition of a stabilizing agent *(e.g.,* at least one amino acid, salt, and/or non-ionic surfactant) when the anti-PVRIG antibody and/or antigen binding portion composition contains at least 10% more antibody binding activity after storage for a period of time, as compared to an anti-PVRIG antibody and/or antigen binding portion composition not containing the stabilizing agent or containing a lower amount of the stabilizing agent. In other embodiments, an anti-PVRIG antibody and/or antigen binding portion composition is considered to have been stabilized by the addition of a stabilizing agent (*e.g.,* at least one amino acid, salt, and/or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage more anti-PVRIG antibody and/or antigen binding portion activity after storage for a period of time, as compared to an anti-PVRIG antibody and/or antigen binding portion composition not containing the stabilizing agent or containing a lower amount of the stabilizing agent.

In one embodiment, a stored anti-PVRIG antibody and/or antigen binding portion composition is considered stable as long as the percentage of anti-PVRIG antibody and/or antigen binding portion present in an aggregated state remains no more than 50%. In some embodiments, a stored anti-PVRIG antibody and/or antigen binding portion thereof composition is considered stable as long as the percentage of the anti-PVRIG antibody and/or antigen binding portion thereof present in an aggregated state remains no more than 45%, 40%, 35%, 30%, 25%, 24%, 23%, 22%, 21 %, 20%, 19%, 18%, 17%, 16%, 15%, 14%, 13%, 12%, 1 1%, 10%, 9%, 8%, 7%, 6%, 5%, 4%, 3%, 2%, 1 %, or less.

In some embodiments, an anti-PVRIG antibody and/or antigen binding portion composition is considered to have been stabilized by the addition of a stabilizing agent (anti-PVRIG antibody and/or antigen binding portion composition, at least one amino acid, salt, and/or non-ionic surfactant) when the composition contains at least 10% less anti-PVRIG antibody and/or antigen binding portion present in an aggregated state after storage for a period of time, as compared to an anti-PVRIG antibody and/or antigen binding portion composition not containing the stabilizing agent or containing a lower amount of the stabilizing agent. In other embodiments, an anti-PVRIG antibody and/or antigen binding portion composition is considered to have been stabilized by the addition of a stabilizing agent (*e.g.,* at least one amino acid, salt, and/or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage less anti-PVRIG antibody and/or antigen binding portion present in an aggregated state after storage for a period of time, as compared to an anti-PVRIG antibody and/or antigen binding portion composition not containing the stabilizing agent or containing a lower amount of the stabilizing agent

In some embodiments, a stored anti-PVRIG antibody and/or antigen binding portion composition is considered stable as long as the composition maintains at least 40% of the starting binding activity (e.g., at time = 0) after being subjected to mechanical stress. In another embodiment, a stored composition is considered stable as long as the composition maintains 45%, 50%, 55%, 60%, 65%, 70%, 75%, 80%, 85%, 90%, 95% or more of the starting binding activity (*e.g.,* at time = 0) after being subjected to mechanical stress. In some embodiments, the mechanical stress is agitation (*e.g.,* shaking).

In some embodiments, an anti-PVRIG antibody and/or antigen binding portion composition is considered to have been stabilized by the addition of a stabilizing agent *(e.g.,* at least one amino acid, salt, or non-ionic surfactant) when the anti-PVRIG antibody and/or antigen binding portion composition contains at least 10% more binding activity after being subjected to mechanical stress, as compared to an anti-PVRIG antibody and/or antigen binding portion composition not containing the stabilizing agent or containing a lower amount of the stabilizing agent. In other embodiments, an anti-PVRIG antibody and/or antigen binding portion composition is considered to have been stabilized by the addition of a stabilizing agent (*e.g.,* a sugar, sugar alcohol, or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage more antibody activity after being subjected to mechanical stress, as compared to an anti-PVRIG antibody and/or antigen binding portion composition not containing the stabilizing agent or containing a lower amount of the stabilizing agent. In a specific embodiment, the mechanical stress is agitation (e.g., shaking).

In some embodiments, a stored anti-PVRIG antibody and/or antigen binding portion composition is considered stable as long as the percentage of anti-PVRIG antibody and/or antigen binding portion present in an aggregated state remains no more than 50% after being subjected to mechanical stress. In other embodiments, a stored anti-PVRIG antibody and/or antigen binding portion composition is considered stable as long as the percentage of anti-PVRIG antibody and/or antigen binding portion present in an aggregated state remains no more than 45%, 40%, 35%, 30%, 25%, 24%, 23%, 22%, 21 %, 20%, 19%, 18%, 17%, 16%, 15%, 14%, 13%, 12%, 11%, 10%, 9%, 8%, 7%, 6%, 5%, 4%, 3%, 2%, 1%, or less after being subjected to mechanical stress. In some embodiments, the mechanical stress is agitation (*e.g.,* shaking).

In some embodiments, an anti-PVRIG antibody and/or antigen binding portion composition is considered to have been stabilized by the addition of a stabilizing agent (*e.g.,* at least one amino acid, salt, or non-ionic surfactant) when the composition contains at least 10% less anti-PVRIG antibody and/or antigen binding portion present in an aggregated state after being subjected to mechanical stress, as compared to an anti-PVRIG antibody and/or antigen binding portion composition not containing the stabilizing agent or containing a lower amount of the stabilizing agent. In some embodiments, an anti-PVRIG antibody and/or antigen binding portion composition is considered to have been stabilized by the addition of a stabilizing agent (*e.g.,* at least one amino acid, salt, or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage less anti-PVRIG antibody and/or antigen binding portion present in an aggregated state after being subjected to mechanical stress, as compared to an anti-PVRIG antibody and/or antigen binding portion composition not containing the stabilizing agent or containing a lower amount of the stabilizing agent. In a specific embodiment, the mechanical stress is agitation (*e.g.,* shaking).

In some embodiments, the highly stabilized formulations of the invention have a shelf life of at least 6 months. As will be appreciated, this shelf life may be at frozen temperatures (*i.e.,* -80°C, -25°C, 0°C), refrigerated (0°C to 10°C), or room temperature (20°C to 32°C) in liquid or lyophilized form. I n further aspects, the highly stabilized formulations of the invention have a shelf life of at least 12, 18, 24, 30, 36, 42, 48, 54, or 60 months.

In some embodiments, shelf life is determined by a percent activity remaining after storage at any of the above temperatures for any of the above periods of time. In some embodiments, shelf life means that the formulation retains at least 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100% of antibody activity as measured by any of the assays described herein or known in the art as compared to activity prior to storage for any of the above amounts of time at any of the above temperatures.

In some embodiments, the present invention provides a stable liquid pharmaceutical formulation of an anti-PVRIG antibody comprising:
(a) an anti-PVRIG antibody, wherein the anti-PVRIG antibody comprises an antibody with CDRs identical to those shown in Figure 3;
(b) 25 mM histidine;
(c) 60 mM NaCl;
(d) 100 mM L-Arginine, and
(e) 0.01% w/v polysorbate 80,
wherein the composition has a pH from 5.5 to 7.0.

In some embodiments, the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL. In some embodiments, the anti-PVRIG antibody is at a concentration of from 10 mg/mL to 40 mg/mL. In some embodiments, the anti-PVRIG antibody is at a concentration of from 15 mg/mL to 40 mg/mL. In some embodiments, the anti-PVRIG antibody is at a concentration of from 15 mg/mL to 30 mg/mL. In some embodiments, the anti-PVRIG antibody is at a concentration of from 10 mg/mL to 25 mg/mL. In some embodiments, the anti-PVRIG antibody is at a concentration of from 15 mg/mL to 25 mg/mL. In some embodiments, the anti-PVRIG antibody is at a concentration of from 10 mg/mL to 25 mg/mL. In some embodiments, the anti-PVRIG antibody is at a concentration of from 15 mg/mL to 25 mg/mL. In some embodiments, the anti-PVRIG antibody is at a concentration of from 20 mg/mL to 25 mg/mL. In some embodiments, the anti-PVRIG antibody is at a concentration of about 20 mg/mL.

In some embodiments, the present invention provides a stable liquid pharmaceutical formulation of an anti-PVRIG antibody comprising:
(a) an anti-PVRIG antibody, wherein the anti-PVRIG antibody comprises an antibody with CDRs identical to those shown in Figure 3;
(b) from 10 mM to 100 mM histidine;
(c) from 30 mM to 100 mM NaCl;
(d) from 20 mM to 150 mM L-Arginine, and
(e) from 0.005% to 0.1% w/v polysorbate 80
wherein the composition has a pH from 5.5 to 7.0.

### B. Amino acids

In some embodiments, the present invention provides a stable liquid pharmaceutical formulation of an anti-PVRIG antibody or antigen binding fragment thereof (e.g., anti-PVRIG antibodies including those with CDRs identical to those shown in Figure 3) comprising at least one amino acid. In some embodiments, the at least one amino acid is histidine. In some embodiments, the at least one amino acid is arginine. In some embodiments, the present invention provides a stable liquid pharmaceutical formulation of an anti-PVRIG antibody or antigen binding fragment thereof comprising at least two amino acids. In some embodiments, the at least two amino acids are histidine and arginine.

In some embodiments, the pharmaceutical formulation comprises from 10 mM to 80 mM histidine, from 15 mM to 70 mM histidine, from 20 mM to 60 mM histidine, from 20 mM to 50 mM histidine, or from 20 mM to 30 mM histidine. In some embodiments, the pharmaceutical formulation comprises from 10 mM to 80 mM histidine. In some embodiments, the pharmaceutical formulation comprises from 15 mM to 70 mM histidine. In some embodiments, the pharmaceutical formulation comprises from 20 mM to 60 mM histidine. In some embodiments, the pharmaceutical formulation comprises from 20 mM to 50 mM histidine. In some embodiments, the pharmaceutical formulation comprises from 20 mM to 30 mM histidine. In some embodiments, the pharmaceutical formulation comprises about 25 mM histidine.

In some embodiments, the pharmaceutical formulation comprises from 10 mM to 80 mM histidine. In some embodiments, the pharmaceutical formulation comprises from 15 mM to 70 mM histidine. In some embodiments, the pharmaceutical formulation comprises from 20 mM to 60 mM histidine. In some embodiments, the pharmaceutical formulation comprises from 20 mM to 50 mM histidine. In some embodiments, the pharmaceutical formulation comprises from 20 mM to 30 mM histidine. In some embodiments, the pharmaceutical formulation comprises about 25 mM histidine.

In some embodiments, the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine. In some embodiments, the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.

In some embodiments, the pharmaceutical formulation comprises from 20 mM to 140 mM L-arginine. In some embodiments, the pharmaceutical formulation comprises from 30 mM to 140 mM L-arginine. In some embodiments, the pharmaceutical formulation comprises from 40 mM to 130 mM L-arginine. In some embodiments, the pharmaceutical formulation comprises from 50 mM to 120 mM L-arginine. In some embodiments, the pharmaceutical formulation comprises from 60 mM to 110 mM L-arginine. In some embodiments, the pharmaceutical formulation comprises from 70 mM to 110 mM L-arginine. In some embodiments, the pharmaceutical formulation comprises from 80 mM to 110 mM L-arginine. In some embodiments, the pharmaceutical formulation comprises from 90 mM to 110 mM L-arginine. In some embodiments, the pharmaceutical formulation comprises about 100 mM L-arginine.

### C. Sugar and/or sugar alcohol

In some embodiments, the present invention provides a stable liquid pharmaceutical formulation of an anti-PVRIG antibody or antigen binding fragment thereof (e.g., anti-PVRIG antibodies including those with CDRs identical to those shown in Figure 3) comprising no sugar and/or sugar alcohol. In some embodiments, the present invention provides a stable liquid pharmaceutical formulation of an anti-PVRIG antibody or antigen binding fragment thereof (*e.g.,* anti-PVRIG antibodies including those with CDRs identical to those shown in Figure 3) comprising no sugar. In some embodiments, the present invention provides a stable liquid pharmaceutical formulation of an anti-PVRIG antibody or antigen binding fragment thereof (*e.g.,* anti-PVRIG antibodies including those with CDRs identical to those shown in Figure 3) comprising no sugar alcohol.

In some embodiments, the present invention provides a stable liquid pharmaceutical formulation of an anti-PVRIG antibody or antigen binding fragment thereof comprising a sugar and/or sugar alcohol. In some embodiments, the sugar is trehalose or sucrose. In some embodiments, the sugar is trehalose. In some embodiments, the sugar is sucrose. In some embodiments, the sugar is only one of trehalose or sucrose but not both.

In some embodiments, the sugar is in an amount of from about 0.5% to 10%, 1 % to 9.5%, 1.5% to 9%, 2.0% to 8.5%, 2.5% to 8%, 3.0% to 7.5%, 3.5% to 7%, 4.0% to 6.5%, 4.5% to 6%, and/or 4.5% to 5.5%. In some embodiments, the sugar is in an amount of from about 0.5% to 10%. In some embodiments, the sugar is in an amount of from about 1 % to 9.5%. In some embodiments, the sugar is in an amount of from about 1.5% to 9%. In some embodiments, the sugar is in an amount of from about 2.0% to 8.5%. In some embodiments, the sugar is in an amount of from about 2.5% to 8%. In some embodiments, the sugar is in an amount of from about 3.0% to 7.5%. In some embodiments, the sugar is in an amount of from about 3.5% to 7%. In some embodiments, the sugar is in an amount of from about 4.0% to 6.5%. In some embodiments, the sugar is in an amount of from about 4.5% to 6%. In some embodiments, the sugar is in an amount of from about 4.5% to 5.5%. In some embodiments, the sugar is in an amount of about 5%

### D. Non-Ionic Surfactants

In some embodiments, the present invention provides a stable liquid pharmaceutical formulation of an anti-PVRIG antibody or antigen binding fragment thereof (*e.g.,* anti-PVRIG antibodies including those with CDRs identical to those shown in Figure 3) comprising a non-ionic surfactant. In some embodiments, the storage stable compositions of an anti-PVRIG antibody or antigen binding fragment comprise a non-ionic surfactant selected from a non-ionic water soluble monoglyceride, a non-ionic water soluble diglyceride, a non-ionic water soluble triglyceride, a non-ionic water soluble monofatty acid esters of polyethyelene glycol, a non-ionic water soluble difatty acid esters of polyethyelene glycol, a non-ionic water soluble sorbitan fatty acid ester, a non-ionic polyglycolyzed glyceride, a non-ionic water soluble triblock copolymer, and a combination thereof. In some embodiments, the non-ionic surfactant is polysorbate 80 (polyoxyethylene (20) sorbitan monooleate).

In some embodiments, the stable liquid pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80. In some embodiments, the stable liquid pharmaceutical formulation comprises from 0.006% to 0.1% w/v polysorbate 80. In some embodiments, the stable liquid pharmaceutical formulation comprises from 0.007% to 0.09% w/v polysorbate 80. In some embodiments, the stable liquid pharmaceutical formulation comprises from 0.008% to 0.08% w/v polysorbate 80. In some embodiments, the stable liquid pharmaceutical formulation comprises from 0.009% to 0.09% w/v polysorbate 80. In some embodiments, the stable liquid pharmaceutical formulation comprises from 0.01% to 0.08% w/v polysorbate 80. In some embodiments, the stable liquid pharmaceutical formulation comprises from 0.01% to 0.07% w/v polysorbate 80. In some embodiments, the stable liquid pharmaceutical formulation comprises from 0.01% to 0.07% w/v polysorbate 80. In some embodiments, the stable liquid pharmaceutical formulation comprises from 0.01% to 0.06% w/v polysorbate 80. In some embodiments, the stable liquid pharmaceutical formulation comprises from 0.009% to 0.05% w/v polysorbate 80. In some embodiments, the stable liquid pharmaceutical formulation comprises about 0.01% polysorbate 80.

### E. Pharmaceutically Acceptable Salts

In some embodiments, the present invention provides a stable liquid pharmaceutical formulation of an anti-PVRIG antibody or antigen binding fragment thereof (*e.g.,* anti-PVRIG antibodies including those with CDRs identical to those shown in Figure 3) comprising a salt, for example, a pharmaceutically acceptable salt.

In some embodiments, the stable liquid pharmaceutical formulation comprising an anti-PVRIG antibody or antigen binding fragment thereof provided herein include a pharmaceutically acceptable salt at a concentration tolerated by the an anti-PVRIG antibody or antigen binding fragment thereof during storage. In some embodiments, the pharmaceutically acceptable salt is a chloride salt. In some embodiments, the pharmaceutically acceptable salt is a monovalent chloride salt. In a more specific embodiment, the pharmaceutically acceptable salt is sodium chloride, potassium chloride, or a combination thereof.

In some embodiments, the stable liquid pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.

In some embodiments, the stable liquid pharmaceutical formulation comprises from 30 mM to 100 mM NaCl. In some embodiments, the stable liquid pharmaceutical formulation comprises from 30 mM to 90 mM NaCl. In some embodiments, the stable liquid pharmaceutical formulation comprises from 40 mM to 80 mM NaCl. In some embodiments, the stable liquid pharmaceutical formulation comprises from 30 mM to 70 mM histidine. In some embodiments, the stable liquid pharmaceutical formulation comprises or from 45 mM to 70 mM NaCl. In some embodiments, pharmaceutical formulation comprises about 60 mM NaCl.

### F. Buffering Agents

In some embodiments, the present invention provides a stable liquid pharmaceutical formulation of an anti-PVRIG antibody or antigen binding fragment thereof (*e.g.,* anti-PVRIG antibodies including those with CDRs identical to those shown in Figure 3) that is buffered at a physiologically acceptable pH. In some embodiments, the physiologically acceptable pH is from about 6.0 to about 7.0.

In some embodiments, stable liquid pharmaceutical formulation of an anti-PVRIG antibody or antigen binding fragment thereof has a pH of from 6 to 7.0. In some embodiments, stable liquid pharmaceutical formulation of an anti-PVRIG antibody or antigen binding fragment thereof has a pH of 6, 6.1, 6.2, 6.3, 6.4, 6.5, 6.6, 6.7, 6.8, 6.9, or 7.0. In some embodiments, the pH is from 6.1 to 6.9. In some embodiments, the pH is from 6.2 to 6.9. In some embodiments, the pH is from 6.3 to 6.8. In some embodiments, the pH is from 6.3 to 6.7. In some embodiments, the pH is from 6.4 to 6.8. In some embodiments, the pH is from 6.5 to 6.8. In some embodiments, the pH is from 6.6 to 6.8. In some embodiments, the pH is 6.3, 6.4, 6.5, 6.6, or 6.7. In some embodiments, the pH is 6.5 +/- 0.2.

### G. Methods for Diluting Aqueous Compositions

[In some embodiments, the method includes adding a dilution buffer, to form a diluted stable liquid pharmaceutical formulation comprising an anti-PVRIG antibody or antigen binding fragment thereof (*e.g.,* anti-PVRIG antibodies including those with CDRs identical to those shown in Figure 3). In some embodiments, the dilution buffer is added at a ratio of from 1:1 (dilution buffer:formulation) to 1000:1 (dilution buffer:formulation). In another embodiment, the dilution buffer is added at a ratio of from 1:1 dilution buffer:formulation) to 500:1 (dilution buffer:formulation). In another embodiment, the dilution buffer is added at a ratio of from 1:1 (dilution buffer:formulation) to 250:1 (dilution buffer:formulation). In another embodiment, the dilution buffer is added at a ratio of from 1:1 (dilution buffer:formulation) to 200:1 (dilution buffer:formulation). In another embodiment, the dilution buffer is added at a ratio of from 1:1 (dilution buffer:formulation) to 100:1 (dilution buffer:formulation). In another embodiment, the dilution buffer is added at a ratio of from 1:1 (dilution buffer:formulation) to 50:1 (dilution buffer:formulation).

In some embodiments, the stable liquid pharmaceutical formulation comprising an anti-PVRIG antibody or antigen binding fragment thereof is diluted from 1-fold to 1000-fold, from 1-fold to 500-fold, from 1-fold to 250-fold, from 1-fold to 200-fold, from 1-fold to 100-fold, from 1-fold to 50-fold, from 1-fold to 10-fold, from 10-fold to 1000-fold, from 10-fold to 500-fold, from 10-fold to 250-fold, from 10-fold to 200-fold, from 10-fold to 100-fold, from 10-fold to 50-fold, from 50-fold to 1000-fold, from 50-fold to 500-fold, from 50-fold to 250-fold, from 50-fold to 200-fold, from 50-fold to 100-fold, from 100-fold to 1000-fold, from 100-fold to 500-fold, from 100-fold to 250-fold, from 100-fold to 200-fold, from 200-fold to 1 ,000-fold, from 200-fold to 500-fold, or from 200-fold to 250-fold.

### H. Stability Assays

As discussed herein, the stable liquid pharmaceutical formulations comprising an anti-PVRIG antibody or antigen binding fragment thereof (*e.g.,* anti-PVRIG antibodies including those with CDRs identical to those shown in Figure 3) show improved stability as compared to control formulations. In one embodiment, improved stability includes retention of a higher percentage of binding activity and/or no reduction in binding activity as compared to control formulations in various stability assays. Such assays can be used to determine if a formulation is a highly stabilized formulation. In some embodiments, the highly stabilized formulation has at least 5%, 10%, 20%, 30%, 40%, 50%, 55%, 60%, 65%, 70%, 75%, 80%, 85%, 90%, 95%, 96%, 97%, 98%, 99% or greater activity than a control formulation when assessed by any of the stability assays discussed herein or known in the art.

In some embodiments, the liquid pharmaceutical formulations comprising an anti-PVRIG antibody or antigen binding fragment thereof are tested under stressor conditions, such as storage at high temperature, agitation, freeze/thaw cycles, or some combination thereof. After such stressors, the formulations are assayed using any of the methods described herein or known in the art to determine the stability under these conditions.

### A280 and Appearance Analysis

In some embodiments, an A280 by SoloVPE assay can be employed to examine the appearance of the stable liquid pharmaceutical formulations comprising an anti-PVRIG antibody or antigen binding fragment thereof.

In some embodiments, the SoloVPE assay can be employed to examine concentrations for the stable liquid pharmaceutical formulations comprising an anti-PVRIG antibody or antigen binding fragment thereof.

A280: Amino acids containing aromatic side chains exhibit strong UV-light absorption at the wavelength of 280nm. Once an absorptivity coefficient has been established for a given protein, the protein's concentration in solution can be calculated from its absorbance. The method is designed to determine the protein concentration by measuring its absorbance at 280nm using the SoloVPE instrument without dilution (https://www.ctechnologiesinc.com/products/solovpe)

Appearance: Sample appearance determination is assessed by holding the sample within a controlled light source and observe the appearance of the material. Gently agitate the solution and determine if the appearance changes when viewed against a black and white background. Use adjectives such as "clear", "turbid", or "slightly turbid" to assess clarity. Be specific with regards to the color of the material. If the material is colorless then state that as a result (*i.e.,* clear, colorless solution). specify the physical state of the sample (*i.e.,* liquid or frozen liquid)

### Binding Assay Analysis

In some embodiments, a binding assay can be performed to examine the activity of the stable liquid pharmaceutical formulations comprising an anti-PVRIG antibody or antigen binding fragment thereof.

### LabChip Analysis

In some embodiments, a LabChip analysis is employed to examine purity, including for example, IgG purity as well as HC + LC percentages for the stable liquid pharmaceutical formulations comprising an anti-PVRIG antibody or antigen binding fragment thereof. In some embodiments, the stable liquid pharmaceutical formulations comprising an anti-PVRIG antibody or antigen binding fragment thereof exhibit IgG purity percentages greater than 94%, greater than 95%, greater than 96%, greater than 97%, or greater than 98%. In some embodiments, the stable liquid pharmaceutical formulations comprising an anti-PVRIG antibody or antigen binding fragment thereof exhibit IgG purity percentages were from about 95% to 98%. In some embodiments, the stable liquid pharmaceutical formulations comprising an anti-PVRIG antibody or antigen binding fragment thereof exhibit IgG purity percentages from about 96% to 97%. In some embodiments, the stable liquid pharmaceutical formulations comprising an anti-PVRIG antibody or antigen binding fragment thereof exhibit HC+LC percentages from about 96% to 100%. In some embodiments, the stable liquid pharmaceutical formulations comprising an anti-PVRIG antibody or antigen binding fragment thereof exhibit HC+LC percentages from about 97% to 100%. In some embodiments, the stable liquid pharmaceutical formulations comprising an anti-PVRIG antibody or antigen binding fragment thereof exhibit HC+LC percentages from about 98% to 100%.

### cIEF Analysis

In some embodiments, a capillary isoelectric focusing (cIEF) can be employed to analyze the stable liquid pharmaceutical formulations comprising an anti-PVRIG antibody or antigen binding fragment thereof for the presence of additional species, including for example, minor acidic species.

### MFI Analysis

Antibodies can form sub-visible particles in response to stressed conditions, such as heat, freeze/thaw cycles, and agitation. In some embodiments, a microflow imaging (MFI) analysis can be employed to analyze the stable liquid pharmaceutical formulations comprising an anti-PVRIG antibody or antigen binding fragment thereof for the formation of particles in response to stressed conditions. In some embodiments, the stable liquid pharmaceutical formulations of the anti-PVRIG antibody or antigen binding fragment thereof provide for a formulation capable of stabilizing the anti-PVRIG antibody or antigen binding fragment thereof against these stressed conditions and protecting against the formation of particles. MFI can be used to evaluate particle counts at different size ranges (< 2 µm, < 5 µm, < 10 µm, and < 25 µm) in different formulations under stressed conditions. Typically, MFI data can be evaluated to choose an appropriate formulation based on generation of the lowest amount of particles/mL for all sizes of particles across all time points, conditions, and formulations.

### SEC Analysis

In some embodiments, size exclusion chromatography (SEC) can be employed to analyze the stable liquid pharmaceutical formulations comprising an anti-PVRIG antibody or antigen binding fragment thereof. The SEC data showed HMW throughout all time points and conditions; however, it remained stable at about 1%. LMW was present in accelerated conditions and 2-8 °C 8 week time point. Within the 40 °C condition, the LMW did increase from about 1% to 3% from Week 1 to Week 2.

### I. Selected Formulation Embodiments

In some embodiments, the present invention provides a stable liquid pharmaceutical formulation of an anti-PVRIG antibody comprising:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation of an anti-PVRIG antibody comprising:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
(a) an anti-PVRIG antibody;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides a stable liquid pharmaceutical formulation comprising:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation comprising:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides a stable liquid pharmaceutical formulation comprising:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation comprising:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides a stable liquid pharmaceutical formulation comprising:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation comprising:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

### IX. ADMINISTRATION OF FORMULATIONS OF ANTI-PVRIG ANTIBODIES

Administration of the pharmaceutical composition comprising anti-PVRIG antibodies and TIGIT antibodies or anti-TIGIT antibodies alone of the present invention (e.g., anti-PVRIG antibodies including those with CDRs identical to those shown in Figure 3 or anti-TIGIT antibodies including those with CDRs identical to those shown in Figure 13), preferably in the form of a sterile aqueous solution, may be done in a variety of ways, As is known in the art, protein therapeutics are often delivered by IV infusion. The antibodies of the present invention may also be delivered using such methods. For example, administration may venious be by intravenous infusion with 0.9% sodium chloride as an infusion vehicle. Such techniques are disclosed in Remington's Pharmaceutical Sciences 16th edition, Osol, A. Ed., 1980.

The dosing amounts and frequencies of administration are, in some embodiments, selected to be therapeutically or prophylactically effective. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art. In order to treat a patient, a therapeutically effective dose of the Fc variant of the present invention may be administered. By "therapeutically effective dose" herein is meant a dose that produces the effects for which it is administered.

### X. DOSING: ANTI-PVRIG ANTIBODY AND/OR ANTIGEN BINDING PORTION THEREOF FORMULATIONS

In some embodiments, the anti-PVRIG antibody and/or antigen binding portion thereof formulations of the present invention can be formulated for administration, including as a unit dosage formulation. In some embodiments, the anti-PVRIG antibody and/or antigen binding portion thereof formulations are administered at a dosage of 0.01 mg/kg of the anti-PVRIG antibody and/or antigen binding portion thereof. In some embodiments, the anti-PVRIG antibody and/or antigen binding portion thereof formulations are administered at a dosage of 0.02 mg/kg of the anti-PVRIG antibody and/or antigen binding portion thereof. In some embodiments, the anti-PVRIG antibody and/or antigen binding portion thereof formulations are administered at a dosage of 0.03 mg/kg of the anti-PVRIG antibody and/or antigen binding portion thereof. In some embodiments, the anti-PVRIG antibody and/or antigen binding portion thereof formulations are administered at a dosage of 0.04 mg/kg of the anti-PVRIG antibody and/or antigen binding portion thereof. In some embodiments, the anti-PVRIG antibody and/or antigen binding portion thereof formulations are administered at a dosage of 0.05 mg/kg of the anti-PVRIG antibody and/or antigen binding portion thereof. In some embodiments, the anti-PVRIG antibody and/or antigen binding portion thereof formulations are administered at a dosage of 0.06 mg/kg of the anti-PVRIG antibody and/or antigen binding portion thereof. In some embodiments, the anti-PVRIG antibody and/or antigen binding portion thereof formulations are administered at a dosage of 0.07 mg/kg of the anti-PVRIG antibody and/or antigen binding portion thereof. In some embodiments, the anti-PVRIG antibody and/or antigen binding portion thereof formulations are administered at a dosage of 0.08 mg/kg of the anti-PVRIG antibody and/or antigen binding portion thereof. In some embodiments, the anti-PVRIG antibody and/or antigen binding portion thereof formulations are administered at a dosage of 0.09 mg/kg of the anti-PVRIG antibody and/or antigen binding portion thereof. In some embodiments, the anti-PVRIG antibody and/or antigen binding portion thereof formulations are administered at a dosage of 0.1 mg/kg of the anti-PVRIG antibody and/or antigen binding portion thereof. In some embodiments, the anti-PVRIG antibody and/or antigen binding portion thereof formulations are administered at a dosage of 0.2 mg/kg of the anti-PVRIG antibody and/or antigen binding portion thereof. In some embodiments, the anti-PVRIG antibody and/or antigen binding portion thereof formulations are administered at a dosage of 0.3 mg/kg of the anti-PVRIG antibody and/or antigen binding portion thereof. In some embodiments, the anti-PVRIG antibody and/or antigen binding portion thereof formulations are administered at a dosage of 0.5 mg/kg of the anti-PVRIG antibody and/or antigen binding portion thereof. In some embodiments, the anti-PVRIG antibody and/or antigen binding portion thereof formulations are administered at a dosage of 0.8 mg/kg of the anti-PVRIG antibody and/or antigen binding portion thereof. In some embodiments, the anti-PVRIG antibody and/or antigen binding portion thereof formulations are administered at a dosage of 1 mg/kg of the anti-PVRIG antibody and/or antigen binding portion thereof. In some embodiments, the anti-PVRIG antibody and/or antigen binding portion thereof formulations are administered at a dosage of 2 mg/kg of the anti-PVRIG antibody and/or antigen binding portion thereof. In some embodiments, the anti-PVRIG antibody and/or antigen binding portion thereof formulations are administered at a dosage of 3 mg/kg of the anti-PVRIG antibody and/or antigen binding portion thereof. In some embodiments, the anti-PVRIG antibody and/or antigen binding portion thereof formulations are administered at a dosage of 4 mg/kg of the anti-PVRIG antibody and/or antigen binding portion thereof. In some embodiments, the anti-PVRIG antibody and/or antigen binding portion thereof formulations are administered at a dosage of 5 mg/kg of the anti-PVRIG antibody and/or antigen binding portion thereof. In some embodiments, the anti-PVRIG antibody and/or antigen binding portion thereof formulations are administered at a dosage of 6 mg/kg of the anti-PVRIG antibody and/or antigen binding portion thereof. In some embodiments, the anti-PVRIG antibody and/or antigen binding portion thereof formulations are administered at a dosage of 7 mg/kg of the anti-PVRIG antibody and/or antigen binding portion thereof. In some embodiments, the anti-PVRIG antibody and/or antigen binding portion thereof formulations are administered at a dosage of 8 mg/kg of the anti-PVRIG antibody and/or antigen binding portion thereof. In some embodiments, the anti-PVRIG antibody and/or antigen binding portion thereof formulations are administered at a dosage of 9 mg/kg of the anti-PVRIG antibody and/or antigen binding portion thereof. In some embodiments, the anti-PVRIG antibody and/or antigen binding portion thereof formulations are administered at a dosage of 10 mg/kg of the anti-PVRIG antibody and/or antigen binding portion thereof. In some embodiments, the anti-PVRIG antibody and/or antigen binding portion thereof formulations are administered at a dosage of 20 mg/kg of the anti-PVRIG antibody and/or antigen binding portion thereof.

In some embodiments, the anti-PVRIG antibody and/or antigen binding portion thereof formulations is administered at a dosage of about 0.01 mg/kg to about 20 mg/kg of the anti-PVRIG antibody. In some embodiments, the anti-PVRIG antibody and/or antigen binding portion thereof formulations is administered at a dosage of about 0.01 mg/kg to about 10 mg/kg of the anti-PVRIG antibody. In some embodiments, the anti-PVRIG antibody and/or antigen binding portion thereof formulations is administered at a dosage of about 20 mg/kg. In some embodiments, the anti-PVRIG antibody and/or antigen binding portion thereof formulations is administered at a dosage of about 20mg/kg each 4 weeks. In some embodiments, the anti-PVRIG antibody and/or antigen binding portion thereof formulations is administered at a dosage of about 20mg/kg IV each 4 weeks. In some embodiments, formulation is administered at a dosage of about 0.1 mg/kg to about 10 mg/kg of the anti-PVRIG antibody. In some embodiments, formulation is administered at a dosage of about 1 mg/kg to about 10 mg/kg of the anti-PVRIG antibody. In some embodiments, formulation is administered at a dosage of about 2 mg/kg to about 10 mg/kg of the anti-PVRIG antibody. In some embodiments, formulation is administered at a dosage of about 3 mg/kg to about 10 mg/kg of the anti-PVRIG antibody. In some embodiments, formulation is administered at a dosage of about 4 mg/kg to about 10 mg/kg of the anti-PVRIG antibody. In some embodiments, formulation is administered at a dosage of about 5 mg/kg to about 10 mg/kg of the anti-PVRIG antibody. In some embodiments, formulation is administered at a dosage of about 5 mg/kg to about 10 mg/kg of the anti-PVRIG antibody. In some embodiments, formulation is administered at a dosage of about 7 mg/kg to about 10 mg/kg of the anti-PVRIG antibody. In some embodiments, formulation is administered at a dosage of about 8 mg/kg to about 10 mg/kg of the anti-PVRIG antibody. In some embodiments, formulation is administered at a dosage of about 9 mg/kg to about 10 mg/kg of the anti-PVRIG antibody. In some embodiments, formulation is administered at a dosage of about 20 mg/kg of the anti-PVRIG antibody. In some embodiments, formulation is administered at a dosage of about 0.01 mg/kg, 0.03 mg/kg, 0.1 mg/kg, 0.3 mg/kg, 1 mg/kg, 3 mg/kg, 10 mg/kg or 20 mg/kg of the anti-PVRIG antibody.

In some embodiments, the an anti-PVRIG antibody and an anti-TIGIT antibody are administered at the same time (including for example on the same date). In some embodiments, the anti-TIGIT antibody alone. In some embodiments, the i) an anti-PVRIG antibody and an anti-TIGIT antibody, or ii) an anti-TIGIT antibody alone are each administered every 1 week, every 2 weeks, every 3 weeks, or every 4 weeks. In some embodiments, the i) an anti-PVRIG antibody and an anti-TIGIT antibody, or ii) an anti-TIGIT antibody alone are each administered every 1 week, every 2 weeks, every 3 weeks, or every 4 weeks. In some embodiments, the i) an anti-PVRIG antibody and an anti-TIGIT antibody, or ii) an anti-TIGIT antibody alone are each administered every 3 weeks. In some embodiments, the i) an anti-PVRIG antibody and an anti-TIGIT antibody, or ii) an anti-TIGIT antibody alone are each administered every 4 weeks. In some embodiments, the i) an anti-PVRIG antibody and an anti-TIGIT antibody are administered every 3 weeks. In some embodiments, the i) an anti-PVRIG antibody and an anti-TIGIT antibody are administered every 4 weeks. In some embodiments, the anti-TIGIT antibody alone is administered every 3 weeks. In some embodiments, the anti-TIGIT antibody alone is administered every 4 weeks. In some embodiments, the anti-TIGIT antibody is administered every 3 weeks. In some embodiments, the anti-TIGIT antibody is administered every 4 weeks. In some embodiments, the anti- PVRIG and/or the anti-TIGIT antibody is administered intravenously. In some embodiments, the anti- PVRIG and/or the anti-TIGIT antibody is administered intravenously every 3 weeks. In some embodiments, the anti- PVRIG and/or the anti-TIGIT antibody is administered intravenously. In some embodiments, the anti- PVRIG and/or the anti-TIGIT antibody is administered intravenously every 4 weeks. In some embodiments, the anti-PVRIG antibody is CHA.7.518.1.H4(S241P) and the anti-TIGIT antibody is CPA.9.086.H4(S241P). In some embodiments, the anti-TIGIT antibody is CPA.9.086.H4(S241P).

### A. Selected Dosing with Formulation Embodiments

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
(a) an anti-PVRIG antibody;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments of the stable liquid pharmaceutical formulation, the formulation is administered with an anti-PD-1 antibody.

In some embodiments of the stable liquid pharmaceutical formulation, the anti-PD-1 antibody is an antibody selected from the group consisting of pembrolizumab and nivolumab.

In some embodiments of the stable liquid pharmaceutical formulation, the anti-PD-1 antibody is nivolumab. In some embodiments of the stable liquid pharmaceutical formulation, the anti-PD-1 antibody is nivolumab is administered at a dosage of about 360 mg or 480 mg. In some embodiments of the stable liquid pharmaceutical formulation, the anti-PD-1 antibody is nivolumab is administered at a dosage of about 360 mg. In some embodiments of the stable liquid pharmaceutical formulation, the anti-PD-1 antibody is nivolumab is administered at a dosage of about 480 mg.

In some embodiments of the stable liquid pharmaceutical formulation, the anti-PD-1 antibody is pembrolizumab.

### XI. DOSING: ANTI-TIGIT ANTIBODY AND/OR ANTIGEN BINDING PORTION THEREOF FORMULATIONS

In some embodiments, the anti-TIGIT antibody and/or antigen binding portion thereof formulations of the present invention can be formulated for administration, including as a unit dosage formulation. In some embodiments, the anti-TIGIT antibody and/or antigen binding portion thereof formulations are administered at a dosage of 0.01 mg/kg of the anti-TIGIT antibody and/or antigen binding portion thereof. In some embodiments, the anti-TIGIT antibody and/or antigen binding portion thereof are administered at a dosage of 0.02 mg/kg of the anti-TIGIT antibody and/or antigen binding portion thereof. In some embodiments, the anti-TIGIT antibody and/or antigen binding portion thereof are administered at a dosage of 0.03 mg/kg of the anti-TIGIT antibody and/or antigen binding portion thereof. In some embodiments, the anti-TIGIT antibody and/or antigen binding portion thereof are administered at a dosage of 0.04 mg/kg of the anti-TIGIT antibody and/or antigen binding portion thereof. In some embodiments, the anti-TIGIT antibody and/or antigen binding portion thereof are administered at a dosage of 0.05 mg/kg of the anti-TIGIT antibody and/or antigen binding portion thereof. In some embodiments, the anti-TIGIT antibody and/or antigen binding portion thereof are administered at a dosage of 0.06 mg/kg of the anti-TIGIT antibody and/or antigen binding portion thereof. In some embodiments, the anti-TIGIT antibody and/or antigen binding portion thereof are administered at a dosage of 0.07 mg/kg of the anti-TIGIT antibody and/or antigen binding portion thereof. In some embodiments, the anti-TIGIT antibody and/or antigen binding portion thereof are administered at a dosage of 0.08 mg/kg of the anti-TIGIT antibody and/or antigen binding portion thereof. In some embodiments, the anti-TIGIT antibody and/or antigen binding portion thereof are administered at a dosage of 0.09 mg/kg of the anti-TIGIT antibody and/or antigen binding portion thereof. In some embodiments, the anti-TIGIT antibody and/or antigen binding portion thereof are administered at a dosage of 0.1 mg/kg of the anti-TIGIT antibody and/or antigen binding portion thereof. In some embodiments, the anti-TIGIT antibody and/or antigen binding portion thereof are administered at a dosage of 0.2 mg/kg of the anti-TIGIT antibody and/or antigen binding portion thereof. In some embodiments, the anti-TIGIT antibody and/or antigen binding portion thereof are administered at a dosage of 0.3 mg/kg of the anti-TIGIT antibody and/or antigen binding portion thereof. In some embodiments, the anti-TIGIT antibody and/or antigen binding portion thereof are administered at a dosage of 0.5 mg/kg of the anti-TIGIT antibody and/or antigen binding portion thereof. In some embodiments, the anti-TIGIT antibody and/or antigen binding portion thereof are administered at a dosage of 0.8 mg/kg of the anti-TIGIT antibody and/or antigen binding portion thereof. In some embodiments, the anti-TIGIT antibody and/or antigen binding portion thereof are administered at a dosage of 1 mg/kg of the anti-TIGIT antibody and/or antigen binding portion thereof. In some embodiments, the anti-TIGIT antibody and/or antigen binding portion thereof are administered at a dosage of 2 mg/kg of the anti-TIGIT antibody and/or antigen binding portion thereof. In some embodiments, the anti-TIGIT antibody and/or antigen binding portion thereof are administered at a dosage of 3 mg/kg of the anti-TIGIT antibody and/or antigen binding portion thereof. In some embodiments, the anti-TIGIT antibody and/or antigen binding portion thereof are administered at a dosage of 4 mg/kg of the anti-TIGIT antibody and/or antigen binding portion thereof. In some embodiments, the anti-TIGIT antibody and/or antigen binding portion thereof are administered at a dosage of 5 mg/kg of the anti-TIGIT antibody and/or antigen binding portion thereof. In some embodiments, the anti-TIGIT antibody and/or antigen binding portion thereof are administered at a dosage of 6 mg/kg of the anti-TIGIT antibody and/or antigen binding portion thereof. In some embodiments, the anti-TIGIT antibody and/or antigen binding portion thereof are administered at a dosage of 7 mg/kg of the anti-TIGIT antibody and/or antigen binding portion thereof. In some embodiments, the anti-TIGIT antibody and/or antigen binding portion thereof are administered at a dosage of 8 mg/kg of the anti-TIGIT antibody and/or antigen binding portion thereof. In some embodiments, the anti-TIGIT antibody and/or antigen binding portion thereof are administered at a dosage of 9 mg/kg of the anti-TIGIT antibody and/or antigen binding portion thereof. In some embodiments, the anti-TIGIT antibody and/or antigen binding portion thereof are administered at a dosage of 10 mg/kg of the anti-TIGIT antibody and/or antigen binding portion thereof.

In some embodiments, the anti-TIGIT antibody and/or antigen binding portion thereof is administered at a dosage of about 0.01 mg/kg to about 10 mg/kg of the anti-TIGIT antibody. In some embodiments, the anti-TIGIT antibody and/or antigen binding portion thereof is administered at a dosage of about 10 mg/kg each 3 weeks. In some embodiments, the anti-TIGIT antibody and/or antigen binding portion thereof isis administered at a dosage of about 0.1 mg/kg to about 10 mg/kg of the anti-TIGIT antibody. In some embodiments, he anti-TIGIT antibody and/or antigen binding portion thereof is administered at a dosage of about 1 mg/kg to about 10 mg/kg of the anti-TIGIT antibody. In some embodiments, the anti-TIGIT antibody and/or antigen binding portion thereof is is administered at a dosage of about 2 mg/kg to about 10 mg/kg of the anti-TIGIT antibody. In some embodiments, the anti-TIGIT antibody and/or antigen binding portion thereof is is administered at a dosage of about 3 mg/kg to about 10 mg/kg of the anti-TIGIT antibody. In some embodiments, the anti-TIGIT antibody and/or antigen binding portion thereof is is administered at a dosage of about 4 mg/kg to about 10 mg/kg of the anti-TIGIT antibody. In some embodiments, the anti-TIGIT antibody and/or antigen binding portion thereof is is administered at a dosage of about 5 mg/kg to about 10 mg/kg of the anti-TIGIT antibody. In some embodiments, the anti-TIGIT antibody and/or antigen binding portion thereof is is administered at a dosage of about 5 mg/kg to about 10 mg/kg of the anti-TIGIT antibody. In some embodiments, the anti-TIGIT antibody and/or antigen binding portion thereof is is administered at a dosage of about 7 mg/kg to about 10 mg/kg of the anti-TIGIT antibody. In some embodiments, the anti-TIGIT antibody and/or antigen binding portion thereof is is administered at a dosage of about 8 mg/kg to about 10 mg/kg of the anti-TIGIT antibody. In some embodiments, the anti-TIGIT antibody and/or antigen binding portion thereof is is administered at a dosage of about 9 mg/kg to about 10 mg/kg of the anti-TIGIT antibody. In some embodiments, the anti-TIGIT antibody and/or antigen binding portion thereof is is administered at a dosage of about 0.01 mg/kg, 0.03 mg/kg, 0.1 mg/kg, 0.3 mg/kg, 1 mg/kg, 3 mg/kg, or 10 mg/kg o of the anti-TIGIT antibody.

### A. Selected Dosing

In some embodiments, the present invention provides that the anti-TIGIT antibody is administered at a dosage of about 0.01 mg/kg, 0.03 mg/kg, 0.1 mg/kg, 0.3 mg/kg, 1 mg/kg, 3 mg/kg, 10 mg/kg. In some embodiments, the anti-TIGIT antibody is administered every 1 week, 2 weeks, 3 weeks, or 4 weeks. In some embodiments, the anti-TIGIT antibody is administered every 3 weeks. In some embodiments, the anti-TIGIT antibody is formulated for a unit dosage administration. In some embodiments, the anti-TIGIT antibody is formulated for a unit dosage administration of about 0.01 mg/kg, 0.03 mg/kg, 0.1 mg/kg, 0.3 mg/kg, 1 mg/kg, 3 mg/kg, 10 mg/kg for every 1 week, 2 weeks, 3 weeks, or 4 weeks. In some embodiments, the anti-TIGIT antibody is formulated for a unit dosage administration of about 0.01 mg/kg, 0.03 mg/kg, 0.1 mg/kg, 0.3 mg/kg, 1 mg/kg, 3 mg/kg, 10 mg/kg for every 3 weeks. In some embodiments, the anti-TIGIT antibody is CPA.9.083.H4(S241P).

In some embodiments, the anti-TIGIT antibody is CPA.9.086.H4(S241P).

In some embodiments, the anti-TIGIT antibody is selected from the group consisting of CPA.9.083.H4(S241P) and CPA.9.086.H4(S241P). In some embodiments, CPA.9.083.H4(S241P) is formulated for a unit dosage administration of about 0.01 mg/kg, 0.03 mg/kg, 0.1 mg/kg, 0.3 mg/kg, 1 mg/kg, 3 mg/kg, 10 mg/kg for every 3 weeks. In some embodiments, CPA.9.086.H4(S241P) is formulated for a unit dosage administration of about 0.01 mg/kg, 0.03 mg/kg, 0.1 mg/kg, 0.3 mg/kg, 1 mg/kg, 3 mg/kg, 10 mg/kg for every 3 weeks.

In some embodiments, the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody. In some embodiments, the CPA.9.086.H4(S241P) is administered at a dosage of about 0.01 mg/kg, 0.03 mg/kg, 0.1 mg/kg, 0.3 mg/kg, 1 mg/kg, 3 mg/kg, or 10 mg/kg.

In some embodiments, the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody every 1 week, 2 weeks, 3 weeks, or 4 weeks. In some embodiments, the CPA.9.086.H4(S241P) is administered at a dosage of about 0.01 mg/kg, 0.03 mg/kg, 0.1 mg/kg, 0.3 mg/kg, 1 mg/kg, 3 mg/kg, or 10 mg/kg of every 1 week, 2 weeks, 3 weeks, or 4 weeks. In some embodiments, the CPA.9.083.H4(S241P) is administered at a dosage of about 0.01 mg/kg, 0.03 mg/kg, 0.1 mg/kg, 0.3 mg/kg, 1 mg/kg, 3 mg/kg, or 10 mg/kg of every 1 week, 2 weeks, 3 weeks, or 4 weeks.

In some embodiments, the anti-TIGIT antibody is administered about 10 mg/kg every 3 weeks. In some embodiments, the CPA.9.086.H4(S241P) is administered about 10 mg/kg every 3 weeks. In some embodiments, the CPA.9.086.H4(S241P) is administered about 3 mg/kg every 3 weeks.

### XII. METHODS OF USING THE ANTI-PVRIG AND/OR ANTI-TIGIT ANTIBODY FORMULATIONS

### A. Therapeutic Uses

The anti-PVRIG antibodies (e.g., anti-PVRIG antibodies including those with CDRs identical to those shown in Figure 3) find use in treating patients, such as human subjects, generally with a condition associated with PVRIG. The term "treatment" as used herein, refers to both therapeutic treatment and prophylactic or preventative measures, which in this example relates to treatment of cancer; Those in need of treatment include those already with cancer as well as those in which the cancer is to be prevented. Hence, the mammal to be treated herein may have been diagnosed as having the cancer or may be predisposed or susceptible to the cancer. As used herein the term "treating" refers to preventing, delaying the onset of, curing, reversing, attenuating, alleviating, minimizing, suppressing, halting the deleterious effects or stabilizing of discernible symptoms of the above-described cancerous diseases, disorders or conditions. It also includes managing the cancer as described above. By "manage" it is meant reducing the severity of the disease, reducing the frequency of episodes of the disease, reducing the duration of such episodes, reducing the severity of such episodes, slowing/reducing cancer cell growth or proliferation, slowing progression of at least one symptom, amelioration of at least one measurable physical parameter and the like. For example, immunostimulatory anti-PVRIG immune molecules should promote T cell or NK or cytokine immunity against target cells, *e.g.,* cancer, infected or pathogen cells and thereby treat cancer by depleting the cells involved in the disease condition.

The PVRIG antibodies of the invention are provided in therapeutically effective dosages. A "therapeutically effective dosage" of an anti-PVRIG immune molecule according to at least some embodiments of the present invention preferably results in a decrease in severity of disease symptoms, an increase in frequency and duration of disease symptom-free periods, an increase in lifespan, disease remission, or a prevention or reduction of impairment or disability due to the disease affliction. For example, for the treatment of PVRIG positive tumors, a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject.

The anti-TIGIT antibodies (e.g., anti-TIGIT antibodies including those with CDRs identical to those shown in Figure 13) find use in treating patients, such as human subjects, generally with a condition associated with TIGIT. The term "treatment" as used herein, refers to both therapeutic treatment and prophylactic or preventative measures, which in this example relates to treatment of cancer; however, also as described below, uses of antibodies and pharmaceutical compositions are also provided for treatment of infectious disease, sepsis, and/or autoimmune conditions, and/or for inhibiting an undesirable immune activation that follows gene therapy. Those in need of treatment include those already with cancer as well as those in which the cancer is to be prevented. Hence, the mammal to be treated herein may have been diagnosed as having the cancer or may be predisposed or susceptible to the cancer. As used herein the term "treating" refers to preventing, delaying the onset of, curing, reversing, attenuating, alleviating, minimizing, suppressing, halting the deleterious effects or stabilizing of discernible symptoms of the above-described cancerous diseases, disorders or conditions. It also includes managing the cancer as described above. By "manage" it is meant reducing the severity of the disease, reducing the frequency of episodes of the disease, reducing the duration of such episodes, reducing the severity of such episodes, slowing/reducing cancer cell growth or proliferation, slowing progression of at least one symptom, amelioration of at least one measurable physical parameter and the like. For example, immunostimulatory anti-TIGIT immune molecules should promote T cell or NK or cytokine immunity against target cells, *e.g.,* cancer, infected or pathogen cells and thereby treat cancer or infectious diseases by depleting the cells involved in the disease condition. Conversely, immunoinhibitory anti-TIGIT immune molecules should reduce T cell or NK activity and/or or the secretion of proinflammatory cytokines which are involved in the disease pathology of some immune disease such as autoimmune, inflammatory, or allergic conditions and thereby treat or ameliorate the disease pathology and tissue destruction that may be associated with such conditions (*e.g.,* joint destruction associated with rheumatoid arthritis conditions).

The TIGIT antibodies of the invention are provided in therapeutically effective dosages. A "therapeutically effective dosage" of an anti-TIGIT immune molecule according to at least some embodiments of the present invention preferably results in a decrease in severity of disease symptoms, an increase in frequency and duration of disease symptom-free periods, an increase in lifespan, disease remission, or a prevention or reduction of impairment or disability due to the disease affliction. For example, for the treatment of TIGIT positive tumors, a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine a therapeutically effective amount based on such factors as the subject's size, the severity of the subject's symptoms, and the particular composition or route of administration selected.

### 1. Cancer Treatment

The PVRIG antibody and formulations of the invention as well as the TIGIT antibody formulations and combinations thereof find particular use in the treatment of cancer. In general, the antibodies of the invention are immunomodulatory, in that rather than directly attack cancerous cells, the anti-PVRIG antibodies and/or anti-TIGIT antibodies of the invention stimulate the immune system, generally by inhibiting the action of PVRIG and TIGIT, respectively. Thus, unlike tumor-targeted therapies, which are aimed at inhibiting molecular pathways that are crucial for tumor growth and development, and/or depleting tumor cells, cancer immunotherapy is aimed to stimulate the patient's own immune system to eliminate cancer cells, providing long-lived tumor destruction. Various approaches can be used in cancer immunotherapy, among them are therapeutic cancer vaccines to induce tumor-specific T cell responses, and immunostimulatory antibodies (i.e. antagonists of inhibitory receptors = immune checkpoints) to remove immunosuppressive pathways.

The TIGIT antibodies and formulations of the invention in the context of monotherapies also find particular use in the treatment of cancer. In general, the antibodies of the invention are immunomodulatory, in that rather than directly attack cancerous cells, the anti-TIGIT antibodies of the invention stimulate the immune system, generally by inhibiting the action of TIGIT. Thus, unlike tumor-targeted therapies, which are aimed at inhibiting molecular pathways that are crucial for tumor growth and development, and/or depleting tumor cells, cancer immunotherapy is aimed to stimulate the patient's own immune system to eliminate cancer cells, providing long-lived tumor destruction. Various approaches can be used in cancer immunotherapy, among them are therapeutic cancer vaccines to induce tumor-specific T cell responses, and immunostimulatory antibodies (*i.e.* antagonists of inhibitory receptors = immune checkpoints) to remove immunosuppressive pathways.

Clinical responses with targeted therapy or conventional anti-cancer therapies tend to be transient as cancer cells develop resistance, and tumor recurrence takes place. However, the clinical use of cancer immunotherapy in the past few years has shown that this type of therapy can have durable clinical responses, showing dramatic impact on long term survival. However, although responses are long term, only a small number of patients respond (as opposed to conventional or targeted therapy, where a large number of patients respond, but responses are transient).

By the time a tumor is detected clinically, it has already evaded the immune-defense system by acquiring immunoresistant and immunosuppressive properties and creating an immunosuppressive tumor microenvironment through various mechanisms and a variety of immune cells.

Accordingly, the anti-PVRIG antibodies and anti-TIGIT antibodies as well as combinations thereof of the invention are useful in treating cancer. Due to the nature of an immuno-oncology mechanism of action, PVRIG and/or TIGIT does not necessarily need to be overexpressed on or correlated with a particular cancer type; that is, the goal is to have the anti-PVRIG and/or anti-TIGIT antibodies antibodies de-suppress T cell and NK cell activation, such that the immune system will go after the cancers.

Moreover, the anti-TIGIT antibodies of the invention are useful as monotherapies in treating cancer. Due to the nature of an immuno-oncology mechanism of action, TIGIT does not necessarily need to be overexpressed on or correlated with a particular cancer type; that is, the goal is to have the anti-TIGIT antibodies de-suppress T cell and NK cell activation, such that the immune system will go after the cancers.

"Cancer," as used herein, refers broadly to any neoplastic disease (whether invasive or metastatic) characterized by abnormal and uncontrolled cell division causing malignant growth or tumor (*e.g.,* unregulated cell growth). The term "cancer" or "cancerous" as used herein should be understood to encompass any neoplastic disease (whether invasive, non-invasive or metastatic) which is characterized by abnormal and uncontrolled cell division causing malignant growth or tumor, non-limiting examples of which are described herein. This includes any physiological condition in mammals that is typically characterized by unregulated cell growth.

"Cancer therapy" herein refers to any method that prevents or treats cancer or ameliorates one or more of the symptoms of cancer. Typically, such therapies will comprises administration of immunostimulatory anti-TIGIT antibodies (including antigen-binding fragments) either alone or in combination with chemotherapy or radiotherapy or other biologics and for enhancing the activity thereof, *i.e.,* in individuals wherein expression of TIGIT suppresses antitumor responses and the efficacy of chemotherapy or radiotherapy or biologic efficacy.

In some embodiments, the anti-PVRIG formulations and/or anti-TIGIT formulations and/or combinations thereof of the present invention can be used in the treatment of solid tumors (including, for example, cancers of the lung, liver, breast, brain, GI tract) and blood cancers (including for example, leukemia and preleukemic disorders, lymphoma, plasma cell disorders) carcinoma, lymphoma, blastoma, sarcoma, and leukemia or lymphoid malignancies. In some embodiments, the cancer is early. In some embodiments, the cancer is advanced (including metastatic). In some embodiments, the cancers amenable for treatment of the invention include cancers that express or do not express PVRIG and/or TIGIT and further include non-metastatic or non-invasive, as well as invasive or metastatic cancers, including cancers where PVRIG and/or TIGIT expression by immune, stromal, or diseased cells suppresses antitumor responses and anti-invasive immune responses. In some embodiments, the anti-PVRIG formulations and/or anti-TIGIT formulations and/or combinations thereof can be used for the treatment of vascularized tumors. In some embodiments, the cancer for treatment using the anti-PVRIG formulations and/or anti-TIGIT formulations and/or combinations thereof of the present invention includes carcinoma, lymphoma, sarcoma, and/or leukemia. In some embodiments, the cancer for treatment using the anti-PVRIG formulations and/or anti-TIGIT formulations of the present invention includes vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma (including hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma), post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, malignant melanoma, pleural mesothelioma, chordoma, endometrial sarcoma, chondrosarcoma, uterine sarcoma, synovial sarcoma, uveal melanoma, amyloidosis, AL-amyloidosis, astrocytoma, and/or Myelodysplastic syndromes (MDS)..

In some embodiments, the cancer for treatment using the anti-PVRIG formulations and/or anti-TIGIT formulations and/or combinations thereof of the present invention includes a cancer selected from the group consisting of prostate cancer, liver cancer (HCC), rectal cancer, colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), uveal melanoma, glioma, renal cell cancer (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cell cancer, MSI-high cancer, KRAS mutant tumors, adult T-cell leukemia/lymphoma, pleural mesothelioma, anal SCC, neuroendocrine lung cancer (including neuroendocrine lung carcinoma), small cell lung cancer, NSCLC, NSCLC large cell, NSCLC squamous cell, NSCLC adenocarcinoma, atypical carcinoid lung cancer, NSCLC with PDL1 >=50% TPS, cervical SCC, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, chordoma, sarcoma, endometrial sarcoma, chondrosarcoma, uterine sarcoma, plasma cell disorders, multiple myeloma, amyloidosis, AL-amyloidosis, glioblastoma, astrocytoma and fallopian tube cancer..

In some embodiments, the cancer for treatment using the anti-PVRIG antibodies and/or anti-TIGIT antibodies and/or combinations thereof, as well as anti-TIGIT antibodies alone of the present invention include advanced cancer, ovarian cancer, lung cancer, non-small-cell lung carcinoma (NSCLC), PD1 refractory or relapsing NSCLC, colon cancer, colorectal cancer (CRC), MSS-CRC, plasma cell neoplasm, head and neck squamous cell carcinomas (HNSCC), PD1 refractory or relapsing HNSCC, and breast cancer.In some embodiments, the cancer for treatment using the anti-PVRIG antibodies and/or anti-TIGIT antibodies and/or combinations thereof, as well as anti-TIGIT antibodies alone is an NSCLC. In some embodiments, the NSCLC is Stage IV or recurrent NSCLC. In some embodiments, the NSCLC patient for treatment exhibits disease recurrence or progression during or after prior treatment that included:
o platinum-based doublet chemotherapy regimen that may include continuation off or switch to maintenance therapy with pemetrexed, bevacizumab, or erlotinib; and/or
o anti-programmed cell death protein-1 (anti-PD-1) or anti-PD-L1-directed therapy.
In some embodiments, the NSCLC patient for treatment is a patient with a known EGFR mutation or ALK translocation who has received or will be receiving an additional line of relevant tyrosine kinase inhibitor therapy (including optional and/or no prior treatment with platinum-based chemotherapy). In some embodiments, the NSCLC patient for treatment has NSCLC tumors harboring EGFR T90M mutation and will be required to have disease progression on Osimertinib. In some embodiments, the NSCLC patient for treatment has had prior treatment with FDA approved targeted therapy is required for subjects with NSCLC tumors harboring other genomic aberrations for which FDA-approved targeted therapy is available (*e.g.,* ROS rearrangement, BRAF V600E mutation)

In some embodiments, the cancer for treatment using the anti-PVRIG antibodies and/or anti-TIGIT antibodies and/or combinations thereof, as well as anti-TIGIT antibodies alone is colorectal cancer (microsatellite stable). In some embodiments, the histologically will have confirmed adenocarcinoma of the colon/rectum and/or Stage IV disease. In some embodiments, the colorectal cancer (microsatellite stable) patient for treatment must has documented MSS status by an FDA approved test *e.g*., genomic testing, IHC for mismatch repair proficient. In some embodiments, the colorectal cancer (microsatellite stable) patient for treatment has disease progression with ≥2 prior systemic therapies for metastatic CRC that one or all of the following: fluroropyrimidines, irinotecan, and oxaliplatin

In some embodiments, the cancer for treatment using the anti-PVRIG antibodies and anti-TIGIT antibodies and/or combinations thereof, as well as anti-TIGIT antibodies alone is HNSCC. In some embodiments, the HNSCC patient for treatment has histologically confirmed recurrent or metastatic HNSCC (oral cavity, pharynx, larynx). In some embodiments, the HNSCC patient for treatment has prior receipt of platinum therapy anti-programmed cell death protein-1 (anti-PD-1) or anti-PD-L1-directed therapy. In some embodiments, the HNSCC patient for treatment has documentation of p16-positive or p16-negative disease to determine human papillomavirus (HPV) status of tumor.

In some embodiments, the cancer for treatment using the a anti-PVRIG antibodies and anti-TIGIT antibodies and/or combinations thereof, as well as anti-TIGIT antibodies alone is multiple myeloma. In some embodiments, the multiple myeloma patient for treatment has previously treated relapsed and refractory multiple myeloma per International Myeloma Working Group consensus criteria (see, Rajkumar et al., 2011). In some embodiments, the multiple myeloma patient for treatment had prior therapy ≥3 lines including an immunomodulatory drug (e.g., lenalidomide, pomalidomide), a proteasome inhibitor (*e.g.,* bortezomib, carfilzomib), and anti-CD38 monoclonal antibody (e.g., daratumumab). In some embodiments, the multiple myeloma patient for treatment has measurable disease of multiple myeloma as defined by at least one of the following (IgD and IgA with monoclonal protein < 0.5 g/dL may be permitted after discussion with PI):
o Serum monoclonal protein ≥ 0.5 g/dL (or quantitative IgA ≥ 1000 mg/dL), or
o ≥ 200 mg of monoclonal protein in the urine on 24-hour urine protein electrophoresis, and/or
o Serum free light chain ≥ 100 mg/L (10 mg/dL) and abnormal serum free kappa to serum free kappa light chain ratio.

"Cancer therapy" herein refers to any method that prevents or treats cancer or ameliorates one or more of the symptoms of cancer. Typically such therapies will comprises administration of immunostimulatory anti-PVRIG antibodies (including antigen-binding fragments) either alone or in combination with chemotherapy or radiotherapy or other biologics and for enhancing the activity thereof, *i.e.,* in individuals wherein expression of PVRIG suppresses antitumor responses and the efficacy of chemotherapy or radiotherapy or biologic efficacy.

In some embodiments, anti-PVRIG antibodies and/or anti-TIGIT formulations and/or combinations thereof, are used in combination with antagonistic antibodies targeting PD-1 (*e.g.,* anti-PD-1 antibodies), including for example but not limited to nivolumab and/or pembrolizumab. In some embodiments, the anti-PD-1 antibody is an antibody selected from the group consisting of nivolumab and pembrolizumab. In some embodiments, the anti-PD-1 antibody is nivolumab. In some embodiments, the anti-PD-1 antibody is pembrolizumab. In some embodiments, the anti-PD-1 antibody is nivolumab is administered at 360mg. In some embodiments, the anti-PD-1 antibody is nivolumab is administered at 360mg IV. In some embodiments, the anti-PD-1 antibody is nivolumab is administered at 360mg IV 3 weeks (*e.g.,* 360mg IV Q3 weeks). In some embodiments, the anti-PD-1 antibody is nivolumab is administered at 480mg. In some embodiments, the anti-PD-1 antibody is nivolumab is administered at 480mg IV. In some embodiments, the anti-PD-1 antibody is nivolumab is administered at 480mg IV 3 weeks (*e.g.,* 480mg IV Q3 weeks). In some embodiments, the subject administered the anti-PVRIG antibody in combination with the anti-PD-1 antibody has exhausted all available standard therapy, including for example, but not limited to ECOG 0-1, prior anti-PD-1, prior anti-PD-L1, prior anti-CTLA-4, prior OX-40, and/or prior CD137 therapies.

### 2. Selected Combination Treatment with Formulation Embodiments

In some embodiments, the present invention provides for treatment of cancer in a subject in need thereof by administration of an anti-TIGIT and/or and an anti-PD1 and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the anti-PD1 is selected from the group consisting of nivolumab, pembrolizumab, BMS-936558/MDX-1106/ONO-4538, AMP224, CT-011, MK-3475.

In some embodiments, the anti-TIGIT antibody comprises a heavy chain variable domain comprising the vhCDR1, vhCDR2, and vhCDR3 from the heavy chain of CPA.9.086.H4(S241P) (SEQ ID NO:877) and a light chain variable domain comprising the vlCDR1, vlCDR2, and vlCDR3 from the light chain of CPA.9.086.H4(S241P) (SEQ ID NO:882). In some embodiments, the anti-TIGIT antibody comprises a heavy chain variable domain of CPA.9.086.H4(S241P) (SEQ ID NO:877) and a light chain variable domain of CPA.9.086.H4(S241P) (SEQ ID NO:882).

In some embodiments, the invention provides the specific combinations of: CHA.7.518.1.H4(S241P) with pembrolizumab; CHA.7.518.1.H4(S241P) with nivolumab; CHA.7.538.1.2.H4(S241P) with pembrolizumab and CHA.7.538.1.2.H4(S241P) with nivolumab. In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
(a) an anti-PVRIG antibody;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

### XIII. MULTIPLE MYELOMA THERAPIES

The present invention also provides for monotherapies and combination therapies with regard to the treatment methods. In some embodiments, combination therapies include combinations with other known multiple myeloma drugs and/or therapies, as well as other immune checkpoints inhibitors.

### a. Monotherapies

In some embodiments, the multiple myeloma therapies contemplated by the methods of the present invention include an anti-PVRIG antibody, including those as described herein. In some embodiments, the multiple myeloma therapies contemplated by the methods of the present invention include an anti-TIGIT antibody, including those as described herein. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma therapy comprises an anti-PVRIG antibody, wherein the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma therapy comprises an anti-PVRIG antibody, wherein the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma combination therapy comprises an anti-TIGIT antibody, wherein the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8 and an anti-DNAM antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma combination therapy comprises an anti-TIGIT antibody, wherein the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8 and an anti-PVRIG antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

### a. Combination Therapies

In some embodiments, the multiple myeloma combination therapies contemplated by the methods of the present invention include combinations of an anti-PVRIG antibody as described herein and an anti-TIGIT antibody, including those as described herein. In some embodiments, the multiple myeloma combination therapies contemplated by the methods of the present invention include combinations of an anti-PVRIG antibody as described herein and an anti-DNAM antibody, including those as described herein. In some embodiments, the multiple myeloma combination therapies contemplated by the methods of the present invention include combinations of an anti-PVRIG antibody as described herein and an immune cell engager, including those as described herein. In some embodiments, the multiple myeloma combination therapies contemplated by the methods of the present invention include combinations of an anti-PVRIG antibody as described herein, and anti-TIGIT antibody as described herein, and an anti-DNAM antibody, including those as described herein. In some embodiments, the multiple myeloma combination therapies contemplated by the methods of the present invention include combinations of an anti-PVRIG antibody as described herein, and anti-TIGIT antibody as described herein, and an immune cell engager, including those as described herein. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

Anti-DNAM antibodies, also referred to as anti-CD226 antibodies, can include 10E5, DX11, 11A8.7.4, or anti-CD226 antibody as shown in Figure 23 (from WO2020/023312; Eli Lilly And Company).

In some embodiments, the multiple myeloma combination therapy comprises an anti-PVRIG antibody, wherein the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050, and an anti-TIGIT antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma combination therapy comprises an anti-PVRIG antibody, wherein the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050, and an anti-TIGIT antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma combination therapy comprises an anti-TIGIT antibody, wherein the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8 and an anti-DNAM antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma combination therapy comprises an anti-TIGIT antibody, wherein the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8, and an anti-DNAM antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma combination therapy comprises an anti-TIGIT antibody, wherein the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8 and an anti-PVRIG antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the multiple myeloma combination therapy comprises an anti-TIGIT antibody, wherein the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8, and an anti-PVRIG antibody. In some embodiments, the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.

In some embodiments, the ant-PVRIG antibody in the multiple myeloma combination therapy is CHA.7.518.1.H4(S241P). In some embodiments, the multiple myeloma combination therapy comprises CHA.7.518.1.H4(S241P) and an anti-TIGIT antibody. In some embodiments, the multiple myeloma combination therapy comprises CHA.7.518.1.H4(S241P) and CPA.9.086.H4(S241P). In some embodiments, the multiple myeloma combination therapy comprises CHA.7.518.1.H4(S241P) and an anti-DNAM antibody.

In some embodiments, the multiple myeloma combination therapy comprises an anti-TIGIT antibody. In some embodiments, the multiple myeloma combination therapy comprises CPA.9.086.H4(S241P). In some embodiments, the multiple myeloma combination therapy comprises CPA.9.086.H4(S241P) and an anti-DNAM antibody. In some embodiments, the multiple myeloma combination therapy comprises CPA.9.086.H4(S241P) and anti-PVRIG antibody.

In some embodiments, the multiple myeloma combination therapy comprises CHA.7.518.1.H4(S241P), CPA.9.086.H4(S241P) and an anti-DNAM antibody.

In some embodiments, the multiple myeloma combination therapies contemplated by the methods of the present invention include combinations of an anti-PVRIG antibody as described herein and an anti-PD-1 antibody, including those as described herein. In some embodiments, the multiple myeloma combination therapies contemplated by the methods of the present invention include combinations of an anti-TIGIT antibody as described herein and an anti-PD-1 antibody, including those as described herein. In some embodiments, the multiple myeloma combination therapies contemplated by the methods of the present invention include combinations of an anti-PVRIG antibody as described herein, an anti-TIGIT antibody as described herein and an anti-PD-1 antibody, including those as described herein.

In some embodiments, the multiple myeloma combination therapy comprises CHA.7.518.1.H4(S241P) and an anti-PD-1 antibody. In some embodiments, the anti-PD-1 antibody of the multiple myeloma combination therapy is nivolumab or pembrolizumab. In some embodiments, the multiple myeloma combination therapy comprises CHA.7.518.1.H4(S241P) and nivolumab. In some embodiments, the multiple myeloma combination therapy comprises CHA.7.518.1.H4(S241P) and pembrolizumab.

In some embodiments, the multiple myeloma combination therapy comprises CPA.9.086.H4(S241P) and an anti-PD-1 antibody. In some embodiments, the anti-PD-1 antibody of the multiple myeloma combination therapy is nivolumab or pembrolizumab. In some embodiments, the multiple myeloma combination therapy comprises CPA.9.086.H4(S241P) and nivolumab. In some embodiments, the multiple myeloma combination therapy comprises CPA.9.086.H4(S241P) and pembrolizumab.

In some embodiments, the multiple myeloma combination therapy comprises CHA.7.518.1.H4(S241P), an anti-TIGIT antibody and an anti-PD-1 antibody. In some embodiments, the multiple myeloma combination therapy comprises an anti-PVRIG antibody, CPA.9.086.H4(S241P) and an anti-PD-1 antibody. In some embodiments, the multiple myeloma combination therapy comprises CHA.7.518.1.H4(S241P), CPA.9.086.H4(S241P) and an anti-PD-1 antibody. In some embodiments, the multiple myeloma combination therapy comprises CHA.7.518.1.H4(S241P), CPA.9.086.H4(S241P) and nivolumab. In some embodiments, the multiple myeloma combination therapy comprises CHA.7.518.1.H4(S241P), CPA.9.086.H4(S241P) and pembrolizumab.

In some embodiments, the multiple myeloma combination therapy comprises CHA.7.518.1.H4(S241P) and an anti-DNAM antibody (*i.e.,* an anti-CD226 antibody). In some embodiments, the multiple myeloma combination therapy comprises CHA.7.518.1.H4(S241P) and the anti-CD226 antibody as shown in Figure 23.

In some embodiments, the multiple myeloma combination therapy comprises CHA.7.538.1.2.H4(S241P) and an anti-DNAM antibody (*i.e.,* an anti-CD226 antibody). In some embodiments, the multiple myeloma combination therapy comprises CHA.7.538.1.2.H4(S241P) and the anti-CD226 antibody as shown in Figure 23.

In some embodiments, the multiple myeloma combination therapy comprises CPA.9.086.H4(S241P) and an anti-DNAM antibody (*i.e.,* an anti-CD226 antibody). In some embodiments, the multiple myeloma combination therapy comprises CPA.9.086.H4(S241P) and the anti-CD226 antibody as shown in Figure 23.

In some embodiments, the multiple myeloma combination therapy comprises CPA.9.083.H4(S241P) and an anti-DNAM antibody (*i.e.,* an anti-CD226 antibody). In some embodiments, the multiple myeloma combination therapy comprises CPA.9.083.H4(S241P) and the anti-CD226 antibody as shown in Figure 23.

In some embodiments, the multiple myeloma combination therapy comprises CHA.7.518.1.H4(S241P), CPA.9.086.H4(S241P), and an anti-DNAM antibody. In some embodiments, the multiple myeloma combination therapy comprises CHA.7.518.1.H4(S241P), CPA.9.086.H4(S241P), and the anti-CD226 antibody as shown in Figure 23.

In some embodiments, the multiple myeloma combination therapy comprises CHA.7.518.1.H4(S241P), CPA.9.083.H4(S241P), and an anti-DNAM antibody. In some embodiments, the multiple myeloma combination therapy comprises CHA.7.518.1.H4(S241P), CPA.9.083.H4(S241P), and the anti-CD226 antibody as shown in Figure 23.

In some embodiments, the combination therapy comprises CHA.7.538.1.2.H4(S241P), CPA.9.086.H4(S241P), and an anti-DNAM antibody. In some embodiments, the multiple myeloma combination therapy comprises CHA.7.538.1.2.H4(S241P), CPA.9.086.H4(S241P), and the anti-CD226 antibody as shown in Figure 23.

In some embodiments, the multiple myeloma combination therapy comprises CHA.7.538.1.2.H4(S241P), CPA.9.083.H4(S241P), and an anti-DNAM antibody. In some embodiments, the multiple myeloma combination therapy comprises CHA.7.538.1.2.H4(S241P), CPA.9.083.H4(S241P), and the anti-CD226 antibody as shown in Figure 23.

In some embodiments, the multiple myeloma combination therapy comprises CHA.7.518.1.H4(S241P) and an immune cell engager.

In some embodiments, the multiple myeloma combination therapy comprises CPA.9.086.H4(S241P) and an immune cell engager.

In some embodiments, the multiple myeloma combination therapy comprises CHA.7.518.1.H4(S241P), an anti-TIGIT antibody and an immune cell engager. In some embodiments, the multiple myeloma combination therapy comprises an anti-PVRIG antibody, CPA.9.086.H4(S241P) and an immune cell engager. In some embodiments, the multiple myeloma combination therapy comprises CHA.7.518.1.H4(S241P), CPA.9.086.H4(S241P) and immune cell engager.

In some embodiments, the multiple myeloma combination therapy comprises CHA.7.538.1.2.H4(S241P) and an immune cell engager.

In some embodiments, the multiple myeloma combination therapy comprises CPA.9.083.H4(S241P) and an immune cell engager.

In some embodiments, the multiple myeloma combination therapy comprises CHA.7.518.1.H4(S241P), CPA.9.086.H4(S241P), and an immune cell engager.

In some embodiments, the multiple myeloma combination therapy comprises CHA.7.518.1.H4(S241P), CPA.9.083.H4(S241P), and an immune cell engager.

In some embodiments, the combination therapy comprises CHA.7.538.1.2.H4(S241P), CPA.9.086.H4(S241P), and immune cell engager.

In some embodiments, the multiple myeloma combination therapy comprises CHA.7.538.1.2.H4(S241P), CPA.9.083.H4(S241P), and a immune cell engager.

### a. Multiple Myeloma Therapies for Combination Therapies:

In some embodiments, the multiple myeloma combination therapy comprises a further component, such as one or more chemotherapies or chemotherapeutics. In some embodiments, the one or more chemotherapeutics include but are not limited to cyclophosphamide (cytoxan), etoposide (VP-16), doxorubicin (adriamycin), liposomal doxorubicin (Doxil), melphalan, melphalan flufenamide, melflufen (Pepaxto), and/or bendamustine (Treanda).

In some embodiments, the multiple myeloma combination therapy comprises a further component, such as one or more corticosteroids. In some embodiments, the one or more corticosteroids include but are not limited to dexamethasone and/or prednisone

In some embodiments, the multiple myeloma combination therapy comprises a further component, such as one or more immunomodulating agents. In some embodiments, the one or more immunomodulating agents include but are not limited to thalidomide (Thalomid), lenalidomide (Revlimid), and/or pomalidomide (Pomalyst).

In some embodiments, the multiple myeloma combination therapy comprises a further component, such as one or more proteasome inhibitors. In some embodiments, the one or more proteasome inhibitors include but are not limited to bortezomib (Velcade), carfilzomib (Kyprolis), and/or ixazomib (Ninlaro).

In some embodiments, the multiple myeloma combination therapy comprises a further component, such as one or more histone deacetylase (HDAC) inhibitors. In some embodiments, the one or more histone deacetylase (HDAC) inhibitors include but are not limited to panobinostat (Farydak).

In some embodiments, the multiple myeloma combination therapy comprises a further component such as one or more immune checkpoint inhibitors.

In some embodiments, the multiple myeloma combination therapy comprises one or more anti-PD-1 monoclonal antibodies, such as nivolumab or pembrolizumab.

In some embodiments, the multiple myeloma combination therapy comprises one or more anti-PD-L1 monoclonal antibodies such as atezolizumab, avelumab, or durvalumab.

In some embodiments, the multiple myeloma combination therapy comprises a further component, such as one or more anti-CD38 monoclonal antibodies. In some embodiments, the one or more anti-CD38 monoclonal antibodies include but are not limited to daratumumab (Darzalex), daratumumab and hyaluronidase (Darzalex Faspro), isatuximab (Sarclisa), and/or belantamab mafodotin-blmf (Blenrep).

In some embodiments, the multiple myeloma combination therapy comprises a further component, such as one or more anti-SLAMF7 antibodies. In some embodiments, the one or more anti- SLAMF7 monoclonal antibodies include but are not limited to elotuzumab (Empliciti).

In some embodiments, the multiple myeloma combination therapy comprises lenalidomide (or pomalidomide or thalidomide) and dexamethasone.

In some embodiments, the multiple myeloma combination therapy comprises carfilzomib (or ixazomib or bortezomib), lenalidomide, and dexamethasone.

In some embodiments, the multiple myeloma combination therapy comprises bortezomib (or carfilzomib), cyclophosphamide, and dexamethasone.

In some embodiments, the multiple myeloma combination therapy comprises elotuzumab (or daratumumab), lenalidomide, and dexamethasone.

In some embodiments, the multiple myeloma combination therapy comprises bortezomib, liposomal doxorubicin, and dexamethasone.

In some embodiments, the multiple myeloma combination therapy comprises panobinostat, bortezomib, and dexamethasone.

In some embodiments, the multiple myeloma combination therapy comprises elotuzumab, bortezomib, and dexamethasone.

In some embodiments, the multiple myeloma combination therapy comprises melphalan and prednisone (MP), in the presence or absence of thalidomide and/or bortezomib

In some embodiments, the multiple myeloma combination therapy comprises vincristine, doxorubicin (Adriamycin), and dexamethasone (called VAD).

In some embodiments, the multiple myeloma combination therapy comprises dexamethasone, cyclophosphamide, etoposide, and cisplatin (called DCEP).

In some embodiments, the multiple myeloma combination therapy comprises dexamethasone, thalidomide, cisplatin, doxorubicin, cyclophosphamide, and etoposide (called DT-PACE), with or without bortezomib.

In some embodiments, the multiple myeloma combination therapy comprises elinexor, bortezomib, and dexamethasone.

### EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

### EXAMPLE 1: COMBINATION STUDY OF CPA.9.086.H4(S241P) AND CHA.7.518.1.H4(S241P) IN PATIENTS WITH ADVANCED MALIGNANCIES

### Study Description & Summary

Phase 1 open label sequential dose escalation and cohort expansion study evaluating the safety, tolerability and preliminary clinical activity of CPA.9.086.H4(S241P) as monotherapy in subjects with advanced malignancies. Including 90 participants.

Condition or disease: Advanced Cancer, Ovarian Cancer, Lung Cancer, Colon Cancer, Plasma Cell Neoplasm, Multiple Myeloma, or HNSCC.

Intervention/treatment Phase: Phase 1
- Drug: Dose escalation: CPA.9.086.H4(S241P) monotherapy.
- Combination Product: Dose escalation: CPA.9.086.H4(S241P) (RDFE) in combination with CHA.7.518.1.H4(S241P).
- Drug: Dose expansion: CPA.9.086.H4(S241P) (RDFE) monotherapy.
- Drug: Dose expansion: CPA.9.086.H4(S241P) (RDFE) in combination with CHA.7.518.1.H4(S241P).

| **Arm** | **Intervention/treatment** |
|---|---|
| Experimental: CPA.9.086.H4(S241P) Monotherapy Dose Escalation Arm. | Drug: Dose escalation: CPA.9.086.H4(S241P) monotherapy. |
| Monotherapy Dose Escalation. CPA.9.086.H4(S241P) monotherapy administered IV every 3 weeks in sequential dose escalation Cohorts using a rules-based design. Up to 7 dose escalation cohorts may be evaluated until a maximum tolerated dose or recommended phase 2 dose is identified. | CPA.9.086.H4(S241P) monotherapy administered IV Q3 weeks in sequential dose escalation doses in cohorts of subjects. |
| Experimental: Combination dose escalation. | Combination Product: Dose escalation: CPA.9.086.H4(S241P) (RDFE) in combination with CHA.7.518.1.H4(S241P). |
| Dose escalation with a 3+3 study design withCPA.9.086.H4(S241P) at the RDFE in combination with CHA.7.518.1.H4(S241P) both IV Q3 wks. | |
| | A 3+3 study design with CPA.9.086.H4(S241P) (RDFE) in combination with CHA.7.518.1.H4(S241P) both administered IV Q3 weeks in sequential dose escalation doses in cohorts of subjects. |
| Experimental: CPA.9.086.H4(S241P) monotherapy dose expansion. | Drug: Dose expansion: CPA.9.086.H4(S241P) (RDFE) monotherapy. |
| CPA.9.086.H4(S241P) monotherapy at the RDFE in select tumor types. | CPA.9.086.H4(S241P) monotherapy (RDFE) in subjects with select tumor types (preferably subjects with multiple myeloma). |
| Experimental: CPA.9.086.H4(S241P) + CHA.7.518.1.H4(S241P) combination dose expansion. | Drug: Dose expansion: CPA.9.086.H4(S241P) (RDFE) in combination with CHA.7.518.1.H4(S241P). |
| CPA.9.086.H4(S241P) (RDFE) + CHA.7.518.1.H4(S241P) administered IV Q3 wks in subjects with select tumor types: HNSCC, CRC (MSS), NSCLC. | CPA.9.086.H4(S241P) (RDFE) in combination with CHA.7.518.1.H4(S241P) in subjects with select tumor types - HNSCC, CRC (MSS), NSCLC. |

### Outcome Measures

### Primary Outcome Measure:

The safety and tolerability of CPA.9.086.H4(S241P) monotherapy and in combination with CHA.7.518.1.H4(S241P). [Time Frame: DLT evaluation window in the 1st cycle (21 Days).]

Incidence of subjects with Adverse Events (AEs) as per CTCAE v5.0 and Dose-Limiting Toxicities (DLTs).

To identify the maximum tolerated dose (MTD) and/or recommended dose for expansion of CPA.9.086.H4(S241P) monotherapy and in combination with CHA.7.518.1.H4(S241P). [Time Frame: 18 months.]

Evaluation of a dose of CPA.9.086.H4(S241P) monotherapy and in combination with CHA.7.518.1.H4(S241P) that is well tolerated by subjects.

To characterize the pharmacokinetic (PK) profile of CPA.9.086.H4(S241P) as monotherapy and in combination with CHA.7.518.1.H4(S241P). [Time Frame: 18 months.]

Evaluation of parameters of CPA.9.086.H4(S241P) monotherapy or in combination with CHA.7.518.1.H4(S241P) exposure such as Maximum Plasma Concentration [Cmax]), AUC.

### Secondary Outcome Measures:

To characterize immunogenicity of CPA.9.086.H4(S241P) monotherapy and in combination with CHA.7.518.1.H4(S241P). [Time Frame: 18 months.]

Evaluation of anti drug antibody to CPA.9.086.H4(S241P) (monotherapy) or CPA.9.086.H4(S241P), CHA.7.518.1.H4(S241P) when administered in combination.

### Other Pre-specified Outcome Measures:

Evaluation of the preliminary antitumor activity of CPA.9.086.H4(S241P) as monotherapy and in combination with CHA.7.518.1.H4(S241P). [Time Frame: 24 months.]

An assessment of antitumor activity eg ORR, PFS, DOR with CPA.9.086.H4(S241P) monotherapy and CPA.9.086.H4(S241P) in combination with CHA.7.518.1.H4(S241P).

### Inclusion Criteria:

Subjects with histologically/cytologically confirmed advanced malignancy (solid tumor) who must have exhausted all available standard therapy; or not a candidate for standard therapy.

Subject is able to provide written, informed consent before initiation of any study related procedures, and is able, in the opinion of the investigator, to comply with all the requirements of the study.

Subject has Eastern Cooperative Oncology Group (ECOG) performance status 0-1.

### Exclusion Criteria:

Prior treatment with a TIGIT inhibitor.

Symptomatic interstitial lung disease or inflammatory pneumonitis.

History of immune-related events that required immunotherapy treatment discontinuation.

### NSCLC Inclusion Criteria:

- Stage IV or recurrent NSCLC.
- Disease recurrence or progression during or after prior treatment that included:
   ∘ platinum-based doublet chemotherapy regimen that may include continuation off or switch to maintenance therapy with pemetrexed, bevacizumab, or erlotinib;
   ∘ anti-programmed cell death protein-1 (anti-PD-1) or anti-PD-L1-directed therapy.
- Subjects with known EGFR mutation or ALK translocation have to have received or be receiving an additional line of relevant tyrosine kinase inhibitor therapy (prior treatment with platinum-based chemotherapy not required)
   ∘ Subjects with NSCLC tumors harboring EGFR T90M mutation will be required to have disease progression on osimertinib;
   ∘ Prior treatment with FDA approved targeted therapy is required for subjects with NSCLC tumors harboring other genomic aberrations for which FDA-approved targeted therapy is available (e.g., ROS rearrangement, BRAF V600E mutation)

### Colorectal cancer (micro satellite stable) inclusion criteria:

- Histologically confirmed adenocarcinoma of the colon/rectum
- Stage IV disease
- Subjects must have documented MSS status by an FDA approved test e.g. genomic testing, IHC for mismatch repair proficient
- Disease progression with ≥2 prior systemic therapies for metastatic CRC that must have included the following: fluroropyrimidines, irinotecan, and oxaliplatin

### HNSCC inclusion criteria:

- Histologically confirmed recurrent or metastatic HNSCC (oral cavity, pharynx, larynx)
- Prior receipt of platinum therapy anti-programmed cell death protein-1 (anti-PD-1) or anti-PD-L1-directed therapy.
- Documentation of p16-positive or p16-negative disease to determine human papillomavirus (HPV) status of tumor.

### Multiple myeloma inclusion criteria:

- Previously treated relapsed and refractory multiple myeloma per International Myeloma Working Group consensus criteria (see, Rajkumar et al., 2011).
- Prior therapy ≥3 lines including an immunomodulatory drug (e.g. lenalidomide, pomalidomide), a proteasome inhibitor (e.g., bortezomib, carfilzomib), and anti-CD38 monoclonal antibody (e.g. daratumumab)
- Measurable disease of multiple myeloma as defined by at least one of the following (IgD and IgA with monoclonal protein < 0.5 g/dL may be permitted after discussion with PI):
   ∘ Serum monoclonal protein ≥ 0.5 g/dL (or quantitative IgA ≥ 1000 mg/dL), or
   ∘ ≥ 200 mg of monoclonal protein in the urine on 24-hour urine protein electrophoresis, and/or
   ∘ Serum free light chain ≥ 100 mg/L (10 mg/dL) and abnormal serum free kappa to serum free kappa light chain ratio.

### EXAMPLE 2: PATIENT DOSED IN PHASE 1 COMBINATION STUDY OF CPA.9.086.H4(S241P) AND CHA.7.518.1.H4(S241P) IN PATIENTS WITH ADVANCED MALIGNANCIES

Combination study evaluates dual blockade of PVRIG and TIGIT, parallel and distinct DNAM axis checkpoint pathways. Phase 1 CPA.9.086.H4(S241P) monotherapy dose escalation study is complete.

First patient has been dosed in its Phase 1 clinical trial evaluating the dual combination of CPA.9.086.H4(S241P), a high-affinity anti-TIGIT antibody and CHA.7.518.1.H4(S241P), an anti-PVRIG antibody in patients with advanced malignancies.

This combination study is an important component in the clinical strategy and forevaluating the dual blockade of DNAM axis members PVRIG and TIGIT. While not being bound by theory, dual blockade of these distinct checkpoint pathways has the potential to expand the number of patients who respond to treatment with immunotherapy. The Phase 1 CPA.9.086.H4(S241P) monotherapy dose expansion and combination with CHA.7.518.1.H4(S241P) dose escalation and cohort expansion arms of this study are designed to assess the safety, tolerability, pharmacokinetics, pharmacodynamics, and preliminary antitumor activity of CPA.9.086.H4(S241P) alone and in combination with CHA.7.518.1.H4(S241P) in patients with advanced malignancies who have exhausted all available standard therapies. The CPA.9.086.H4(S241P) monotherapy dose expansion study, will be in subjects with select tumor types preferably subjects with multiple myeloma. The CPA.9.086.H4(S241P) in combination with CHA.7.518.1.H4(S241P) dose expansion arm is expected to be in select tumor types, HNSCC, CRC (MSS) and NSCLC.

### CPA.9.086.H4(S241P)

CPA.9.086.H4(S241P) is a high affinity, fully human antibody that blocks the interaction of TIGIT with PVR, its ligand, and consequently enhances T cell function. Data suggests that CPA.9.086.H4(S241P) has *in vitro* activity comparable or superior to TIGIT antibodies in clinical development. It is currently being evaluated in a Phase 1 clinical trial in patients with advanced malignancies who have exhausted all available standard therapies. Compugen has demonstrated in preclinical studies that simultaneous inhibition of TIGIT and PVRIG, the two coinhibitory arms of the DNAM axis, can increase antitumor immune responses, which may be further enhanced with the addition of PD-1 blockade. These data suggest that treatment with CHA.7.518.1.H4(S241P) and CPA.9.086.H4(S241P), targeting PVRIG and TIGIT, respectively, alone or in combination with a PD-1 inhibitor, has the potential to expand immuno-oncology treatment to patient populations who are non-responsive or refractory to existing immunotherapies..

### CHA.7.518.1.H4(S241P)

CHA.7.518.1.H4(S241P) is a humanized antibody that binds with high affinity to PVRIG, a novel immune checkpoint discovered computationally by Compugen, blocking the interaction with its ligand, PVRL2. Blockade of PVRIG by CHA.7.518.1.H4(S241P) has demonstrated in preclinical studies potent, reproducible enhancement of T cell activation, consistent with the desired mechanism of action of activating T cells in the tumor microenvironment to generate anti-tumor immune responses. PVRIG and TIGIT, constitute parallel immune checkpoint pathways that counteract DNAM, a costimulatory molecule on T cells and NK cells. As such, preclinical data suggest that the inhibition of PVRIG together with TIGIT and/or PD-1 has the potential to further enhance anti-tumor immune response and improve patient outcomes in a broad variety of tumor types.

### EXAMPLE 3: DOMINANT EXPRESSION OF DNAM-1 AXIS MEMBERS IN BM OF MM PATIENTS

### A. Introduction

Blocking inhibitory immune checkpoints holds the potential to treat multiple myeloma (MM) patients. However, thus far checkpoint inhibitors have not shown significant clinical benefits for MM patients, warranting the need for alternative checkpoint blockers. The immune checkpoint TIGIT was recently shown to be the most upregulated immune inhibitory receptor on CD8⁺ T cells in MM patients' bone marrow (BM), compared to other checkpoints (Guillerey C., Blood. 2018). Preclinical models demonstrated the dominant effects of TIGIT blockade or depletion, by significantly improving mice survival, reducing myeloma cell numbers and exhausted T cell hallmarks (Minnie S., Blood. 2018). As a result, several clinical trials using anti-TIGIT monoclonal antibody as monotherapy or in combination with PDL-1 and LAG-3 inhibitors have been recently initiated in MM patients.

The DNAM-1 family, in addition to TIGIT, also includes the inhibitory receptor PVRIG, that competes with the co-activating receptor DNAM-1 for the binding to the shared ligand PVRL2, similarly to the TIGIT/PVR/DNAM-1 interaction. Accordingly, TIGIT and PVRIG co-blockade were shown to synergize in enhancing T cell activity and anti-tumor activity in preclinical models (Whelan S., Cancer Immunol. Res. 2019). PVRL2 together with PVR (ligand of TIGIT) were shown to be highly expressed on plasma cells and on CD14⁺ cells in BM of MM patients (Lozano E., Clin. Cancer Res. 2020). This study aimed at evaluating DNAM-1 axis receptors expression in MM patients' BM.

### B. Methods

Fresh BM aspirates were collected from 21 MM patients with progressive disease (PD) or in complete response (CR) after obtaining IRB approval. BM mononuclear cells were isolated and single cell suspensions were obtained followed by staining with anti-human antibodies to evaluate DNAM-1 axis members and PD-1 expression. BM biopsies from 6 MM patients (each patient had 4 score on the Tissue Micro-Array T291 USBiomax) were stained for PVRL2 expression by immune-histochemistry (IHC).

### C. Results

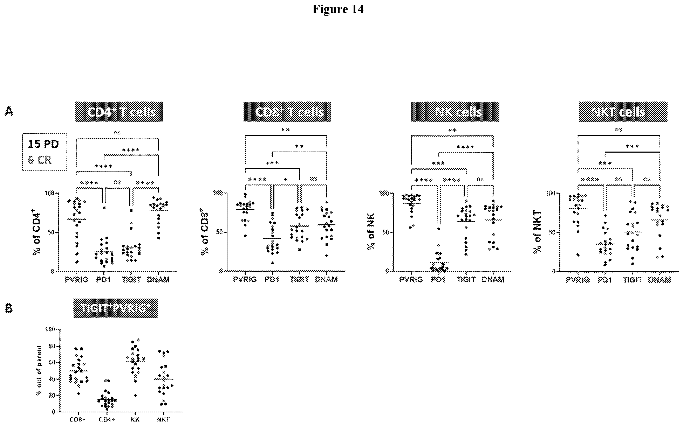

Flow cytometry analysis of PD-1 and DNAM-1 axis receptors revealed a significant lower fraction of PD-1⁺ cells among cell populations examined compared with other receptors. TIGIT expression was the highest on NK, CD8⁺ and NKT cells compared to CD4⁺ T cells, which is in line with previous published data (Lozano E. Clin. Cancer Res. 2020). In contrast, DNAM-1 was expressed on CD8⁺ T, NK and NKT cells with prominently high expression on CD4⁺ T cells (Fig. 14A). The highest expression among the receptors was of PVRIG on all lymphoid populations, except CD4⁺ where DNAM-1 was more highly expressed. Importantly, 50% of CD8⁺ T cells co-expressed TIGIT and PVRIG, supporting a combinatorial therapeutic approach (Fig. 14B).

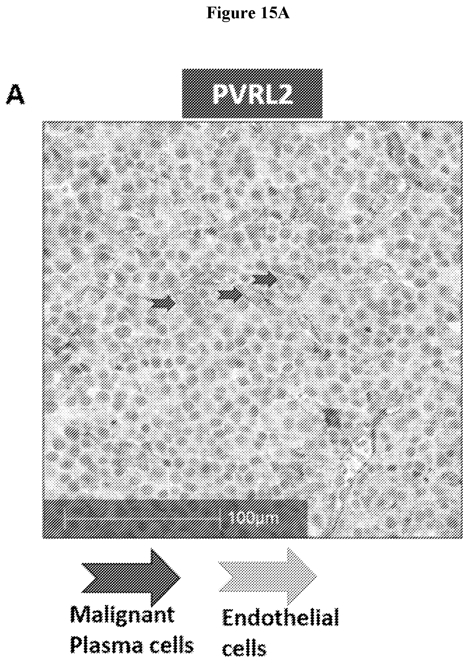

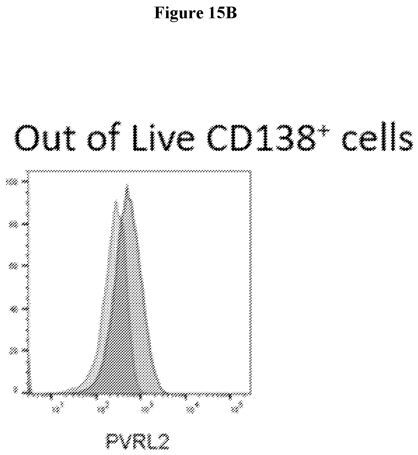

Additionally, the expression of the PVRL2 ligand on MM plasma and endothelial cells was demonstrated by IHC. FACS analysis further supported PVRL2 expression on plasma cells in MM BM (Fig. 15).

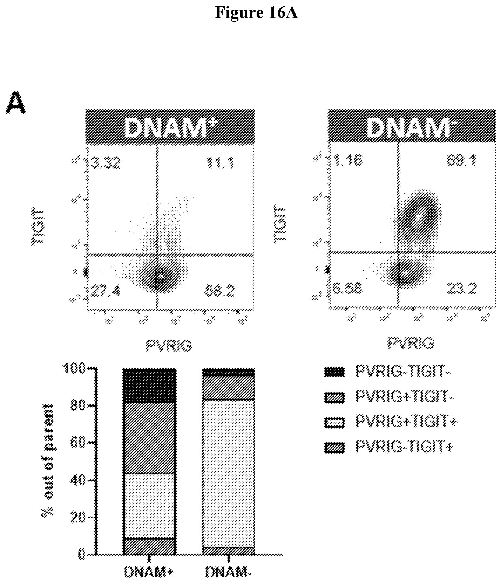

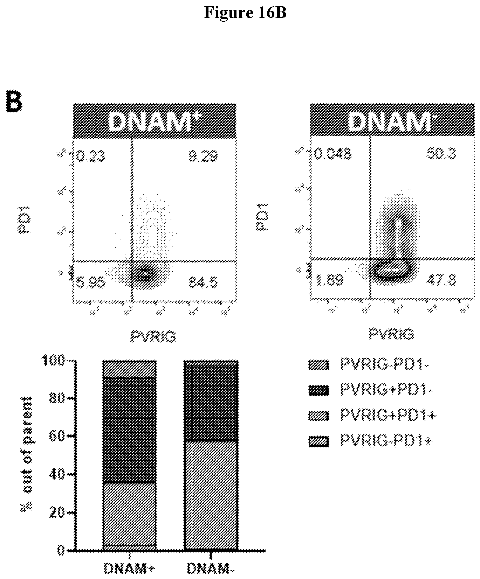

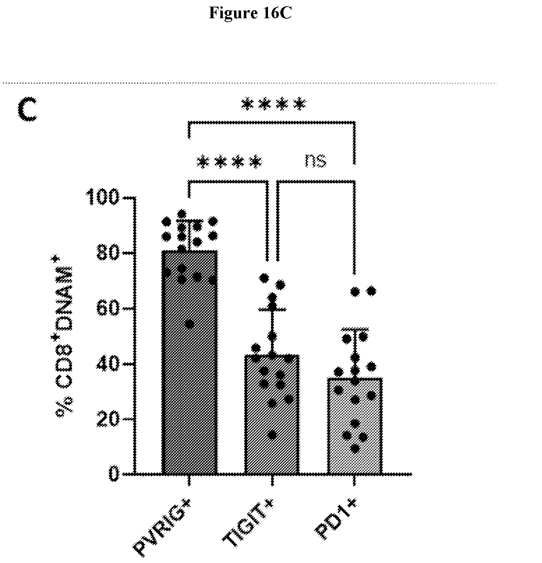

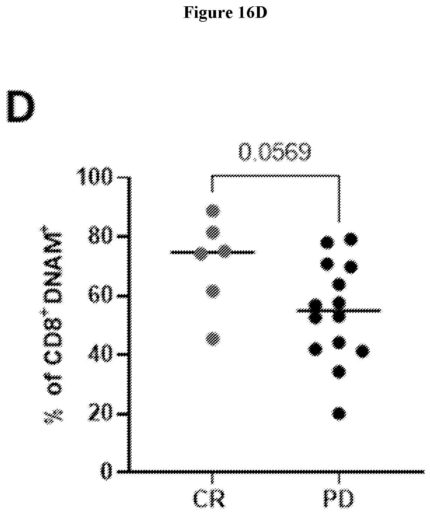

A higher expression of PVRIG, TIGIT and PD-1 was present on DNAM-1 negative CD8⁺ T cells (Figs. 16A, B), suggesting accumulation of exhausted cells in MM tumor microenvironment (TME) as previously described (Minnie S., Blood. 2018). PVRIG had significantly higher expression on DNAM⁺ cells, compared to PD-1 and TIGIT (Fig 3C), suggesting the potential of its blockade to enhance DNAM-1 activation and subsequent proliferation of earlier differentiated memory cells in MM TME. Finally, CR patients had a trend for higher DNAM-1 expression on CD8⁺ T cells compared to those with PD (Fig. 16D). This is consistent with other reports in mice showing that the expression of DNAM-1 negatively correlates with BM myeloma cell numbers (Minnie S., Blood. 2018)*.*

### D. Conclusions

DNAM-1 axis receptors are dominantly expressed on lymphocytes in BM of MM patients, with PVRIG exhibiting the most prominent expression. The reduced expression of DNAM-1 in PD patients TME, compared to CR patients, suggests a link between DNAM-1 axis and clinical outcomes. As recently TIGIT became an attractive target for blockade in MM, our new findings highlight the dominant expression of PVRIG and suggest that combined blockade of TIGIT with PVRIG may potentially benefit MM patients, placing the DNAM-1 axis as a dominant pathway in MM therapy.

### EXAMPLE 4: EXPRESSION OF PVRIG AND TIGIT IN MULTIPLE MYELOMA BONE MARROW

### I. BACKGROUND/STUDY RATIONALE

### E. Myeloma treatment

Multiple myeloma (MM) is a bone marrow (BM)-based malignancy characterized by the expansion of plasma cells that produce monoclonal immunoglobulin (IgG, IgA, IgD, IgE, or free λ or κ light chains). The overall survival of patients with MM varies greatly from a few months to many years; the mean is approximately seven years. Anemia, hypercalcemia and bone lesions correlate directly with total mass of myeloma cells and have important prognostic significance. Other prognostic factors include age, the plasma cell labeling index, serum albumin, b2-microglobulin, C-reactive protein, thymidine kinase, and soluble interleukin-6 receptor. Recently, serum B-cell maturation antigen has been identified as a new prognostic factor for MM. Major complications, such as infection and renal insufficiency, are the main causes of death for myeloma patients.

Alkylating agents such as melphalan, bendamustine and cyclophosphamide are among the most effective drugs against MM. Other drugs, including vinca alkaloids, nitrosoureas and anthracyclines, are also effective in treating MM when combined with steroids but do not improve survival compared to the combination of oral melphalan and prednisone. Nor has the commonly used combination of vincristine, doxorubicin and dexamethasone (VAD) improved median survival compared to other regimens in randomized clinical trials. Its main advantage is that it rapidly induces remissions which may be important in the subset of patients that present with renal failure or hypercalcemia.

High-dose chemotherapy followed by autologous hematopoietic support also represents another treatment option for MM patients. However, the disease remains incurable with any currently available therapeutic modality. Anti-myeloma agents such as the proteasome inhibitors bortezomib, carfilzomib and ixazomib, the DNA-damaging anthracycline agent doxorubicin or the derivative pegylated liposomal doxorubicin, the immunomodulatory agents thalidomide, lenalidomide and pomalidomide, monoclonal antibodies daratumumab and elotuzumab, the selective inhibitor of nuclear export selinexor, and arsenic trioxide (ATO) have proven to be potent inhibitors of MM cell growth and are now therapeutic options for these patients, especially when used in combination with other anti-MM agents. Recently, the monoclonal antibodies daratumumab and elotuzumab have shown anti-MM effects. We have shown, using our unique animal models, that myeloma cells are sensitive to treatment with the proteasome inhibitor bortezomib (Campbell *et al,* 2006a). Specifically, LAGl-1-bearing mice treated with high dose bortezomib showed significant decreased tumor volume and paraprotein levels versus low dose bortezomib and vehicle. In addition, the combination of melphalan and bortezomib demonstrated marked synergism *in vitro* (Ma *et al,* 2003). Clinically, we conducted a Phase I study and established the safety and efficacy of low-dose oral melphalan and bortezomib in relapsed/refractory MM patients (Berenson *et al,* 2006a). Using the maximally tolerated dose of this combination with oral ascorbic acid, we have used this combination as frontline treatment for myeloma and demonstrated a high response rate with minimal toxicity (Berenson *et al,* 2007). During the past decade, we have used these SCID-hu MM models to evaluate many different drugs and combinations which have led to a variety of different successful treatments for MM patients, including the JAK inhibitor ruxolitinib (RUX) in combination with lenalidomide and steroids (Chen *et al,* 2018).

Immunomodulatory drugs (IMiDs) have now been shown to block several pathways important for disease progression in multiple myeloma. First established as agents with antiangiogenic properties, thalidomide and IMiDs inhibit the production of interleukin (IL)-6, which is a growth factor for the proliferation of myeloma cells. In addition, they activate apoptotic pathways through caspase 8-mediated cell death. At the mitochondrial level, they are responsible for c-jun terminal kinase (JNK)-dependent release of cytochrome-c and Smac into the cytosol of cells, where they regulate the activity of molecules that affect apoptosis. By activating T cells to produce IL-2, thalidomide and IMiDs alter natural killer (NK) cell numbers and function, thus augmenting the activity of NK-dependent cytotoxicity. Lenalidomide, a thalidomide derivative, has also been shown to have a different toxicity profile. Lenalidomide (Revlimid) has been shown to have anti-tumor effects in MM and other hematological malignancies. It enhances the expression of tumor suppressor genes (p21 & p27), induces G1 cell cycle arrest, caspase activation, and apoptosis. Furthermore, this drug shows immunomodulatory effects and co-stimulates T and NK cells. Lenalidomide in combination with dexamethasone is indicated for the treatment of MM patients who have received at least one prior therapy. In addition, recent studies show high response rates among relapsed and refractory patients when lenalidomide is combined with dexamethasone and bortezomib.

Despite improvements in anti-MM therapy, the disease remains incurable and nearly all patients eventually develop resistance to these therapies. Thus, additional tumor target and therapeutic options that are capable of overcoming drug resistance are critical to help improve the outcome for these patients.

### F. Targeting of PVRIG and TIGIT in MM

Therapeutic antibodies targeting the CTLA4/PD-1 pathways have revolutionized cancer immunotherapy by encouraging anti-tumor responses in patients with solid tumors and hematological malignancies. The expression of the inhibitory receptors CTLA-4, PD-1, LAG-3, and TIM-3 in MM patients may indicate underlying mechanisms of T-cell dysfunction (Chung DJ et al, Cancer Immunol Res 2016), which could be potentially reversed by blocking antibodies.

Although initial data supported the rationale for PD-1 blockade to stimulate anti-MM immunity, therapeutic antibody nivolumab as a single agent did not show a significant improvement in the treatment of patients with multiple myeloma (Suen H et al, Leukemia 2015) highlighting the need to investigate other immune regulatory pathways relevant for MM patients.

The poliovirus receptor-related Ig domain-containing protein (PVRIG) and T-cell immune-receptor with Ig and ITIM domains (TIGIT) are non-redundant co-inhibitory receptors expressed in T cells and NK cells. The cognate ligand of TIGIT is poliovirus receptor (PVR, CD155), whereas the cognate receptor of PVRIG is PVR-related 2 (PVRL2, Nectin-2, CD112). DNAM-1 (CD226) expressed on the surface of natural killer (NK) cells, T lymphocytes, platelets, monocytes, and a subset of B cells. PVRL2 and PVR were identified as DNAM-1 ligands, and upon their ligation, a co-stimulatory effect is mediated.

### G. Aims of the study

There is very limited data on the expression of TIGIT in MM. A recent report showed TIGIT expression on CD8+ T cells in BM of MM patients (Guillerey et al., Blood 2018). However, the expression of PVRIG on CD4+ and CD8+ T, NK and NKT cells in MM remains to be investigated. In this study, we will investigate the expression of PVRIG, TIGIT, DNAM-1 and PD-1 on lymphocytes infiltrating BM aspirates of MM patients. The expression of PVRL2, PVRIG ligand will be investigated by IHC in MM biopsies.

### II. EXPERIMENTAL DESIGN AND METHODS

Determination of PVRIG and TIGIT expression on bone marrow and peripheral blood mononuclear cells from MM patients including tumor cells, T-cells and NK cells

### 1. Preparation of MM bone marrow tumor cells

Twenty-one fresh BM aspirates from MM patients (15 Progressive disease (PD)/ relapsed MM patients and 6 complete remission CR patients) were collected from donors following informed consent (Figure 23). An Institutional Review Board approval was received to obtain blood and bone marrow for research purposes through Western IRB (Protocol 101). BM mononuclear cells (MCs) were isolated using density gradient centrifugation with Histopaque-1077 (Sigma, St Louis) according standard protocol.

### 2. Antibodies to be used for flow cytometric analysis are listed in Table 4.

**Table 4. Antibodies used in the flow cytometric analysis.**

| **Antibody** | **Clone** | **Vendor** | **Cat#** |
|---|---|---|---|
| CD3-BV510 | OKT3 | Biolegend | 317332 |
| CD4- Percp-efluor 710 | SK3 | Invitrogen | 46-0047-42 |
| CD8- FITC | SK1 | Biolegend | 344704 |
| CD56- APC-Fire750 | 5.1H11 | Biolegend | 362554 |
| CD138-BUV805 | MI15 | BD | 748350 |
| PVRIG-PE | W16216D | Biolegend | 301504 |
| TIGIT-BV421 | A15153G | Biolegend | 372710 |
| PD1-APC | EH12.2H7 | Biolegend | 329908 |
| DNAM-BV786 | DX11 | BD | 742497 |
| Live/dead: UV450 | | Tonbo bioscience | 13-08668-T100 |
| Human TruStain FcX blocking solution | | Biolegend | 422302 |

### 3. Flow cytometric assay to be performed with antibodies to the following proteins in the following groups (all double staining will include viability stain):

Flow cytometric assay was performed with antibodies for targeted markers (anti-human PVRIG, anti-human TIGIT, anti-human DNAM, anti-human CD3, anti-human CD4, anti-human CD8, anti-human CD56, and anti-human CD138) as well as their matched isotypes. Full minus one (FMO) isotype controls were included for each experiment. Briefly, fresh MM BM tumor cells were isolated from MM patients, and then blocked using 5% FBS for preventing non-specific binding. The cells were treated with different antibodies and isotype controls, and then fixed using 2% paraformaldehyde for 30 minutes on ice. Flow cytometric analyses were performed using a Becton Dickinson FACSymphony S6 (excitation at 355, 406, 488, 552, and 640 nm). The 20 BM samples included in the study are listed in Table 5.

BM stands for Patient Bone Marrow sample ID; CR= complete response; PD= progressive disease

**Table 5. List of MM patients participating in the study.**

| | BM | Date | Status |
|---|---|---|---|
| 1 | **2910** | 11/11/2020 | PD |
| 2 | **3481** | 11/11/2020 | PD |
| 3 | **3603** | 11/10/2020 | PD |
| 4 | **1974** | 11/30/2020 | PD |
| 5 | **3086** | 12/7/2020 | CR |
| 6 | **3530** | 12/9/2020 | CR |
| 7 | **2145** | 12/9/2020 | CR |
| 8 | **2460** | 12/14/2020 | CR |
| 9 | **2672** | 12/16/2020 | PD |
| 10 | **3570** | 12/18/2020 | CR |
| 11 | **3611** | 12/21/2020 | PD |
| 12 | **2501** | 12/28/2020 | PD |
| 13 | **2632** | 1/11/2021 | PD |
| 14 | **2399** | 1/11/2021 | PD |
| 15 | **2188** | 1/14/2021 | CR |
| 16 | **3610** | 1/15/2021 | PD |
| 17 | **3427** | 3/17/2021 | PD |
| 18 | **1429** | 3/25/2021 | PD |
| 19 | **3439** | 3/30/2021 | PD |
| 20 | **3458** | 4/7/2021 | PD |
| 21 | **3501** | | PD |

Clinical Data and treatment history of all the patients is provided in Figure 23.

### IHC

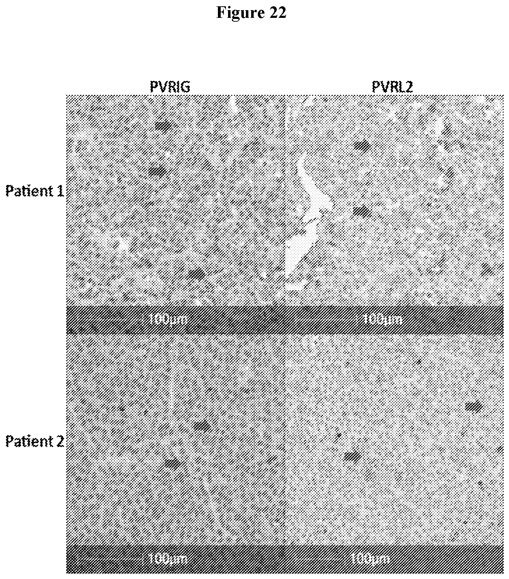

In parallel to FACS analysis, in order to validate protein expression of PVRL2 and PVRIG on MM cells in infiltrating TILs, respectively. We have preformed IHC analysis on small TMA of MM (T291d TMA by Biomax US). Both PVRL2 and PVRIG were detected in this small TMA, PVRL2 on endothelial and malignant cells, and PVRIG on infiltrating lymphocytes (Figure 22). Figure 22 presents IHC staining, using mAb, of PVRIG and PVRL2 on MM bone marrow biopsy TMA. PVRL2 stained endothelial cells (blue arrows) and some malignant plasma cells (orange arrows), in malignant plasma cells PVRL2 could be detected on membrane and/or cytoplasmatic speckles. PVRIG stains infiltrating lymphocytes (red arrows). Out of the 4 biopsies on the TMA, two had lymphocytic infiltration and two were absent of infiltration, hance no PVRIG could be evaluated. The two positive samples are presented.

### III. RESULTS - SUMMARY

### 1. PVRIG is highly expressed on CD4⁺ and CD8⁺ T-cells as well as NK and CD3+CD56+ NKT cells in MM bone marrow mononuclear cells.

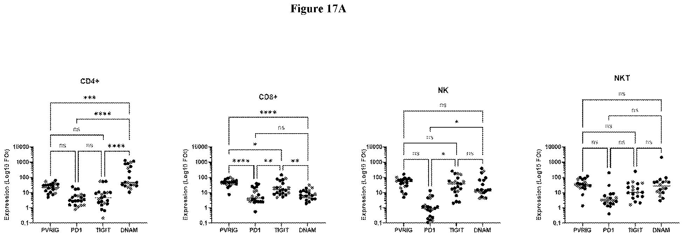

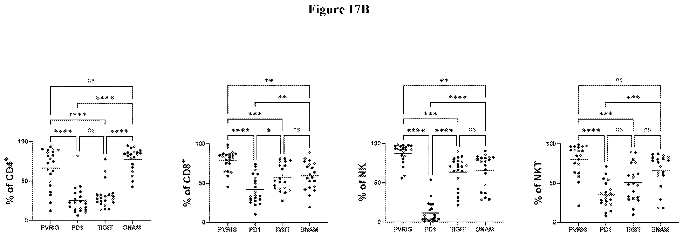

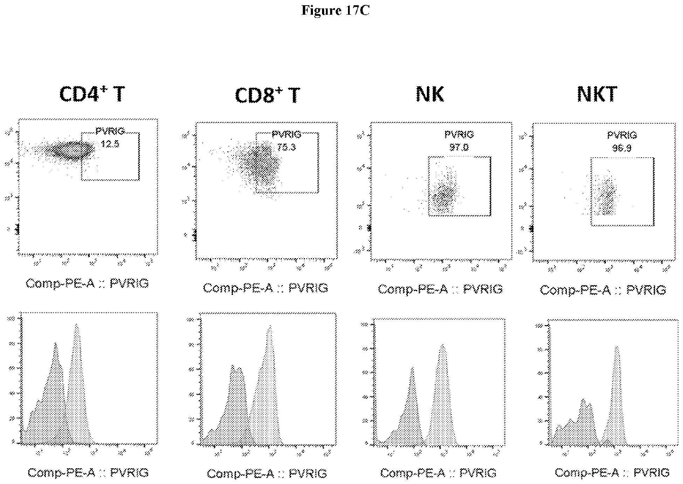

The expression of PVRIG, TIGIT, PD-1 and DNAM-1 was assessed in BM of MM CR and PD patients. As shown in figure 17, all inhibitory receptors are expressed in varying levels on all examined cell populations. PVRIG was preferentially expressed on CD8+ T cells, as well as on NK and NKT cells compared to other inhibitory molecules, whereas CD4+ T cells dominantly expressed DNAM-1 (Figure 17). The expression of PD-1 (figure 17A) and the percentage of PD-1+ lymphocytes (Figure 17B) were the lowest, relatively to the expression of other molecules. This indicates on the dominant expression of the DNAM-1 axis molecules, PVRIG, TIGIT and DNAM-1, in BM of MM patients.

Figure 17A presents the expression of PVRIG, TIGIT, PD-1 and DNAM-1 on CD4, CD8 T cells and NK, NKT cells, as Log10 of the fold of stain mean florescence intensity (MFI) over isotype control MFI.

Figure 17B presents the expression of PVRIG, TIGIT, PD-1 and DNAM-1 on CD4, CD8 T cells and NK, NKT cells, as percentages of the population.

Figure 17C presents a representative histogram showing the expression of PVRIG on MM patient BM lymphocytes.

### 2. PVRIG is co-expressed with TIGIT on CD8⁺ T-cells as well as NK and CD3⁺CD56⁺ NKT cells in MM bone marrow mononuclear cells.

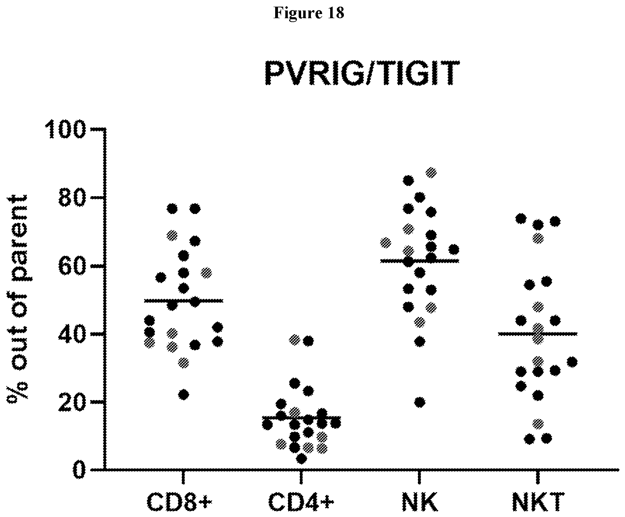

We also examined the co-expression of PVRIG with TIGIT on lymphocytes in BM of MM patients. As shown in Figure 18, PVRIG and TIGIT are co-expressed on high percentage of CD8+ T cells, NK and NKT cells.

### 3. PVRIG is co-expressed with DNAM-1 on CD8⁺ and CD4⁺ T-cells as well as NK and CD3⁺CD56⁺ NKT cells in MM bone marrow mononuclear cells.

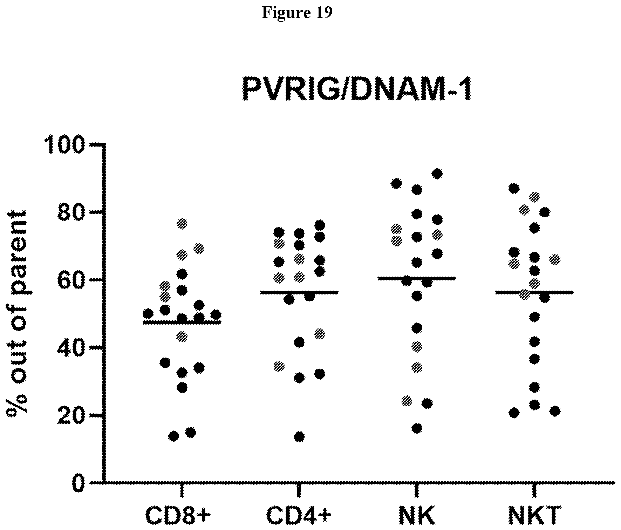

PVRIG and DNAM-1 are co-expressed on above 50% of lymphocytes in BM of MM patients (Figure 19).

We also examined the co-expression of PVRIG with DNAM1 on lymphocytes in BM of MM patients. Figure 19 presents DNAM1 and PVRIG co-expression on CD4, CD8 T cells and NK, NKT cells.

As shown in Figure 19, PVRIG and DNAM1 are co-expressed on CD8+ T cells, NK and NKT cells.

### 4. TIGIT and DNAM-1 are co-expressed on NK cells.

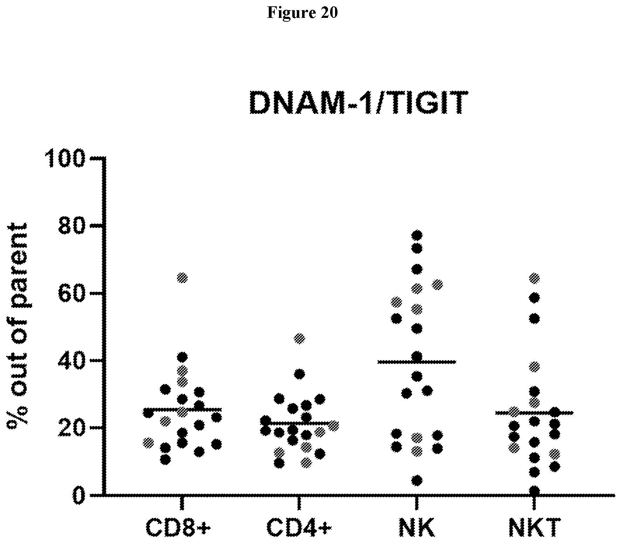

We also tested co-expression of TIGIT and DNAM-1 on T, NK and NKT cells. Figure 20 shows DNAM-1 and TIGIT co-expression on CD4, CD8 T cells, NK cells and NKT cells.

As shown in Figure 20, TIGIT and DNAM-1 are mainly co-expressed on NK cells (with mean of about 40% of total NK cells). Lower degree of co-expression was observed on T cells or NK cells (less than 30% of ceslls).

### 5. DNAM-1 expression levels on CD8 T cells positively correlate with complete response in MM patients.

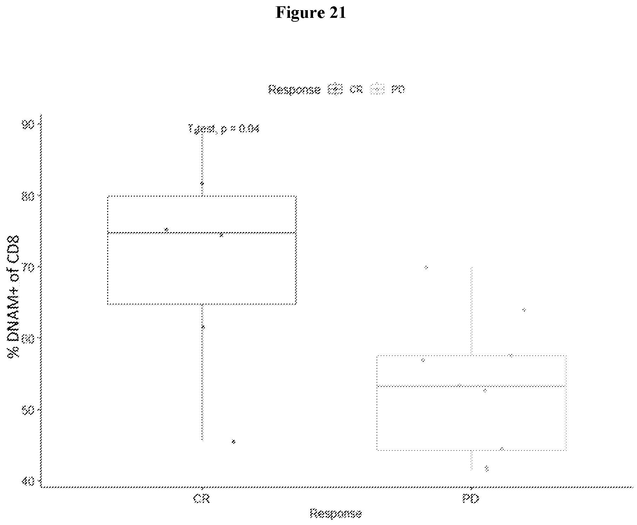

T-test comparing complete responders to progressive disease was preformed on all data points from the FACS analysis (% positive cells to specific marker and MFI of the specific marker per cell type). The significant results (p-value ≤ 0.05) were found for the precent of CD8⁺ T cell positive for DNAM, which was significantly higher in the complete responders (Figure 21).

### 6. PVRIG and PVRL2 expression in bone marrow biopsy of MM patient

In parallel to FACS analysis, in order to validate protein expression of PVRL2 and PVRIG on MM cells in infiltrating TILs, respectively. We have preformed IHC analysis on small TMA of MM (T291d TMA by Biomax US). Both PVRL2 and PVRIG were detected in this small TMA, PVRL2 on endothelial and malignant cells, and PVRIG on infiltrating lymphocytes (Figure 22). Figure 22 presents IHC staining, using mAb, of PVRIG and PVRL2 on MM bone marrow biopsy TMA.

### EXAMPLE 5: THE EXPRESSION OF PVRIG AND TIGIT PATHWAY IS DOMINANT IN MULTIPLE MYELOMA PATIENTS BONE MARROW

Blocking inhibitory immune receptors have shown limited clinical benefits in multiple myeloma (MM) patients, warranting the need for alternative checkpoint blockers. The regulatory receptor TIGIT was shown to be highly expressed on CD8+ T cells in MM patients' bone marrow (BM) and its blockade in preclinical models resulted in significantly improved mice survival and reduced T cell exhaustion. Accordingly, clinical trials using anti-TIGIT antibodies have been recently initiated. In addition to TIGIT, the DNAM-1 axis includes the novel inhibitory receptor PVRIG. Both TIGIT and PVRIG deliver inhibitory signal into T and NK cells and compete with the co-activating receptor DNAM-1 for binding to PVR and PVRL2 respectively. Accordingly, TIGIT/PVRIG co-blockade was shown to synergize in enhancing T cell responses and anti-tumor activity in preclinical models. PVRL2 and PVR were shown to be expressed on plasma cells in the MM BM. In this study, we evaluated DNAM-1 axis receptors expression in BM of MM patients.

BM mononuclear cells derived from 21 MM patients with progressive disease (PD), or complete response (CR) were analyzed for PD-1 and DNAM-1 axis expression by flow cytometry. Additionally, BM biopsies from 6 patients were assessed for PVRL2 expression by immuno-histochemistry (IHC).

Flow cytometric analysis of receptors expression revealed a significantly lower fraction of PD 1+ cells across the examined lymphoid cell populations, compared to other receptors. Notably, PVRIG demonstrated the highest expression among the evaluated receptors on NK, NKT and CD8+ T cells, while TIGIT showed significantly higher expression on NK and CD8+ T cells compared to PD-1. Importantly, 50% of CD8+ and 60% of NK cells co-expressed TIGIT and PVRIG, and all examined cell populations showed high (>50%) DNAM-1 expression. Moreover, the expression of PVRIG ligand, PVRL2 on BM plasma cells was demonstrated by IHC and flow cytometry.

Co-expression of PVRIG/TIGIT/PD1 was abundant on DNAM-1- CD8+ T cells, suggesting the accumulation of an exhausted CD8+ T cell population in the MM tumor microenvironment. PVRIG showed significantly higher expression on DNAM+ CD8+ T cells (81%), compared to TIGIT (43%) and PD-1 (34%), supporting the potential of PVRIG blockade to enhance DNAM-1 signaling and subsequently activate CD8+ T cells. Finally, CR patients had a trend towards higher DNAM-1 expression on CD8+ T cells (75%) compared to PD patients (54%, p=0.057).

To conclude, DNAM-1 axis receptors are dominantly expressed on MM BM lymphocytes, with PVRIG exhibiting the most prominent expression. The reduced expression of DNAM-1 in PD patients suggests a link between DNAM-1 axis and clinical outcome. Our findings highlight for the first time the dominant expression of PVRIG, as well as TIGIT and suggest that combined blockade of these checkpoints may potentially benefit MM patients, placing the DNAM-1 axis as a promising therapeutic pathway in MM.

### EXAMPLE 6: CPA.9.086.H4(S241P) (ANTI-TIGIT ANTIBODY) MONOTHERAPY - PRELIMINARY EVALUATION OF SAFETY, TOLERABILITY, PHARMACOKINETICS AND RECEPTOR OCCUPANCY IN PATIENTS WITH ADVANCED SOLID TUMORS

Background: CPA.9.086.H4(S241P) is an IgG4 fully human high-affinity monoclonal antibody, inhibitor of TIGIT (T cell Ig and immunoreceptor tyrosine-based inhibitory motif [ITIM] domain) binding to poliovirus receptor (PVR). It has been demonstrated that TIGIT blockade enhances synergistic activation of T-cells (*see,* Whelan, et al., Cancer Immunol Res. 2019). It has been emonstrated that CPA.9.086.H4(S241P) enhance T and NK activity *in-vitro* and *in-vivo* (Hansen, et al., Cancer Immunol. Immunother., 2021)*.*

TIGIT blockade by CPA.9.086.H4(S241P) was shown to enhance anti-tumor immunity in pre-clinical models. CPA.9.086.H4(S241P) as monotherapy will have an acceptable safety, tolerability, antitumor activity, pharmacokinetics, immunophenotyping and cytokine analyses, including those who have exhausted all available standard therapies.

This example provides preliminary results of pts in dose escalation on safety, tolerability, pharmacokinetics, receptor occupancy and lymphocytes immunophenotyping.

**Methods:** Utilizing an accelerated titration and 3+3 study design we enrolled 18 patients (pts) at the following CPA.9.086.H4(S241P) doses 0.01, 0.03, 0.1, 0.3, 1, 3 and 10 mg/kg IV Q3 wks. These 18 patients had advanced solid tumors who had exhausted all available standard therapies. Key primary objectives were to evaluate the safety, tolerability (CTCAE v5.0), to characterize the pharmacokinetics (PK) and to select a recommended dose for expansion (RDFE). An exploratory objective was evaluation of peripheral receptor occupancy (RO). Key inclusion criteria: Age ≥18 yrs, histologically/cytologically confirmed advanced malignancy who have exhausted all available standard therapy or not a candidate for standard therapy. Patients with performance status ECOG 0-1, prior ICI permissible. Dose-limiting toxicities (DLTs) were evaluated within a 21-day window in the 1st cycle of dose escalation.

Doses of CPA.9.086.H4(S241P) evaluated were 0.01, 0.03, 0.1, 0.3, 1, 3, 10 [mg/kg IV Q3W]. Dose-limiting toxicity was evaluated in the 1^{st} 21 days in the 1^{st} cycle of dose escalation. In the single-subject cohorts [0.01 - 0.3 mg/kg]: If the single toxicity Grade ≥2 at least possibly related to CPA.9.086.H4(S241P) is reported during the DLT period, 2 additional subjects were enrolled. Other dose cohorts utilized a 3+3 study design. Antitumor activity was evaluated per RECIST v1.1 with CT scans Q6W and response assessment per investigator. Safety assessment evaluated per CTCAE v5.0.

### Inclusion Criteria:

- Age ≥18 yrs
- Histologically confirmed locally advanced or metastatic solid malignancy
- Has exhausted all available standard treatment or is not a candidate for available standard therapy
- ECOG 0-1
- Measurable disease not required in dose escalation

### Exclusion Criteria:

- Active autoimmune disease requiring systemic treatment
- History of inflammatory pneumonitis or interstitial lung disease
- History of immune-related toxicities on prior immunotherapy treatment leading to discontinuation

### Primary Objectives:

- Safety and tolerability of CPA.9.086.H4(S241P) monotherapy (dose escalation for this presentation)
- The MTD and/or recommended dose for expansion of CPA.9.086.H4(S241P) monotherapy
- PK profile of CPA.9.086.H4(S241P) monotherapy

### Secondary Objectives:

- Immunogenicity of CPA.9.086.H4(S241P) monotherapy and in combination with anti-PVRIG

### Exploratory Objectives:

- Preliminary antitumor activity of CPA.9.086.H4(S241P) monotherapy and in combination with anti-PVRIG
- CPA.9.086.H4(S241P)-mediated pharmacodynamic effect in blood, immune-related changes (cytokines, immunophenotyping)

A total of 18 pts were enrolled with 2 DLTs reported
- Single pt dose cohort - 1 pt with G2 vomiting assessed by the investigator as possibly related to study drug
- 3+3 dose cohort - 1 pt with G3 atrial fibrillation assessed by the investigator as possibly related to study drug

Maximum tolerated dose of CPA.9.086.H4(S241P) was not reached. The most frequent TEAEs were fatigue 7 pts (39%) [G1, 5 (28%)/G2 2 (11%)], G1 diarrhoea 3 pts (17%). Serious adverse events [all causality] were reported in 2 pts (11%) - a pt with prostate cancer, G3 atrial fibrillation assessed by the investigator as possibly related to study drug; a pt with widely metastatic rectal cancer with pulmonary metastases, spinal cord compression (G3 acute pulmonary failure, spinal cord compression and urinary retention) - assessed by the investigator as related to disease progression, not related to study drug. Median (min, max) number of prior therapies was 7 (2, 16).

Study treatment discontinuation reported in 1 pt (6%). CPA.9.086.H4(S241P) peripheral receptor occupancy was above 90% at doses ≥0.1 mg/kg. Preliminary PK profiles of CPA.9.086.H4(S241P) were generally dose proportional. No depletion of major lymphocyte populations expressing TIGIT (NK, CD4 and CD8 T cells).

**Results:** In the safety population [N=18], 17 pts reported treatment emergent adverse events (TEAEs). The most frequent TEAES [≥2pts] were fatigue 7 pts (39%) all G1/2, diarrhea 3 pts (17%) all G1/2. Two pts reported DLTs deemed related to study drug, a pt with G2 nausea (single pt cohort, 0.01 mg/kg) and a pt with G3 atrial fibrillation (1 mg/kg). Serious adverse events were reported in 2 pts, 1 pt with atrial fibrillation (deemed by the investigator as possibly related to CPA.9.086.H4(S241P)) and 1 pt with spinal cord compression (deemed by the investigator as unrelated to CPA.9.086.H4(S241P), related to disease). Preliminary PK profile were generally dose proportional and peripheral RO above 90% was reported from 0.1 mg/kg dose.

A total of 18 pts were treated and MTD of CPA.9.086.H4(S241P) was not reached, CPA.9.086.H4(S241P) was well tolerated and has a favorable safety profile. A patient in a single patient dose cohort [0.01 mg/kg] reported DLT of G2 vomiting and a pt[1 mg/kg] with DLT of G3 atrial fibrillation; these were assessed by the investigator as possibly related to study drug. No DLTs were reported at any other CPA.9.086.H4(S241P) doses including higher doses [3 mg/kg, 10 mg/kg]. Best response of stable disease in 9 pts (50%) with 6 pts (67%) with confirmed SD and 3 pts (17%) with SD ≥6 months [includes 1pt (#08) with SD ≥6 months within the datacut date, data entry delayed]. The most frequent TEAE was fatigue 7 pts (39%) [G1, 5 (28%), G2 2 (11%)]. Serious adverse events reported in 2 pts (11%) -G3 atrial fibrillation assessed by the investigator as possibly related to study drug; a ptwith G3 acute pulmonary failure, spinal cord compression and urinary retention with widely metastatic rectal cancer (pulmonary metastases, spinal cord compression), assessed by the investigator as related to disease progression, not related to study drug. Median (min, max) number of prior therapies was 7 (2, 16). CPA.9.086.H4(S241P) peripheral receptor occupancy was above 90% at doses ≥0.1 mg/kg. Preliminary PK profiles of CPA.9.086.H4(S241P) were generally dose proportional. No depletion of major lymphocyte populations expressing TIGIT (NK, CD4 and CD8 T cells).

**Conclusion:** CPA.9.086.H4(S241P) has an acceptable safety, tolerability and PK profile. A CPA.9.086.H4(S241P) 3 mg/kg IV Q3 wks is the RDFE. Enrollment into combination cohort (CPA.9.086.H4(S241P) + anti-PVRIG antibody), for evaluation of safety/tolerability at the RDFE of both study drugs, combination dose expansion (CPA.9.086.H4(S241P) + anti-PVRIG antibody) in patients with HNSCC, NSCLC and CRC-MSS and CPA.9.086.H4(S241P) monotherapy dose expansion (pts with multiple myeloma) all at the RDFE of study drug(s) are planned.

CPA.9.086.H4(S241P) monotherapy has a favorable safety and toxicity profile. CPA.9.086.H4(S241P) monotherapy demonstrates preliminary signals of antitumor activity in a heavily pretreated and heterogenous pt population with median (min, max) prior therapies 7 (2, 16). CPA.9.086.H4(S241P) peripheral receptor occupancy >90% from 0.1 mg/kg dose. Preliminary PK profiles of CPA.9.086.H4(S241P) were generally dose proportional. CPA.9.086.H4(S241P) 3 mg/kg IV Q3W has been selected as the RDFE. CPA.9.086.H4(S241P) did not deplete major TIGIT+ expressing lymphocytes (CD4, CD8, NK cells). The CPA.9.086.H4(S241P) monotherapy expansion cohort is enrolling patients. A PD-L(1)-free regimen of CPA.9.086.H4(S241P) in combination with anti-PVRIG antibody is part of the expansion cohort evaluating pts with R/R CRC, NSCLC, HNSCC.

Encouraging signal of antitumor activity with SD 9 pts (50%) and 6 pts (67%) with confirmed SD; 3 pts (17%) with SD ≥6 months [includes an additional ptwith SD ≥6 months within the datacut date, data entry delayed] in a heavily pretreated and heterogenous ptpopulation with median (min, max) 7 (2, 16) prior therapies. CPA.9.086.H4(S241P) peripheral receptor occupancy >90% from 0.1 mg/kg dose. Preliminary PK profiles of CPA.9.086.H4(S241P) were generally dose proportional. CPA.9.086.H4(S241P) 3 mg/kg IV Q3W has been selected as the RDFE. CPA.9.086.H4(S241P) did not deplete major TIGIT+ expressing lymphocytes (CD4, CD8, NK cells). The CPA.9.086.H4(S241P) monotherapy expansion cohort is enrolling patients. A PD-L(1)-free regimen (CPA.9.086.H4(S241P) in combination with anti-PVRIG) is part of the expansion cohort evaluating pts with relapsed/refractory colorectal cancer, Non small cell lung cancer, Head and neck squamous cell cancer.

### EXAMPLE 7: TREATMENT OF MULTIPLE MYELOMA USING BCMA/CD3 T CELL ENGAGER IN COMBINATION WITH PVRIG AND TIGIT ANTIBODIES

### Materials and Methods:

The fresh bone marrow cells from multiple myeloma (MM) patient were co-cultured with 5 nM recombinant anti-BCMA x anti-CD3 Bispecific Antibody (scIgG) BCMA/CD3 bispecific antibody (see, the World Wide Web at creative-biolabs.com/bsab/bispecific-bcma-cd3-scigg-12165.htm) with either hIgG4 isotype control antibody, or anti-human PVRIG blocking antibodies (10 µg/ml), or anti-TIGIT blocking antibodies (10 µg/ml), or a combination of both anti-human PVRIG and anti-TIGIT blocking antibodies for 48 hours. Supernatants of culture medium from each experimental group were collected, and then assayed according to the IFN-gamma (R&D Systems^{™}, Cat#: DIF50C) and Granzyme B (R&D Systems^{™}, Cat# DGZB00) ELISA protocol, without dilution. Each data was the average of duplicate samples.

### Results:

Bone marrow cells were harvested from patient with MM, and co-cultured with anti-BCMA/CD3 bi-specific antibody with either anti-human PVRIG blocking antibodies, anti-TIGIT blocking antibodies, a combination of both anti-human PVRIG and anti-TIGIT blocking antibodies or with hIgG4 isotype control.

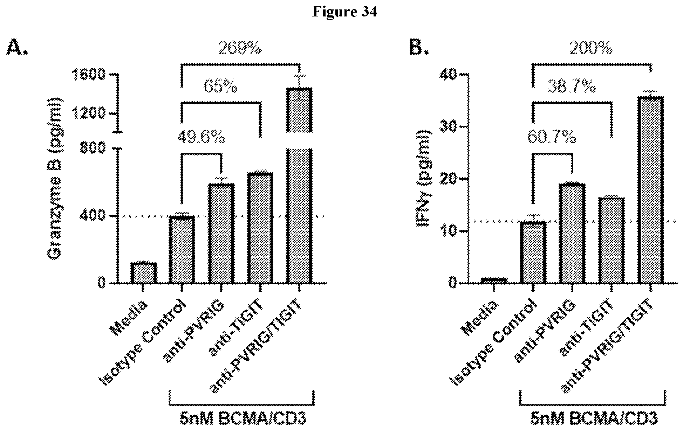

Anti-BCMA/CD3 bi-specific antibody induces immune activation as shown by secretion of Granzyme B and IFNγ. When cells were co-cultured with anti-BCMA/CD3 and anti-PVRIG antibody there was increased immune activation compared to anti-BCMA/CD3 alone, as measured by secretion of Granzyme B (Figure 34A, 49.6%) and IFNγ (Figure 34B, 60.7%). When cells were co-cultured with anti-BCMA/CD3 and anti-TIGIT antibody there was increased immune activation compared to anti-BCMA/CD3 alone, as measured by secretion of Granzyme B (Figure 34A, 65%) and IFNγ (Figure 34B, 38.7%). Triple combination of anti-BCMA/CD3, anti-PVRIG and anti-TIGIT antibodies, resulted in a robust immune activation compared to anti-BCMA/CD3 alone, as measured by secretion of Granzyme B (Figure 34A, 269%) and IFNγ (Figure 34B, 200%).

**Table 6. List of exemplary T cell engagers.**

| **Indications** | **Drug** | **Target** |
|---|---|---|
| Refractory/relapsed B-NHL | Epcoritamab | CD20/CD3 |
| Refractory/relapsed B-NHL | Odronextamab | CD20/CD3 |
| Refractory/relapsed FL | Mosunetuzumab | CD20/CD3 |
| Refractory/relapsed NHL and CLL | XmAb13676 | CD20/CD3 |
| Refractory/relapsed NHL | Glofitamab | CD20/CD3 |
| Refractory/relapsed NHL | IGM-2323 | CD20/CD3 |
| Refractory/relapsed AML | AMG 330 | CD33/CD3 |
| Refractory/relapsed AML | AMG 673 | CD33/CD3 |
| Refractory/relapsed MM | AMG 420 | BCMA/CD3 |
| Refractory/relapsed MM | AMG 701 | BCMA/CD3 |
| Refractory/relapsed MM | Teclistamab | BCMA/CD3 |
| Refractory/relapsed MM | REGN5458 | BCMA/CD3 |
| Refractory/relapsed MM | PF-06863135 | BCMA/CD3 |
| Refractory/relapsed MM | TNB-383B | BCMA/CD3 |
| Refractory/relapsed MM | Cevostamab | FcRH5/CD3 |
| Refractory/relapsed MM | Talquetamab | GPRC5D/CD3 |
| Primary induction failure AML | Flotetuzumab | CD123/CD3 |
| Metastatic castration-resistant prostate cancer | Pasotuxizumab | CD3/PSMA |
| Metastatic castration-resistant prostate cancer | AMG 160 | CD3/PSMA |
| Refractory/relapsed Glioblastoma | AMG 596 | CD3/EGFRvIII |
| Refractory/relapsed Small-cell lung cancer | AMG 757 | CD3/DLL3 |
| Gastric cancer | AMG 199 | CD3/MUC 17 |
| Gastric cancer | AMG 910 | CD3/CLDN18.2 |
| Refractory/relapsed solid tumors | AMV564 | CD3/CD33 |
| CEA+ advanced solid tumors | Cibisatamab | CD3/CEA |
| HER2+ advanced solid tumors | M802 | CD3/HER2 |
| Malignant ascites | M701 | CD3/EpCAM |
| Solid malignancies | ERY974 | CD3/GPC3 |
| Refractory/relapsed Metastatic colorectal carcinoma | MGD007 | CD3/gpA33 |
| Metastatic castration-resistant prostate cancer | ES414 | CD3/PSMA |
| Refractory/relapsed Neuroblastoma, osteosarcoma, and other GD2+ solid tumors | Hu3F8-bsAb | CD3/GD2 |
| HER2+ metastatic breast cancers | ISB 1302 | CD3/HER2 |
| Locally advanced or metastatic HER2+ cancers | BTRC4017A | CD3/HER2 |
| Locally advanced or metastatic solid tumors | GEN1044 | CD3/5 T4 |

### EXAMPLE 8: PVRIG EXPRESSION IN SYNOVIAL SARCOMA

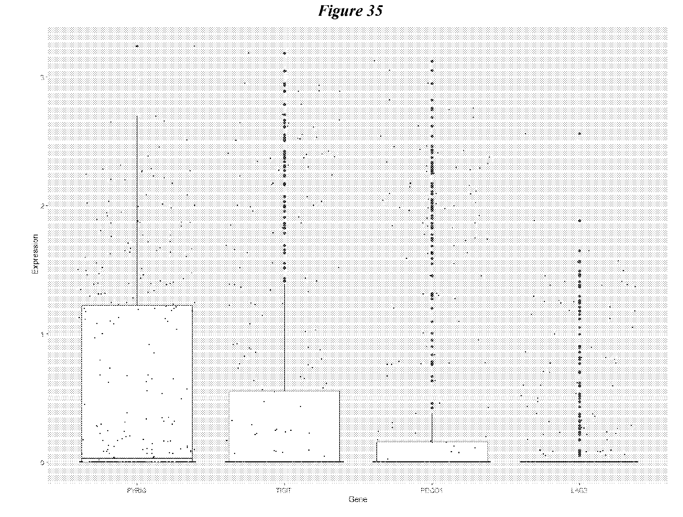

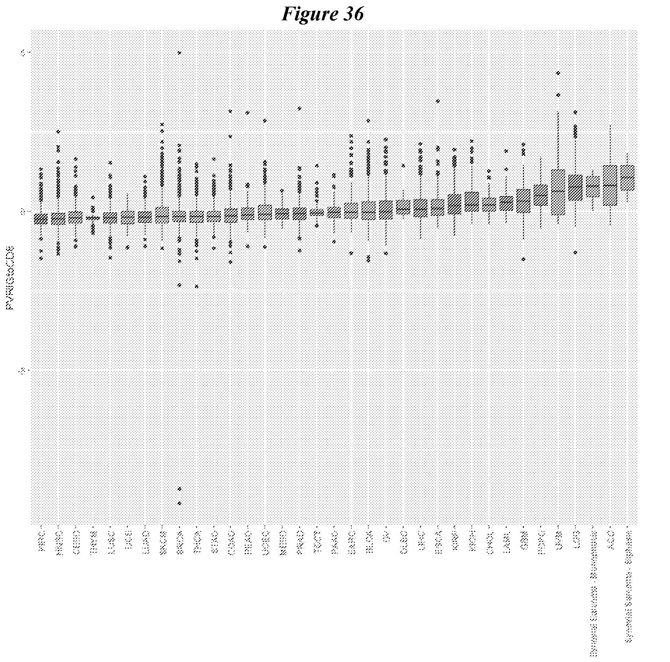

Specifically in synovial sarcoma PVRIG has dominant expression over other immune checkpoints on CD8 T-cells (Figure 35), PVRIG counterpart, PVRL2(NECTIN2) is also expressed by stromal cells (data not shown). Moreover analyzing of all tumors in TCGA, subseting synovial sarcoma from the general sarcoma TCGA classification (SARC), PVRIG has the highest expression in synovial sarcoma, when normalized to CD8 expression (Figure 36). Therefore, anti-PVRIG treatment could be highly beneficial for synovial sarcoma, (including but not limited to subtypes of synovial sarcoma monophasic spindle cell, biphasic, poorly differentiated, myxoid, ossifying and monophasic epithelial) treatment alone and/or in combination with standard of and/or other immune checkpoints.

### Material and methods

### Single cell analysis

Cunt and cells annotation data was downloaded from GEO (https://www.ncbi.nlm.nih.gov/geo/query/acc.cgi?acc=GSE131309), and analyzed using Seurat pipeline (https://satijalab.org/seurat/articles/pbmc3k_tutorial.html).

### TCGA analysis;

RPKM values for PVRIG, CD8A and CD8B were downloaded from the TCGA portal. CD8 was calculated as the geometric mean of CD8A and CD8B. ratio of PVRIG to CD8 was plotted in ascending order of the median ratio across the samples in each tumor type. Tumor type abbreviations can be found in (https://gdc.cancer.gov/resources-tcga-users/tcga-code-tables/tcga-study-abbreviations).

The examples set forth above are provided to give those of ordinary skill in the art a complete disclosure and description of how to make and use the embodiments of the compositions, systems and methods of the invention, and are not intended to limit the scope of what the inventors regard as their invention. Modifications of the above-described modes for carrying out the invention that are obvious to persons of skill in the art are intended to be within the scope of the following claims. All patents and publications mentioned in the specification are indicative of the levels of skill of those skilled in the art to which the invention pertains.

All headings and section designations are used for clarity and reference purposes only and are not to be considered limiting in any way. For example, those of skill in the art will appreciate the usefulness of combining various aspects from different headings and sections as appropriate according to the spirit and scope of the invention described herein.

All references cited herein are hereby incorporated by reference herein in their entireties and for all purposes to the same extent as if each individual publication or patent or patent application was specifically and individually indicated to be incorporated by reference in its entirety for all purposes.

Many modifications and variations of this application can be made without departing from its spirit and scope, as will be apparent to those skilled in the art. The specific embodiments and examples described herein are offered by way of example only, and the application is to be limited only by the terms of the appended claims, along with the full scope of equivalents to which the claims are entitled.

The invention also includes the following aspects:
1. A method of treating cancer comprising administering i) an anti-PVRIG antibody and an anti-TIGIT antibody, ii) an anti-TIGIT antibody alone, or iii) an anti-PVRIG antibody alone.
2. The method of aspect 1, wherein said anti-PVRIG antibody is CHA.7.518.1.H4(S241P) and said anti-TIGIT antibody is CPA.9.086.H4(S241P).
3. The method of aspect 1, wherein said anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
4. The method of aspect 1, wherein said anti-TIGIT antibody comprises:
   i) a heavy chain variable domain comprising the vhCDR1, vhCDR2, and vhCDR3 from the heavy chain of CPA.9.086.H4(S241P) (SEQ ID NO:877), and
   ii) a light chain variable domain comprising the vlCDR1, vlCDR2, and vlCDR3 from the light chain of CPA.9.086.H4(S241P) (SEQ ID NO:882).
5. The method of any one of aspects 1-4, wherein said anti-TIGIT antibody is administered every 3 weeks or every 4 weeks.
6. The method of any one of aspects 1-5, wherein said anti-TIGIT antibody and said anti-PVRIG antibody are each individually administered every 3 weeks or every 4 weeks.
7. The method of any one of aspects 1-6, wherein said anti-TIGIT and/or said anti-TIGIT antibody is administered intravenously.
8. The method of any one of aspects 1-7, wherein the anti-PVRIG antibody is administered as a stable liquid pharmaceutical formulation of an anti-PVRIG antibody comprising:
   (a) an anti-PVRIG antibody, wherein said anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:4), and
      ii) a light chain variable domain comprising the vlCDR1, vlCDR2, and vlCDR3 from the light chain of CHA.7.518.1.H4(S241P) (SEQ ID NO:9);
   (b) from 10 mM to 100 mM histidine;
   (c) from 30 mM to 100 mM NaCl;
   (d) from 20 mM to 150 mM L-Arginine; and
   (e) from 0.005% to 0.1% w/v polysorbate 80,
   wherein the composition has a pH from 5.5 to 7.0.
9. The method according to any one of aspects 1-8, wherein said anti-PVRIG antibody and/or anti-TIGIT antibody comprises a CH1-hinge-CH2-CH3 sequence of IgG4 (SEQ ID NO:17 or SEQ ID NO:50), wherein said hinge region optionally comprises mutations.
10. The method according to any one of aspects 1-9, wherein said anti-PVRIG antibody and/or anti-TIGIT antibody comprises the CH1-hinge-CH2-CH3 region from IgG1, IgG2, IgG3, or IgG4, wherein said hinge region optionally comprises mutations.
11. The method according to any one of aspects 1-10, wherein for said anti-PVRIG antibody said heavy chain variable domain is from the heavy chain of CHA.7.518.1.H4(S241P) (SEQ ID NO:4) and said light chain variable domain is from the light chain of CHA.7.518.1.H4(S241P) (SEQ ID NO:9) and for said anti-TIGIT antibody said heavy chain variable domain is from the heavy chain of CPA.9.086 (S241P) (SEQ ID NO:877) and said light chain variable domain is from the light chain of CPA.9.086 (S241P) (SEQ ID NO:882).
12. The method according to any one of aspects 1-11, wherein said anti-PVRIG antibody comprises a CL region of human kappa 2 light chain.
13. The method according to any one of aspects 1-12, wherein said pharmaceutical formulation comprises from 10 mM to 80 mM histidine, from 15 mM to 70 mM histidine, from 20 mM to 60 mM histidine, from 20 mM to 50 mM histidine, or from 20 mM to 30 mM histidine.
14. The method according to any one of aspects 1-13, wherein said pharmaceutical formulation comprises about 25 mM histidine.
15. The method according to any one of aspects 1-14, wherein said pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.
16. The method according to any one of aspects 1-15, wherein said pharmaceutical formulation comprises about 60 mM NaCl.
17. The method according to any one of aspects 1-16, wherein said pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.
18. The method according to any one of aspects 1-17, wherein said pharmaceutical formulation comprises about 100 mM L-arginine.
19. The method according to any one of aspects 1-18, wherein said pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.
20. The method according to any one of aspects 1-19, wherein said pharmaceutical formulation comprises about 0.01% polysorbate 80.
21. The method according to any one of aspects 1-20, wherein said pH is from 6 to 7.0.
22. The method according to any one of aspects 1-21, wherein said pH is from 6.3 to 6.8.
23. The method according to any one of aspects 1-22, wherein said pH is 6.5 +/- 0.2.
24. The method according to any one of aspects 1-23, wherein said anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.
25. The method according to any one of aspects 1-24, wherein said formulation is stable at 2°C to 8°C for at least 1 week, 2 weeks, 3 weeks, 4 weeks, 5 weeks, 6 weeks, 7 weeks, 8 weeks, 9 weeks, 10 weeks, 1 month, 3 months, 6 months, 9 months, 12 months, 18 months, 24 months, 30 months, 36 months, or 42 months.
26. The method according to any one of aspects 1-25, wherein said formulation is stable at about 20°C to 25 °C for at least 1 week, 2 weeks, 3 weeks, 4 weeks, 5 weeks, 6 weeks, 1 months, 3 months, 6 months, or 9 months, 12 months, 18 months, or 36 months.
27. The method according to any one of aspects 1-26, wherein said formulation is stable at 35°C to 40°C for at least 1 week, 2 weeks, 3 weeks, 4 weeks, or 5 weeks.
28. The method according to any one of aspects 1-27, wherein said anti-PVRIG antibody is at a concentration of about 20 mg/mL.
29. The method according to any one of aspects 1-28, wherein said anti-PVRIG antibody formulation comprises:
   a) a heavy chain comprising:
      i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   b) a light chain comprising:
      i) a VL-CL, wherein the VL from CHA.7.518.1.H4(S241P) (SEQ ID NO:9) and wherein the CL region is from human kappa 2 light chain.
30. The method according to aspect 29, wherein said hinge region optionally comprises mutations.
31. The method according to aspect 30, wherein said hinge region optionally comprises mutations.
32. The method according to any one of aspects 1-31, wherein said anti-PVRIG antibody formulation comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13).
33. The method according to any one of aspects 1-32, said anti-PVRIG antibody formulation comprising:
   (a) an anti-PVRIG antibody, wherein said anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:4), and
      ii) a light chain variable domain comprising the vlCDR1, vlCDR2, and vlCDR3 from the light chain of CHA.7.518.1.H4(S241P) (SEQ ID NO:9);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
34. The method according to any one of aspects 1-33, said anti-PVRIG antibody formulation comprising:
   (a) an anti-PVRIG antibody, wherein said anti-PVRIG antibody comprises:
      i) a heavy chain comprising the heavy chain from CHA.7.518.1.H4(S241P) (SEQ ID NO:8); and
      ii) a light chain comprising the light chain from CHA.7.518.1.H4(S241P) (SEQ ID NO: 13);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
35. The method according to any one of aspects 1-34, wherein said anti-PVRIG antibody is administered at a dosage of about 0.01 mg/kg to about 20 mg/kg of the anti-PVRIG antibody or about 0.01 mg/kg to about 10 mg/kg of the anti-PVRIG antibody.
36. The method according to any one of aspects 1-35, wherein said anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.
37. The smethod according to any one of aspects 1-36, wherein said anti-PVRIG antibody is administered 20 mg/kg every 4 weeks.
38. The method according to any one of aspects 1-37, wherein said stable liquid pharmaceutical formulation is administered for the treatment of cancer.
39. The method according to any one of aspects 1-38, for use in a method of treating cancer.
40. The method according aspect 38 or 39, wherein said cancer selected from the group consisting of vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS ](microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma (including hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma), post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, malignant melanoma, pleural mesothelioma, chordoma, endometrial sarcoma, chondrosarcoma, uterine sarcoma, synovial sarcoma, uveal melanoma, amyloidosis, AL-amyloidosis, astrocytoma, and/or Myelodysplastic syndromes (MDS).
41. The method according to any one of aspects 38-40, wherein said cancer selected from the group consisting advanced cancer, ovarian cancer, lung cancer, colon cancer, plasma cell neoplasm, multiple myeloma, and HNSCC.
42. The method according to any one of aspects 38-40, wherein said cancer selected from the group consisting of HNSCC, CRC (MSS), NSCLC, and multiple myeloma.
43. A method for treating multiple myeloma comprising administering an anti-PVRIG antibody, optionally wherein the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.
44. The method according to aspect 43, wherein the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.
45. The method according to any one of aspects 43 to 44, wherein the anti-PVRIG antibody comprises a heavy chain variable domain from the heavy chain of CHA.7.518.1.H4(S241P) (SEQ ID NO:8) and a light chain variable domain from the light chain of CHA.7.518.1.H4(S241P) (SEQ ID NO:13).
46. The method according to any one of aspects 43 to 45, wherein the anti-PVRIG antibody comprises a heavy chain variable domain from the heavy chain of CHA.7.538.1.2.H4(S241P) (SEQ ID NO:266) and a light chain variable domain from the light chain of CHA.7.538.1.2.H4(S241P) (SEQ ID NO:271).
47. The method according to any one of aspects 43 to 46, wherein the anti-PVRIG antibody is administered as a stable liquid pharmaceutical formulation of the anti-PVRIG antibody comprising:
   (a) an anti-PVRIG antibody, wherein the anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:8), and
      ii) a light chain variable domain comprising the vlCDR1, vlCDR2, and vlCDR3 from the light chain of CHA.7.518.1.H4(S241P) (SEQ ID NO:13);
   (b) from 10 mM to 100 mM histidine;
   (c) from 30 mM to 100 mM NaCl;
   (d) from 20 mM to 150 mM L-Arginine; and
   (e) from 0.005% to 0.1% w/v polysorbate 80,
   wherein the composition has a pH from 5.5 to 7.0.
48. The method according to any one of aspects 43 to 47, wherein the anti-PVRIG antibody comprises a CH1-hinge-CH2-CH3 sequence of IgG4 (SEQ ID NO:657 or SEQ ID NO:658), wherein the hinge region optionally comprises mutations.
49. The method according to any one of aspects 43 to 48, wherein the anti-PVRIG antibody comprises a CH1-hinge-CH2-CH3 region from IgG1, IgG2, IgG3, or IgG4, wherein the hinge region optionally comprises mutations.
50. The method according to any one of aspects 43 to 49, wherein the heavy chain variable domain of the anti-PVRIG antibody is from the heavy chain of CHA.7.518.1.H4(S241P) (SEQ ID NO:8) and the light chain variable domain of the anti-PVRIG antibody is from the light chain of CHA.7.518.1.H4(S241P) (SEQ ID NO:13).
51. The method according to any one of aspects 43 to 50, wherein the anti-PVRIG antibody comprises a CL region of human kappa 2 light chain.
52. The method according to any one of aspects 47 to 51, wherein the stable liquid formulation comprises from 10 mM to 80 mM histidine, from 15 mM to 70 mM histidine, from 20 mM to 60 mM histidine, from 20 mM to 50 mM histidine, or from 20 mM to 30 mM histidine.
53. The method according to any one of aspects 47 to 52, wherein the pharmaceutical formulation comprises about 25 mM histidine.
54. The method according to any one of aspects 47 to 53, wherein the pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.
55. The method according to any one of aspects 47 to 54, wherein the pharmaceutical formulation comprises about 60 mM NaCl.
56. The method according to any one of aspects 47 to 55, wherein the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.
57. The method according to any one of aspects 47 to 56, wherein the pharmaceutical formulation comprises about 100 mM L-arginine.
58. The method according to any one of aspects 47 to 57, wherein the pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.
59. The method according to any one of aspects 47 to 58, wherein the pharmaceutical formulation comprises about 0.01% polysorbate 80.
60. The method according to any one of aspects 47 to 59, wherein the pH is from 6 to 7.0.
61. The method according to any one of aspects 47 to 60, wherein the pH is from 6.3 to 6.8.
62. The method according to any one of aspects 47 to 61, wherein the pH is 6.5 +/- 0.2.
63. The method according to any one of aspects 47 to 62, wherein the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.
64. The method according to any one of aspects 47 to 63, wherein the anti-PVRIG antibody is at a concentration of about 20 mg/mL.
65. The method according to any one of aspects 47 to 64, wherein the anti-PVRIG antibody formulation comprises:
   a) a heavy chain comprising:
      i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   b) a light chain comprising:
      i) a VL-CL, wherein the VL from CHA.7.518.1.H4(S241P) (SEQ ID NO:9) and wherein the CL region is from human kappa 2 light chain.
66. The method according to aspect 65, wherein the hinge region optionally comprises mutations.
67. The method according to aspect 66, wherein the hinge region optionally comprises mutations.
68. The method according to any one of aspects 47 to 67, wherein the anti-PVRIG antibody formulation comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13).
69. The method according to any one of aspects 47 to 67, the anti-PVRIG antibody formulation comprising:
   (a) an anti-PVRIG antibody, wherein the anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:8), and
      ii) a light chain variable domain comprising the vlCDR1, vlCDR2, and vlCDR3 from the light chain of CHA.7.518.1.H4(S241P) (SEQ ID NO:13);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
70. The method according to any one of aspects 47 to 67, the anti-PVRIG antibody formulation comprising:
   (a) an anti-PVRIG antibody, wherein the anti-PVRIG antibody comprises:
      i) a heavy chain comprising the heavy chain from CHA.7.518.1.H4(S241P) (SEQ ID NO:8); and
      ii) a light chain comprising the light chain from CHA.7.518.1.H4(S241P) (SEQ ID NO: 13);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
71. The method according to any one of aspects 43 to 70, wherein the anti-PVRIG antibody is administered at a dosage of about 0.01 mg/kg to about 20 mg/kg of the anti-PVRIG antibody or about 0.01 mg/kg to about 10 mg/kg of the anti-PVRIG antibody.
72. The method according to any one of aspects 43 to 71, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.
73. The method according to any one of aspects 43 to 72, wherein the anti-PVRIG antibody is administered about 20 mg/kg every 4 weeks.
74. The method according to any one of aspects 43 to 73, wherein the anti-PVRIG antibody is administered in combination with an anti-DNAM antibody.
75. The method according to aspect 74, wherein the anti-DNAM antibody is selected from the group consisting of 10E5, DX11, 11A8.7.4, and the anti-CD226 antibody as shown in Figure 23.
76. The method according to any one of aspects 43 to 75, wherein the anti-PVRIG antibody is administered in combination with an anti-PD-1 antibody.
77. The method according to any one of aspects 43 to 76, wherein the anti-PVRIG antibody is administered in combination with an anti-TIGIT antibody.
78. The method according to any one of aspects 43 to 77, wherein the anti-PVRIG antibody is administered in combination with an anti-PD-1 antibody and an anti-TIGIT antibody.
79. The method according aspects 76 to 78, wherein the anti-PD-1 antibody is selected from the group consisting of nivolumab and pembrolizumab.
80. The method according to aspects 77 to 78, the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
81. The method according to aspects 77 to 80, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
82. The method according to any one of aspects aspects 77 to 81, wherein the anti-TIGIT antibody is selected from the group consisting of CPA.9.083.H4(S241P) and CPA.9.086.H4(S241P).
83. The method according to any one of aspects 77 to 82, wherein the anti-TIGIT antibody comprises:
   a) a heavy chain comprising VH-CH1-hinge-CH2-CH3; and
   b) a light chain comprising VL-VC, wherein VC is either kappa or lambda.
84. The method according to aspect83, wherein the sequence of the CH1-hinge-CH2-CH3 is selected from human IgG1, IgG2 and IgG4, and variants thereof.
85. The method according to any one of aspects 77 to 83, wherein the anti-PVRIG antibody is CHA.7.518.1.H4(S241P), the anti-TIGIT antibody is CPA.9.086.H4(S241P) and the anti-PD-1 antibody is nivolumab.
86. The method according to any one of aspects 77 to 83, wherein the anti-PVRIG antibody is CHA.7.518.1.H4(S241P), the anti-TIGIT antibody is CPA.9.083.H4(S241P) and the anti-PD-1 antibody is nivolumab.
87. The method according to any one of aspects 77 to 83, wherein the anti-PVRIG antibody is CHA.7.518.1.H4(S241P), the anti-TIGIT antibody is CPA.9.086.H4(S241P) and the anti-PD-1 antibody is pembrolizumab.
88. The method according to any one of aspects 77 to 83, wherein the anti-PVRIG antibody is CHA.7.518.1.H4(S241P), the anti-TIGIT antibody is CPA.9.083.H4(S241P) and the anti-PD-1 antibody is pembrolizumab.
89. The method according to any one of aspects 77 to 83, wherein the anti-PVRIG antibody is CHA.7.538.1.2.H4(S241P), the anti-TIGIT antibody is CPA.9.086.H4(S241P) and the anti-PD-1 antibody is nivolumab.
90. The method according to any one of aspects 77 to 83, wherein the anti-PVRIG antibody is CHA.7.538.1.2.H4(S241P), the anti-TIGIT antibody is CPA.9.083.H4(S241P) and the anti-PD-1 antibody is nivolumab.
91. The method according to any one of aspects 77 to 83, wherein the anti-PVRIG antibody is CHA.7.538.1.2.H4(S241P), the anti-TIGIT antibody is CPA.9.086.H4(S241P) and the anti-PD-1 antibody is pembrolizumab.
92. The method according to any one of aspects 77 to 83, wherein the anti-PVRIG antibody is CHA.7.538.1.2.H4(S241P), the anti-TIGIT antibody is CPA.9.083.H4(S241P) and the anti-PD-1 antibody is pembrolizumab.
93. The method according to any of the preceding aspects, wherein the anti-PVRIG antibody and/or the anti-TIGIT antibody is administered in combination with a further multiple myeloma therapy.
94. The method according to any of the preceding aspects, wherein the anti-PVRIG antibody and/or the anti-TIGIT antibody is administered in combination with one or more chemotherapies or chemotherapeutics.
95. The method according to aspect 94, wherein the one or more chemotherapeutics or chemotherapeutics is selected from the group consisting of cyclophosphamide (cytoxan), etoposide (VP-16), doxorubicin (adriamycin), liposomal doxorubicin (Doxil), melphalan, melphalan flufenamide, melflufen (Pepaxto), and bendamustine (Treanda).
96. The method according to any of the preceding aspects, wherein the anti-PVRIG antibody and/or the anti-TIGIT antibody is administered in combination with one or more corticosteroids.
97. The method according to aspect 96, wherein the the one or more corticosteroids are selected from the group consisting of dexamethasone and/ prednisone.
98. The method according to any of the preceding aspects, wherein the anti-PVRIG antibody and/or the anti-TIGIT antibody is administered in combination with one or more immunomodulating agents.
99. The method according to aspect 98, wherein the one or more immunomodulating agents include are selected from the group consisting of thalidomide (Thalomid), lenalidomide (Revlimid), and pomalidomide (Pomalyst).
100. The method according to any of the preceding aspects, wherein the anti-PVRIG antibody and/or the anti-TIGIT antibody is administered in combination with one or more proteasome inhibitors.
101. The method according to aspect 100, wherein the one or more proteasome inhibitors are selected from the group consisting of bortezomib (Velcade), carfilzomib (Kyprolis), and ixazomib (Ninlaro).
102. The method according to any of the preceding aspects, wherein the anti-PVRIG antibody and/or the anti-TIGIT antibody is administered in combination with one or more histone deacetylase (HDAC) inhibitors.
103. The method according to aspect 102, wherein the one or more histone deacetylase (HDAC) inhibitors is panobinostat (Farydak).
104. The method according to any of the preceding aspects, wherein the anti-PVRIG antibody and/or the anti-TIGIT antibody is administered in combination with one or more anti-CD38 monoclonal antibodies.
105. The method according to aspect 104, wherein the one or more anti-CD38 monoclonal antibodies are selected from the group consisting of daratumumab (Darzalex), daratumumab and hyaluronidase (Darzalex Faspro), isatuximab (Sarclisa), and belantamab mafodotin-blmf (Blenrep).
106. The method according to any of the preceding aspects, wherein the anti-PVRIG antibody and/or the anti-TIGIT antibody is administered in combination with one or more anti-SLAMF7 antibodies.
107. The method according to aspect 106, the anti-SLAMF7 monoclonal antibody is elotuzumab (Empliciti).
108. The method according to any of the preceding aspects wherein the anti-PVRIG antibody and/or the anti-TIGIT antibody is administered in combination with lenalidomide (or pomalidomide or thalidomide) and dexamethasone.
109. The method according to any of the preceding aspects wherein the anti-PVRIG antibody and/or the anti-TIGIT antibody is administered in combination with carfilzomib (or ixazomib or bortezomib), lenalidomide, and dexamethasone.
110. The method according to any of the preceding aspects wherein the anti-PVRIG antibody and/or the anti-TIGIT antibody is administered in combination with bortezomib (or carfilzomib), cyclophosphamide, and dexamethasone.
111. The method according to any of the preceding aspects wherein the anti-PVRIG antibody and/or the anti-TIGIT antibody is administered in combination with elotuzumab (or daratumumab), lenalidomide, and dexamethasone.
112. The method according to any of the preceding aspects wherein the anti-PVRIG antibody and/or the anti-TIGIT antibody is administered in combination with bortezomib, liposomal doxorubicin, and dexamethasone.
113. The method according to any of the preceding aspects wherein the anti-PVRIG antibody and/or the anti-TIGIT antibody is administered in combination with panobinostat, bortezomib, and dexamethasone.
114. The method according to any of the preceding aspects wherein the anti-PVRIG antibody and/or the anti-TIGIT antibody is administered in combination with elotuzumab, bortezomib, and dexamethasone.
115. The method according to any of the preceding aspects wherein the anti-PVRIG antibody and/or the anti-TIGIT antibody is administered in combination with melphalan and prednisone (MP), in the presence or absence of thalidomide and/or bortezomib
116. The method according to any of the preceding aspects wherein the anti-PVRIG antibody and/or the anti-TIGIT antibody is administered in combination with vincristine, doxorubicin (Adriamycin), and dexamethasone (called VAD).
117. The method according to any of the preceding aspects wherein the anti-PVRIG antibody and/or the anti-TIGIT antibody is administered in combination with dexamethasone, cyclophosphamide, etoposide, and cisplatin (called DCEP).
118. The method according to any of the preceding aspects wherein the anti-PVRIG antibody and/or the anti-TIGIT antibody is administered in combination with dexamethasone, thalidomide, cisplatin, doxorubicin, cyclophosphamide, and etoposide (called DT-PACE), with or without bortezomib.
119. The method according to any of the preceding aspects wherein the anti-PVRIG antibody and/or the anti-TIGIT antibody is administered in combination with elinexor, bortezomib, and dexamethasone.
120. The method according to any of the preceding aspects, wherein the subject to be administered the therapy has multiple myleoma with high DNAM expression.
121. The method according to any of the preceding aspects, wherein the subject to be administered the therapy has multiple myleoma with high DNAM expression in CD8+ cells and/or a high percentage of CD8+ cells are DNAM positive.
122. The method according to any of the preceding aspects wherein the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.
123. A method for treating multiple myeloma comprising administering an anti-TIGIT antibody, optionally wherein the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
124. The method according to aspect 123, wherein the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.80.
125. The method according to aspect 123 or 124, wherein the anti-TIGIT antibody comprises a heavy chain variable domain from the heavy chain of CPA.9.086.H4(S241P) (SEQ ID NO:877) and a light chain variable domain from the light chain of CPA.9.086.H4(S241P) (SEQ ID NO:882).
126. The method according to any one of aspects 123 to 125, wherein the anti-TIGIT antibody comprises a heavy chain variable domain from the heavy chain of CPA.9.086.H4(S241P) and a light chain variable domain from the light chain of CPA.9.086.H4(S241P).
127. The method according to any one of aspects 123 to 126, wherein the anti-TIGIT antibody is administered in combination with an anti-PVRIG antibody according to any one of aspects 43 to 46.
128. The method according to any one of aspects 123 to 127, wherein the anti-TIGIT antibody is administered in combination with an anti-PD-1 antibody.
129. The method according to aspect 128, wherein the anti-PD-1 antibody is selected from the group consisting of nivolumab and pembrolizumab.
130. The method according to any one of aspects 123 to 129, wherein the anti-TIGIT antibody is administered in combination with an anti-DNAM antibody.
131. The method according to aspect 130, wherein the anti-DNAM antibody is selected from the group consisting of 10E5, DX11, 11A8.7.4, and the anti-CD226 antibody as shown in Figure 23.
132. The method according to aspects 123 to 131, wherein the multiple myeloma is selected from the group consisting of hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma.
133. A method of treatment for cancer comprising administering an anti-TIGIT antibody comprising the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8,
   wherein the anti-TIGIT antibody is administered at a dosage of about 0.01 mg/kg to about 10 mg/kg.
134. The method according to aspect 133, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
135. The method according to any one of aspects 133 to 134, wherein the anti-TIGIT antibody comprises:
   a) a heavy chain comprising VH-CH1-hinge-CH2-CH3; and
   b) a light chain comprising VL-VC, wherein VC is either kappa or lambda.
136. The method according to any one of aspects 133 to 135, wherein the sequence of the CH1-hinge-CH2-CH3 is selected from human IgG1, IgG2 and IgG4, and variants thereof.
137. The method according to one of aspects 133 to 136, wherein the hinge region optionally comprises mutations.
138. The method according to any one of aspects 133 to 137, wherein the anti-TIGIT antibody is selected from the group consisting of CPA.9.083.H4(S241P) and CPA.9.086.H4(S241P).
139. The method according to any one of aspects 133 to 138, wherein the anti-TIGIT antibody is CPA.9.083.H4(S241P).
140. The method according to any one of aspects 133 to 139, wherein the anti-TIGIT antibody is CPA.9.086.H4(S241P).
141. The method according to any one of aspects 133 to 140, wherein the anti-TIGIT antibody is administered every 1 week, 2 weeks, 3 weeks, or 4 weeks.
142. The method according to any one of aspects 133 to 141, wherein the anti-TIGIT antibody is administered every 3 weeks.
143. The method according to any one of aspects 133 to 142, wherein the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody.
144. The method according to any one of aspects 133 to 143, wherein the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody every 1 week, 2 weeks, 3 weeks, or 4 weeks.
145. The method according to any one of aspects 133 to 144, wherein the anti-TIGIT antibody is administered about 10 mg/kg every 3 weeks or every 4 weeks.
146. The method according to any one of aspects 133 to 144, wherein the anti-TIGIT antibody is administered about 3 mg/kg every 3 weeks or every 4 weeks.
147. The method of treatment according to any one of aspects 133 to 146, wherein the cancer is selected from the group consisting of vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma (including hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma), post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, malignant melanoma, pleural mesothelioma, chordoma, endometrial sarcoma, chondrosarcoma, uterine sarcoma, synovial sarcoma, uveal melanoma, amyloidosis, AL-amyloidosis, astrocytoma, and/or Myelodysplastic syndromes (MDS).
148. The use of an anti-TIGIT according to any one of aspects 133 to 147 for the treatment of cancer.
149. The use according to aspect 148, wherein the cancer is selected from the group consisting of prostate cancer, liver cancer (HCC), rectal cancer, colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), uveal melanoma, glioma, renal cell cancer (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cell cancer, MSI-high cancer, KRAS mutant tumors, adult T-cell leukemia/lymphoma, pleural mesothelioma, anal SCC, neuroendocrine lung cancer (including neuroendocrine lung carcinoma), small cell lung cancer, NSCLC, NSCLC large cell, NSCLC squamous cell, NSCLC adenocarcinoma, atypical carcinoid lung cancer, NSCLC with PDL1 >=50% TPS, cervical SCC, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, chordoma, sarcoma, endometrial sarcoma, chondrosarcoma, uterine sarcoma, plasma cell disorders, multiple myeloma, amyloidosis, AL-amyloidosis, glioblastoma, astrocytoma, and fallopian tube cancer.
150. A method of treating cancer comprising administering a therapeutic combination selected from the group consisting of:
   i) an anti-PVRIG antibody, an anti-TIGIT antibody, and an immune cell engager;
   ii) an anti-TIGIT antibody and an immune cell engager; and
   iii) an anti-PVRIG antibody and an immune cell engager.
151. The method of aspect 150, wherein the immune cell engager is bispecific or is a bispecifc antibody.
152. The method of aspects 150 or 151, wherein the immune cell engager comprises a tumor-targeting moiety.
153. The method according to any one of aspects 150 to 152, wherein the tumor-targeting moiety comprises an antibody molecule or a binding portion thereof, optionally which specifically binds to the targeted tumor and/or tumor cells.
154. The method according to any one of aspects 150 to 153, wherein the immune cell engager comprises an immune cell-targeting moiety.
155. The method according to any one of aspects 150 to 154, wherein the immune cell-targeting moiety comprises an antibody molecule or a binding portion thereof, optionally which specifically binds to a target immune cell antigen.
156. The method according to aspect 155, wherein the immune cell antigen is selected from the group consisting of a T cell antigen, a NK cell antigen, a B cell antigen, a dendritic cell antigen, and a macrophage cell antigen.
157. The method according to any one of aspects 150 to 156, wherein the immune cell engager is a T cell engager.
158. The method according to any one of aspects 150 to 157, wherein the T cell engager is selected from the group consising of a Bispecific T cell engager (BiTE), a F(ab')2, F(ab')-ScFv2, a di-scFv, a diabody, a minibody, a scFv-Fc, a DART, a TandAb, a ScDiabody, a ScDiabody-CH3, Diabody-CH3, a triple body, a miniantibody, a minibody, a TriBi minibody, a ScFv-CH3 KIH (knobs in holes), a Fab-ScFv, a SCFv-CH-CL-scFv, a scFv-KIH, a Fab-scFv-Fc, a Tetravalent HCAb, a scDiabody-Fc, a Diabody-Fc, a intrabody, dock and lock antibodies, a ImmTAC, a HSAbody, a ScDiabody-HAS, a humabody and a Tandem ScFv-toxic.
159. The method according to any one of aspects 150 to 158, wherein the immune cell engager comprises a fusion protein comprising two or more polypeptides.
160. The method of aspect 159, wherein the immune cell engager comprises two or more single-chain variable fragments (scFvs), wehrein at least two of the scFvs comprise binding domains from different antibodies, and wherein at least one scFv comprises an effector cell surface molecule binding domain and binds to an effector cell surface molecule and at least one scFv binds to a tumor cell, optionally a tumor specific cell surface molecule binding domain and binds to a tumor specific cell surface molecule.
161. The method of aspect 160, wherein the effector cell surface molecule is selected from the group consisting of CD3, CD28, CD5, CD16, NKG2D, CD64, CD32, CD89, NKG2C, CD44v6, IL-12, PD-L1, Vγ9Vδ2, NKp46, and BCMA.
162. The method of aspect 160, wherein the tumor specific cell surface molecule is selected from the group consisting of BCMA, B7H3, CD10, CD19, CD20, CD22, CD24, CD30, CD33, CD34, CD38, CD44, CD79a, CD79b, CD123, CD138, CD179b, CEA, CLDN18.2, CLEC12A, CS-1, DLL3, EGFR, EGFRvIII, EGFRxFcγRI, EGFRvIIIxCD3, EGFRxCD3, EPCAM, FLT-3, FOLR1, FOLR3, GD2, gpA33, GPC3, HER2, HM1.24, LGR5, MSLN, MCSP, MICA/B, MUC 17 (mucin-17), PSMA, PAMA, P-cadherin, ROR1, PD-1, CLEC12A, WT1, EphA2, ADAM17, and PSCA.
163. The method according to any one of aspects 161-162, wherein the immune cell engager effector cell surface molecule: tumor specific cell surface molecule pairs are selected from the group consisting of CD3:CD19, CD16:CD30, CD64:CD30, CD16:BCMA, CD64:BCMA, CD3:BCMA, and CD3:CD33.
164. The method of aspect 163, wherein the effector cell surface molecule: tumor specific cell surface molecule pair is CD3:BCMA.
165. The method according to any one of aspects 159 to 164, wherein the immune cell engager further comprises a linker, optionally between the effector cell surface molecule binding domain and the tumor specific cell antigen binding domain.
166. The method according to any one of aspects 150 to 165, wherein the immune cell engager is a bi-specific T cell engager (BiTE)
167. The method of aspect 166, wherein the BiTE is specific for the CD3ε subunit of the TCR complex of a T-cell and tumor-targeting moiety.
168. The method of aspect 167, wherein the BiTEis selected from the group consisting of Blinatumomab (Blyncyto^{®}), Solitomab, Epcoritamab, Odronextamab, Mosunetuzumab, XmAb13676, Glofitamab, scIgG, IGM-2323, AMG 330, AMG 673, AMG 420, AMG 701, Teclistamab, REGN5458, PF-06863135, TNB-383B, Cevostamab, Talquetamab, Flotetuzumab, Pasotuxizumab, AMG 160, AMG 596, AMG 757, AMG 199, AMG 910, AMV564, Cibisatamab, M802, M701, ERY974, MGD007, ES414, Hu3F8-bsAb, ISB 1302, BTRC4017A, GEN1044, CAdDuo, CAdTrio, CD44v6, Vγ9Vδ2, NKp46, BCMA, PD-L1, CLEC12A, WT1, bscEphA2xCD, A300E, PSCA, EGFRxFcγRI, EGFRvIIIxCD3, and EGFRxCD3.
169. The method according to any one of aspects 150 to 167, wherein the immune cell engager binds to CD3 and BCMA.
170. The method according to any one of aspects 150 to 167 or 169, wherein the immune cell engager is a bispecific antibody targeting CD3 and BCMA.
171. The method according to any one of aspects 150 to 170, wherein the treatment further comprises administering one or more additional immmmune checkpoint inhibitor antibodies.
172. The method according to any one of aspects 150 to 171, wherein the immmmune checkpoint inhibitor antibody is selected from the group consisting of anti-PVRIG antibodies, anti- PD-1 antibodies, anti-CTLA-4 antibodies, anti-PD-L1 antibodies, anti-LAG-3 antibodies, anti-TIM-3 antibodies, anti-BTLA antibodies, anti- DNAM1 antibodies, anti-ICOS antibodies, anti-4-1bb antibodies, anti-GITR antibodies, anti-OX40 antibodies, anti-CD96 antibodies, anti-B7-H4 antibodies, anti-B7-H3 antibodies, anti-VISTA antibodies, anti-CD27 antibodies, anti-CD40 antibodies, and anti-CD137 antibodies.
173. The method according to any one of aspects 150 to 172, wherein the the anti-PVRIG antibody and/or the anti-TIGIT antibody, and/or the immune cell engager and/or the immmmune checkpoint inhibitor antibody are each administered sequentially or simultaneously, including in any order.
174. The method of treatment according to any one of aspects 150 to 172, wherein the cancer is selected from the group consisting of vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma (including hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma), post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, malignant melanoma, pleural mesothelioma, chordoma, endometrial sarcoma, chondrosarcoma, uterine sarcoma, synovial sarcoma, uveal melanoma, amyloidosis, AL-amyloidosis, astrocytoma, and Myelodysplastic syndromes (MDS).
175. The use according to aspect 173, wherein the cancer is selected from the group consisting of prostate cancer, liver cancer (HCC), rectal cancer, colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), uveal melanoma, glioma, renal cell cancer (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cell cancer, MSI-high cancer, KRAS mutant tumors, adult T-cell leukemia/lymphoma, pleural mesothelioma, anal SCC, neuroendocrine lung cancer (including neuroendocrine lung carcinoma), small cell lung cancer, NSCLC, NSCLC large cell, NSCLC squamous cell, NSCLC adenocarcinoma, atypical carcinoid lung cancer, NSCLC with PDL1 >=50% TPS, cervical SCC, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, chordoma, sarcoma, endometrial sarcoma, chondrosarcoma, uterine sarcoma, plasma cell disorders, multiple myeloma, amyloidosis, AL-amyloidosis, glioblastoma, astrocytoma, and fallopian tube cancer.
176. The method according to any one of aspects 150 to 175, wherein the anti-PVRIG antibody and/or the anti-TIGIT antibody is an antibody according any one of aspects 1-149.
177. The method according to any one of aspects 1 to 175, wherein the method is for use in the treatment of cancer.
178. Use of the anti-PVRIG antibodies and/or anti-TIGIT antibodies and/or immune cell engagers according to any one of aspects 1 to 175 in a method for treating cancer.
179. The anti-PVRIG antibodies and/or anti-TIGIT antibodies and/or immune cell engagers according to any one of aspects 1 to 175, for use in the preparation of a medicament for use in a method for treating cancer.

## Claims

1. An anti-TIGIT antibody for use in a method of treatment for cancer comprising administering the anti-TIGIT antibody, wherein the anti-TIGIT antibody comprises the vhCDR1, vhCDR2, vhCDR3, vlCDR1, vlCDR2, and vlCDR3 sequences from an antibody having a variable heavy domain sequence of SEQ ID NO:877 and a variable light domain sequence of SEQ ID NO:882, wherein the vhCDR1 sequence is of SEQ ID NO:878, the vhCDR2 sequence is of SEQ ID NO:879, the vhCDR3 sequence is of SEQ ID NO:880, the vlCDR1 sequence is of SEQ ID NO:883, the vlCDR2 sequence is of SEQ ID NO:884 and the vlCDR3 sequence is of SEQ ID NO:885,
or a set of CDRs having no more than 2 changes as compared to one of said sets of CDRs, wherein the anti-TIGIT antibody is administered at a dosage of about 0.01 mg/kg to about 10 mg/kg.

2. The anti-TIGIT antibody for use according to claim 1, wherein the anti-TIGIT antibody comprises the variable heavy domain and the variable light domain sequences from an antibody having a variable heavy domain sequence of SEQ ID NO:877 and a variable light domain sequence of SEQ ID NO:882.

3. The anti-TIGIT antibody for use according to any one of claims 1 to 2, wherein the anti-TIGIT antibody comprises:
a) a heavy chain comprising VH-CH1-hinge-CH2-CH3; and
b) a light chain comprising VL-VC, wherein VC is either kappa or lambda.

4. The anti-TIGIT antibody for use according to any one of claims 1 to 3, wherein the sequence of the CH1-hinge-CH2-CH3 is selected from human IgG1, IgG2 and IgG4, and variants thereof.

5. The anti-TIGIT antibody for use according to one of claims 1 to 4, wherein the hinge region optionally comprises mutations.

6. The anti-TIGIT antibody for use according to any one of claims 1 to 5, wherein the anti-TIGIT antibody is an antibody having a full length heavy chain sequence of SEQ ID NO:881 and a full length light chain sequence of SEQ ID NO:886.

7. The anti-TIGIT antibody for use according to any one of claims 1 to 6, wherein the anti-TIGIT antibody is administered every 1 week, 2 weeks, 3 weeks, or 4 weeks.

8. The anti-TIGIT antibody for use according to any one of claims 1 to 7, wherein the anti-TIGIT antibody is administered every 3 weeks.

9. The anti-TIGIT antibody for use according to any one of claims 1 to 8, wherein the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody.

10. The anti-TIGIT antibody for use according to any one of claims 1 to 9, wherein the anti-TIGIT antibody is administered at a dosage of about 0.01 mg/kg, 0.03 mg/kg, 0.1 mg/kg, 0.3 mg/kg, 1 mg/kg, 3 mg/kg, or 10 mg/kg of the anti-TIGIT antibody every 1 week, 2 weeks, 3 weeks, or 4 weeks.

11. The anti-TIGIT antibody for use according to any one of claims 1 to 10, wherein the anti-TIGIT antibody is administered about 10 mg/kg every 3 weeks or every 4 weeks.

12. The anti-TIGIT antibody for use according to any one of claims 1 to 10, wherein the anti-TIGIT antibody is administered about 3 mg/kg every 3 weeks or every 4 weeks.

13. The anti-TIGIT antibody for use according to any one of claims 1 to 12, wherein the cancer is selected from the group consisting of vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma (including hyperdiploid multiple myeloma (HMM), non-hyperdiploid or hypodiploid, light Chain Myeloma, non-secretory Myeloma, solitary Plasmacytoma, extramedullary Plasmacytoma, Monoclonal Gammopathy of Undetermined Significance (MGUS), Smoldering Multiple Myeloma (SMM), immunoglobulin D (IgD) Myeloma, and immunoglobulin E (IgE) Myeloma), post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, malignant melanoma, pleural mesothelioma, chordoma, endometrial sarcoma, chondrosarcoma, uterine sarcoma, synovial sarcoma, uveal melanoma, amyloidosis, AL-amyloidosis, astrocytoma, and/or Myelodysplastic syndromes (MDS).
